# EUROPEAN PATENT SPECIFICATION

(11) **EP 4 099 416 B1**
(45) Date of publication and mention of the grant of the patent: **06.11.2024**
(21) Application number: 22176426.9
(22) Date of filing: 31.05.2022
(51) Int. Cl.: C07D 333/00, C08G 61/00, C08G 73/00, H10K 85/10, H10K 85/60, H10K 30/20, H10K 39/32

(54) **INFRARED ABSORPTION COMPOSITION, AND INFRARED ABSORPTION FILM, PHOTOELECTRIC DEVICE, SENSOR, IMAGE SENSOR, AND ELECTRONIC DEVICE INCLUDING THE SAME**
INFRAROTABSORPTIONSZUSAMMENSETZUNG UND INFRAROTABSORPTIONSFILM, PHOTOELEKTRISCHE VORRICHTUNG, SENSOR, BILDSENSOR UND ELEKTRONISCHES GERÄT DAMIT
COMPOSITION D'ABSORPTION INFRAROUGE ET FILM D'ABSORPTION INFRAROUGE, DISPOSITIF PHOTOÉLECTRIQUE, CAPTEUR, CAPTEUR D'IMAGE ET DISPOSITIF ÉLECTRONIQUE LES COMPRENANT

(30) Priority: 04.06.2021 KR 20210073033
(43) Date of publication of application: 07.12.2022
(73) Proprietor: Samsung Electronics Co., Ltd., Suwon-si, Gyeonggi-do 16677 (KR)
(72) Inventor: PARK, Insun, Suwon-si (KR); KIM, Changki, Suwon-si (KR); KIM, Rae Sung, Hwaseong-si (KR); LEEM, Dong-Seok, Seongnam-si (KR); KWON, Ohkyu, Songpa-gu (KR)
(74) Representative: Elkington and Fife LLP

(56) References cited:
- ZHANG YAJING ET AL: "Designing a thiophene-fused quinoxaline unit to build D-A copolymers for non-fullerene organic solar cells", DYES AND PIGMENTS, ELSEVIER APPLIED SCIENCE PUBLISHERS BARKING, GB, vol. 174, 7 November 2019 (2019-11-07), XP085974768, ISSN: 0143-7208, [retrieved on 20191107], DOI: 10.1016/J.DYEPIG.2019.108022
- JIANGSHENG YU ET AL: "Thiadiazole quinoxaline-based copolymers with approximately 1.0 eV bandgap for ternary polymer solar cells", POLYMER, vol. 79, 1 November 2015 (2015-11-01), AMSTERDAM, NL, pages 12 - 20, XP055281962, ISSN: 0032-3861, DOI: 10.1016/j.polymer.2015.09.047

## Description

### FIELD OF THE INVENTION

Infrared absorption compositions and infrared absorption films, photoelectric devices, sensors, image sensors, and electronic devices including the same are disclosed.

### BACKGROUND OF THE INVENTION

An imaging device is used in a digital camera and a camcorder, etc., to capture an image and to store it as an electrical signal, and the imaging device includes a sensor separating incident light according to a wavelength and converting each component to an electrical signal.

Recently, an infrared photoelectric device for improving sensitivity of a sensor in a low illumination environment or for use as a biometric device has been studied.

ZHANG YAJING ET AL disclose a thiophene-fused quinoxaline unit to build D-A copolymers for non-fullerene organic solar cells: "Designing a thiophene-fused quinoxaline unit to build D-A copolymers for non-fullerene organic solar cells",DYES AND PIGMENTS, ELSEVIER APPLIED SCIENCE PUBLISHERS BARKING, GB, vol. 174, 7 November 2019 (2019-11-07).

Jiangsheng Yu ET AL disclose thiadiazole quinoxaline-based copolymers for polymer solar cells: "Thiadiazole quinoxaline-based copolymers with approximately 1.0 eV bandgap for ternary polymer solar cells",Polymer, vol. 79, 1 November 2015 (2015-11-01), pages 12-20.

### SUMMARY OF THE INVENTION

Some example embodiments provide an infrared absorption composition having excellent infrared light absorption characteristics.

Some example embodiments provide an infrared absorption film including the infrared absorption composition.

Some example embodiments provide a photoelectric device including the infrared absorption composition.

Some example embodiments provide an organic sensor including the infrared absorption composition or the photoelectric device.

Some example embodiments provide an electronic device including the photoelectric device or the organic sensor.

According to some example embodiments, an infrared absorption composition includes a p-type semiconductor compound including a first structural unit represented by Chemical Formula 1 and a second structural unit including an electron donating moiety; and an n-type semiconductor compound represented by Chemical Formula 2.

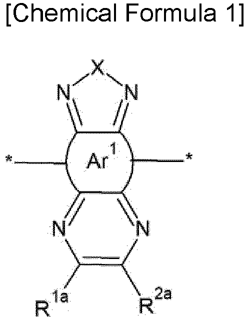

In Chemical Formula 1,
Ar¹ is a substituted or unsubstituted C6 to C30 aromatic ring, a substituted or unsubstituted C3 to C30 heteroaromatic ring, or any combination thereof,
X is O, S, Se, Te, S(=O), S(=O₂), NR^{a}, CR^{b}R^{c}, SiR^{d}R^{e}, GeR^{f}R^{g}, CR^{h}=CRⁱ, or CR^{hh}=CRⁱⁱ, wherein R^{a}, R^{b}, R^{c}, R^{d}, R^{e}, R^{f}, R^{g}, R^{h}, and Rⁱ are each independently hydrogen, deuterium, a C1 to C6 alkyl group, a C1 to C6 haloalkyl group, a C6 to C14 aryl group, a C3 to C12 heteroaryl group, a halogen, a cyano group, or any combination thereof, and R^{hh} and Rⁱⁱ are each independently a C1 to C6 alkylene group or a C2 to C6 heteroalkylene group and linked to each other to provide an aromatic or heteroaromatic ring,
R^{1a} and R^{2a} are each independently a substituted or unsubstituted C6 to C30 aryl group or a substituted or unsubstituted C3 to C30 heteroaryl group or R^{1a} and R^{2a} are linked to each other to provide a substituted or unsubstituted C6 to C30 arene group or a substituted or unsubstituted C3 to C30 heteroarene group, and
* is a linking point within the p-type semiconductor compound, 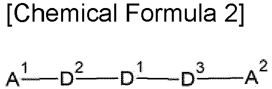
wherein, in Chemical Formula 2
D¹ is a first electron donating moiety having any one of the structures represented by Chemical Formulas 3A to 3E,
D² and D³ are each independently a single bond or a second electron donating moiety, and
A¹ and A² are each independently a third electron accepting moiety of a substituted or unsubstituted C6 to C30 hydrocarbon ring group having at least one functional group of C=O, C=S, C=Se, C=Te, or C=C(CN)₂; a substituted or unsubstituted C2 to C30 heterocyclic group having at least one functional group of C=O, C=S, C=Se, C=Te, or C=C(CN)₂; or a fused ring thereof, 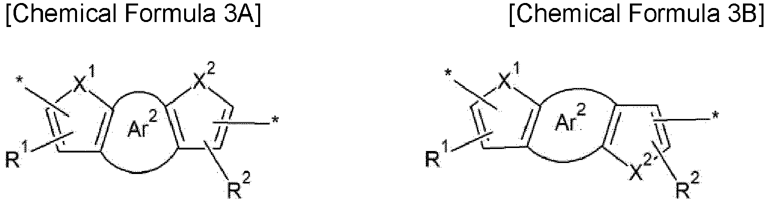 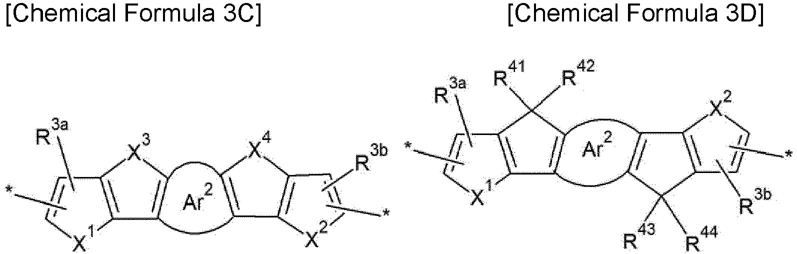 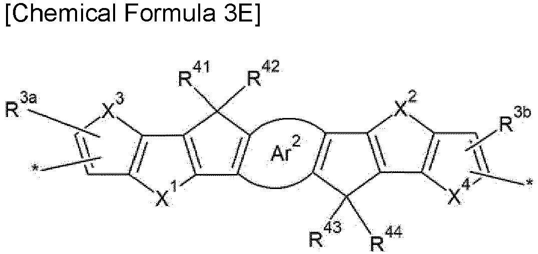
wherein, in Chemical Formulas 3A to 3E,
Ar² is a substituted or unsubstituted C6 to C30 arene group; a substituted or unsubstituted C3 to C30 heterocyclic group including at least one of N, O, S, Se, Te, or Si; a fused ring thereof; or any combination thereof,
X¹, X², X³, and X⁴ are each independently S, Se, or Te,
R⁴¹, R⁴², R⁴¹, and R⁴⁴ are each independently a substituted or unsubstituted C1 to C30 alkyl group, a substituted or unsubstituted C1 to C30 alkoxy group, a substituted or unsubstituted C6 to C20 aryl group, or a substituted or unsubstituted C3 to C20 heteroaryl group,
R¹, R², R^{3a} and R^{3b} are each independently hydrogen or a C1 to C10 alkyl group, and
* denotes a linking point within Chemical Formula 2.

In Chemical Formula 1, Ar¹ may be a benzene ring, a substituted or unsubstituted naphthalene ring, a substituted or unsubstituted anthracene ring, a substituted or unsubstituted phenanthrene ring, a substituted or unsubstituted tetracene ring, a substituted or unsubstituted pyrene ring, a substituted or unsubstituted quinoline ring, a substituted or unsubstituted isoquinoline ring, a substituted or unsubstituted quinoxaline ring, a substituted or unsubstituted quinazoline ring, or a substituted or unsubstituted phenanthroline ring.

In Chemical Formula 1, Ar¹ may be one moiety of the moieties represented by Chemical Formula 1A-1.

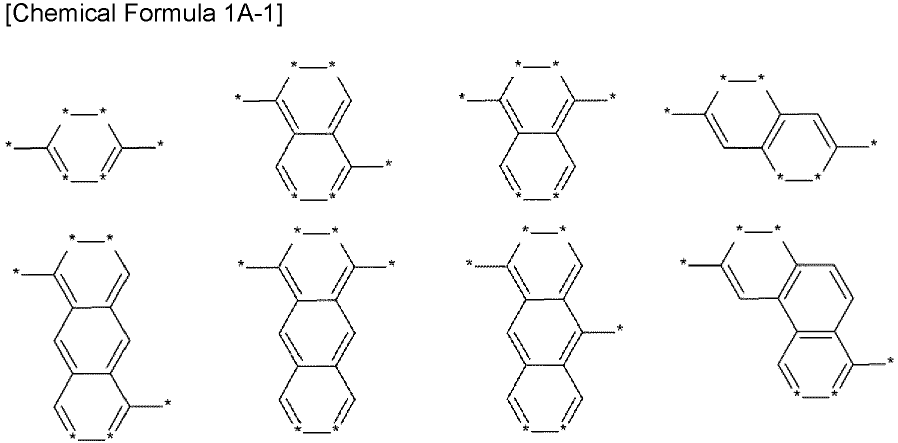

In Chemical Formula 1A-1,
at least one hydrogen of each aromatic ring may be hydrogen or may be replaced by deuterium, a halogen, a cyano group, a C1 to C10 alkyl group, a C1 to C10 haloalkyl group, a -SiH₃ group, or a C1 to C10 alkylsilyl group, and
adjacent pairs of *'s inside at least one aromatic ring are linking points with an N-X-N-containing ring and a pyrazine ring of Chemical Formula 1.

In Chemical Formula 1, Ar¹ may be one moiety of the moieties represented by Chemical Formula 1A-2.

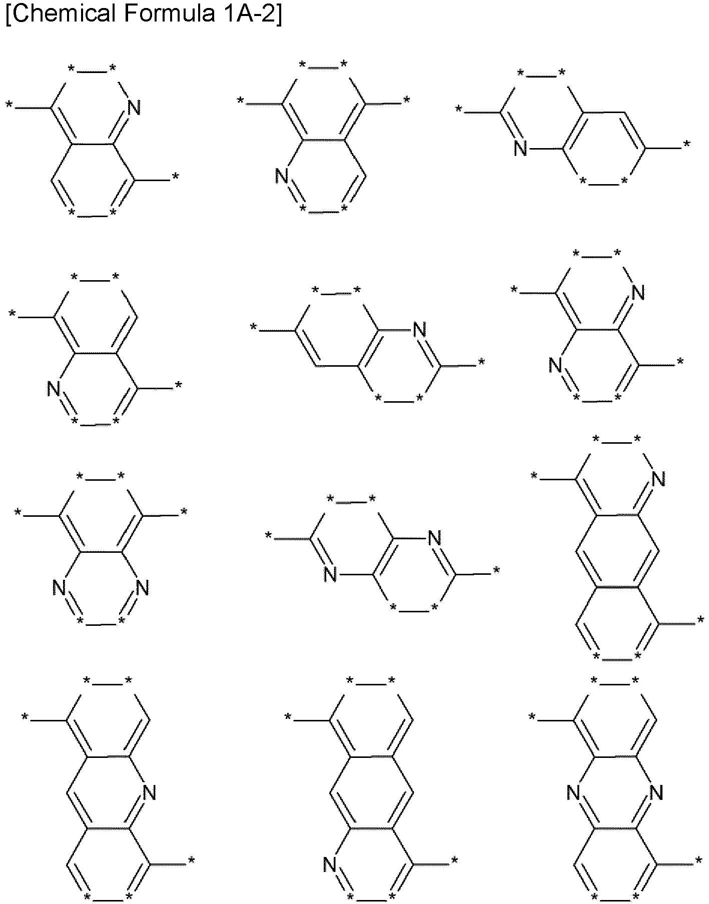

In Chemical Formula 1A-2,
at least one hydrogen of each aromatic or heteroaromatic ring may be hydrogen or may be replaced by deuterium, a halogen, a cyano group, a C1 to C10 alkyl group, a C1 to C10 haloalkyl group, a -SiH₃ group, or a C1 to C10 alkylsilyl group, and
adjacent pairs of *'s inside at least one aromatic or heteroaromatic ring are linking points with an N-X-N-containing ring and a pyrazine ring of Chemical Formula 1.

In Chemical Formula 1, R^{1a} and R^{2a} may be linked to each other, and the substituted or unsubstituted C6 to C30 arene group and the substituted or unsubstituted C3 to C30 heteroarene group formed by linking R^{1a} and R^{2a} to each other may be a substituted or unsubstituted benzene ring, a substituted or unsubstituted naphthalene ring, a substituted or unsubstituted acenaphthene ring, a substituted or unsubstituted anthracene ring, a substituted or unsubstituted phenanthrene ring, a substituted or unsubstituted tetracene ring, or a substituted or unsubstituted pyrene ring; or a substituted or unsubstituted quinoline ring, a substituted or unsubstituted isoquinoline ring, a substituted or unsubstituted quinoxaline ring, a substituted or unsubstituted quinazoline ring, a substituted or unsubstituted phenanthroline ring, a substituted or unsubstituted pyrimidine ring, or a substituted or unsubstituted benzodithiophene ring.

In Chemical Formula 1, R^{1a} and R^{2a} may be linked to each other, and the substituted or unsubstituted C6 to C30 arene group and the substituted or unsubstituted C3 to C30 heteroarene group formed by linking R^{1a} and R^{2a} to each other may be one moiety of moieties represented by Chemical Formulas 1B-1 and 1B-2.

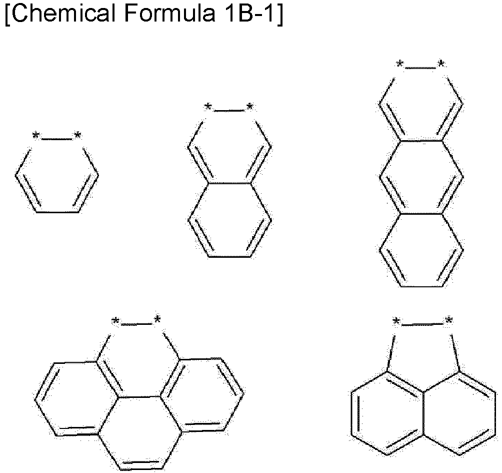

In Chemical Formula 1B-1,
at least one hydrogen of each aromatic ring may be hydrogen or may be replaced by a halogen, a cyano group, a C1 to C30 alkyl group, a C1 to C30 alkoxy group, a C1 to C30 haloalkyl group, a -SiH₃ group, a C1 to C30 alkylsilyl group, a C6 to C30 aryl group, a C6 to C30 aryloxy group, or a C3 to C30 heteroaryl group, and
each * is a point bonded to the pyrazine ring of Chemical Formula 1.

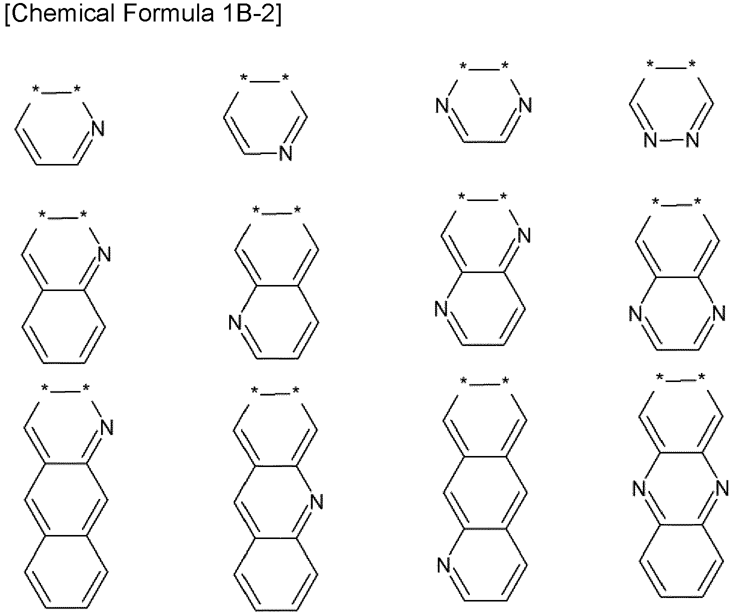

In Chemical Formula 1B-2,
at least one hydrogen of each aromatic or heteroaromatic ring may be hydrogen or may be replaced by a halogen, a cyano group, a C1 to C30 alkyl group, a C1 to C30 alkoxy group, a C1 to C30 haloalkyl group, a -SiH₃ group, a C1 to C30 alkylsilyl group, a C6 to C30 aryl group, a C6 to C30 aryloxy group, or a C3 to C30 heteroaryl group, and
each * is a point bonded to the pyrazine ring of Chemical Formula 1.

In Chemical Formula 1, R^{1a} and R^{2a} may be linked to each other, and the substituted or unsubstituted C6 to C30 arene group and the substituted or unsubstituted C3 to C30 heteroarene group formed by linking R^{1a} and R^{2a} to each other may each independently be one moiety of moieties having an aromatic or heteroaromatic ring represented by Chemical Formula 1B-3 or 1B-4.

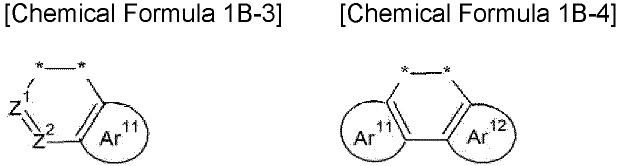

In Chemical Formulas 1B-3 and 1B-4,
Ar¹¹ and Ar¹² are each independently a substituted or unsubstituted C6 to C30 arene group or a substituted or unsubstituted C3 to C30 heteroarene group,
in Chemical Formula 1B-3, Z¹ and Z² are each independently N or CR^{x}, wherein R^{x} is hydrogen, deuterium, C1 to C10 alkyl group, C1 to C10 haloalkyl group, a -SiH₃ group, C1 to C10 alkylsilyl group, a -NH₂ group, a C1 to C10 alkylamine group, a C6 to C10 arylamine group, a C6 to C14 aryl group, a C3 to C12 heteroaryl group, a halogen, a cyano group, or any combination thereof, and
each * inside the aromatic or heteroaromatic ring is a point bonded to a pyrazine ring of Chemical Formula 1.

The moiety represented by Chemical Formula 1B-3 may be represented by Chemical Formula 1B-3-1.

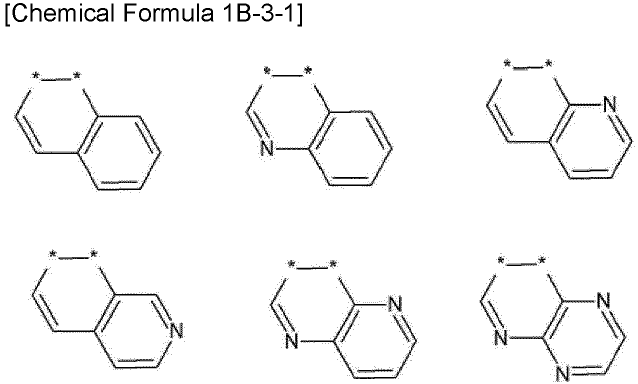

In Chemical Formula 1B-3-1,
at least one hydrogen of each aromatic or heteroaromatic ring may be hydrogen or may be replaced by a halogen, a cyano group, a C1 to C30 alkyl group, a C1 to C30 alkoxy group, a C1 to C30 haloalkyl group, a -SiH₃ group, a C1 to C30 alkylsilyl group, a C6 to C30 aryl group, a C6 to C30 aryloxy group, or a C3 to C30 heteroaryl group, and
each * inside the aromatic or heteroaromatic ring is a point bonded to the pyrazine ring of Chemical Formula 1.

The moiety represented by Chemical Formula 1B-4 may be represented by Chemical Formula 1B-4-1.

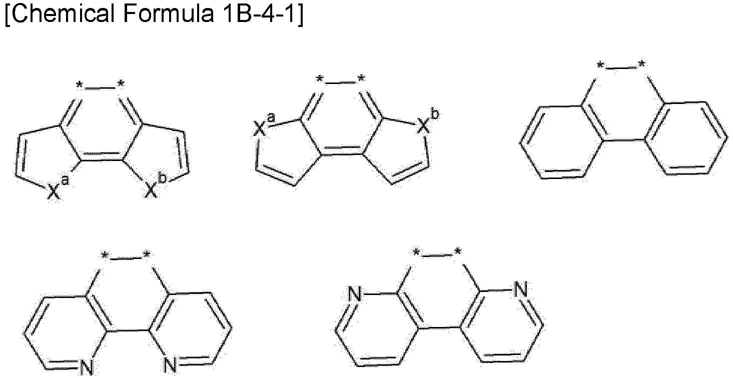

In Chemical Formula 1B-4-1,
at least one hydrogen of each aromatic or heteroaromatic ring may be hydrogen or may be replaced by a halogen, a cyano group, a C1 to C30 alkyl group, a C1 to C30 alkoxy group, a C1 to C30 haloalkyl group, a -SiH₃ group, a C1 to C30 alkylsilyl group, a C6 to C30 aryl group, a C6 to C30 aryloxy group, or a C3 to C30 heteroaryl group,
X^{a} and X^{b} are each independently O, S, Se, Te, NR^{a}, SiR^{b}R^{c}, or GeR^{d}R^{e}, wherein R^{a}, R^{b}, R^{c}, R^{d}, and R^{e} are each independently hydrogen, a halogen, a substituted or unsubstituted C1 to C10 alkyl group, or a substituted or unsubstituted C6 to C10 aryl group, and
each * inside the aromatic ring is a point bonded to the pyrazine ring of Chemical Formula 1.

The first structural unit of the p-type semiconductor compound may be represented by Chemical Formula 1C.

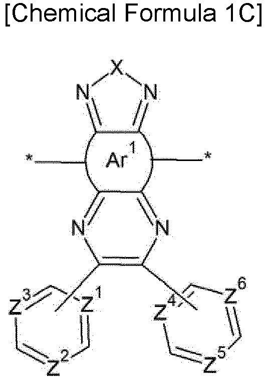

In Chemical Formula 1C,
Ar¹ is a substituted or unsubstituted C6 to C30 aromatic ring, a substituted or unsubstituted C3 to C30 heteroaromatic ring, or any combination thereof,
X is O, S, Se, Te, S(=O), S(=O₂), NR^{a}, CR^{b}R^{c}, SiR^{d}R^{e}, GeR^{f}R^{g}, CR^{h}=CRⁱ, or CR^{hh}=CRⁱⁱ, wherein R^{a}, R^{b}, R^{c}, R^{d}, R^{e}, R^{f}, R^{g}, R^{h}, and Rⁱ are each independently hydrogen, deuterium, a C1 to C6 alkyl group, a C1 to C6 haloalkyl group, a C6 to C14 aryl group, a C3 to C12 heteroaryl group, a halogen, a cyano group, or any combination thereof, and R^{hh} and Rⁱⁱ are each independently a C1 to C6 alkylene group or a C2 to C6 heteroalkylene group and linked to each other to provide an aromatic or heteroaromatic ring,
Z¹ to Z⁶ are each independently N or CR^{x}, wherein R^{x} is hydrogen, deuterium, a C1 to C20 alkyl group, a C1 to C20 haloalkyl group, a -SiH₃ group, a C1 to C20 alkylsilyl group, a -NH₂ group, a C1 to C20 alkylamine group, a C6 to C12 aryl group, a C3 to C12 heteroaryl group, a halogen, a cyano group, or any combination thereof,
at least one hydrogen of each aromatic or heteroaromatic ring may be hydrogen or may be replaced by deuterium, a halogen, a cyano group, a C1 to C30 alkyl group, a C1 to C30 alkoxy group, a C1 to C30 haloalkyl group, a C6 to C30 aryl group, a C6 to C30 aryloxy group, a -SiH₃ group, or a C1 to C30 alkylsilyl group, and
* is a linking point within the p-type semiconductor compound.

The electron donating moiety included in the second structural unit of the p-type semiconductor compound may be a substituted or unsubstituted C6 to C30 arene group, a substituted or unsubstituted divalent C3 to C30 heterocyclic group including at least one of N, O, S, Se, Te, or Si, a fused ring thereof, or any combination thereof.

The electron donating moiety included in the second structural unit of the p-type semiconductor compound may include at least one moiety of the moieties of Group 1 (Chemical Formulas 4A to 4J).

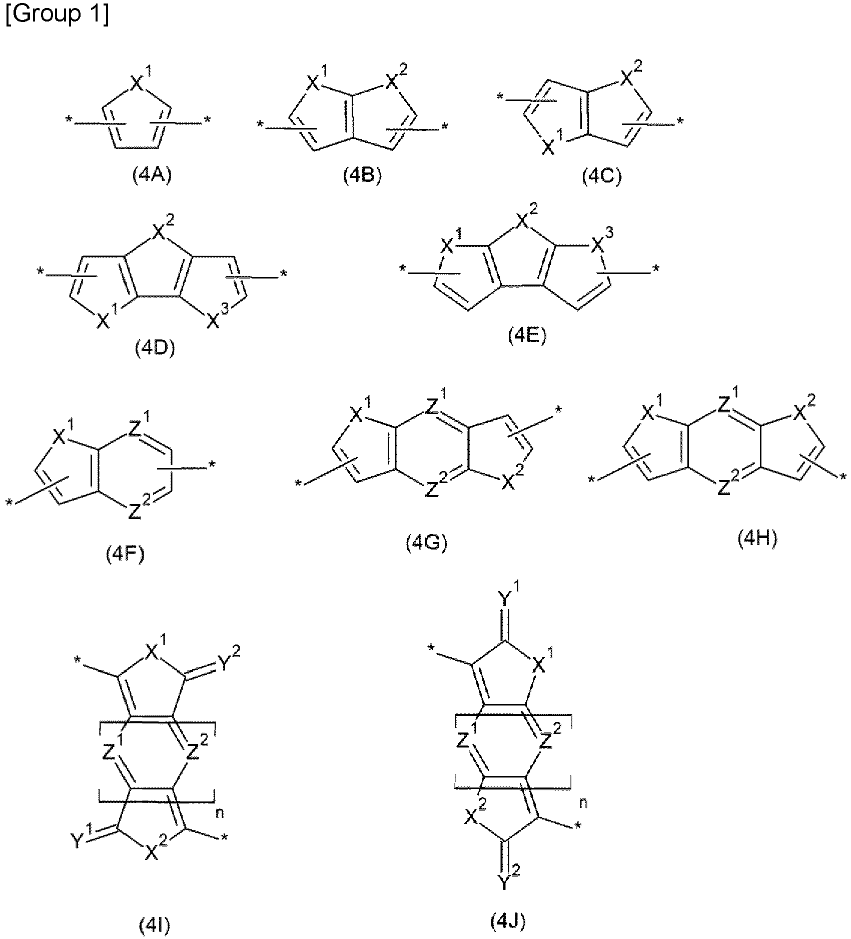

In Group 1,
X¹ to X³ may each independently be S, Se, Te, S(=O), S(=O₂), NR^{a}, SiR^{d}R^{e}, or GeR^{f}R^{g}, wherein R^{a}, R^{b}, R^{c}, R^{d}, R^{e}, R^{f}, and R^{g} may each independently be hydrogen, deuterium, a C1 to C20 alkyl group, a C1 to C20 alkoxy group, a C1 to C20 haloalkyl group, a C6 to C20 aryl group, a C3 to C20 heteroaryl group, a halogen, a cyano group, or any combination thereof,
Z¹ and Z² may each independently be N or CR^{x}, wherein R^{x} may be hydrogen, deuterium, a C1 to C10 alkyl group, a C1 to C10 haloalkyl group, a -SiH₃ group, a C1 to C10 alkylsilyl group, a -NH₂ group, a C1 to C10 alkylamine group, a C6 to C12 aryl group, a C3 to C12 heteroaryl group, a halogen, a cyano group, or any combination thereof,
Y¹ and Y² may each independently be O, S, Se, or Te,
n may be 0 or 1, and
at least one hydrogen of each aromatic or heteroaromatic ring may be hydrogen or may be replaced by deuterium, a halogen, a cyano group, a C1 to C30 alkyl group, a C1 to C30 alkoxy group, a C1 to C30 haloalkyl group, a C6 to C30 aryl group, a C6 to C30 aryloxy group, a -SiH₃ group, or a C1 to C30 alkylsilyl group.

The p-type semiconductor compound may be a polymer including about 20 mol% to about 50 mol% of the first structural unit and about 50 mol% to about 80 mol% of the second structural unit.

The p-type semiconductor compound may be configured to exhibit a peak absorption wavelength in a wavelength range of about 1000 nm to about 3000 nm.

In Chemical Formulas 3A to 3C, Ar² may be a moiety having one structure of the structures of Group 2 (Chemical Formulas 5A to 5K).

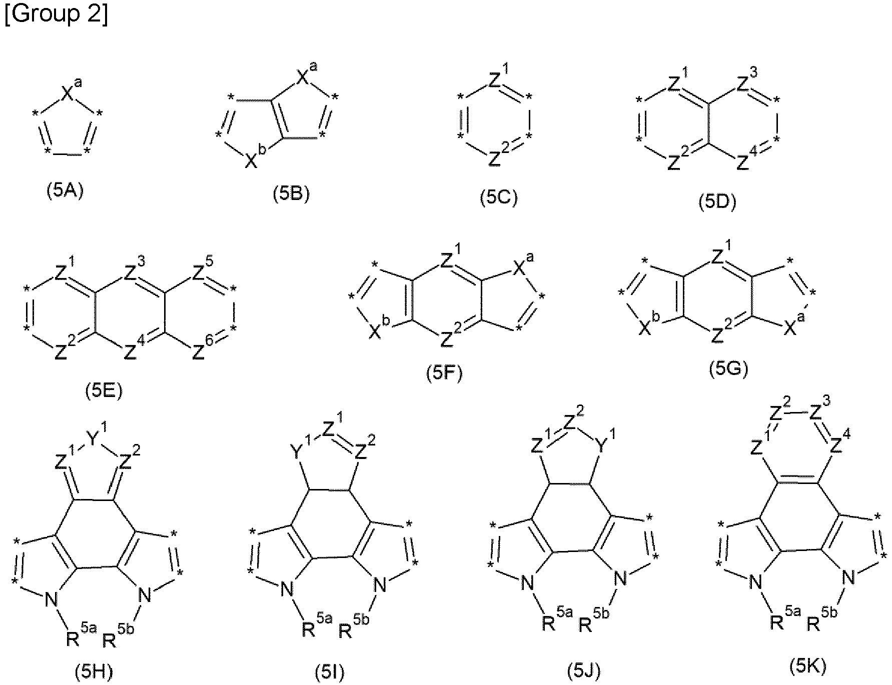

In Group 2 (Chemical Formulas 5A to 5K),
X^{a} and X^{b} may each independently be CR^{x}R^{y}, S, Se, or Te, wherein R^{x} and R^{y} are each independently a substituted or unsubstituted C1 to C30 alkyl group, a substituted or unsubstituted C1 to C30 alkoxy group, a substituted or unsubstituted C6 to C20 aryl group, or a substituted or unsubstituted C3 to C20 heteroaryl group,
R^{5a} and R^{5b} may each independently be hydrogen, a C1 to C20 alkyl group, a C1 to C20 alkoxy group, a C6 to C10 aryl group, or a C2 to C10 heteroaryl group,
Y¹ may be CR^{p}R^{q}, NR^{r}, O, S, Se, or Te, wherein R^{p}, R^{q}, and R^{r} may each independently be hydrogen or a C1 to C20 alkyl group, and
Z¹ to Z⁶ may each independently be CR^{s} or N, wherein, R^{s} may be hydrogen or a C1 to C20 alkyl group.

In Chemical Formulas 3D and 3E, R⁴¹, R⁴², R⁴³, and R⁴⁴ may each independently be a C1 to C20 (e.g., C4 to C15 or C4 to C10) alkyl group; a C1 to C20 (e.g., C4 to C15 or C4 to C10) alkoxy group; a C6 to C20 aryl group substituted with a C1 to C20 (e.g., C4 to C15 or C4 to C10) alkyl group, or a C1 to C20 (e.g., C4 to C15 or C4 to C10) alkoxy group; or a C3 to C20 heteroaryl group substituted with a C1 to C20 (e.g., C4 to C15 or C4 to C10) alkyl group or a C1 to C20 (e.g., C4 to C15 or C4 to C10) alkoxy group.

In Chemical Formulas 3D and 3E, R⁴¹, R⁴², R⁴³, and R⁴⁴ may each independently be a substituted or unsubstituted C3 to C30 branched alkyl group or a substituted or unsubstituted C3 to C30 branched alkoxy group.

The electron donating moiety represented by Chemical Formula 3A may be a moiety represented by Chemical Formula 3A-1.

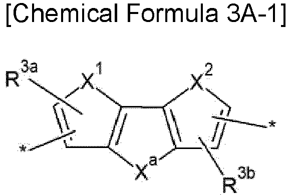

In Chemical Formula 3A-1,
X^{a} is CR^{x}R^{y}, S, Se, or Te, wherein R^{x} and R^{y} are each independently a substituted or unsubstituted C1 to C30 alkyl group, a substituted or unsubstituted C1 to C30 alkoxy group, a substituted or unsubstituted C6 to C20 aryl group, or a substituted or unsubstituted C3 to C20 heteroaryl group,
X¹ and X² are each independently S, Se, or Te,
R^{3a} and R^{3b} are each independently hydrogen or a C1 to C10 alkyl group, and
* denotes a linking point within Chemical Formula 2.

The electron donating moiety represented by Chemical Formula 3B may be a moiety represented by Chemical Formula 3B-1.

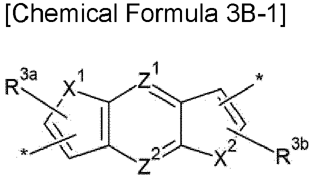

In Chemical Formula 3B-1,
Z¹ and Z² are each independently CR^{s} or N, wherein, R^{s} is hydrogen or a C1 to C20 alkyl group,
X¹ and X² are each independently S, Se, or Te,
R^{3a} and R^{3b} are each independently hydrogen or a C1 to C10 alkyl group, and
* denotes a linking point within Chemical Formula 2.

The electron donating moiety represented by Chemical Formula 3C may be a moiety represented by Chemical Formula 3C-1.

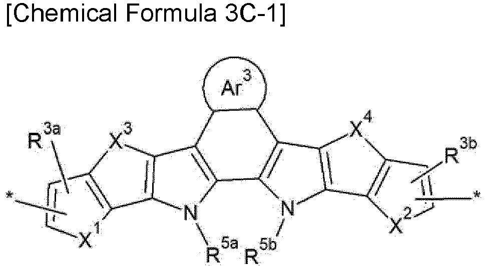

In Chemical Formula 3C-1,
Ar³ is one moiety of moieties having a ring and represented by Chemical Formula 3C-1a,
X¹, X², X³, and X⁴ are each independently S, Se, or Te,
R^{3a} and R^{3b} are each independently hydrogen or a C1 to C10 alkyl group,
R^{5a} and R^{5b} are each independently hydrogen, a C1 to C20 alkyl group, a C1 to C20 alkoxy group, a C6 to C10 aryl group, or a C2 to C10 heteroaryl group, and
* denotes a linking point within Chemical Formula 2.

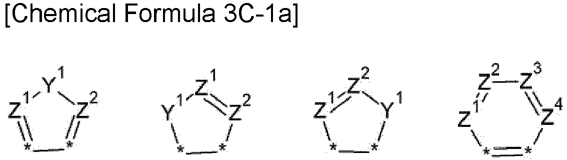

In Chemical Formula 3C-1a,
Y¹ may be CR^{p}R^{q}, NR^{r}, O, S, Se, or Te, wherein R^{p}, R^{q}, and R^{r} may each independently be hydrogen or a C1 to C20 alkyl group,
Z¹ to Z⁴ may each independently be CR^{s} or N, wherein, R^{s} may be hydrogen or a C1 to C20 alkyl group, and
* inside the ring denotes a point linked to Chemical Formula 3C-1.

The electron donating moiety represented by Chemical Formula 3D may be a moiety represented by Chemical Formula 3D-1.

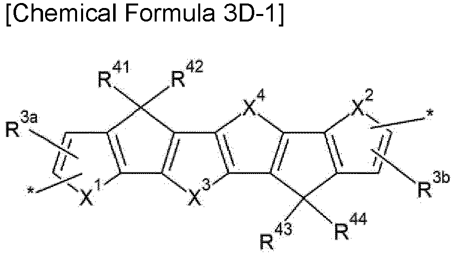

In Chemical Formula 3D-1,
X¹, X², X³, and X⁴ are each independently S, Se, or Te,
R⁴¹, R⁴², R⁴³, and R⁴⁴ are each independently a substituted or unsubstituted C1 to C30 alkyl group, a substituted or unsubstituted C1 to C30 alkoxy group, a substituted or unsubstituted C6 to C20 aryl group, or a substituted or unsubstituted C3 to C20 heteroaryl group,
R^{3a} and R^{3b} are each independently hydrogen or a C1 to C10 alkyl group, and
* denotes a linking point within Chemical Formula 2.

The electron donating moiety represented by Chemical Formula 3D may be a moiety represented by Chemical Formula 3D-2.

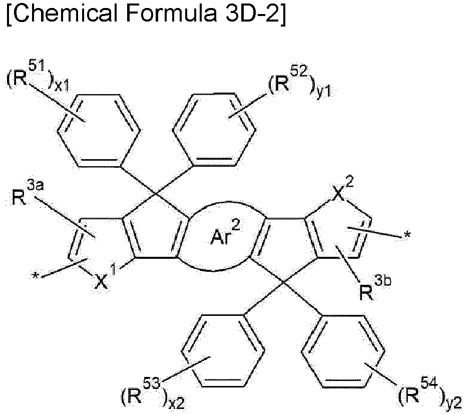

In Chemical Formula 3D-2,
Ar² may be a substituted or unsubstituted C6 to C30 arene group; a substituted or unsubstituted C3 to C30 heterocyclic group including at least one of N, O, S, Se, Te, or Si; a fused ring thereof; or any combination thereof,
X¹ and X² may each independently be S, Se, or Te,
R⁵¹, R⁵², R⁵³, and R⁵⁴ may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group or a substituted or unsubstituted C2 to C20 heteroaryl group,
x1, y1, x2, and y2 may each independently be an integer of 0 to 5,
R^{3a} and R^{3b} may each independently be hydrogen or a C1 to C10 alkyl group, and
* denotes a linking point within Chemical Formula 2.

The electron donating moiety represented by Chemical Formula 3E may be a moiety represented by Chemical Formula 3E-1.

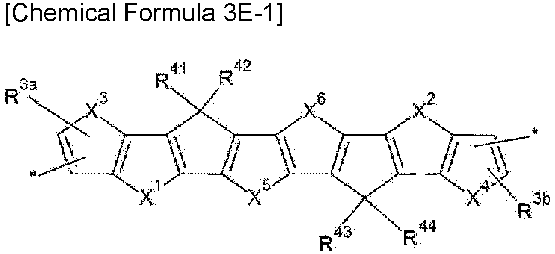

In Chemical Formula 3E-1,
X¹, X², X³, X⁴, X⁵, and X⁶ are each independently S, Se, or Te,
R⁴¹, R⁴², R⁴³, and R⁴⁴ may each independently be a substituted or unsubstituted C1 to C30 alkyl group, a substituted or unsubstituted C1 to C30 alkoxy group, a substituted or unsubstituted C6 to C20 aryl group, or a substituted or unsubstituted C3 to C20 heteroaryl group,
R^{3a} and R^{3b} may each independently be hydrogen or a C1 to C10 alkyl group, and
* denotes a linking point within Chemical Formula 2.

The electron donating moiety represented by Chemical Formula 3E may be a moiety represented by Chemical Formula 3E-2.

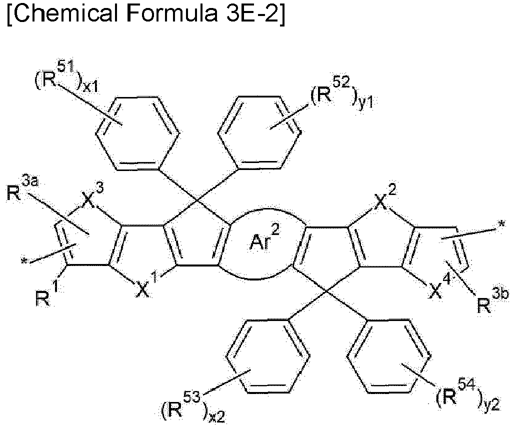

In Chemical Formula 3E-2,
Ar² is a substituted or unsubstituted C6 to C30 arene group; a substituted or unsubstituted C3 to C30 heterocyclic group including at least one of N, O, S, Se, Te, or Si; a fused ring thereof; or any combination thereof,
X¹, X², X³, and X⁴ may each independently be S, Se, or Te,
R⁵¹, R⁵², R⁵³, and R⁵⁴ may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C2 to C20 heteroaryl group,
x1, y1, x2, and y2 may each independently be an integer of 0 to 5,
R^{3a} and R^{3b} may each independently be hydrogen or a C1 to C10 alkyl group, and
* denotes a linking point within Chemical Formula 2.

In Chemical Formula 2, D² and D³ may each independently be one moiety of moieties represented by Group 1 (Chemical Formulas 4A to 4J).

In Chemical Formula 2, A¹ and A² may each independently be an electron accepting moiety represented by any one of Chemical Formulas 6A to 6F.

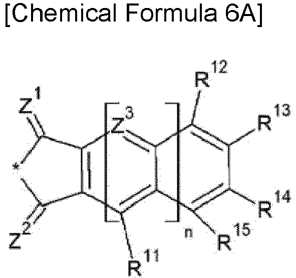

In Chemical Formula 6A,
Z¹ and Z² may each independently be O, S, Se, Te, or CR^{a}R^{b}, wherein R^{a} and R^{b} may each independently be hydrogen, a substituted or unsubstituted C1 to C10 alkyl group, or a cyano group,
Z³ is N or CR^{c}, wherein R^{c} is hydrogen, deuterium, or a substituted or unsubstituted C1 to C10 alkyl group,
R¹¹, R¹², R¹³, R¹⁴, and R¹⁵ may be the same or different and may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), a cyano-containing group, or any combination thereof, and R¹², R¹³, R¹⁴, and R¹⁵ may each independently be present or at least one pair of R¹² and R¹³ and R¹⁴ and R¹⁵ may be linked to each other to provide a fused aromatic ring,
n may be 0 or 1, and
* may be a linking point within Chemical Formula 2.

In some example embodiments, at least one of CR¹¹, CR¹², CR¹³, CR¹⁴, or CR¹⁵ of Chemical Formula 6A may be replaced with nitrogen (N). That is, the substituted or unsubstituted benzene ring moiety of Chemical Formula 6A may include a hetero atom (N).

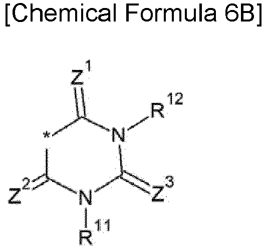

In Chemical Formula 6B,
Z¹ and Z² may each independently be O, S, Se, Te, or CR^{a}R^{b}, wherein R^{a} and R^{b} may each independently be hydrogen, a substituted or unsubstituted C1 to C10 alkyl group, or a cyano group,
Z³ may be O, S, Se, Te, or C(R^{a})(CN), wherein R^{a} is hydrogen, a cyano group (-CN), or a C1 to C10 alkyl group,
R¹¹ and R¹² may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* may be a linking point.

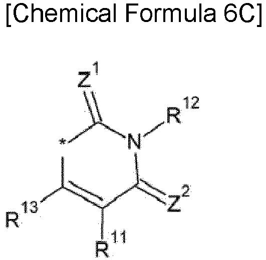

In Chemical Formula 6C,
Z¹ and Z² may each independently be O, S, Se, Te, or CR^{a}R^{b}, wherein R^{a} and R^{b} may each independently be hydrogen, a substituted or unsubstituted C1 to C10 alkyl group, or a cyano group,
R¹¹, R¹², and R¹³ may be same or different from each other and may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* may be a linking point within Chemical Formula 2.

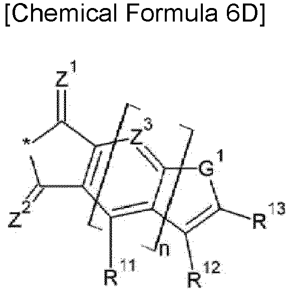

In Chemical Formula 6D,
Z¹ and Z² may each independently be O, S, Se, Te, or CR^{a}R^{b}, wherein R^{a} and R^{b} may each independently be hydrogen, a substituted or unsubstituted C1 to C10 alkyl group, or a cyano group,
Z³ may be N or CR^{c}, wherein R^{c} may be hydrogen or a substituted or unsubstituted C1 to C10 alkyl group,
G¹ may be O, S, Se, Te, SiR^{x}R^{y}, or GeR^{z}R^{w}, wherein R^{x}, R^{y}, R^{z}, and R^{w} may be same or different from each other and may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C2 to C20 heteroaryl group,
R¹¹, R¹², and R¹³ may be same or different from each other and may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof, and R¹² and R¹³ may each independently be present or may be linked to each other to provide a fused aromatic ring,
n may be 0 or 1, and
* may be a linking point within Chemical Formula 2.

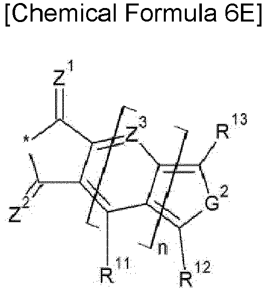

In Chemical Formula 6E,
Z¹ and Z² may each independently be O, S, Se, Te, or CR^{a}R^{b}, wherein R^{a} and R^{b} may each independently be hydrogen, a substituted or unsubstituted C1 to C10 alkyl group, or a cyano group,
Z³ may be N or CR^{c}, wherein R^{c} may be hydrogen or a substituted or unsubstituted C1 to C10 alkyl group,
G² may be O, S, Se, Te, SiR^{x}R^{y}, or GeR^{z}R^{w}, wherein R^{x}, R^{y}, R^{z}, and R^{w} may be the same or different and may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C2 to C20 heteroaryl group,
R¹¹, R¹², and R¹³ may be the same or different and may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof,
n may be 0 or 1, and
* may be a linking point within Chemical Formula 2.

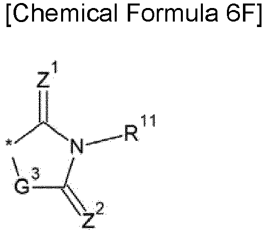

In Chemical Formula 6F,
Z¹ and Z² may each independently be O, S, Se, Te, or CR^{a}R^{b}, wherein R^{a} and R^{b} may each independently be hydrogen, a substituted or unsubstituted C1 to C10 alkyl group, or a cyano group,
R¹¹ may be hydrogen, deuterium, a substituted or unsubstituted C1 to C30 alkyl group, a substituted or unsubstituted C6 to C30 aryl group, a substituted or unsubstituted C4 to C30 heteroaryl group, a halogen, a cyano group (-CN), a cyano-containing group, or any combination thereof,
G³ may be O, S, Se, Te, SiR^{x}R^{y}, or GeR^{z}R^{w}, wherein R^{x}, R^{y}, R^{z}, and R^{w} may be same or different from each other and may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C2 to C20 heteroaryl group, and
* is a linking point within Chemical Formula 2.

A weight ratio of the p-type semiconductor compound to the n-type semiconductor compound (p-type semiconductor compound : n-type semiconductor compound) may be in a range of about 1:0.1 to about 1:10.

The infrared absorption composition may be configured to exhibit a peak absorption wavelength in a wavelength region of about 1000 nm to about 3000 nm.

According to some example embodiments, an infrared absorption film showing a face-on alignment structure in grazing incident small angle x-ray scattering (GISAXS) analysis of a film may include the infrared absorption composition.

According to some example embodiments, an infrared absorption film having a surface roughness of less than or equal to about 2 nm in atomic force microscopy analysis of a film comprising the infrared absorption composition including the p-type semiconductor compound and the n-type semiconductor compound is provided.

According to some example embodiments, a photoelectric device includes a first electrode and a second electrode facing each other, and a photoactive layer between the first electrode and the second electrode, wherein the photoactive layer includes the infrared absorption composition.

According to some example embodiments, a photoelectric device includes a first electrode and a second electrode facing each other, and a photoactive layer between the first electrode and the second electrode, wherein the photoactive layer includes the infrared absorption composition and an external quantum efficiency at - 3V of the photoelectric device is between about 10% and 100%.

According to some example embodiments, a photoelectric device includes a first electrode and a second electrode facing each other, and a photoactive layer between the first electrode and the second electrode, wherein the photoactive layer includes the infrared absorption composition, and an external quantum efficiency at - 3V of the photoelectric device is increased by at least about 80% compared to a photoelectric device including a photoactive layer including the same p-type semiconductor compound and an n-type semiconductor compound of fullerene or a fullerene derivative.

According to some example embodiments, a sensor including the photoelectric device is provided.

According to some example embodiments, an electronic device including the photoelectric device or the sensor is provided.

According to some example embodiments, a photoelectric device may include a first electrode and a second electrode facing each other, a photoactive layer between the first electrode and the second electrode, and a charge auxiliary layer between the photoactive layer and the first electrode, or the photoactive layer and the second electrode. At least one of the photoactive layer or the charge auxiliary layer includes the infrared absorption composition.

A sensor may include the photoelectric device.

According to some example embodiments, an image sensor may include a semiconductor substrate, a first photoelectric device on the semiconductor substrate, the first photoelectric device configured to selectively absorb light in a first infrared wavelength region, and an additional sensor configured to selectively absorb light in a separate wavelength region that is different from the first infrared wavelength region. The first photoelectric device may include the infrared absorption composition.

The additional sensor may be an infrared light sensor at least partially embedded within the semiconductor substrate, and the separate wavelength region may be a separate infrared wavelength region that is different from the first infrared wavelength region, and the first photoelectric device and the infrared light sensor may overlap in a vertical direction that is perpendicular to an upper surface of the semiconductor substrate.

The additional sensor may include a plurality of photodiodes at least partially embedded within the semiconductor substrate, the plurality of photodiodes configured to selectively absorb light in separate visible wavelength regions, and the first photoelectric device and the plurality of photodiodes may overlap in a vertical direction that is perpendicular to an upper surface of the semiconductor substrate.

The additional sensor may include at least one additional photoelectric device vertically stacked between the first photoelectric device and the semiconductor substrate, each separate photoelectric device of the at least one additional photoelectric device including a separate photoelectric conversion layer and configured to selectively absorb light in a separate, respective wavelength region that is different from the first infrared wavelength region.

The first photoelectric device may include a first electrode and a second electrode facing each other, and a photoactive layer between the first electrode and the second electrode, wherein the photoactive layer includes the infrared absorption composition.

The first photoelectric device may include a first electrode and a second electrode facing each other, a photoactive layer between the first electrode and the second electrode, and a charge auxiliary layer between the photoactive layer and the first electrode, or the photoactive layer and the second electrode. The charge auxiliary layer may include the infrared absorption composition.

Since the infrared absorption composition exhibits good absorption characteristics in the infrared region, it may be effectively used for photoelectric devices and/or sensors.

### BRIEF DESCRIPTION OF THE DRAWINGS

FIG. 1 is a cross-sectional view showing a photoelectric device according to some example embodiments,
FIG. 2 is a cross-sectional view showing a photoelectric device according to some example embodiments,
FIG. 3 is a cross-sectional view showing an image sensor according to some example embodiments,
FIG. 4 is a cross-sectional view showing an image sensor according to some example embodiments,
FIG. 5 is a cross-sectional view showing an image sensor according to some example embodiments,
FIG. 6 is a cross-sectional view showing an image sensor according to some example embodiments,
FIG. 7 is a cross-sectional view showing an image sensor according to some example embodiments,
FIG. 8 is a cross-sectional view showing an image sensor according to some example embodiments,
FIG. 9 is a cross-sectional view showing an image sensor according to some example embodiments,
FIG. 10 is a cross-sectional view showing an image sensor according to some example embodiments,
FIG. 11 is a block diagram of a digital camera including an image sensor according to some example embodiments,
FIG. 12 is a block diagram of an electronic device according to some example embodiments,
FIGS. 13A and 13B are atomic force microscopy analysis photographs of a film made of the infrared absorption composition according to Preparation Examples 1-11b and a film made of the infrared absorption composition according to Comparative Preparation Examples 1-11b, respectively, according to some example embodiments, and
FIGS. 14A, 14B, and 14C are figures showing the analysis result of GISAXS (grazing incident small angle x-ray scattering) of a film made of the infrared absorption composition according to Preparation Example 1-8, a film made of the infrared absorption composition according to Comparative Preparation Examples 1-8, and a film made of the polymer (Polymer 8) according to Synthesis Examples 1-8, respectively, according to some example embodiments.

### DETAILED DESCRIPTION OF THE EMBODIMENTS

Hereinafter, some example embodiments will hereinafter be described in detail, and may be easily performed by a person having an ordinary skill in the related art. However, the inventive concepts may be embodied in many different forms and is not to be construed as limited to the example embodiments set forth herein.

In the drawings, the thickness of layers, films, panels, regions, etc., are exaggerated for clarity.

It will be understood that when an element such as a layer, film, region, or substrate is referred to as being "on" another element, it may be directly on the other element or intervening elements may also be present. In contrast, when an element is referred to as being "directly on" another element, there are no intervening elements present. It will further be understood that when an element is referred to as being "on" another element, it may be above or beneath or adjacent (e.g., horizontally adjacent) to the other element.

It will be understood that elements and/or properties thereof (e.g., structures, surfaces, directions, or the like), which may be referred to as being "perpendicular," "parallel," "coplanar," or the like with regard to other elements and/or properties thereof (e.g., structures, surfaces, directions, or the like) may be "perpendicular," "parallel," "coplanar," or the like or may be "substantially perpendicular," "substantially parallel," "substantially coplanar," respectively, with regard to the other elements and/or properties thereof.

Elements and/or properties thereof (e.g., structures, surfaces, directions, or the like) that are "substantially perpendicular" with regard to other elements and/or properties thereof will be understood to be "perpendicular" with regard to the other elements and/or properties thereof within manufacturing tolerances and/or material tolerances and/or have a deviation in magnitude and/or angle from "perpendicular," or the like with regard to the other elements and/or properties thereof that is equal to or less than 10% (e.g., a. tolerance of ±10%).

Elements and/or properties thereof (e.g., structures, surfaces, directions, or the like) that are "substantially parallel" with regard to other elements and/or properties thereof will be understood to be "parallel" with regard to the other elements and/or properties thereof within manufacturing tolerances and/or material tolerances and/or have a deviation in magnitude and/or angle from "parallel," or the like with regard to the other elements and/or properties thereof that is equal to or less than 10% (e.g., a. tolerance of ±10%).

Elements and/or properties thereof (e.g., structures, surfaces, directions, or the like) that are "substantially coplanar" with regard to other elements and/or properties thereof will be understood to be "coplanar" with regard to the other elements and/or properties thereof within manufacturing tolerances and/or material tolerances and/or have a deviation in magnitude and/or angle from "coplanar," or the like with regard to the other elements and/or properties thereof that is equal to or less than 10% (e.g., a. tolerance of ±10%).

It will be understood that elements and/or properties thereof may be recited herein as being "the same" or "equal" as other elements, and it will be further understood that elements and/or properties thereof recited herein as being "identical" to, "the same" as, or "equal" to other elements may be "identical" to, "the same" as, or "equal" to or "substantially identical" to, "substantially the same" as or "substantially equal" to the other elements and/or properties thereof. Elements and/or properties thereof that are "substantially identical" to, "substantially the same" as or "substantially equal" to other elements and/or properties thereof will be understood to include elements and/or properties thereof that are identical to, the same as, or equal to the other elements and/or properties thereof within manufacturing tolerances and/or material tolerances. Elements and/or properties thereof that are identical or substantially identical to and/or the same or substantially the same as other elements and/or properties thereof may be structurally the same or substantially the same, functionally the same or substantially the same, and/or compositionally the same or substantially the same.

It will be understood that elements and/or properties thereof described herein as being the "substantially" the same and/or identical encompasses elements and/or properties thereof that have a relative difference in magnitude that is equal to or less than 10%. Further, regardless of whether elements and/or properties thereof are modified as "substantially," it will be understood that these elements and/or properties thereof should be construed as including a manufacturing or operational tolerance (e.g., ±10%) around the stated elements and/or properties thereof.

While the term "same," "equal" or "identical" may be used in description of some example embodiments, it should be understood that some imprecisions may exist. Thus, when one element is referred to as being the same as another element, it should be understood that an element or a value is the same as another element within a desired manufacturing or operational tolerance range (e.g., ±10%).

When the terms "about" or "substantially" are used in this specification in connection with a numerical value, it is intended that the associated numerical value includes a manufacturing or operational tolerance (e.g., ±10%) around the stated numerical value. Moreover, when the words "about" and "substantially" are used in connection with geometric shapes, it is intended that precision of the geometric shape is not required but that latitude for the shape is within the scope of the disclosure. Further, regardless of whether numerical values or shapes are modified as "about" or "substantially," it will be understood that these values and shapes should be construed as including a manufacturing or operational tolerance (e.g., ±10%) around the stated numerical values or shapes. When ranges are specified, the range includes all values therebetween such as increments of 0.1%.

In the drawings, parts having no relationship with the description are omitted for clarity of some example embodiments, and the same or similar constituent elements are indicated by the same reference numeral throughout the specification.

As used herein, "at least one of A, B, or C," "one of A, B, C, or any combination thereof" and "one of A, B, C, and any combination thereof" refer to each constituent element, and any combination thereof (e.g., A; B; C; A and B; A and C; B and C; or A, B and C).

Hereinafter, "combination" includes a mixture of two or more, inter-substitution, and a laminate structure of two or more.

As used herein, when specific definition is not otherwise provided, "substituted" refers to replacement of a hydrogen of a compound or a functional group by a substituent of a halogen atom, a hydroxy group, a nitro group, a cyano group, an amino group, an azido group, an amidino group, a hydrazino group, a hydrazono group, a carbonyl group, a carbamyl group, a thiol group, an ester group, a carboxyl group or a salt thereof, a sulfonic acid group or a salt thereof, a phosphoric acid group or a salt thereof, a silyl group, a C1 to C20 alkyl group, a C2 to C20 alkenyl group, a C2 to C20 alkynyl group, a C6 to C30 aryl group, a C7 to C30 arylalkyl group, a C1 to C30 alkoxy group, a C1 to C20 heteroalkyl group, a C3 to C20 heteroaryl group, a C3 to C20 heteroarylalkyl group, a C3 to C30 cycloalkyl group, a C3 to C15 cycloalkenyl group, a C6 to C15 cycloalkynyl group, a C3 to C30 heterocycloalkyl group, or any combination thereof.

As used herein, when specific definition is not otherwise provided, "'hetero" refers to one including 1 to 4 heteroatoms selected from N, O, S, Se, Te, Si, and P.

As used herein, when a definition is not otherwise provided, "aromatic ring" refers to a functional group in which all atoms in the cyclic functional group have a p-orbital, and wherein these p-orbitals are conjugated and "heteroaromatic ring" refers to the aromatic ring including a heteroatom. The "aromatic ring" refers to a C6 to C30 arene group, for example a C6 to C20 arene group or a C6 to C30 aryl group, for example a C6 to C20 aryl group, and the "heteroaromatic ring" refers to a C3 to C30 heteroarene group, for example a C3 to C20 heteroarene group or a C3 to C30 heteroaryl group, for example a C3 to C20 heteroaryl group.

As used herein, when a definition is not otherwise provided, "arene group" refers to a hydrocarbon group having an aromatic ring, and includes monocyclic and polycyclic hydrocarbon groups, and the additional ring of the polycyclic hydrocarbon group may be an aromatic ring or a nonaromatic ring. "Heteroarene group" refers to an arene group including 1 to 3 heteroatoms selected from N, O, S, Se, Te, P, and Si in a ring group.

As used herein, when a definition is not otherwise provided, "aryl group" refers to a group including at least one hydrocarbon aromatic moiety. All elements of the hydrocarbon aromatic moiety have p-orbitals which form conjugation, for example a phenyl group, a tolyl group, a xylyl group, a naphthyl group, and the like; two or more hydrocarbon aromatic moieties may be linked by a sigma bond and may be, for example a biphenyl group, a terphenyl group, a quaterphenyl group, and the like; or two or more hydrocarbon aromatic moieties are fused directly or indirectly to provide a non-aromatic fused ring, for example a fluorenyl group. The aryl group may include a monocyclic, polycyclic, or fused polycyclic (i.e., rings sharing adjacent pairs of carbon atoms) functional group.

As used herein, when a definition is not otherwise provided, "heteroaryl group" refers to inclusion of at least one heteroatom of N, O, S, Se, Te, P, and Si instead of carbon (C) in the ring. When the heteroaryl group is a fused ring, at least one of the rings constituting the heteroaryl group may have a heteroatom, or each ring may have a heteroatom.

As used herein, when a definition is not otherwise provided, "halogen" may be any one of F, Cl, Br, and I, and the haloalkyl group is one in which at least one hydrogen of an alkyl group is replaced by a halogen, for example, a perfluoroalkyl group such as -CF₃.

As used herein, when a definition is not otherwise provided, "hydrocarbon ring group" refers to an aromatic ring (arene ring) or a fused ring of an aromatic ring and a non-aromatic ring (alicyclic ring). The aromatic ring may be, for example, at least one aromatic ring such as a C6 to C30 aryl group, a C6 to C20 aryl group, or a C6 to C10 aryl group, and the fused ring may include a cyclic group in which at least one aromatic ring (arene ring) such as a C6 to C30 aryl group, a C6 to C20 aryl group or a C6 to C10 aryl group, and at least one non-aromatic ring (alicyclic ring) such as a C3 to C30 cycloalkyl group, a C3 to C20 cycloalkyl group or a C3 to C10 cycloalkyl group are fused to each other. The aromatic ring or non-aromatic ring (alicyclic ring) may include a hetero atom (e.g., N, O, S, Se, Te, P, and/or Si) in the ring.

As used herein, when a definition is not otherwise provided, the "heterocyclic group" refers to a ring group in which 1 to 3 carbons of an aromatic hydrocarbon group (e.g., a C6 to C30 aryl group, a C6 to C20 aryl group, or a C6 to C10 aryl group), an alicyclic hydrocarbon group (e.g., a C3 to C30 cycloalkyl group, a C3 to C20 cycloalkyl group, or a C3 to C10 cycloalkyl group), or a fused ring thereof are replaced by a heteroatom of N, O, S, Se, Te, P, or Si. In addition, at least one carbon atom of the heterocyclic group may be replaced by a thiocarbonyl group (C=S).

As used herein, when a definition is not otherwise provided, "fused ring" refers to a condensed ring formed by bonding two or more cyclic groups selected from an aromatic hydrocarbon group (e.g., a C6 to C30 aryl group, a C6 to C20 aryl group, or a C6 to C10 aryl group) and an alicyclic hydrocarbon group (e.g., a C3 to C30 cycloalkyl group, a C3 to C20 cycloalkyl group, or a C3 to C10 cycloalkyl group).

As used herein, when a definition is not otherwise provided, "cyano-containing group" refers to a monovalent group such as a C1 to C30 alkyl group, a C2 to C30 alkenyl group, or a C2 to C30 alkynyl group where at least one hydrogen is replaced by a cyano group. The cyano-containing group also refers to a divalent group such as =CR^{x'}-(CR^{x}R^{y})ₚ-CR^{y}(CN)₂ wherein R^{x}, R^{y}, R^{x'}, and R^{y'} are independently hydrogen or a C1 to C10 alkyl group and p may be an integer of 0 to 10 (or 1 to 10). Specific examples of the cyano-containing group may be a dicyanomethyl group, a dicyanovinyl group, a cyanoethynyl group, and the like.

As used herein, when a definition is not otherwise provided, "alkyl group" refers to a linear or branched alkyl group, a C1 to C30 alkyl group, for example a C1 to C20 alkyl group, or a C1 to C10 alkyl group.

As used herein, when a definition is not otherwise provided, the "infrared wavelength region" includes a near infrared ray/infrared wavelength region with a wavelength region of about 1000 nm to about 3000 nm.

Hereinafter, an infrared absorption composition according to some example embodiments is described. The infrared absorption composition includes a p-type semiconductor compound and an n-type semiconductor compound and the p-type semiconductor compound and the n-type semiconductor compound provide a bulk heterojunction (BHJ) structure.

The p-type semiconductor compound may be a compound (e.g., a polymer) including a first structural unit represented by Chemical Formula 1 and a second structural unit including an electron donating moiety. Hereinafter, the p-type semiconductor compound is also described as a polymer.

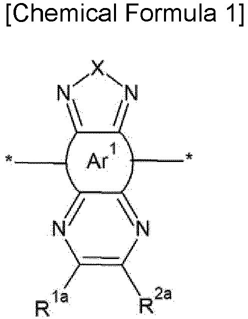

In Chemical Formula 1,
Ar¹ is a substituted or unsubstituted C6 to C30 aromatic ring, a substituted or unsubstituted C3 to C30 heteroaromatic ring, or any combination thereof,
X is O, S, Se, Te, S(=O), S(=O₂), NR^{a}, CR^{b}R^{c}, SiR^{d}R^{e}, GeR^{f}R^{g}, CR^{h}=CRⁱ, or CR^{hh}=CRⁱⁱ, wherein R^{a}, R^{b}, R^{c}, R^{d}, R^{e}, R^{f}, R^{g}, R^{h}, and Rⁱ are each independently hydrogen, deuterium, a C1 to C6 alkyl group, a C1 to C6 haloalkyl group, a C6 to C14 aryl group, a C3 to C12 heteroaryl group, a halogen, a cyano group, or any combination thereof, R^{hh} and Rⁱⁱ may each independently be a C1 to C6 alkylene group or a C2 to C6 heteroalkylene group and linked to each other to provide an aromatic or heteroaromatic ring,
R^{1a} and R^{2a} are each independently a substituted or unsubstituted C6 to C30 aryl group or a substituted or unsubstituted C3 to C30 heteroaryl group or R^{1a} and R^{2a} may be linked to each other to provide a substituted or unsubstituted C6 to C30 arene group or a substituted or unsubstituted C3 to C30 heteroarene group, and
* is a linking point within the p-type semiconductor compound.

A material absorbing long-wavelength light such as near infrared ray/infrared light may have a small HOMO-LUMO bandgap energy. The p-type semiconductor compound may be designed (e.g., configured) to have a small energy bandgap because the conjugate length can be easily adjusted. Research on materials (especially polymers) having a small energy bandgap have been conducted in various ways so far, but the absorption characteristics in the infrared region are very low, and thus the device efficiency in the infrared region tends to be almost nonexistent.

The p-type semiconductor compound includes a first structural unit represented by Chemical Formula 1 and a second structural unit including an electron donating moiety to provide a structure having strong charge transfer characteristics and a small energy bandgap. Accordingly, the polymer may effectively absorb light in the near-infrared/infrared wavelength region (e.g., about 1000 nm to about 3000 nm or about 1100 nm to about 3000 nm), thereby providing improved performance of a device (e.g., a photoactive layer, a thin film, etc.) that includes an infrared absorption composition that includes the p-type semiconductor. In addition, since the thin film may be formed by a solution process, the manufacturing cost of the device may be reduced based on the device including the infrared absorption composition in the thin film.

In Chemical Formula 1, Ar¹ may be a benzene ring, a substituted or unsubstituted naphthalene ring, a substituted or unsubstituted anthracene ring, a substituted or unsubstituted phenanthrene ring, a substituted or unsubstituted tetracene ring, or a substituted or unsubstituted pyrene ring. In addition, in Chemical Formula 1, Ar¹ may be a substituted or unsubstituted quinoline ring, a substituted or unsubstituted isoquinoline ring, a substituted or unsubstituted quinoxaline ring, a substituted or unsubstituted quinazoline ring, or a substituted or unsubstituted phenanthroline ring.

In Chemical Formula 1, Ar¹ may be a benzene ring, a substituted or unsubstituted naphthalene ring, a substituted or unsubstituted anthracene ring, a substituted or unsubstituted phenanthrene ring, a substituted or unsubstituted tetracene ring, a substituted or unsubstituted pyrene ring, a substituted or unsubstituted quinoline ring, a substituted or unsubstituted isoquinoline ring, a substituted or unsubstituted quinoxaline ring, a substituted or unsubstituted quinazoline ring, or a substituted or unsubstituted phenanthroline ring.

In Chemical Formula 1, Ar¹ may be one of moieties (e.g., one moiety of the moieties) represented by Chemical Formula 1A-1.

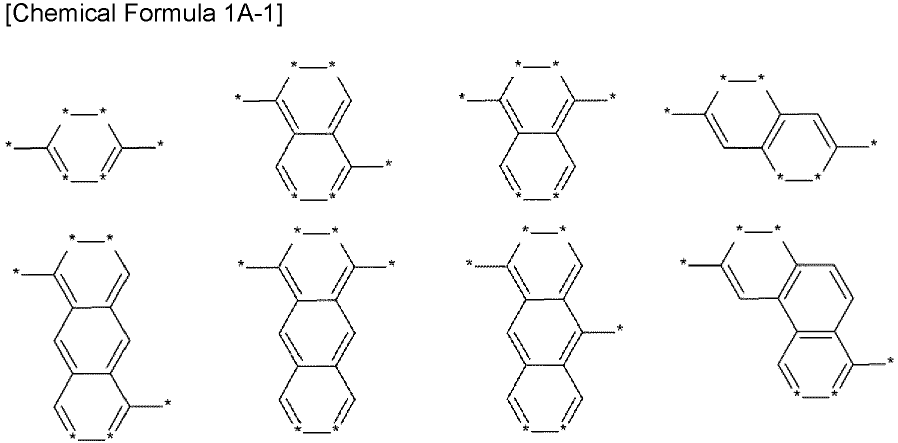

In Chemical Formula 1A-1,
at least one hydrogen of each aromatic ring may be retained as hydrogen or may be replaced by deuterium, a halogen, a cyano group, a C1 to C10 alkyl group, a C1 to C10 haloalkyl group, a -SiH₃ group, or a C1 to C10 alkylsilyl group, and
adjacent pairs of *'s inside at least one aromatic ring are linking points with an N-X-N-containing ring and a pyrazine ring of Chemical Formula 1.

In Chemical Formula 1, Ar¹ may be one of moieties (e.g., one moiety of the moieties) represented by Chemical Formula 1A-2.

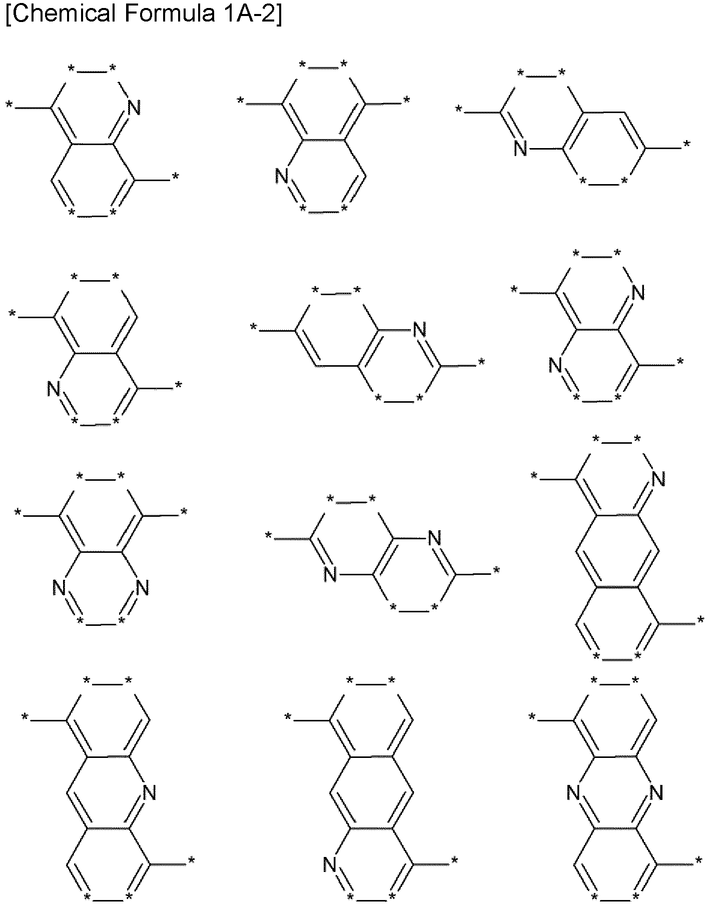

In Chemical Formula 1A-2,
at least one hydrogen of each aromatic or heteroaromatic ring may be retained as hydrogen or may be replaced by deuterium, a halogen, a cyano group, a C1 to C10 alkyl group, a C1 to C10 haloalkyl group, a -SiH₃ group, or a C1 to C10 alkylsilyl group, and
adjacent pairs of *'s inside at least one aromatic or heteroaromatic ring are linking points with an N-X-N-containing ring and a pyrazine ring of Chemical Formula 1.

In Chemical Formula 1, when R^{1a} and R^{2a} are a substituted or unsubstituted C6 to C30 aryl group or a substituted or unsubstituted C3 to C30 heteroaryl group, the C6 to C30 aryl group or C3 to C30 heteroaryl group may be substituted with a C1 to C30 alkoxy group; or a C6 to C30 aryloxy group substituted with a C1 to C30 alkyl group or a C1 to C30 alkoxy group. In this case, the solubility of the polymer in the solvent may be improved.

In Chemical Formula 1, when R^{1a} and R^{2a} are a substituted or unsubstituted C6 to C30 aryl group or a substituted or unsubstituted C3 to C30 heteroaryl group, Chemical Formula 1 may be represented by Chemical Formula 1C.

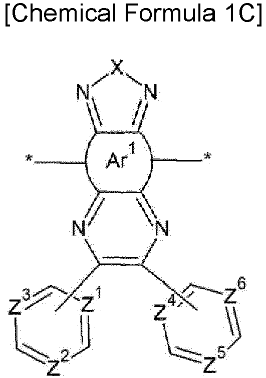

In Chemical Formula 1C,
Ar¹ and X are the same as in Chemical Formula 1, such that Ar¹ is a substituted or unsubstituted C6 to C30 aromatic ring, a substituted or unsubstituted C3 to C30 heteroaromatic ring, or any combination thereof, and X is O, S, Se, Te, S(=O), S(=O₂), NR^{a}, CR^{b}R^{c}, SiR^{d}R^{e}, GeR^{f}R^{g}, CR^{h}=CRⁱ, or CR^{hh}=CRⁱⁱ, wherein R^{a}, R^{b}, R^{c}, R^{d}, R^{e}, R^{f}, R^{g}, R^{h}, and Rⁱ are each independently hydrogen, deuterium, a C1 to C6 alkyl group, a C1 to C6 haloalkyl group, a C6 to C14 aryl group, a C3 to C12 heteroaryl group, a halogen, a cyano group, or any combination thereof, and R^{hh} and Rⁱⁱ are each independently a C1 to C6 alkylene group or a C2 to C6 heteroalkylene group and linked to each other to provide an aromatic or heteroaromatic ring,
Z¹ to Z⁶ may each independently be N or CR^{x}, wherein R^{x} is hydrogen, deuterium, a C1 to C20 alkyl group, a C1 to C20 haloalkyl group, a -SiH₃ group, a C1 to C20 alkylsilyl group, an -NH₂ group, a C1 to C20 alkylamine group, a C6 to C12 aryl group, a C3 to C12 heteroaryl group, a halogen, a cyano group, or any combination thereof,
at least one hydrogen of each aromatic or heteroaromatic ring may be retained as hydrogen or may be replaced by deuterium, a halogen, a cyano group, a C1 to C30 alkyl group, a C1 to C30 alkoxy group, a C1 to C30 haloalkyl group, a C6 to C30 aryl group, a C6 to C30 aryloxy group, a -SiH₃ group, or a C1 to C30 alkylsilyl group, and
* is a linking point within the p-type semiconductor compound.

The substituted or unsubstituted C6 to C30 arene group and the substituted or unsubstituted C3 to C30 heteroarene group formed by linking R^{1a} and R^{2a} to each other may shift an absorption wavelength of the polymer to a longer wavelength and increase stability of the polymer.

In Chemical Formula 1, R^{1a} and R^{2a} may be linked to each other, and the substituted or unsubstituted C6 to C30 arene group and the substituted or unsubstituted C3 to C30 heteroarene group formed by linking R^{1a} and R^{2a} to each other may be a substituted or unsubstituted benzene ring, a substituted or unsubstituted naphthalene ring, a substituted or unsubstituted acenaphthene ring, a substituted or unsubstituted anthracene ring, a substituted or unsubstituted phenanthrene ring, a substituted or unsubstituted tetracene ring, or a substituted or unsubstituted pyrene ring; or a substituted or unsubstituted quinoline ring, a substituted or unsubstituted isoquinoline ring, a substituted or unsubstituted quinoxaline ring, a substituted or unsubstituted quinazoline ring, a substituted or unsubstituted phenanthroline ring, a substituted or unsubstituted pyrimidine ring, or a substituted or unsubstituted benzodithiophene ring.

In Chemical Formula 1, R^{1a} and R^{2a} may be linked to each other, and the substituted or unsubstituted C6 to C30 arene group and the substituted or unsubstituted C3 to C30 heteroarene group formed by linking R^{1a} and R^{2a} to each other may be one of moieties (e.g., one moiety of the moieties) represented by Chemical Formulas 1B-1 and 1B-2.

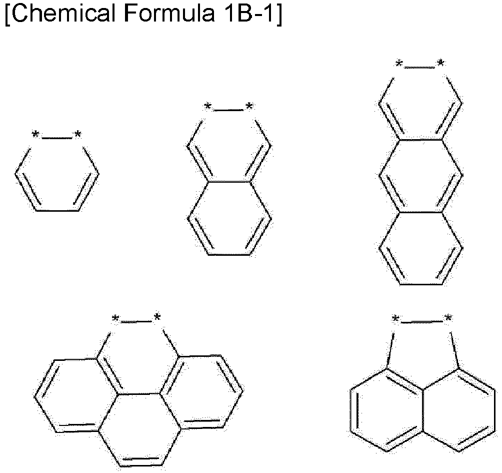

In Chemical Formula 1B-1,
at least one hydrogen of each aromatic ring may be retained as hydrogen or may be replaced by a halogen, a cyano group, a C1 to C30 alkyl group, a C1 to C30 alkoxy group, a C1 to C30 haloalkyl group, a -SiH₃ group, a C1 to C30 alkylsilyl group, a C6 to C30 aryl group, a C6 to C30 aryloxy group, or a C3 to C30 heteroaryl group, and
each * inside the aromatic ring is a point bonded to the pyrazine ring of Chemical Formula 1.

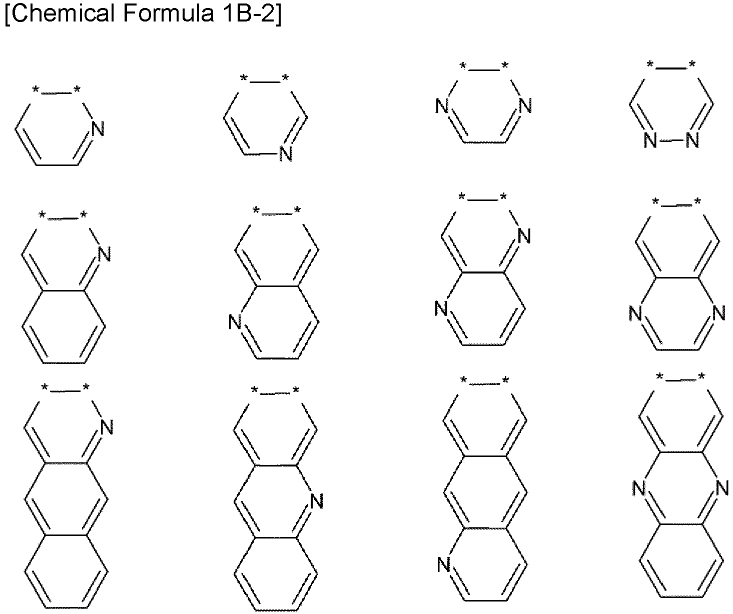

In Chemical Formula 1B-2,
at least one hydrogen of each aromatic or heteroaromatic ring may be retained as hydrogen or may be replaced by a halogen, a cyano group, a C1 to C30 alkyl group, a C1 to C30 alkoxy group, a C1 to C30 haloalkyl group, a -SiH₃ group, a C1 to C30 alkylsilyl group, a C6 to C30 aryl group, a C6 to C30 aryloxy group, or a C3 to C30 heteroaryl group, and
each * inside the aromatic or heteroaromatic ring is a point bonded to the pyrazine ring of Chemical Formula 1.

In Chemical Formula 1, R^{1a} and R^{2a} may be linked to each other, and the substituted or unsubstituted C6 to C30 arene group and the substituted or unsubstituted C3 to C30 heteroarene group formed by linking R^{1a} and R^{2a} to each other may each independently one of moieties (e.g., one moiety of the moieties) having an aromatic or heteroaromatic ring and represented by Chemical Formula 1B-3 or 1B-4.

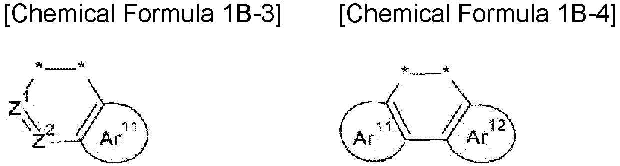

In Chemical Formulas 1B-3 and 1B-4,
Ar¹¹ and Ar¹² may each independently be a substituted or unsubstituted C6 to C30 arene group or a substituted or unsubstituted C3 to C30 heteroarene group,
in Chemical Formula 1B-3, Z¹ and Z² may each independently be N or CR^{x}, wherein R^{x} is hydrogen, deuterium, a C1 to C10 alkyl group, a C1 to C10 haloalkyl group, a -SiH₃ group, a C1 to C10 alkylsilyl group, a -NH₂ group, a C1 to C10 alkylamine group, a C6 to C10 arylamine group, a C6 to C14 aryl group, a C3 to C12 heteroaryl group, a halogen, a cyano group, or any combination thereof, and
each * inside the aromatic ring is a point bonded to the pyrazine ring of Chemical Formula 1.

The moiety represented by Chemical Formula 1B-3 may be represented by Chemical Formula 1B-3-1.

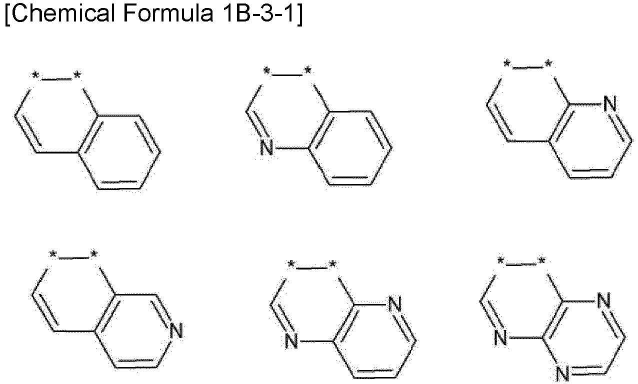

In Chemical Formula 1B-3-1,
at least one hydrogen of each aromatic or heteroaromatic ring may be retained as hydrogen or may be replaced by a halogen, a cyano group, a C1 to C30 alkyl group, a C1 to C30 alkoxy group, a C1 to C30 haloalkyl group, a -SiH₃ group, a C1 to C30 alkylsilyl group, a C6 to C30 aryl group, a C6 to C30 aryloxy group, or a C3 to C30 heteroaryl group, and
each * inside the aromatic or heteroaromatic ring is a point bonded to the pyrazine ring of Chemical Formula 1.

The moiety represented by Chemical Formula 1B-4 may be represented by Chemical Formula 1B-4-1.

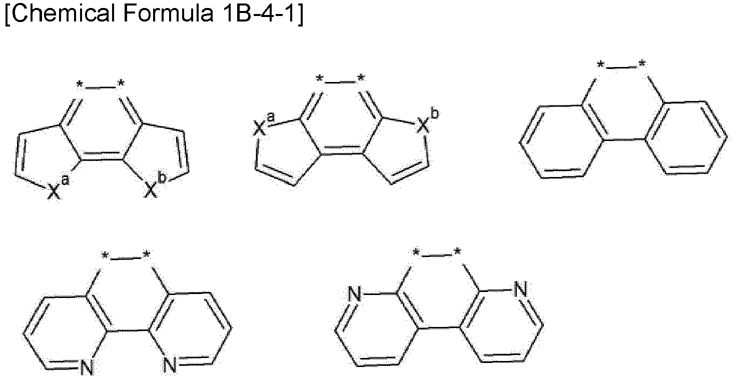

In Chemical Formula 1B-4-1,
at least one hydrogen of each aromatic or heteroaromatic ring may be retained as hydrogen or may be replaced by a halogen, a cyano group, a C1 to C30 alkyl group, a C1 to C30 alkoxy group, a C1 to C30 haloalkyl group, a -SiH₃ group, a C1 to C30 alkylsilyl group, a C6 to C30 aryl group, a C6 to C30 aryloxy group, or a C3 to C30 heteroaryl group,
X^{a} and X^{b} may each independently be O, S, Se, Te, NR^{a}, SiR^{b}R^{c}, or GeR^{d}R^{e}, wherein R^{a}, R^{b}, R^{c}, R^{d}, or R^{e} are each independently hydrogen, a halogen, a substituted or unsubstituted C1 to C10 alkyl group, or a substituted or unsubstituted C6 to C10 aryl group, and
each * inside the aromatic or heteroaromatic ring is a point bonded to the pyrazine ring of Chemical Formula 1.

The substituted or unsubstituted C6 to C30 arene group and the substituted or unsubstituted C3 to C30 heteroarene group formed by linking R^{1a} and R^{2a} to each other may increase a conjugate length and increase a planarization of the polymer structure to reduce the energy bandgap of the polymer. Thereby, the absorption of the long wavelength region of the polymer may be increased.

In Chemical Formula 1B-1, 1B-2, 1B-3, 1B-4, 1B-3-1, and/or 1B-4-1, when hydrogen of the aromatic or heteroaromatic ring is substituted with a C1 to C30 alkoxy group; or a C6 to C30 aryloxy group substituted with a C1 to C30 alkyl group or a C1 to C30 alkoxy group, the solubility of the polymer may be improved.

The p-type semiconductor compound including the first structural unit further includes a second structural unit including an electron donating moiety. The second structural unit increases planarity of the polymer and shifts the absorption wavelength of the polymer to a longer wavelength, thereby exhibiting excellent absorption characteristics in the infrared region (e.g., extinction coefficient in the infrared region). The first structural unit may serve as an acceptor and the second structural unit may serve as a donor to improve charge transfer characteristics.

The electron donating moiety included in the second structural unit of the p-type semiconductor compound may include at least one of moieties (e.g., at least one moiety of the moieties) of Group 1 (Chemical Formulas 4A to 4J).

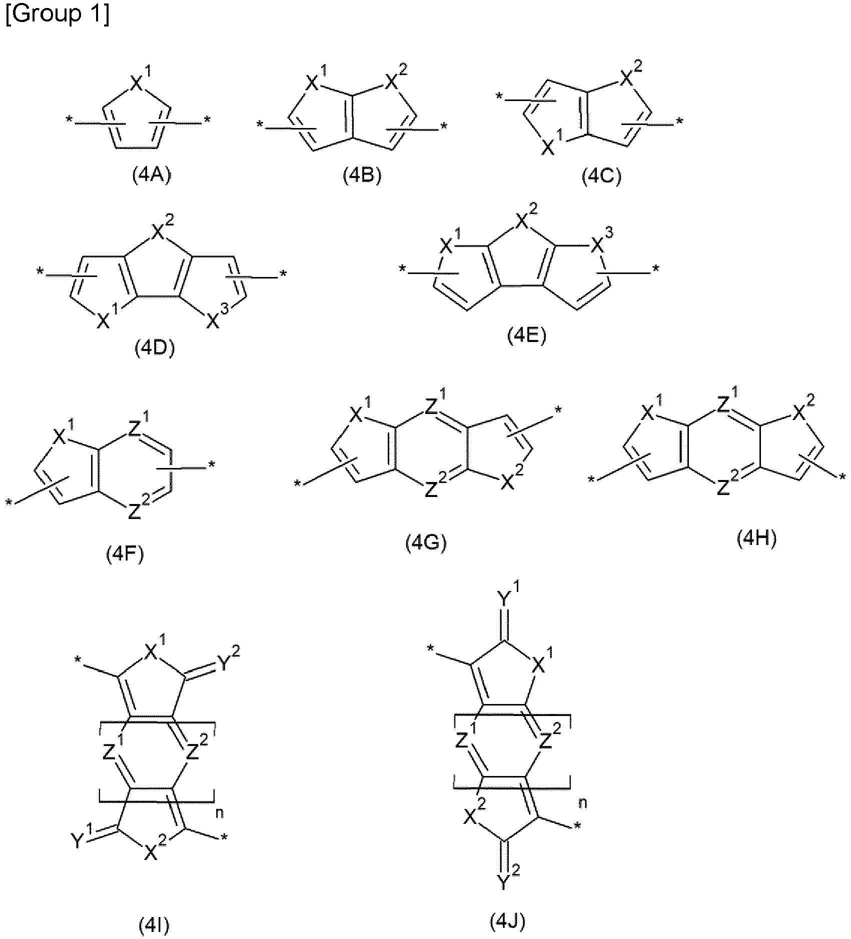

In Group 1,
X¹ to X³ may each independently be S, Se, Te, S(=O), S(=O₂), NR^{a}, SiR^{d}R^{e}, or GeR^{f}R^{g}, wherein R^{a}, R^{b}, R^{c}, R^{d}, R^{e}, R^{f}, and R^{g} may each independently be hydrogen, deuterium, a C1 to C20 alkyl group, a C1 to C20 alkoxy group, a C1 to C20 haloalkyl group, a C6 to C20 aryl group, a C3 to C20 heteroaryl group, a halogen, a cyano group, or any combination thereof,
Z¹ and Z² may each independently be N or CR^{x}, wherein R^{x} may be hydrogen, deuterium, a C1 to C10 alkyl group, a C1 to C10 haloalkyl group, a -SiH₃ group, a C1 to C10 alkylsilyl group, a -NH₂ group, a C1 to C10 alkylamine group, a C6 to C12 aryl group, a C3 to C12 heteroaryl group, a halogen, a cyano group, or any combination thereof,
Y¹ and Y² may each independently be O, S, Se, or Te,
n may be 0 or 1, and
at least one hydrogen of each aromatic or heteroaromatic ring may be retained as hydrogen or may be replaced by deuterium, a halogen, a cyano group, a C1 to C30 alkyl group, a C1 to C30 alkoxy group, a C1 to C30 haloalkyl group, a C6 to C30 aryl group, a C6 to C30 aryloxy group, a -SiH₃ group, or a C1 to C30 alkylsilyl group.

In Group 1 (Chemical Formulas 4A to 4J), for example, in Chemical Formula 4A, a moiety in which X¹ is Se and a moiety in which X¹ is S may be included together.

In Group 1 (Chemical Formulas 4A to 4J), when hydrogen of the aromatic or heteroaromatic ring is replaced by a C1 to C30 alkoxy group; or a C6 to C30 aryloxy group substituted with a C1 to C30 alkyl group or a C1 to C30 alkoxy group, solubility of the p-type semiconductor compound may be improved.

The first structural unit may be included in an amount of greater than or equal to about 20 mol%, greater than or equal to about 21 mol%, greater than or equal to about 22 mol%, greater than or equal to about 23 mol%, greater than or equal to about 24 mol%, or greater than or equal to about 25 mol% and less than or equal to about 50 mol%, less than or equal to about 49 mol%, less than or equal to about 48 mol%, less than or equal to about 47 mol%, less than or equal to about 46 mol%, less than or equal to about 45 mol%, or less than or equal to about 44 mol% based on 100 mol% of the p-type semiconductor compound. The second structural unit may be included in an amount of greater than or equal to about 50 mol%, greater than or equal to about 51 mol%, greater than or equal to about 52 mol%, greater than or equal to about 53 mol%, greater than or equal to about 54 mol%, or greater than or equal to about 55 mol% and less than or equal to about 80 mol%, less than or equal to about 79 mol%, less than or equal to about 78 mol%, less than or equal to about 77 mol%, less than or equal to about 76 mol%, less than or equal to about 75 mol%, or less than or equal to about 74 mol% based on 100 mol% of the p-type semiconductor compound. In the above amount range, a p-type semiconductor compound having improved infrared absorption characteristics may be obtained.

The p-type semiconductor compound may further include a third structural unit selected from Group 1 (Chemical Formulas 4A to 4J) and having a different structure from the second structural unit. The third structural unit may be included in an amount of greater than or equal to about 40 parts by mole, greater than or equal to about 41 parts by mole, greater than or equal to about 42 parts by mole, greater than or equal to about 43 parts by mole, greater than or equal to about 44 parts by mole, or greater than or equal to about 45 parts by mole and less than or equal to about 300 parts by mole, less than or equal to about 290 parts by mole, less than or equal to about 280 parts by mole, less than or equal to about 270 parts by mole, less than or equal to about 260 parts by mole, less than or equal to about 250 parts by mole, or less than or equal to about 240 parts by mole, based on 100 parts by mole in total of the first structural unit and the second structural unit. In the above amount range, a p-type semiconductor compound having improved infrared absorption characteristics may be obtained.

The p-type semiconductor compound including the first structural unit, the second structural unit and optionally the third structural unit may be an alternating copolymer, a random copolymer, or a block copolymer.

The p-type semiconductor compound may have a number average molecular weight of greater than or equal to about 1,000 g/mol, for example greater than or equal to about 1,500 g/mol, greater than or equal to about 2,000 g/mol, greater than or equal to about 2,500 g/mol, greater than or equal to about 3,000 g/mol, greater than or equal to about 3,500 g/mol, or greater than or equal to about 4,000 g/mol and less than or equal to about 80,000 g/mol, for example less than or equal to about 75,000 g/mol, less than or equal to about 70,000 g/mol, less than or equal to about 65,000 g/mol, less than or equal to about 60,000 g/mol, less than or equal to about 55,000 g/mol, or less than or equal to about 50,000 g/mol.

For example, the p-type semiconductor compound may be a polymer including at least one of the structural units represented by Group 3.

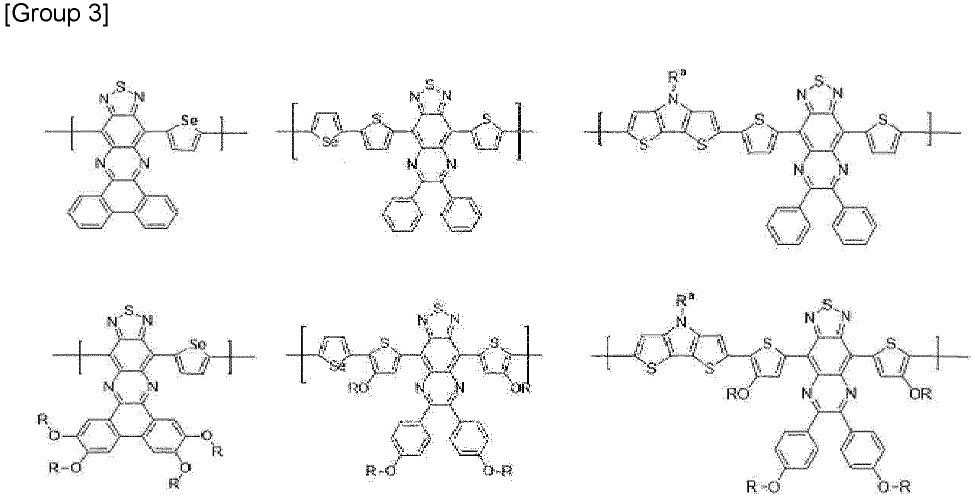

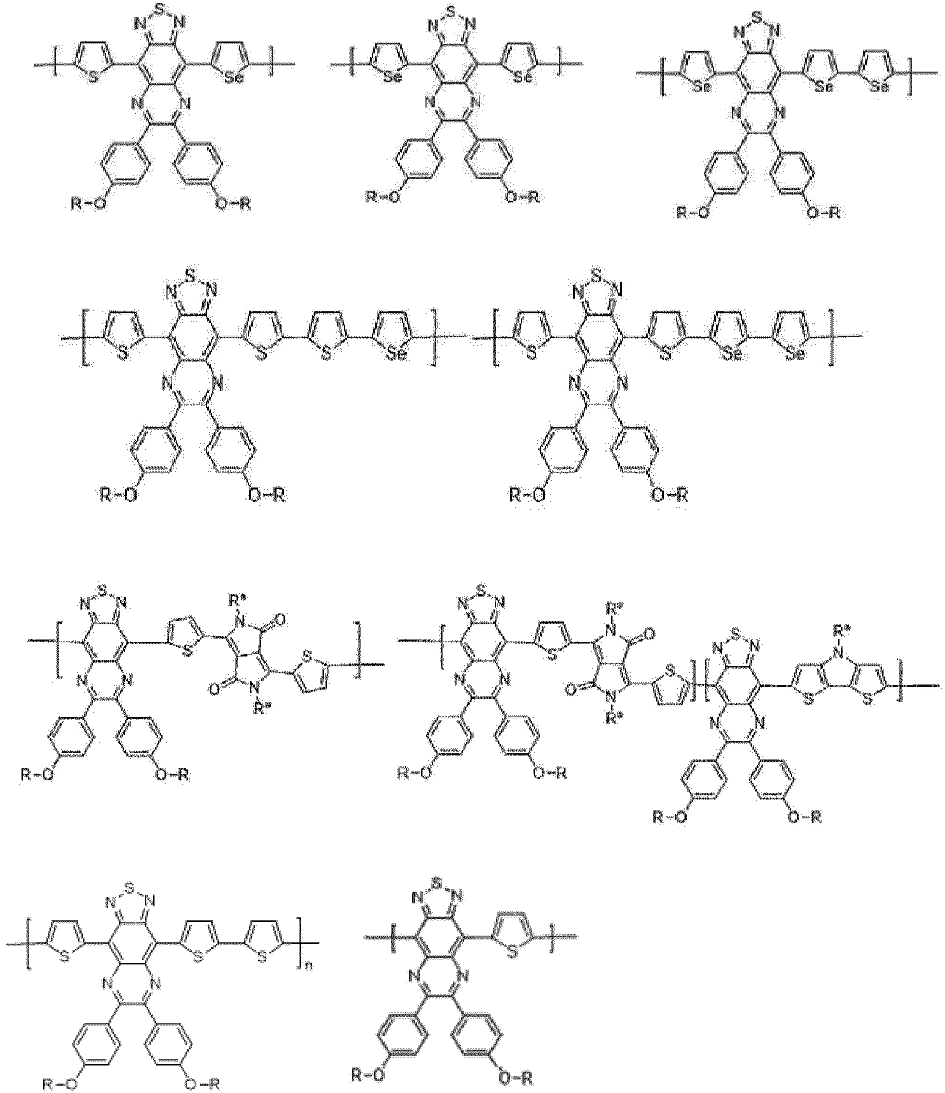

In Group 3,
OR may be a C1 to C30 alkoxy group or a C6 to C30 aryloxy group (e.g., a C1 to C30 alkyl group or a C1 to C30 alkoxy group substituted C6 to C30 aryloxy group), and a plurality of ORs in one polymer are the same or different from each other.
R^{a} may be hydrogen, a halogen, a substituted or unsubstituted C1 to C10 alkyl group, or a substituted or unsubstituted C6 to C10 aryl group, and
at least one hydrogen of each aromatic or heteroaromatic ring (e.g., thiophene ring, selenophene ring, benzene ring, etc.) may be replaced by deuterium, a halogen, a cyano group, a C1 to C10 alkyl group, a C1 to C10 haloalkyl group, a - SiH₃ group, or a C1 to C10 alkylsilyl group.

The p-type semiconductor compound may absorb light in the near-infrared/infrared wavelength region and a peak absorption wavelength (λₘₐₓ) of the p-type semiconductor compound (e.g., a peak absorption wavelength that the p-type semiconductor compound is configured to exhibit) may be for example greater than or equal to about 1000 nm, for example greater than or equal to about 1010 nm, greater than or equal to about 1020 nm, greater than or equal to about 1030 nm, greater than or equal to about 1040 nm, greater than or equal to about 1050 nm, greater than or equal to about 1060 nm, greater than or equal to about 1070 nm, greater than or equal to about 1080 nm, greater than or equal to about 1090 nm, or greater than or equal to about 1100 nm. The peak absorption wavelength of the p-type semiconductor compound (e.g., a peak absorption wavelength that the p-type semiconductor compound is configured to exhibit) may for example belong to a wavelength region of less than or equal to about 3000 nm, less than or equal to about 2900 nm, less than or equal to about 2800 nm, less than or equal to about 2700 nm, less than or equal to about 2600 nm, less than or equal to about 2500 nm, less than or equal to about 2400 nm, less than or equal to about 2300 nm, less than or equal to about 2200 nm, or less than or equal to about 2100 nm. The peak absorption wavelength of the p-type semiconductor compound may for example belong to a wavelength region of about 1000 nm to about 3000 nm, and within the above range, for example about 1000 nm to about 2500 nm, for example about 1010 nm to about 2200 nm, for example about 1010 nm to about 2100 nm, for example about 1010 nm to about 2000 nm, for example about 1020 nm to about 2000 nm, for example about 1030 nm to about 2000 nm, or for example about 1040 nm to about 2000 nm.

The n-type semiconductor compound providing the BHJ structure with the p-type semiconductor compound_may be represented by Chemical Formula 2.

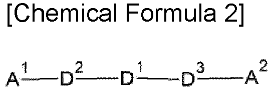

In Chemical Formula 2
D¹ may be a first electron donating moiety having any one structure of the structures represented by Chemical Formulas 3A to 3E,
D² and D³ may each independently be a single bond or a second electron donating moiety, and
A¹ and A² may each independently be an electron accepting moiety of a substituted or unsubstituted C6 to C30 hydrocarbon ring group having at least one functional group of C=O, C=S, C=Se, C=Te, or C=C(CN)₂; a substituted or unsubstituted C2 to C30 heterocyclic group having at least one functional group of C=O, C=S, C=Se, C=Te, or C=C(CN)₂; or a fused ring thereof,

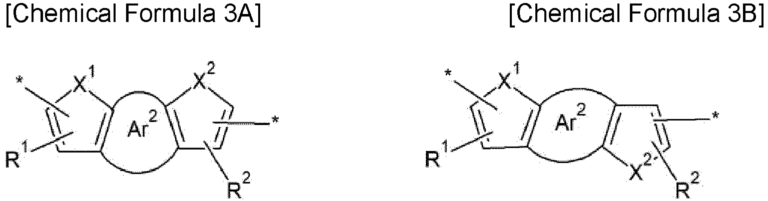

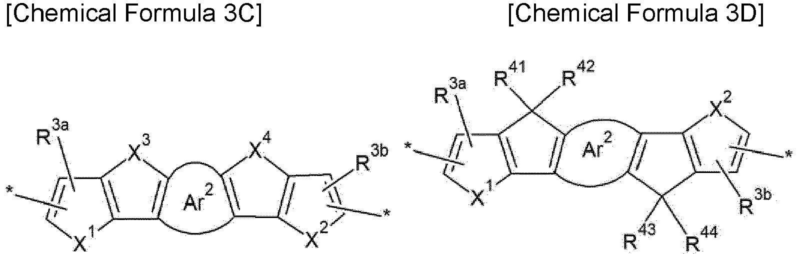

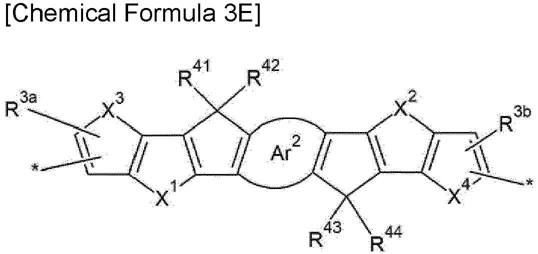

In Chemical Formulas 3A to 3E,
Ar² may be a substituted or unsubstituted C6 to C30 arene group; a substituted or unsubstituted C3 to C30 heterocyclic group including at least one of N, O, S, Se, Te, or Si; a fused ring thereof; or any combination thereof,
X¹, X², X³, and X⁴ may each independently be S, Se, or Te,
R⁴¹, R⁴², R⁴³, and R⁴⁴ may each independently be a substituted or unsubstituted C1 to C30 alkyl group, a substituted or unsubstituted C1 to C30 alkoxy group, a substituted or unsubstituted C6 to C20 aryl group, or a substituted or unsubstituted C3 to C20 heteroaryl group,
R¹, R², R^{3a} and R^{3b} may each independently be hydrogen or a C1 to C10 alkyl group, and
* denotes a linking point within Chemical Formula 2.

In Chemical Formulas 3A to 3C, Ar² may be a moiety having one structure of structures of Group 2 (Chemical Formulas 5A to 5K).

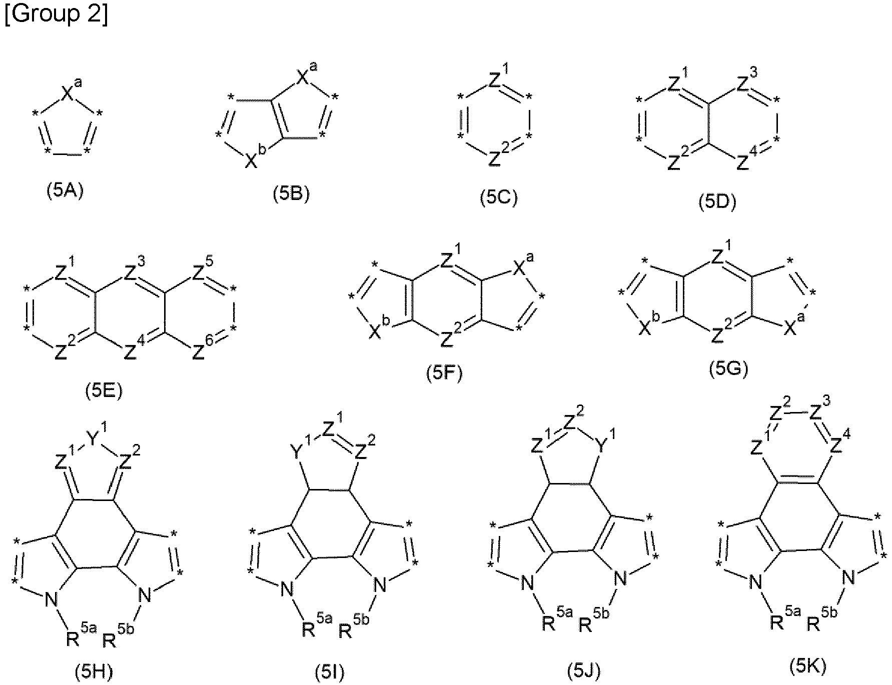

In Group 2,
X^{a} and X^{b} may each independently be CR^{x}R^{y}, S, Se, or Te, wherein R^{x} and R^{y} may each independently be a substituted or unsubstituted C1 to C30 alkyl group, a substituted or unsubstituted C1 to C30 alkoxy group, a substituted or unsubstituted C6 to C20 aryl group, or a substituted or unsubstituted C3 to C20 heteroaryl group,
R^{5a} and R^{5b} may each independently be hydrogen, a C1 to C20 alkyl group, a C1 to C20 alkoxy group, a C6 to C10 aryl group, or a C2 to C10 heteroaryl group,
Y¹ may be CR^{p}R^{q}, NR^{r}, O, S, Se, or Te, wherein R^{p}, R^{q}, and R^{r} may each independently be hydrogen or a C1 to C20 alkyl group, and
Z¹ to Z⁶ may each independently be CR^{s} or N, wherein, R^{s} is hydrogen or a C1 to C20 alkyl group.

In Chemical Formulas 3D and 3E, R⁴¹, R⁴², R⁴³, and R⁴⁴ may each independently be a C1 to C20 (e.g., C4 to C15 or C4 to C10) alkyl group; a C1 to C20 (e.g., C4 to C15 or C4 to C10) alkoxy group; a C6 to C20 aryl group substituted with a C1 to C20 (e.g., C4 to C15 or C4 to C10) alkyl group, or a C1 to C20 (e.g., C4 to C15 or C4 to C10) alkoxy group; or a C3 to C20 heteroaryl group substituted with a C1 to C20 (e.g., C4 to C15 or C4 to C10) alkyl group or a C1 to C20 (e.g., C4 to C15 or C4 to C10) alkoxy group.

In Chemical Formulas 3D and 3E, R⁴¹, R⁴², R⁴³, and R⁴⁴ may be a substituted or unsubstituted C3 to C30 branched alkyl group or a substituted or unsubstituted C3 to C30 branched alkoxy group.

The first electron donating moiety represented by Chemical Formula 3A may be a moiety represented by Chemical Formula 3A-1.

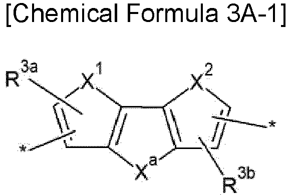

In Chemical Formula 3A-1,
X^{a} may be CR^{x}R^{y}, S, Se, or Te, wherein R^{x} and R^{y} may each independently be a substituted or unsubstituted C1 to C30 alkyl group, a substituted or unsubstituted C1 to C30 alkoxy group, a substituted or unsubstituted C6 to C20 aryl group, or a substituted or unsubstituted C3 to C20 heteroaryl group,
X¹ and X² may each independently be S, Se, or Te,
R^{3a} and R^{3b} may each independently be hydrogen or a C1 to C10 alkyl group, and
* denotes a linking point within Chemical Formula 2.

The first electron donating moiety represented by Chemical Formula 3B may be a moiety represented by Chemical Formula 3B-1.

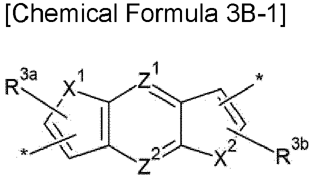

In Chemical Formula 3B-1,
Z¹ and Z² may each independently be CR^{s} or N, wherein R^{s} may be hydrogen or a C1 to C20 alkyl group,
X¹ and X² may each independently be S, Se, or Te,
R^{3a} and R^{3b} may each independently be hydrogen or a C1 to C10 alkyl group, and
* denotes a linking point within Chemical Formula 2.

The electron donating moiety represented by Chemical Formula 3C may be a moiety represented by Chemical Formula 3C-1.

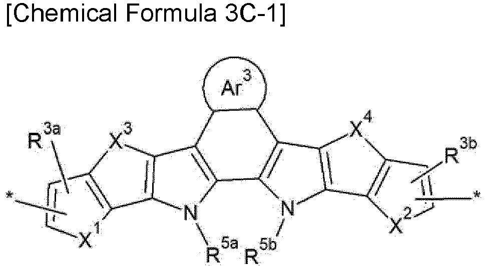

In Chemical Formula 3C-1,
Ar³ is one of moieties (e.g., one moiety of the moieties) represented by Chemical Formula 3C-1a,
X¹, X², X³, and X⁴ may each independently be S, Se, or Te,
R^{3a} and R^{3b} may each independently be hydrogen or a C1 to C10 alkyl group,
R^{5a} and R^{5b} may each independently be hydrogen, a C1 to C20 alkyl group, a C1 to C20 alkoxy group, a C6 to C10 aryl group, or a C2 to C10 heteroaryl group,
* denotes a linking point within Chemical Formula 2.

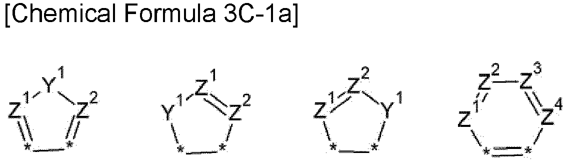

In Chemical Formula 3C-1a,
Y' may be CR^{p}R^{q}, NR^{r}, O, S, Se, or Te, wherein R^{p}, R^{q}, and R^{r} may each independently be hydrogen or a C1 to C20 alkyl group, and
Z¹ to Z⁴ may each independently be CR^{s} or N, wherein, R^{s} may be hydrogen or a C1 to C20 alkyl group, and
* inside the ring denotes a linking point with Chemical Formula 3C-1.

In Chemical Formula 3C-1a, at least one of Z¹ or Z² and/or at least one, for example, at least two of, Z¹ to Z⁴ may be N.

The moiety represented by Chemical Formula 3C-1a may be one of moieties (e.g., one moiety of the moieties) represented by Chemical Formula 3C-1aa.

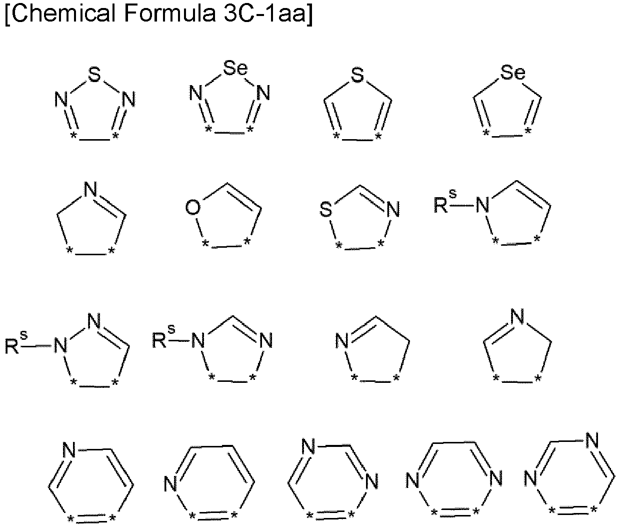

In Chemical Formula 3C-1aa,
R^{s} may be hydrogen or a C1 to C20 alkyl group (e.g., C1 to C15 alkyl group or C1 to C10 alkyl group),
at least one hydrogen of each heteroaromatic ring may be replaced by deuterium, a halogen, a cyano group, a C1 to C10 alkyl group, a C1 to C10 haloalkyl group, a -SiH₃ group, or a C1 to C10 alkylsilyl group, and
* inside the ring denotes a linking point with Chemical Formula 3C-1.

The electron donating moiety represented by Chemical Formula 3D may be a moiety represented by Chemical Formula 3D-1.

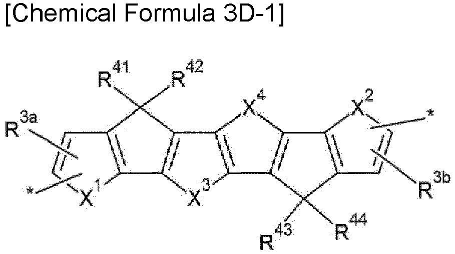

In Chemical Formula 3D-1,
X¹, X², X³, and X⁴ may each independently be S, Se, or Te,
R⁴¹, R⁴², R⁴³, and R⁴⁴ may each independently be a substituted or unsubstituted C1 to C30 alkyl group, a substituted or unsubstituted C1 to C30 alkoxy group, a substituted or unsubstituted C6 to C20 aryl group, or a substituted or unsubstituted C3 to C20 heteroaryl group,
R^{3a} and R^{3b} may each independently be hydrogen or a C1 to C10 alkyl group, and
* denotes a linking point within Chemical Formula 2.

The electron donating moiety represented by Chemical Formula 3D may be a moiety represented by Chemical Formula 3D-2.

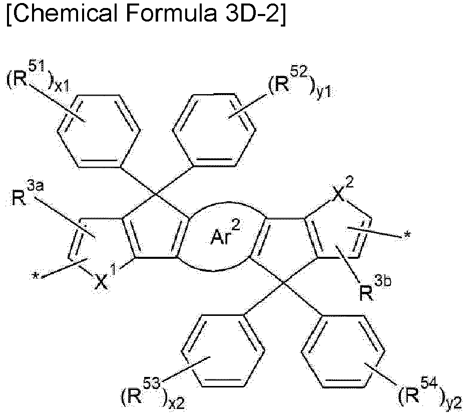

In Chemical Formula 3D-2,
Ar² may be a substituted or unsubstituted C6 to C30 arene group; a substituted or unsubstituted C3 to C30 heterocyclic group including at least one of N, O, S, Se, Te, or Si; a fused ring thereof; or any combination thereof,
X¹ and X² may each independently be S, Se, or Te,
R⁵¹, R⁵², R⁵³, and R⁵⁴ may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group or a substituted or unsubstituted C2 to C20 heteroaryl group,
x1, y1, x2, and y2 may each independently be an integer of 0 to 5,
R^{3a} and R^{3b} may each independently be hydrogen or a C1 to C10 alkyl group, and
* denotes a linking point within Chemical Formula 2.

In Chemical Formula 3D-2, R⁵¹, R⁵², R⁵³, and R⁵⁴ may be present in the para position of the benzene ring.

The electron donating moiety represented by Chemical Formula 3E may be a moiety represented by Chemical Formula 3E-1.

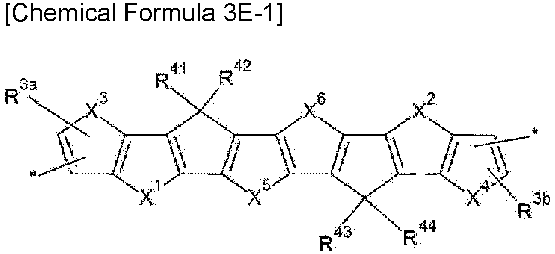

In Chemical Formula 3E-1,
X¹, X², X³, X⁴, X⁵, and X⁶ may each independently be S, Se, or Te,
R⁴¹, R⁴², R⁴³, and R⁴⁴ may each independently be a substituted or unsubstituted C1 to C30 alkyl group, a substituted or unsubstituted C1 to C30 alkoxy group, a substituted or unsubstituted C6 to C20 aryl group, or a substituted or unsubstituted C3 to C20 heteroaryl group,
R¹, R², R^{3a} and R^{3b} may each independently be hydrogen or a C1 to C10 alkyl group, and
* denotes a linking point within Chemical Formula 2.

The electron donating moiety represented by Chemical Formula 3E may be a moiety represented by Chemical Formula 3E-2.

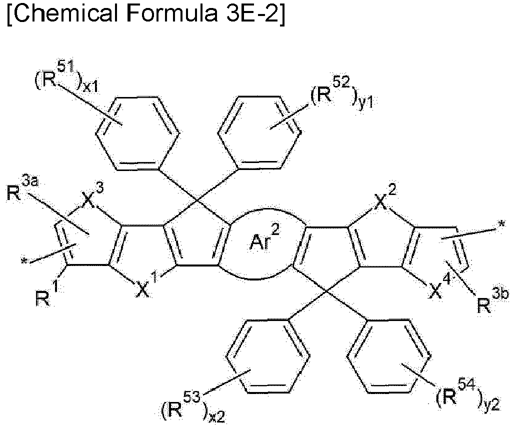

In Chemical Formula 3E-2,
Ar² may be a substituted or unsubstituted C6 to C30 arene group; a substituted or unsubstituted C3 to C30 heterocyclic group including at least one of N, O, S, Se, Te, or Si; a fused ring thereof; or any combination thereof,
X¹, X², X³, and X⁴ may each independently be S, Se, or Te,
R⁵¹, R⁵², R⁵³, and R⁵⁴ may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group or a substituted or unsubstituted C2 to C20 heteroaryl group,
x1, y1, x2, and y2 may each independently be an integer of 0 to 5,
R¹, R^{3a} and R^{3b} may each independently be hydrogen or a C1 to C10 alkyl group, and
* denotes a linking point within Chemical Formula 2.

In Chemical Formula 3E-2, R⁵¹, R⁵², R⁵³, and R⁵⁴ may be present in the para position of the benzene ring.

In Chemical Formula 2, D² and D³ may each independently represent a single bond or one of moieties (e.g., one moiety of the moieties) represented by Group 1 (Chemical Formulas 4A to 4J), and D² and D³ may be the same as or different from each other. D² and D³ may be the same as or different from D¹. When D¹, D², and D³ are the same or different electron donating moieties, charge transfer characteristics of the n-type semiconductor compound represented by Chemical Formula 2 may be improved.

In Chemical Formulas 3A to 3E, R⁴¹, R⁴², R⁴³, and R⁴⁴ may each independently be a C1 to C30 alkyl group substituted with a C1 to C20 (e.g., C4 to C15 or C4 to C10) alkyl group or a C1 to C20 (e.g., C4 to C15 or C4 to C10) alkoxy group; a C1 to C30 alkoxy group substituted with a C1 to C20 (e.g., C4 to C15 or C4 to C10) alkyl group or a C1 to C20 (e.g., C4 to C15 or C4 to C10) alkoxy group; a C6 to C10 aryl group substituted with a C1 to C20 (e.g., C4 to C15 or C4 to C10) alkyl group or a C1 to C20 (e.g., C4 to C15 or C4 to C10) alkoxy group; or a C3 to C10 heteroaryl group substituted with a C1 to C20 (e.g., C4 to C15 or C4 to C10) alkyl group or a C1 to C20 (e.g., C4 to C15 or C4 to C10) alkoxy group.

In some example embodiments, R^{5a} and R^{5b} of Chemical Formulas 5H to 5K of Group 2 may each independently be a substituted or unsubstituted branched C3 to C20 alkyl group (e.g., isopropyl group, isobutyl group, 2-propylpentyl group, 2-propyloctyl group, t-butyl group, isopentyl group, neopentyl group, 2-ethylbutyl group, 2-methylpentyl group, 3-methylpentyl group, 2,3-dimethylbutyl group, 2-ethylpentyl group, 3-ethylpentyl group, 2-methylhexyl group, 2,3-dimethylpentyl group, 2,4-dimethylpentyl group, 2-methyloctyl group, 2-ethyloctyl group, 4-methyloctyl group, 3,3-dimethyloctyl group, 4-ethyloctyl group, 2-methylheptyl group, 3-methylheptyl group, 4-methylheptyl group, 2-ethylhexyl group, 3-ethylhexyl group, 2,2,4-trimethylpentyl group, 2,4-dimethylhexyl group, 2-methyl-3-ethylpentyl group, 3-methyl-4-methylhexyl group, 3,3,4-trimethylhexyl group, 3,4,5-trimethylhexyl group, 4-ethylheptyl group, 5-methylnonyl group, 3-methyl-2-ethylheptyl group, 1-methylnonyl group, 2,3,5-trimethylheptyl group, 3-methyl-4-ethylheptyl group, 2,2,3,3-tetramethylhexyl group, 4-propylheptyl group, or 2,4-dimethyl-3-ethylhexyl group).

In Chemical Formula 2, A¹ and A² may each independently be an electron accepting moiety represented by any one of Chemical Formulas 6A to 6F.

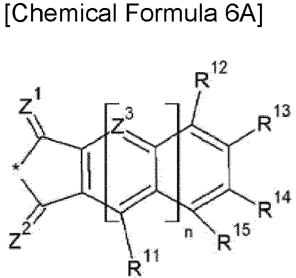

In Chemical Formula 6A,
Z¹ and Z² may each independently be O, S, Se, Te, or CR^{a}R^{b}, wherein R^{a} and R^{b} may each independently be hydrogen, a substituted or unsubstituted C1 to C10 alkyl group, or a cyano group,
Z³ may be N or CR^{c}, wherein R^{c} may be hydrogen, deuterium, or a substituted or unsubstituted C1 to C10 alkyl group,
R¹¹, R¹², R¹³, R¹⁴, and R¹⁵ may be the same or different and may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), a cyano-containing group, or any combination thereof, and R¹², R¹³, R¹⁴, and R¹⁵ may each independently be present or at least one pair of R¹² and R¹³ and R¹⁴ and R¹⁵ may be linked to each other to provide a fused aromatic ring,
n may be 0 or 1, and
* may be a linking point within Chemical Formula 2.

In some example embodiments, at least one of CR¹¹, CR¹², CR¹³, CR¹⁴, and CR¹⁵ of Chemical Formula 6A may be replaced by nitrogen (N). That is, the substituted or unsubstituted benzene ring moiety of Chemical Formula 6A may include a heteroatom (N).

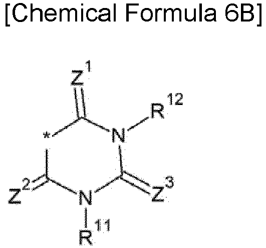

In Chemical Formula 6B,
Z¹ and Z² may each independently be O, S, Se, Te, or CR^{a}R^{b}, wherein R^{a} and R^{b} may each independently be hydrogen, a substituted or unsubstituted C1 to C10 alkyl group, or a cyano group,
Z³ may be O, S, Se, Te, or C(R^{a})(CN), wherein R^{a} is hydrogen, a cyano group (-CN), or a C1 to C10 alkyl group,
R¹¹ and R¹² may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* may be a linking point within Chemical Formula 2.

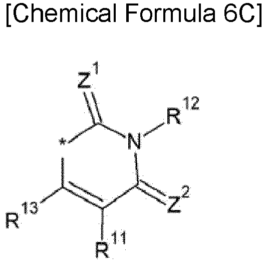

In Chemical Formula 6C,
Z¹ and Z² may each independently be O, S, Se, Te, or CR^{a}R^{b}, wherein R^{a} and R^{b} may each independently be hydrogen, a substituted or unsubstituted C1 to C10 alkyl group, or a cyano group,
R¹¹, R¹², and R¹³ may be the same or different from each other and may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* may be a linking point within Chemical Formula 2.

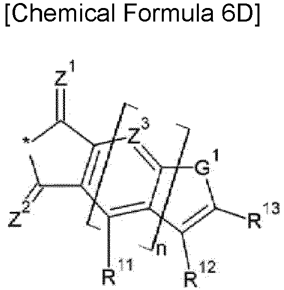

In Chemical Formula 6D,
Z¹ and Z² may each independently be O, S, Se, Te, or CR^{a}R^{b}, wherein R^{a} and R^{b} may each independently be hydrogen, a substituted or unsubstituted C1 to C10 alkyl group, or a cyano group,
Z³ may be N or CR^{c}, wherein R^{c} may be hydrogen or a substituted or unsubstituted C1 to C10 alkyl group,
G¹ may be O, S, Se, Te, SiR^{x}R^{y}, or GeR^{z}R^{w}, wherein R^{x}, R^{y}, R^{z} and R^{w} may be the same or different from each other and may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C2 to C20 heteroaryl group,
R¹¹, R¹², and R¹³ may be the same or different from each other and may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof, and R¹² and R¹³ may each independently be present or may be linked to each other to provide a fused aromatic ring,
n may be 0 or 1, and
* may be a linking point within Chemical Formula 2.

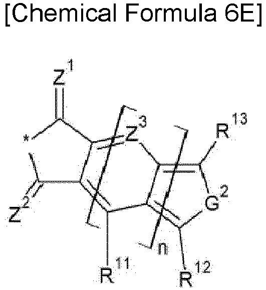

In Chemical Formula 6E,
Z¹ and Z² may each independently be O, S, Se, Te, or CR^{a}R^{b}, wherein R^{a} and R^{b} may each independently be hydrogen, a substituted or unsubstituted C1 to C10 alkyl group, or a cyano group,
Z³ is N or CR^{c}, wherein R^{c} is hydrogen or a substituted or unsubstituted C1 to C10 alkyl group,
G² may be O, S, Se, Te, SiR^{x}R^{y}, or GeR^{z}R^{w}, wherein R^{x}, R^{y}, R^{z}, and R^{w} may be the same or different and may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C2 to C20 heteroaryl group,
R¹¹, R¹², and R¹³ may be the same or different and may each independently be hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof,
n may be 0 or 1, and
* may be a linking point within Chemical Formula 2.

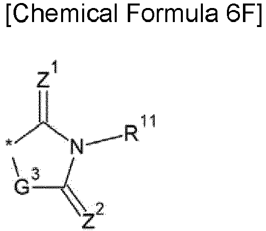

In Chemical Formula 6F,
Z¹ and Z² may each independently be O, S, Se, Te, or CR^{a}R^{b}, wherein R^{a} and R^{b} may each independently be hydrogen, a substituted or unsubstituted C1 to C10 alkyl group, or a cyano group,
R¹¹ may be hydrogen, deuterium, a substituted or unsubstituted C1 to C30 alkyl group, a substituted or unsubstituted C6 to C30 aryl group, a substituted or unsubstituted C4 to C30 heteroaryl group, a halogen, a cyano group(-CN), a cyano-containing group, or any combination thereof, and
G³ may be O, S, Se, Te, SiR^{x}R^{y}, or GeR^{z}R^{w}, wherein R^{x}, R^{y}, R^{z}, and R^{w} may be the same or different and may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C2 to C20 heteroaryl group, and
* may be a linking point within Chemical Formula 2.

In Chemical Formula 2, when D¹ is an electron donating moiety of Chemical Formula 3A, D² and D³ are each independently a single bond or a second electron donating moiety represented by Chemical Formula 4A of Group 1, and A¹ and A² are respective electron accepting moieties represented by Chemical Formula 6A, the compound represented by Chemical Formula 2 may be one of compounds represented by Chemical Formulas 7A-1 to 7A-6.

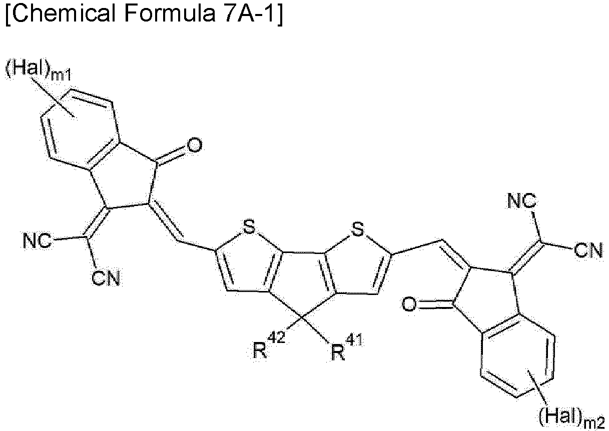

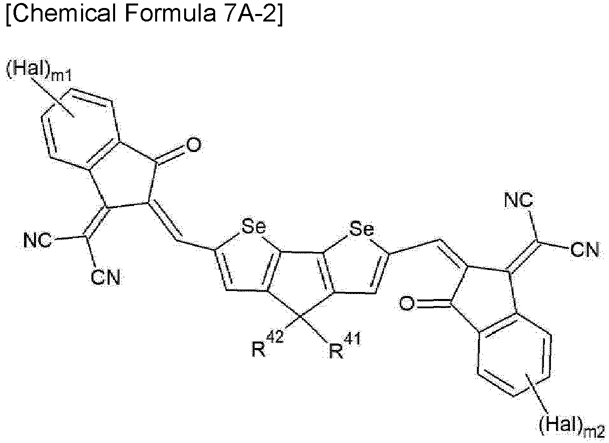

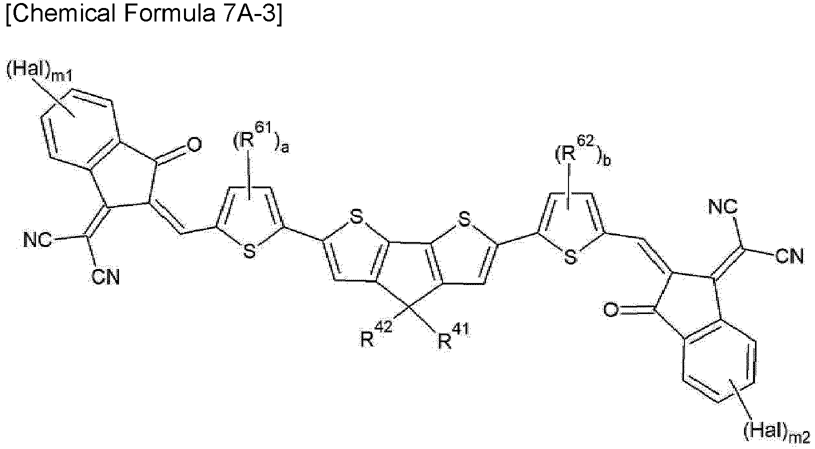

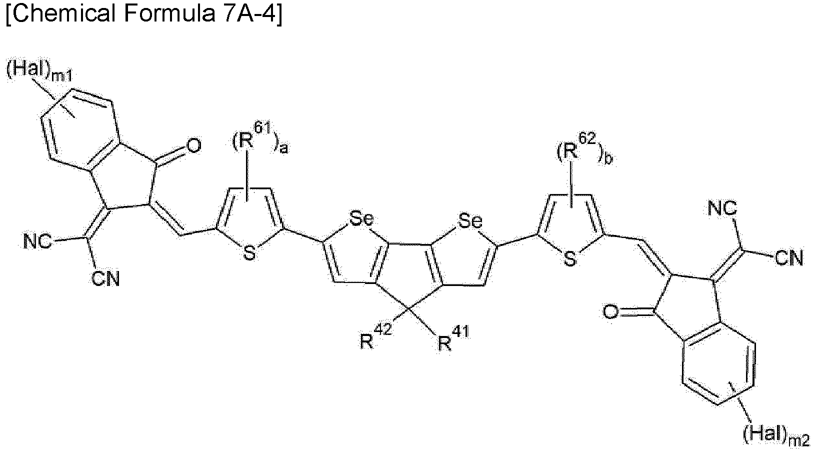

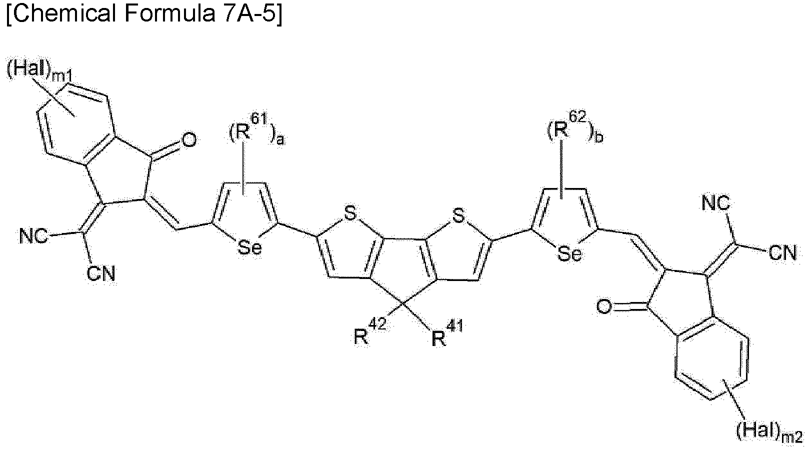

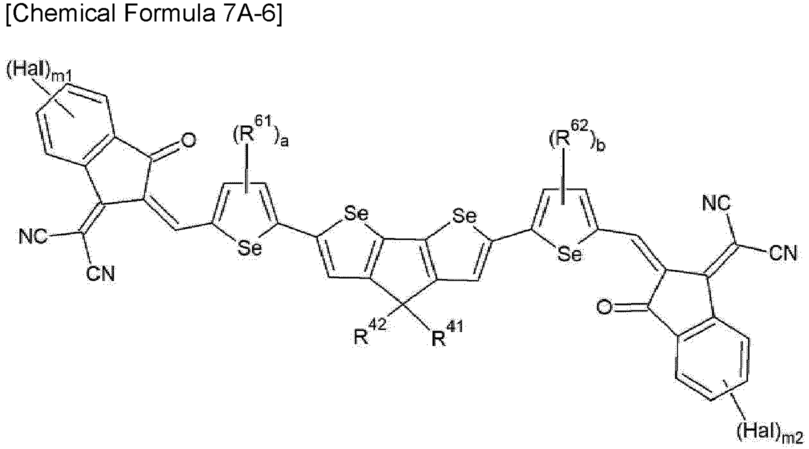

In Chemical Formulas 7A-1 to 7A-6,
R⁴¹ and R⁴² may each independently be a substituted or unsubstituted C1 to C30 alkyl group, a substituted or unsubstituted C1 to C30 alkoxy group, a substituted or unsubstituted C6 to C20 aryl group, or a substituted or unsubstituted C3 to C20 heteroaryl group,
R⁶¹ and R⁶² may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group or a substituted or unsubstituted C3 to C20 heteroaryl group,
a and b may each independently be an integer of 1 or 2,
Hal denotes a halogen (e.g., F, Cl, Br, or I), and
m1 and m2 may each independently be an integer of 0 to 4 (e.g., 0 to 2).

In Chemical Formulas 7A-1 to 7A-6, the moiety represented as an example of Chemical Formula 6A as the electron accepting moiety is illustrated, but an electron accepting moiety represented by any one of Chemical Formulas 6B to 6F may be also included.

In Chemical Formula 2, when D¹ is an electron donating moiety of Chemical Formula 3C, D² and D³ are each independently a single bond, and A¹ and A² are electron accepting moieties represented by Chemical Formula 6A, the compound represented by Chemical Formula 2 may be a compound represented by one of compounds represented by Chemical Formula 7C-1 to 7C-4.

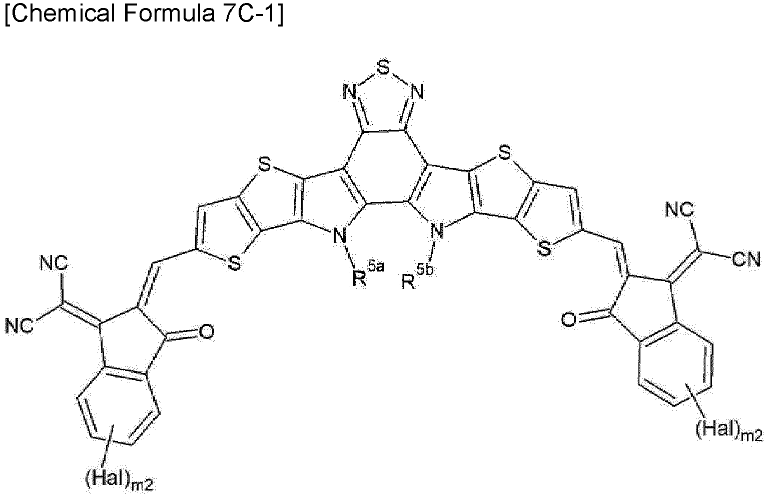

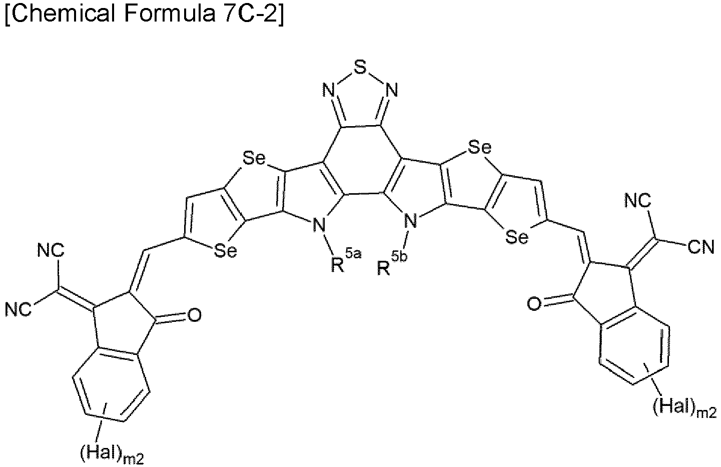

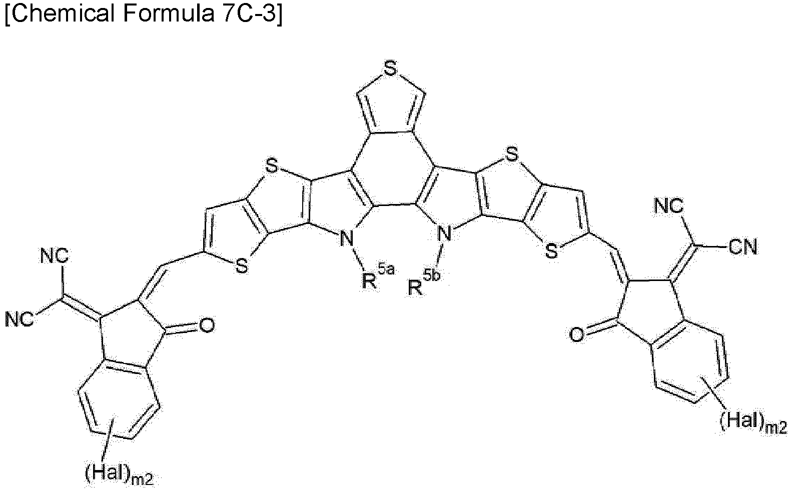

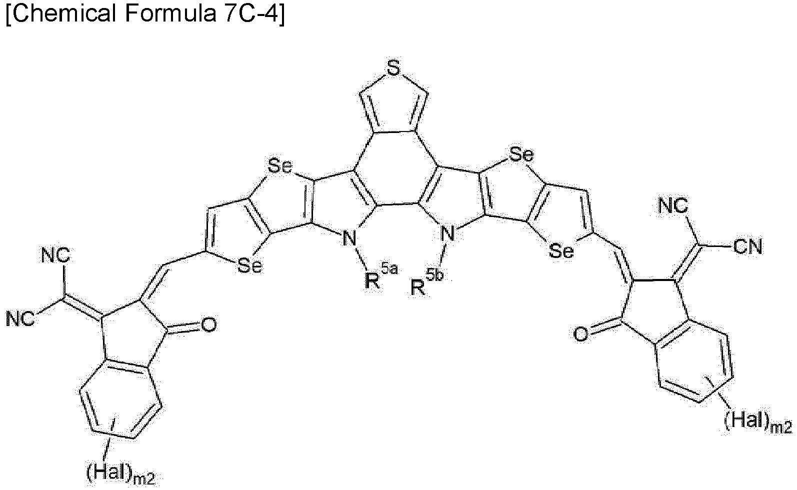

In Chemical Formulas 7C-1 to 7C-4,
R^{5a} and R^{5b} may each independently be hydrogen, a C1 to C20 alkyl group, a C1 to C20 alkoxy group, a C6 to C10 aryl group, or a C2 to C10 heteroaryl group,
Hal denotes a halogen (e.g., F, Cl, Br, or I), and
m1 and m2 may each independently be an integer of 0 to 4 (e.g., 0 to 2).

In Chemical Formulas 7C-1 to 7C-4, the moiety represented as an example of Chemical Formula 6A as the electron accepting moiety is illustrated, but an electron accepting moiety represented by any one of Chemical Formulas 6B to 6F may be also included.

In Chemical Formula 2, when D¹ is an electron donating moiety of Chemical Formula 3D, D² and D³ are each independently a single bond, and A¹ and A² are electron accepting moieties represented by Chemical Formula 6A, the compound represented by Chemical Formula 2 may be one of compounds represented by Chemical Formulas 7D-1 to 7D-4.

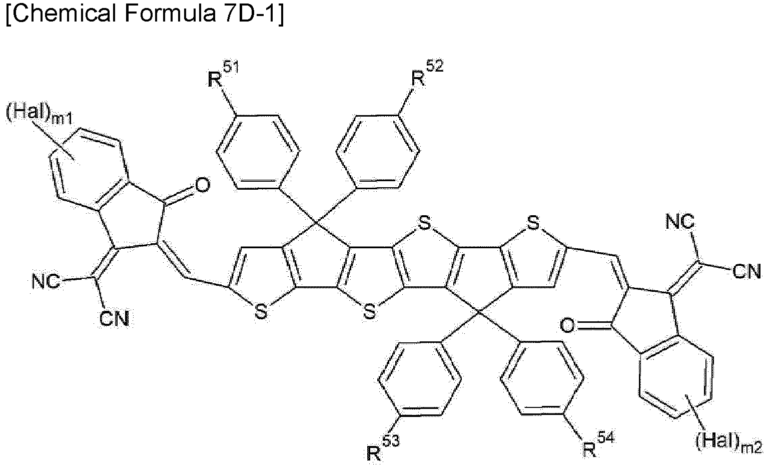

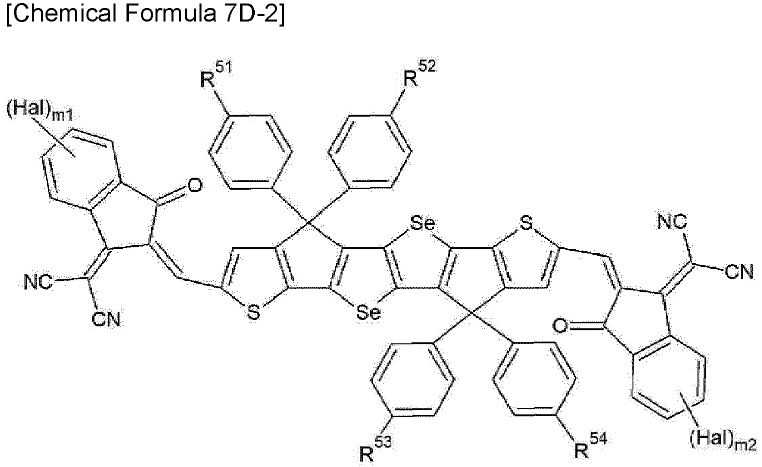

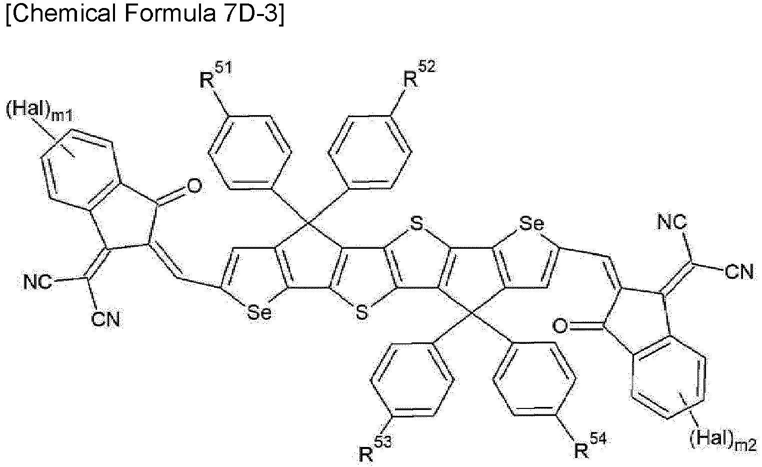

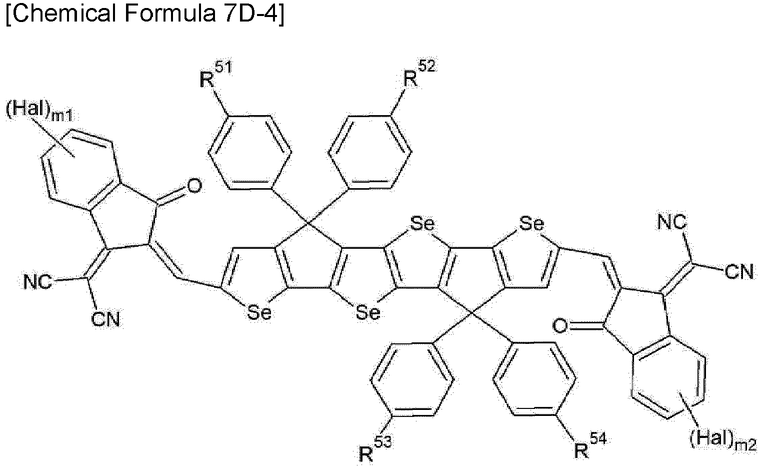

In Chemical Formulas 7D-1 to 7D-4,
R⁵¹, R⁵², R⁵³, and R⁵⁴ may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C2 to C20 heteroaryl group,
Hal denotes a halogen (e.g., F, Cl, Br, or I), and
m1 and m2 may each independently be an integer of 0 to 4 (e.g., 0 to 2).

In Chemical Formulas 7D-1 to 7D-4, the moiety represented as an example of Chemical Formula 6A as the electron accepting moiety is illustrated, but an electron accepting moiety represented by any one of Chemical Formulas 6B to 6F may be also included.

In Chemical Formula 2, when D¹ is an electron donating moiety of Chemical Formula 3E, D² and D³ are each independently a single bond, and A¹ and A² are electron accepting moieties represented by Chemical Formula 6A, the compound represented by Chemical Formula 2 may be one of compounds represented by Chemical Formulas 7E-1 to 7E-6.

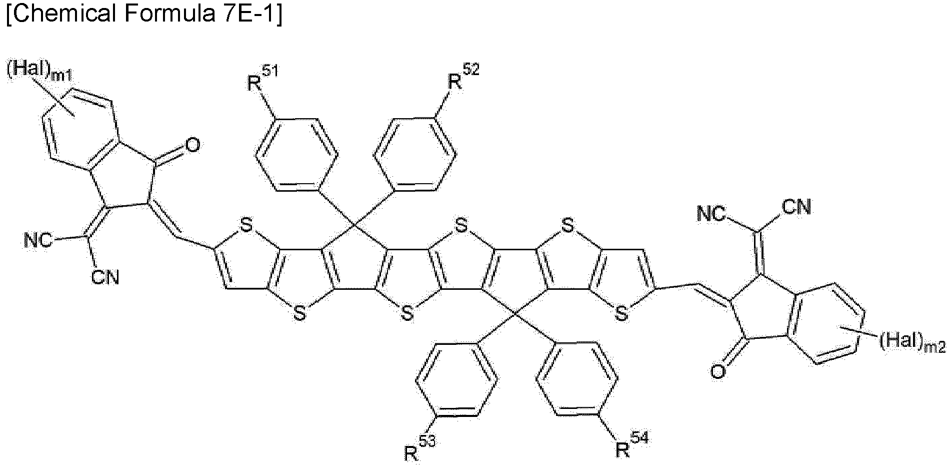

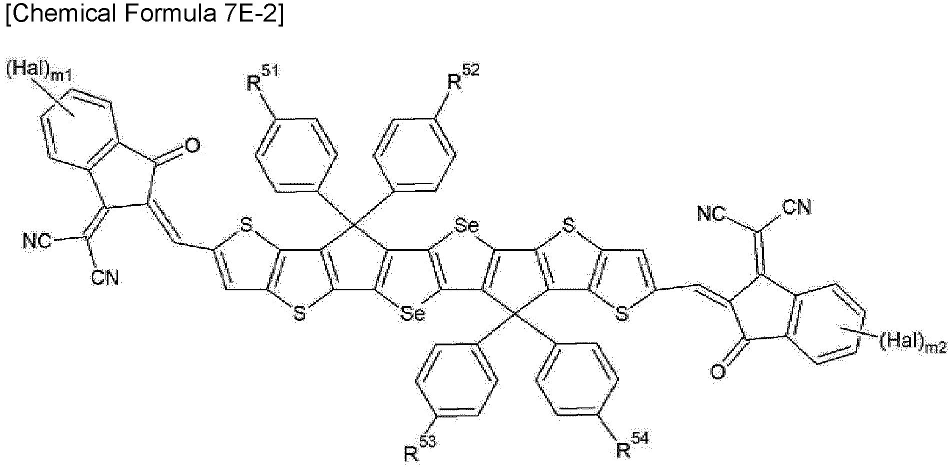

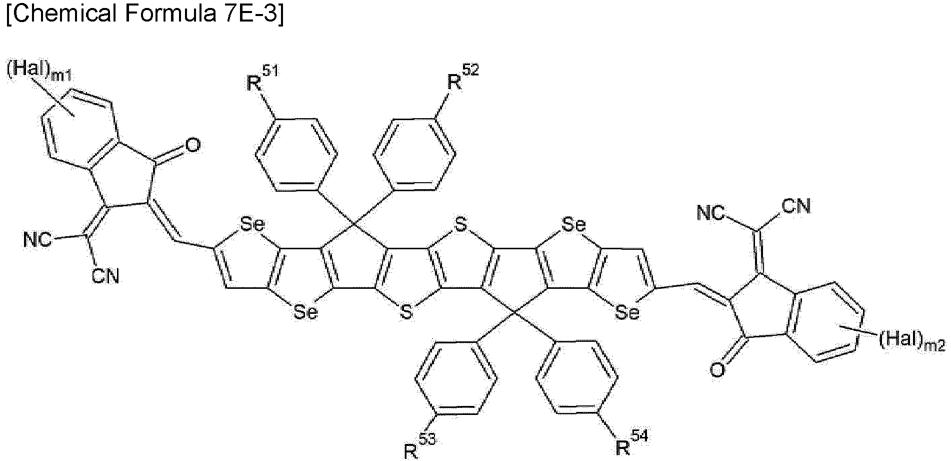

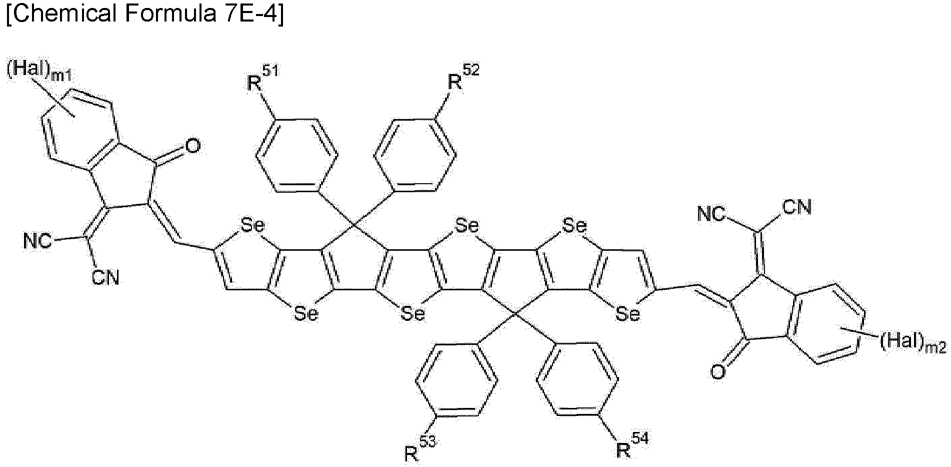

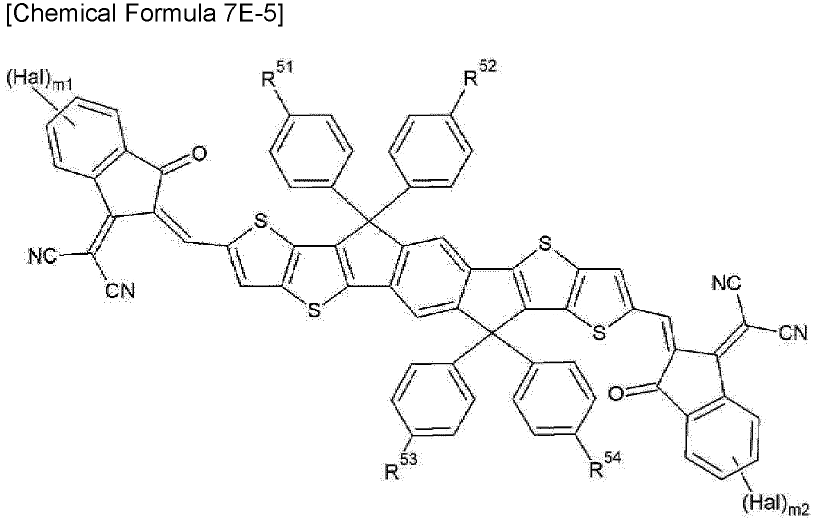

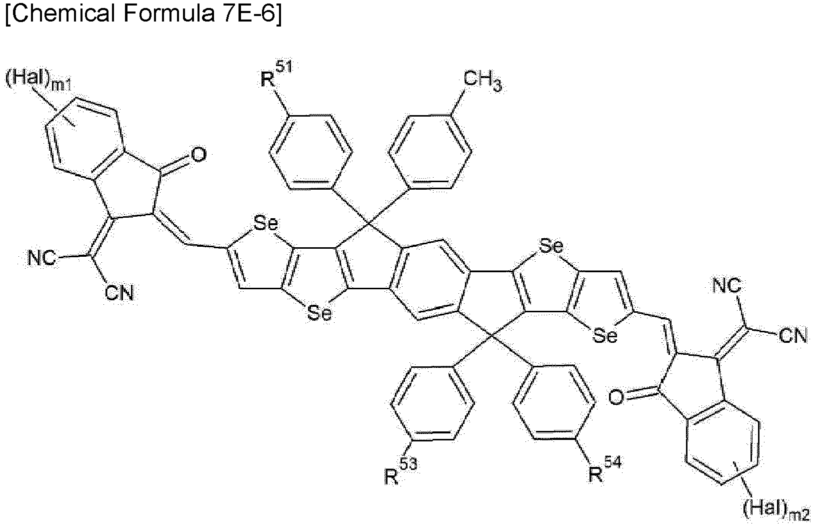

In Chemical Formulas 7E-1 to 7E-6,
R⁵¹, R⁵², R⁵³, and R⁵⁴ may each independently be hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C2 to C20 heteroaryl group,
Hal denotes a halogen (e.g., F, Cl, Br, or I), and m1 and m2 may each independently be an integer of 0 to 4 (e.g., 0 to 2).

In Chemical Formulas 7E-1 to 7E-6, the moiety represented as an example of Chemical Formula 6A as the electron accepting moiety is illustrated, but an electron accepting moiety represented by any one of Chemical Formulas 6B to 6F may be also included.

The compound of Chemical Formula 7A-3 may be a compound of Chemical Formula 7A-3a.

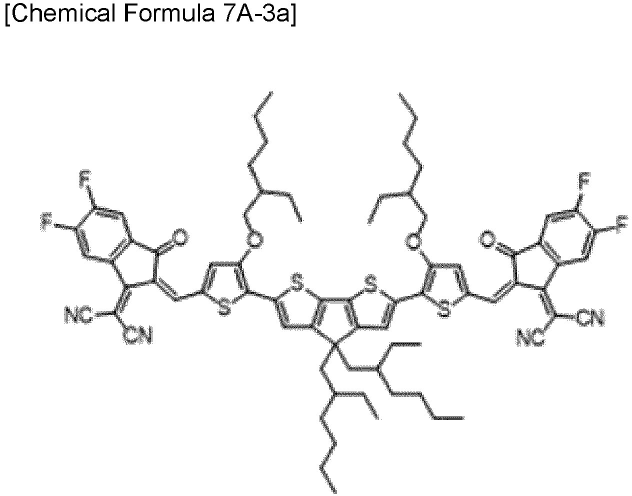

The compound of Chemical Formula 7C-1 may be a compound of Chemical Formula 7C-1a or 7C-1b.

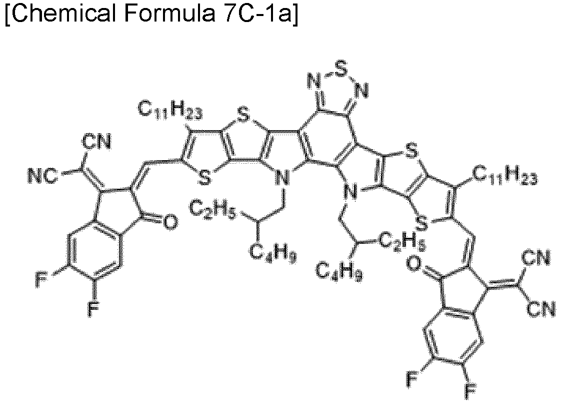

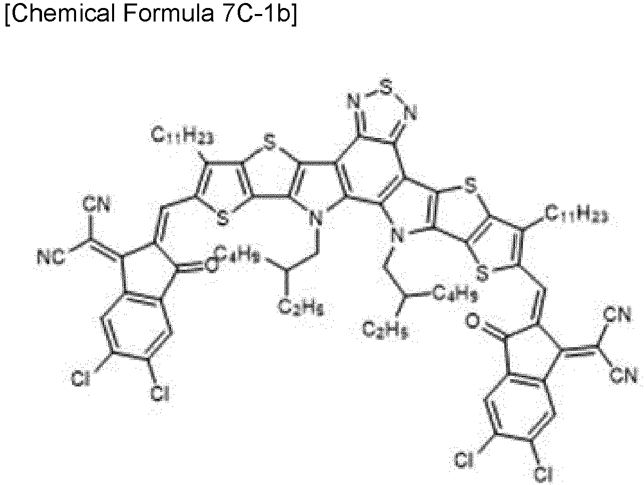

The compound of Chemical Formula 7D-1 may be a compound of Chemical Formula 7D-1a.

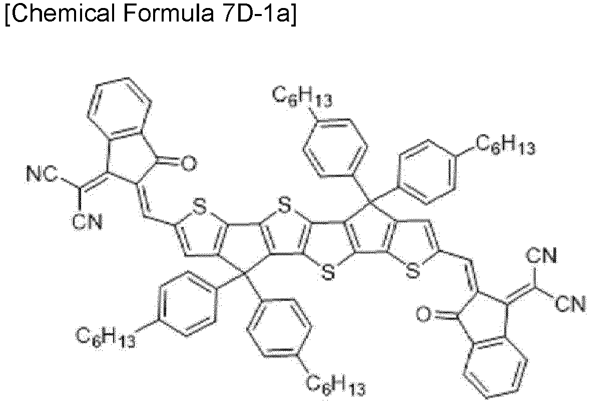

The compound of Chemical Formula 7E-1 may be a compound of Chemical Formula 7E-1a or 7E-1b.

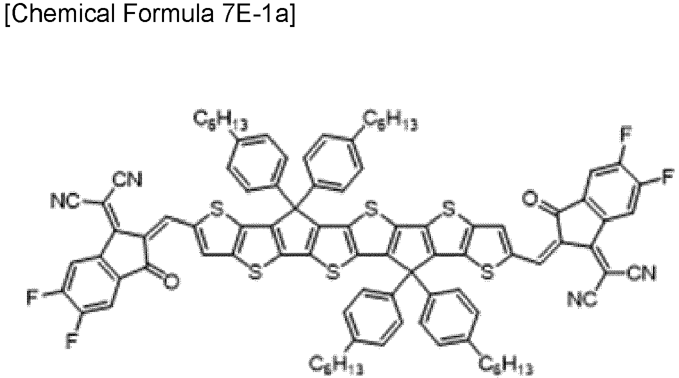

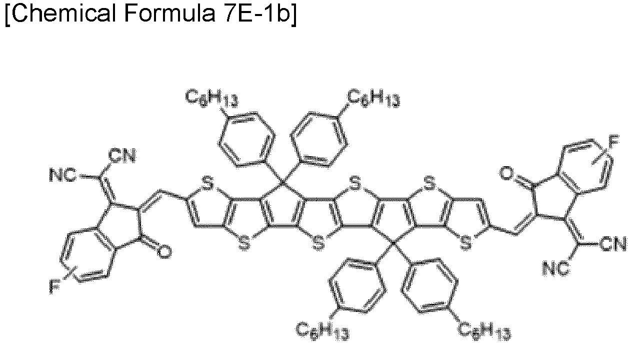

Substitution positions of F in Chemical Formula 7E-1b may be symmetric or asymmetric.

The compound of Chemical Formula 7E-5 may be a compound of Chemical Formula 7E-5a or 7E-5b.

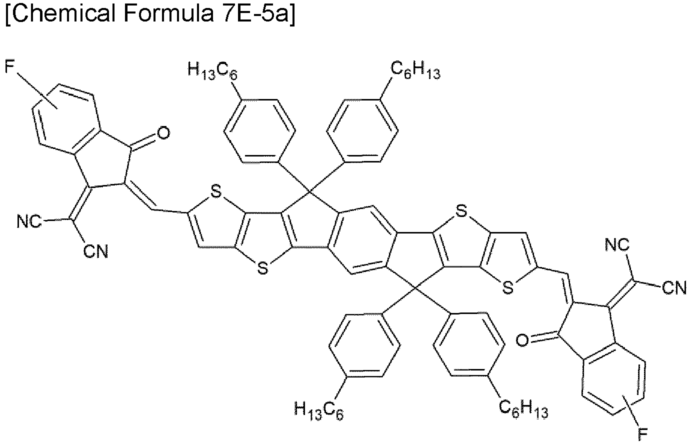

Substitution positions of F in Chemical Formula 7E-5a may be symmetrical or asymmetrical to each other.

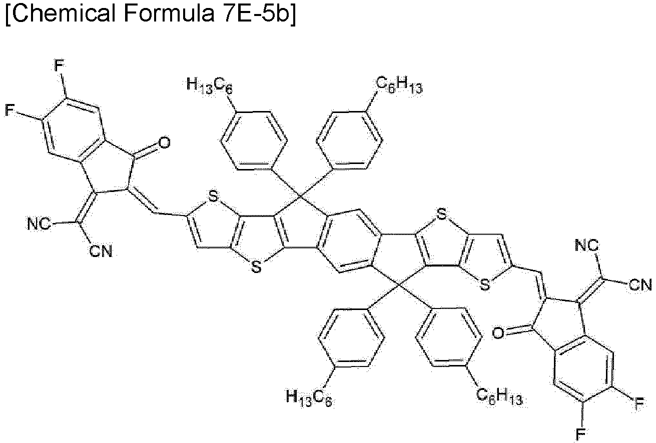

A weight ratio of the p-type semiconductor compound to the n-type semiconductor compound (p-type semiconductor compound : n-type semiconductor compound) may be in a range of about 1:0.1 to about 1:10, for example about 1:0.5 to about 1:3 (about 1:0.5 to about 1:1 or about 1:1 to about 1:3). That is, the weight ratio of the n-type semiconductor compound/p-type semiconductor compound may be greater than or equal to about 0.1, greater than or equal to about 0.2, greater than or equal to about 0.3, greater than or equal to about 0.4, greater than or equal to about 0.5, greater than or equal to about 0.6, or greater than or equal to about 0.7 and less than or equal to about 10, less than or equal to about 9, less than or equal to about 8, less than or equal to about 7, less than or equal to about 6, less than or equal to about 5, less than or equal to about 4, less than or equal to about 3, less than or equal to about 2, less than or equal to about 1.5, less than or equal to about 1.4, or less than or equal to about 1.3. Within the above range, a BHJ structure having excellent infrared absorption characteristics may be provided.

The infrared absorption composition may absorb light in an infrared wavelength region, and a peak absorption wavelength (λₘₐₓ) of the infrared absorption composition (e.g., a peak absorption wavelength that the infrared absorption composition is configured to exhibit) may be for example in a wavelength region of greater than or equal to about 1000 nm, greater than or equal to about 1010 nm, greater than or equal to about 1020 nm, greater than or equal to about 1030 nm, greater than or equal to about 1040 nm, greater than or equal to about 1050 nm, greater than or equal to about 1060 nm, greater than or equal to about 1070 nm, greater than or equal to about 1080 nm, greater than or equal to about 1090 nm, or greater than or equal to about 1100 nm. The peak absorption wavelength of the infrared absorption composition may be for example in a wavelength region of less than or equal to about 3000 nm, less than or equal to about 2900 nm, less than or equal to about 2800 nm, less than or equal to about 2700 nm, less than or equal to about 2600 nm, less than or equal to about 2500 nm, less than or equal to about 2400 nm, less than or equal to about 2300 nm, less than or equal to about 2200 nm, or less than or equal to about 2100 nm.

The infrared absorption composition has excellent photoelectric conversion efficiency for absorbing light and converting it into an electrical signal, so that it may be effectively used as a photoelectric conversion material of a photoelectric device.

According to some example embodiments, an infrared absorption film showing a face-on alignment structure in GISAXS (grazing incident small angle x-ray scattering) analysis of a film made of the aforementioned infrared absorption composition including the p-type semiconductor compound and the n-type semiconductor compound is provided. This shows an alignment structure different from that of the conventional composition including the p-type semiconductor compound and the n-type semiconductor compound exhibiting an edge-on structure.

In addition, according to some example embodiments, in atomic force microscopy analysis of a film made of the infrared absorption composition including the p-type semiconductor compound and the n-type semiconductor compound, an infrared absorption film may have a surface roughness of less than or equal to about 2 nm, for example, less than or equal to about 1.9 nm, less than or equal to about 1.8 nm, less than or equal to about 1.7 nm, less than or equal to about 1.6 nm, less than or equal to about 1.5 nm, or less than or equal to about 1.4 nm.

The p-type semiconductor compound and the n-type semiconductor compound may form a thin film (e.g., an infrared absorption film) through a solution process, so that a large-area photoelectric device may be manufactured at low cost.

The infrared absorption composition may be applied to various fields requiring absorption characteristics in an infrared wavelength region.

The infrared absorption composition has both absorption characteristics and photoelectric characteristics in the infrared wavelength region, and may significantly reduce dark current of a photoelectric device, and thus can be effectively used as a photoelectric conversion material for a photoelectric device.

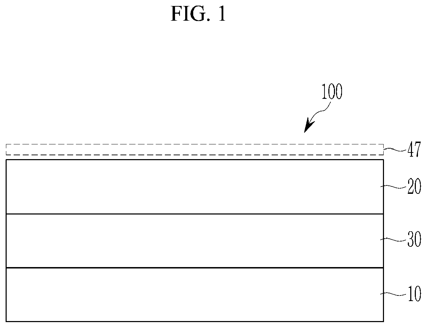

FIG. 1 is a cross-sectional view illustrating a photoelectric device according to some example embodiments.

Referring to FIG. 1, a photoelectric device 100 according to some example embodiments includes a first electrode 10 and a second electrode 20 facing each other, and a photoactive layer 30 disposed between the first electrode 10 and the second electrode 20.

A substrate (not shown) may be disposed under the first electrode 10 and on the second electrode 20. The substrate may be for example an inorganic substrate such as a glass plate or silicon wafer or an organic substrate made of an organic material such as polycarbonate, polymethylmethacrylate, polyethyleneterephthalate, polyethylenenaphthalate, polyamide, polyethersulfone, or any combination thereof. The substrate may be omitted.

One of the first electrode 10 and the second electrode 20 may be an anode and the other may be a cathode. For example, the first electrode 10 may be an anode and the second electrode 20 may be a cathode.

At least one of the first electrode 10 or the second electrode 20 may be a light-transmitting electrode and the light-transmitting electrode may be for example made of a conductive oxide such as an indium tin oxide (ITO), indium zinc oxide (IZO), zinc oxide (ZnO), tin oxide (SnO₂), aluminum tin oxide (AlTO), and/or fluorine doped tin oxide (FTO), or a metal thin layer of a single layer or a multilayer. When one of the first electrode 10 and the second electrode 20 is a non-light-transmitting electrode, it may be made of, for example, an opaque conductor such as aluminum (Al), silver (Ag), or gold (Au). For example, the first electrode 10 and the second electrode 20 may be all light-transmitting electrodes. For example, the second electrode 20 may be a light receiving electrode disposed at a light receiving side.

The photoactive layer 30 includes the aforementioned infrared absorption composition including the p-type semiconductor compound and the n-type semiconductor compound. Accordingly, it will be understood that the photoactive layer 30 may at least partially comprise the aforementioned infrared absorption composition. The photoactive layer 30, and thus the photoelectric device 100 may have improved infrared light absorption characteristics (e.g., may have improved sensitivity to light in an infrared wavelength region, improved absorbance of light in the infrared wavelength region, etc.) and thus improved photoelectric conversion performance based on the photoactive layer 30 including the aforementioned infrared absorption composition. In some example embodiments, the photoactive layer 30 may be an infrared absorption film that includes the infrared absorption composition.

The photoactive layer 30 may be a layer (intrinsic layer, I layer) including a p-type semiconductor and an n-type semiconductor configured to provide a pn junction and may produce excitons by receiving light from outside and then separating holes and electrons from the produced excitons.

The photoactive layer 30 may further include a p-type layer and/or an n-type layer in addition to the intrinsic layer. The p-type layer may include a p-type semiconductor compound, and the n-type layer may include an n-type semiconductor compound. For example, the photoactive layer 30 may include various combinations of p-type layer/l layer, I layer/n-type layer, p-type layer/l layer/n-type layer, and the like.

The photoelectric device 100 may further include an auxiliary layer disposed between the first electrode 10 and the photoactive layer 30 and/or between the second electrode 20 and the photoactive layer 30. The auxiliary layer may be a charge auxiliary layer or an optical auxiliary layer.

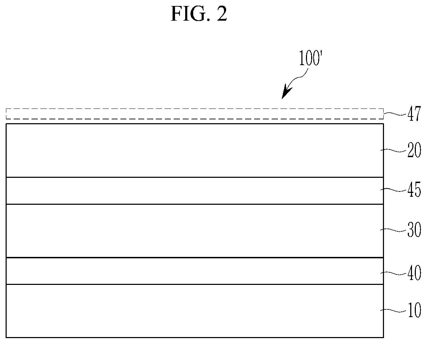

A photoelectric device having such a structure is shown in FIG. 2.

FIG. 2 is a cross-sectional view showing a photoelectric device according to some example embodiments.

Referring to FIG. 2, a photoelectric device 100' according to some example embodiments, including the example embodiments shown in FIG. 2, includes a first electrode 10 and a second electrode 20 facing each other, and a photoactive layer 30 disposed between the first electrode 10 and second electrode 20, like some example embodiments, including the example embodiments shown in FIG. 1.

However, the photoelectric device 100' according to some example embodiments, including the example embodiments shown in FIG. 2, further includes charge auxiliary layers 40 and 45 (also referred to herein as first and second charge auxiliary layers, respectively) between the first electrode 10 and the photoactive layer 30, and the second electrode 20 and the photoactive layer 30, unlike some example embodiments, including the example embodiments shown in FIG. 1. The charge auxiliary layers 40 and 45 may facilitate the transfer of holes and electrons separated from the photoactive layer 30, so as to increase efficiency of the photoelectric device 100'. In some example embodiments, only one of the first charge auxiliary layer 40 or the second charge auxiliary layer 45 is included in the photoelectric device 100'.

The charge auxiliary layers 40 and 45 may be at least one selected from a hole injection layer (HIL) for facilitating hole injection, a hole transport layer (HTL) for facilitating hole transport, an electron blocking layer (EBL) for preventing electron transport, an electron injection layer (EIL) for facilitating electron injection, an electron transport layer (ETL) for facilitating electron transport, and a hole blocking layer (HBL) for preventing hole transport.

The charge auxiliary layers 40 and 45 may include, for example, an organic material, an inorganic material, or an organic/inorganic material. The organic material may be an organic compound having hole or electron characteristics, and the inorganic material may be, for example, a metal oxide such as molybdenum oxide, tungsten oxide, nickel oxide, and the like.

The charge auxiliary layers 40 and 45 may include, for example, the aforementioned infrared absorption composition. In some example embodiments, the charge auxiliary layers 40 and/or 45 may include the aforementioned infrared absorption composition and the photoactive layer 30 may also include the aforementioned infrared absorption composition. In some example embodiments, the charge auxiliary layers 40 and/or 45 may include the infrared absorption composition and the photoactive layer 30 may not include the aforementioned infrared absorption composition. The charge auxiliary layers 40 and/or 45, and thus the photoelectric device 100', may have improved infrared light absorption characteristics and thus improved photoelectric conversion performance and/or efficiency, based on the charge auxiliary layers 40 and/or 45 including the aforementioned infrared absorption composition.

In some example embodiments, a photoelectric device 100' may include a first electrode 10 and a second electrode 20 facing each other, a photoactive layer 30 between the first electrode 10 and the second electrode 20, and one or more charge auxiliary layers 40 and/or 45, where the one or more charge auxiliary layers 40 and/or 45 may include a first charge auxiliary layer 40 that is between the photoactive layer 30 and the first electrode 10 and/or a second charge auxiliary layer 45 that is between the photoactive layer 30 and the second electrode 20, and wherein at least one of the photoactive layer 30 or the one or more charge auxiliary layers 40 and/or 45 include the aforementioned infrared absorption composition.

The optical auxiliary layer may be disposed in a light incident direction of the photoelectric device and may be for example disposed on the photoactive layer 30 when the second electrode 20 is a light receiving electrode (e.g., the electrode proximate to a surrounding environment from which light is received at the photoelectric device 100'). For example, the optical auxiliary layer may be disposed between the second electrode 20 and the photoactive layer 30.

The photoelectric devices 100 and 100' may further include an anti-reflection layer 47 on one surface of the first electrode 10 or the second electrode 20. The anti-reflection layer 47 is disposed at a light incidence side and lowers reflectance of light of incident light and thereby light absorbance is further improved. For example, when light is incident on the first electrode 10, the anti-reflection layer 47 may be disposed on one surface of the first electrode 10, and when light is incident on the second electrode 20, the anti-reflection layer 47 may be disposed on one surface of the second electrode 20.

The anti-reflection layer 47 may include, for example a material having a refractive index of about 1.6 to about 2.5 and may include for example at least one of a metal oxide, a semi-metal oxide, a metal sulfide, or an organic material having a refractive index within the above ranges. The anti-reflection layer 47 may include, for example a metal oxide or a semi-metal oxide (e.g., chalcogen oxide) such as an aluminum-containing oxide, a molybdenum-containing oxide, a tungsten-containing oxide, a vanadium-containing oxide, a rhenium-containing oxide, a niobium-containing oxide, a tantalum-containing oxide, a titanium-containing oxide, a nickel-containing oxide, a copper-containing oxide, a cobalt-containing oxide, a manganese-containing oxide, a chromium-containing oxide, a tellurium-containing oxide, or any combination thereof; a metal sulfide such as zinc sulfide; or an organic material such as an amine derivative, but is not limited thereto.

In the photoelectric devices 100 and 100', when light enters photoelectric device 100 and/or 100' and thus enters the photoactive layer 30 thereof from (e.g., via) from the first electrode 10 and/or second electrode 20 and when the photoactive layer 30 thus absorbs the light in a particular (or, alternatively, predetermined) wavelength region, excitons may be produced from the inside. The excitons are separated into holes and electrons in the photoactive layer 30, and the separated holes are transported to an anode that is one of the first electrode 10 and the second electrode 20 and the separated electrons are transported to the cathode that is the other of the first electrode 10 or the second electrode 20 so as to flow (e.g., induce, generate, etc.) a current in the photoelectric devices 100 and 100'.

The photoelectric devices 100 and 100' may have an external quantum efficiency of greater than or equal to about 10%, for example, greater than or equal to about 12%, or greater than or equal to about 15% at -3V in the infrared wavelength region. The photoelectric devices 100 and 100' may have an external quantum efficiency between about 10% and 100%, for example, between about 12% and 100%, or between about 15% and 100% at -3V in the infrared wavelength region.

The photoelectric devices 100 and 100' may have an external quantum efficiency increase of about 80% or more, for example about 90% or more, or about 100% or more at -3V in the infrared wavelength region compared to a photoelectric device including a photoactive layer including the same p-type semiconductor compound and fullerene or fullerene derivative. The fullerene derivative may be Phenyl-C61-butyric acid methyl ester (PCBM).

The photoelectric devices 100 and 100' may be applied to (e.g., included in) a sensor such as an image sensor (e.g., CMOS image sensor), a photodetector, an optical sensor (infrared light sensor), or a solar cell, but example embodiments are not limited thereto.

In some example embodiments, the photoelectric device 100 may include the infrared absorption composition in any of the elements thereof, including, in addition to or alternative to the photoactive layer 30, one or more of the first electrode 10 or the second electrode 20. In some example embodiments, the photoelectric device 100' may include the infrared absorption composition in any of the elements thereof, including, in addition to or alternative to the photoactive layer 30 and/or one or more of the charge auxiliary layers 40/45, one or more of the first electrode 10 or the second electrode 20.

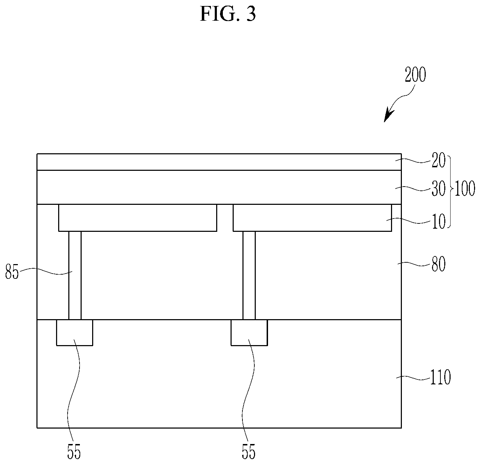

FIG. 3 is a cross-sectional view illustrating an image sensor according to some example embodiments.

The image sensor 200 according to some example embodiments includes a semiconductor substrate 110, an insulation layer 80, and a photoelectric device 100. Although the image sensor 200 including the photoelectric device 100 of FIG. 1 is illustrated in FIG. 3, the image sensor 200 may include the photoelectric device 100' of FIG. 2.

The semiconductor substrate 110 may be a silicon substrate, and a transmission transistor (not shown) and a charge storage 55 are integrated therein. The charge storage 55 may be integrated for each pixel. The charge storage 55 is electrically connected to the photoelectric device 100, and information in the charge storage 55 may be transferred by a transmission transistor.

A metal wire (not shown) and a pad (not shown) are also formed on the semiconductor substrate 110. In order to decrease signal delay, the metal wire and pad may be made of a metal having low resistivity, for example, aluminum (Al), copper (Cu), silver (Ag), and alloys thereof, but is not limited thereto. However, the structure is not limited thereto, and the metal wire and pad may be disposed under the semiconductor substrate 110.

An insulation layer 80 is formed on the metal wire and pad. The insulation layer 80 may be made of an inorganic insulating material such as a silicon oxide and/or a silicon nitride, or a low dielectric constant (low K) material such as SiC, SiCOH, SiCO, and SiOF. The insulation layer 80 has a trench 85 exposing the charge storage 55. The trench 85 may be filled with fillers.

The aforementioned photoelectric device 100 is formed on the insulation layer 80. As described above, the photoelectric device 100 includes a first electrode 10, a photoactive layer 30, and a second electrode 20. Even though a structure in which the first electrode 10, the photoactive layer 30, and the second electrode 20 are sequentially stacked is shown as an example in the drawing, the present inventive concepts is not limited to this structure, and the second electrode 20, the photoactive layer 30, and the first electrode 10 may be arranged in this order.

The first electrode 10 and the second electrode 20 may both be transparent electrodes, and the photoactive layer 30 may be the same as described above with reference to FIGS. 1 and 2. The photoactive layer 30 may selectively absorb light in an infrared wavelength region. Incident light from the side of the second electrode 20 may be photoelectrically converted by mainly absorbing light in an infrared wavelength region in the photoactive layer 30. As noted above with reference to FIG. 1, the photoactive layer 30 may include the aforementioned infrared absorption composition and thus may have improved sensitivity to infrared light, such that the operational performance and/or efficiency of the image sensor 200 in absorbing and/or converting incident infrared light into electrical signals (e.g., photoelectric conversion performance and/or efficiency) may be improved.

Focusing lens (not shown) may be further formed on the photoelectric device 100. The focusing lens may control a direction of incident light and gather the light in one region. The focusing lens may have a shape of, for example, a cylinder or a hemisphere, but is not limited thereto.

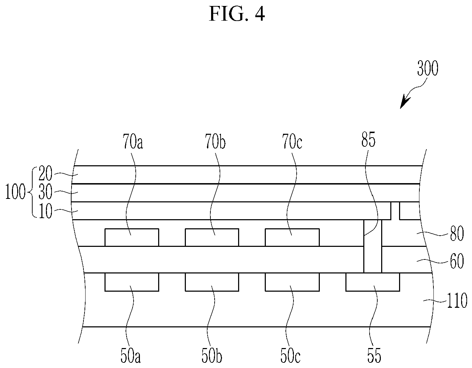

FIG. 4 is a cross-sectional view showing an image sensor according to some example embodiments.

Referring to FIG. 4, an image sensor 300 according to some example embodiments includes a semiconductor substrate 110 integrated with photo-sensing devices (e.g., photodiodes, including silicon-based photodiodes) 50a, 50b, and 50c, a transmission transistor (not shown), and a charge storage 55, a lower insulation layer 60, color filters 70a, 70b, and 70c, an upper insulation layer 80, and a photoelectric device 100.

FIG. 4 illustrates an image sensor 300 including the photoelectric device 100 of FIG. 1, but the image sensor 300 may also include the photoelectric device 100' of FIG. 2.

The semiconductor substrate 110 may be integrated with photo-sensing devices 50a, 50b, and 50c, a transmission transistor (not shown), and a charge storage 55. The photo-sensing devices 50a, 50b, and 50c may be photodiodes.

The photo-sensing devices 50a, 50b, and 50c, the transmission transistor, and/or the charge storage 55 may be integrated in each pixel. For example, the photo-sensing device 50a may be included in a red pixel, the photo-sensing device 50b may be included in a green pixel, and the photo-sensing device 50c may be included in a blue pixel.

The photo-sensing devices 50a, 50b, and 50c sense (e.g., selectively absorb and/or convert (into electrical signals, e.g., photoelectrically convert)) incident light, the information sensed by the photo-sensing devices may be transferred by the transmission transistor, the charge storage 55 is electrically connected to the photoelectric device 100, and the information of the charge storage 55 may be transferred by the transmission transistor.

A metal wire (not shown) and a pad (not shown) are formed on the semiconductor substrate 110. In order to decrease signal delay, the metal wire and pad may be made of a metal having low resistivity, for example, aluminum (Al), copper (Cu), silver (Ag), and alloys thereof, but are not limited thereto. Further, it is not limited to the structure, and the metal wire and pad may be disposed under the photo-sensing devices 50a and 50b.

The lower insulation layer 60 is formed on the metal wire and the pad. The lower insulation layer 60 may include a same or different material composition as the insulation layer 80.

Color filters 70a, 70b, and 70c are formed on the lower insulation layer 60. The color filters 70a, 70b, and 70c includes a red filter 70a formed in a red pixel, a green filter 70b formed in a green pixel, and a blue filter 70c formed in a blue pixel.

The upper insulation layer 80 is formed on the color filters 70a, 70b, and 70c. The upper insulation layer 80 eliminates step differences caused by the color filters 70a, 70b, and 70c and planarizes the surface.

The aforementioned photoelectric device 100 is formed on the upper insulation layer 80. As described above, the photoelectric device 100 includes a first electrode 10, a photoactive layer 30, and a second electrode 20. Even though a structure in which the first electrode 10, the photoactive layer 30, and the second electrode 20 are sequentially stacked is shown as an example in the drawing, the present inventive concepts is not limited to this structure, and the second electrode 20, the photoactive layer 30, and the first electrode 10 may be arranged in this order.

The first electrode 10 and the second electrode 20 may both be transparent electrodes, and the photoactive layer 30 is the same as described above. The photoactive layer 30 may selectively absorb light in a near-infrared/infrared wavelength region. As noted above with regard to photoelectric devices 100 and 100', any portion of the photoelectric device 100 (e.g., first electrode 10, second electrode 20, and/or photoactive layer 30) may include the aforementioned infrared absorption composition.

Incident light from the side of the second electrode 20 may be photoelectrically converted by mainly absorbing light in a near infra-red wavelength region in the photoactive layer 30. Light in the remaining wavelength region may pass through the first electrode 10 and the color filters 70a, 70b, and 70c, the light in a red wavelength region passing through the color filter 70a may be sensed by the photo-sensing device 50a, the light in a green wavelength region passing through the color filter 70b may be sensed by the photo-sensing device 50b, and the light in a blue wavelength region passing through the color filter 70c may be sensed by the photo-sensing device 50c.

As noted above with reference to FIG. 1, the photoactive layer 30 may include the aforementioned infrared absorption composition and thus may have improved sensitivity to infrared light, such that the operational performance and/or efficiency of the image sensor 300 in absorbing and/or converting incident infrared light into electrical signals (e.g., photoelectric conversion performance and/or efficiency) may be improved.

Accordingly, where an image sensor 300 includes a photoelectric device 100 that includes the infrared absorption composition and is configured to selectively absorb and/or convert (into electrical signals, e.g., photoelectrically convert) light in a first infrared wavelength region, the image sensor may include an additional sensor that includes a plurality of photodiodes (e.g., photo-sensing devices 50a, 50b, 50c) at least partially embedded within the semiconductor substrate and configured to selectively absorb and/or convert (into electrical signals, e.g., photoelectrically convert) light in separate visible wavelength regions that are different from the first infrared wavelength region (e.g., red, green, and/or blue light).

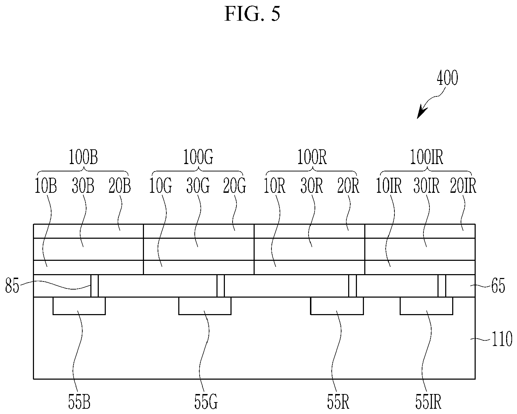

FIG. 5 is a cross-sectional view showing an image sensor according to some example embodiments.

Referring to FIG. 5, an image sensor 400 according to some example embodiments includes a semiconductor substrate 110 integrated with an infrared light charge storage 55IR, a blue light charge storage 55B, a green light charge storage 55G, a red light charge storage 55R, and a transmission transistor (not shown), a lower insulation layer 65, a blue photo-sensing device 100B, a green photo-sensing device 100G, a red photo-sensing device 100R, and an infrared photo-sensing device 100IR.

The semiconductor substrate 110 may be a silicon substrate, and the infrared light charge storage 55IR, blue light charge storage 55B, the green light charge storage 55G, the red light charge storage 55R, and the transfer transistor (not shown) are integrated therein. The blue light charge storage 55B, the green light charge storage 55G, and the red light charge storage 55R may be integrated for each blue pixel, green pixel, and red pixel.

Charges generated in the infrared photo-sensing device 100IR, the blue photo-sensing device 100B, the green photo-sensing device 100G, and the red photo-sensing device 100R are collected in the infrared light charge storage 55IR, the blue light charge storage 55B, the green light charge storage 55G, and the red light charge storage 55R, which are electrically connected to each of the infrared photo-sensing device 100IR, the blue photo-sensing device 100B, the green photo-sensing device 100G, and the red photo-sensing device 100R.

A metal wire (not shown) and a pad (not shown) are formed on the semiconductor substrate 110. In order to decrease signal delay, the metal wires and pads may be made of a metal having low resistivity, for example, aluminum (Al), copper (Cu), silver (Ag), and alloys thereof, but are not limited thereto.

The lower insulation layer 65 may be formed on the metal wire and pad. The lower insulation layer 65 may be made of an inorganic insulation material such as a silicon oxide and/or a silicon nitride, or a low dielectric constant (low K) material such as SiC, SiCOH, SiCO, and SiOF.

The blue photo-sensing device 100B, the green photo-sensing device 100G, the red photo-sensing device 100R, and the infrared photo-sensing device 100IR are formed on the lower insulation layer 65. The blue photo-sensing device 100B may include a first electrode 10B, a second electrode 20B, and a photoactive layer 30B configured to selectively absorb light in a blue wavelength region, the green photo-sensing device 100G may include a first electrode 10G, a second electrode 20G, and a photoactive layer 30G configured to selectively absorb light in a green wavelength region, the red photo-sensing device 100R may include a first electrode 10R, a second electrode 20R, and a photoactive layer 30R configured to selectively absorb light in a red wavelength region, and the infrared photo-sensing device 100IR may include a first electrode 10IR, a second electrode 20IR, and a photoactive layer 30IR configured to selectively absorb light in an infrared light wavelength region.

The first electrodes 10B, 10G, 10R, and 10IR and the second electrodes 20B, 20G, 20R, and 20IR may be light-transmitting electrodes and may be made of, for example, a transparent conductor such as indium tin oxide (ITO), indium zinc oxide (IZO), zinc oxide (ZnO), tin oxide (SnO₂), aluminum tin oxide (AlTO), and fluorine-doped tin oxide (FTO), or may be a metal thin film having a thin thickness of several nanometers to several tens of nanometers or a metal thin film having a thin thickness of several nanometers to several tens of nanometers doped with a metal oxide.

The photoactive layers 30B, 30G, 30R, and 30IR may include a p-type semiconductor material and an n-type semiconductor material. The photoactive layer 30B of the blue photo-sensing device 100B may include a p-type semiconductor material configured to selectively absorb light in a blue wavelength region and an n-type semiconductor material configured to selectively absorb light in a blue wavelength region, the photoactive layer 30G of the green photo-sensing device 100G may include a p-type semiconductor material configured to selectively absorb light in a green wavelength region and an n-type semiconductor material configured to selectively absorb light in a green wavelength region, the photoactive layer 30R of the red photo-sensing device 100R may include a p-type semiconductor material configured to selectively absorb light in a red wavelength region and an n-type semiconductor material configured to selectively absorb light in a red wavelength region, and the photoactive layer 30IR of the infrared photo-sensing device 100IR may include the aforementioned infrared absorption composition. The infrared photo-sensing device 100IR may selectively absorb light in an infrared region of greater than or equal to about 1000 nm and less than or equal to about 3000 nm without absorption of the visible light region.

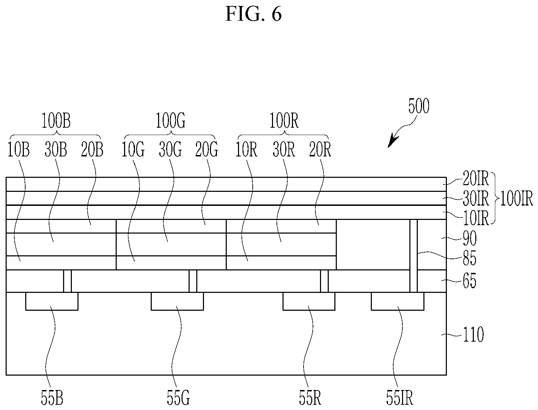

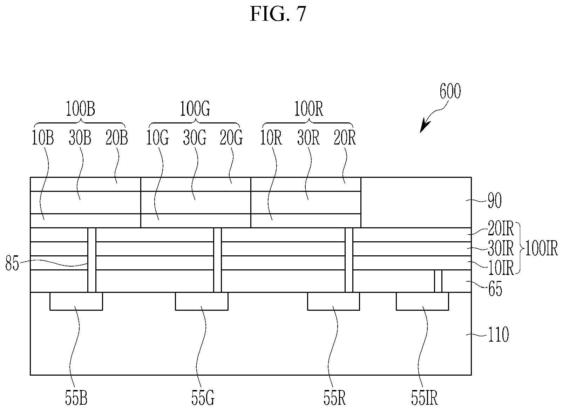

FIG. 6 is a cross-sectional view showing an image sensor according to some example embodiments. FIG. 7 is a cross-sectional view showing an image sensor according to some example embodiments.

Referring to FIG. 6, an image sensor 500 may include a semiconductor substrate 110 integrated with an infrared light charge storage 55IR, a blue light charge storage 55B, a green light charge storage 55G, a red light charge storage 55R, and a transmission transistor (not shown), a lower insulation layer 65, a blue photo-sensing device 100B, a green photo-sensing device 100G, a red photo-sensing device 100R, and an infrared photo-sensing device 100IR. The infrared photo-sensing device 100IR is formed on the whole surface of the blue photo-sensing device 100B, the green photo-sensing device 100G, and the red photo-sensing device 100R and on the upper insulating layer 90. The rest of the configuration is the same as that of the image sensor shown in FIG. 5.

In the configuration of FIG. 6, the infrared photo-sensing device 100IR may be present on the lower insulation layer 65, and the blue photo-sensing device 100B, the green photo-sensing device 100G, the red photo-sensing device 100R may be disposed thereon. An image sensor 600 having such a configuration is shown in FIG. 7. In this case, the upper insulation layer 90 may be disposed on a portion of the infrared photo-sensing device 100IR connected to the infrared light charge storage 55IR.

The infrared photo-sensing device 100IR may be configured to selectively absorb light in an infrared region of greater than or equal to about 1000 nm and less than or equal to about 3000 nm, and have a large absorption area to improve efficiency.

The sensor according to some example embodiments may include a plurality of sensors having different functions. For example, at least one of the plurality of sensors having different functions may be a biometric sensor, and the biometric sensor may be for example an iris sensor, a depth sensor, a fingerprint sensor, a blood vessel distribution sensor, and the like, but is not limited thereto.

For example, one of the plurality of sensors having different functions may be an iris sensor and the other may be a depth sensor. The iris sensor identifies a person by using unique iris characteristics of every person and specifically, taking an image of an eye of a user within an appropriate distance, processing the image, and comparing it with his/her stored image. The depth sensor identifies a shape and a location of an object from its three-dimensional information by taking an image of the object within an appropriate distance with a user and processing the image. This depth sensor may be for example used as a face recognition sensor.

In some example embodiments, a plurality of sensors may include, for example a first infrared light sensor configured to sense light in an infrared region having a first wavelength (λ₁) in an infrared wavelength region and a second infrared light sensor configured to sense light in an infrared region having a second wavelength (λ₂) in an infrared wavelength region.

The first wavelength (λ₁) and the second wavelength (λ₂) may be for example different in a wavelength region of about 1000 nm to about 3000 nm, and for example a difference between the first wavelength (λ₁) and the second wavelength (λ₂) may be greater than or equal to about 30 nm, greater than or equal to about 50 nm, greater than or equal to about 70 nm, greater than or equal to about 80 nm, or greater than or equal to about 90 nm.

For example, one of the first wavelength (λ₁) or the second wavelength (λ₂) may belong to a wavelength region of about 1000 nm to 1500 nm and the other of the first wavelength (λ₁) or the second wavelength (λ₂) may belong to a wavelength region of greater than about 1500 nm and less than or equal to about 2000 nm.

For example, one of the first wavelength (λ₁) or the second wavelength (λ₂) may belong to a wavelength region of about 1000 nm to about 1500 nm and the other of the first wavelength (λ₁) or the second wavelength (λ₂) may belong to a wavelength region of greater than or equal to about 2000 nm and less than or equal to about 3000 nm.

For example, one of the first wavelength (λ₁) or the second wavelength (λ₂) may be about 1000 nm and the other of the first wavelength (λ₁) or the second wavelength (λ₂) may be about 1200 nm.

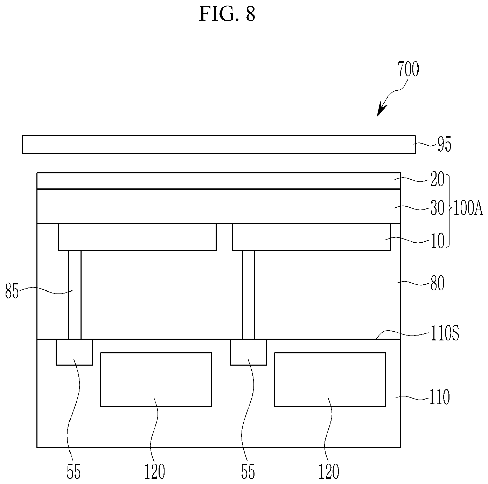

FIG. 8 is a cross-sectional view illustrating an image sensor including a plurality of sensors according to some example embodiments.

The image sensor 700 according to some example embodiments includes a dual bandpass filter 95, a first infrared light sensor 100A, an insulation layer 80 (also referred to herein as an upper insulation layer), and a semiconductor substrate 110 integrated with a second infrared light sensor 120, such that the second infrared light sensor 120 is at least partially embedded within the semiconductor substrate 110. The first infrared light sensor 100A and the second infrared light sensor 120 are stacked, e.g., may overlap in a vertical direction that is perpendicular to the upper surface 110S of the semiconductor substrate 110.

The dual bandpass filter 95 may be disposed on a front side of the first infrared light sensor 100A and may selectively transmit infrared light including the first wavelength (λ₁) and infrared light including the second wavelength (λ₂) and may block and/or absorb other light. Herein, other light may include light in an ultraviolet (UV) and visible region.

The first infrared light sensor 100A includes a first electrode 10, a photoactive layer 30, and a second electrode 20. The first infrared light sensor 100A may be the same as the photoelectric device 100 according to some example embodiments, including the example embodiments described with reference to FIG. 1, but it will be understood that, in some example embodiments, the first infrared light sensor 100A may be the same as the photoelectric device 100' according to some example embodiments, including the example embodiments described with reference to FIG. 2.

The second infrared light sensor 120 may be integrated in the semiconductor substrate 110 (e.g., encompassed within a volume space defined by outer surfaces of the semiconductor substrate 110) and may be a photo-sensing device. The semiconductor substrate 110 may be for example a silicon substrate and may be integrated with the second infrared light sensor 120, the charge storage 55, and a transmission transistor (not shown).

The second infrared light sensor 120 may be a photodiode and may sense entered light, and sensed information is transferred by the transmission transistor. Herein, the light entered into the second infrared light sensor 120 is light that passes the dual bandpass filter 95 and the first infrared light sensor 100A and may be infrared light in a particular (or, alternatively, predetermined) region including the second wavelength (λ₂). All infrared light in a particular (or, alternatively, predetermined) region including the first wavelength (λ₁) may be absorbed by the photoactive layer 30 and may not reach the second infrared light sensor 120. In this case, a separate filter for wavelength selectivity with respect to the light entered into the second infrared light sensor 120 is not separately needed. However, for the time when all infrared light in a particular (or, alternatively, predetermined) region including the first wavelength (λ₁) is not absorbed by the photoactive layer 30, a filter between the first infrared light sensor 100A and the second infrared light sensor 120 may be further disposed.

Accordingly, in the image sensor 700, the first infrared light sensor 100A may be understood to include a photoelectric device (e.g., photoelectric device 100 and/or 200) configured to sense (e.g., selectively absorb and/or convert (into electrical signals, e.g., photoelectrically convert)) light in a first infrared wavelength region of incident light (e.g., a first infrared wavelength region including a first wavelength (λ₁)), and the second infrared light sensor 120 may be understood to be an additional sensor configured to selectively absorb and/or convert (into electrical signals, e.g., photoelectrically convert) light in a separate wavelength region of incident light (e.g., a second infrared wavelength region that is different from the first infrared wavelength region and includes a second wavelength (λ₂) and excludes the first wavelength (λ₁)).

As noted above with reference to FIG. 1, the photoactive layer 30, or any portion of the photoelectric device 100 and/or 100', may include the aforementioned infrared absorption composition and thus may have improved sensitivity to and/or absorbance of infrared light, such that the operational performance and/or efficiency of the image sensor 700 in absorbing and/or photoelectrically converting incident infrared light into electrical signals (e.g., photoelectric conversion performance and/or efficiency) may be improved. In some example embodiments, the second infrared light sensor 120 may include the aforementioned infrared absorption composition and thus may have improved sensitivity to and/or absorbance of infrared light, such that the operational performance and/or efficiency of the image sensor 700 in absorbing and/or converting incident infrared light into electrical signals (e.g., photoelectric conversion performance and/or efficiency) may be improved.

The sensor according to some example embodiments may include two infrared light sensors respectively performing separately functions and thus may work as a combination sensor. In addition, two sensors performing separately functions are stacked in each pixel, and thus the number of pixel performing functioning of each sensor is twice increased while maintaining a size and resultantly, sensitivity may be much improved.

The aforementioned image sensors may be applied to various electronic devices, for example and the electronic devices may include for example a camera, a camcorder, a mobile phone internally having them, a display device, a security device, or a medical device, but are not limited thereto.

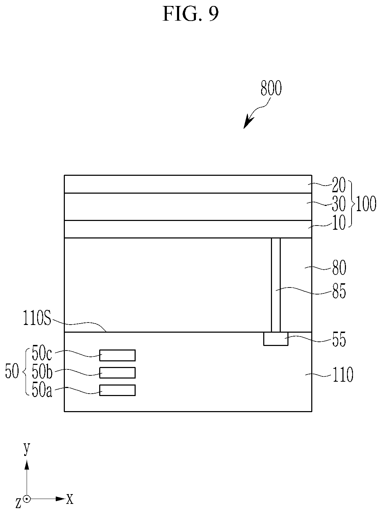

FIG. 9 is a cross-sectional view showing an image sensor according to some example embodiments.

Referring to FIG. 9, the image sensor 800 according to some example embodiments includes the visible light sensor 50, and the photoelectric device 100 like that of some example embodiments. As shown in FIG. 9, the visible light sensor 50 includes a red photo-sensing device 50a, a green photo-sensing device 50b, and a blue photo-sensing device 50c integrated in (e.g., at least partially embedded within) the semiconductor substrate 110, wherein the red photo-sensing device 50a, the green photo-sensing device 50b, and the blue photo-sensing device 50c may be photodiodes and may be configured to selectively absorb light in separate visible wavelength regions.

In the image sensor 800 according to some example embodiments, the red photo-sensing device 50a, the green photo-sensing device 50b, and the blue photo-sensing device 50c integrated in the semiconductor substrate 110 are stacked (e.g., overlap with each other) in a vertical direction (e.g., the Y direction, extending perpendicular to the upper surface 110S of the semiconductor substrate 110) and overlap with the photoelectric device 100 in the vertical direction. The red photo-sensing device 50a, the green photo-sensing device 50b, and the blue photo-sensing device 50c may be configured to selectively absorb and/or convert (into electrical signals, e.g., photoelectrically convert) light in each wavelength region depending on a stacking depth from the upper surface 110S and thus sense it. In other words, the red photo-sensing device 50a configured to selectively absorb and/or convert (into electrical signals, e.g., photoelectrically convert) red light in a long wavelength region is disposed deeper from the upper surface 110S of the semiconductor substrate 110 than the blue photo-sensing device 50c configured to selectively absorb and/or convert (into electrical signals, e.g., photoelectrically convert) blue light in a short wavelength region, and the green photo-sensing device 50b configured to selectively absorb and/or convert (into electrical signals, e.g., photoelectrically convert) green light in a medium wavelength region is disposed deeper from the upper surface 110S of the semiconductor substrate 110 than the blue photo-sensing device 50c and closer to the upper surface 110S of the semiconductor substrate 110 than the red photo-sensing device 50a. In this way, the color filters 70a, 70b, and 70c may be omitted by separating absorption wavelengths depending on the stacking depth.

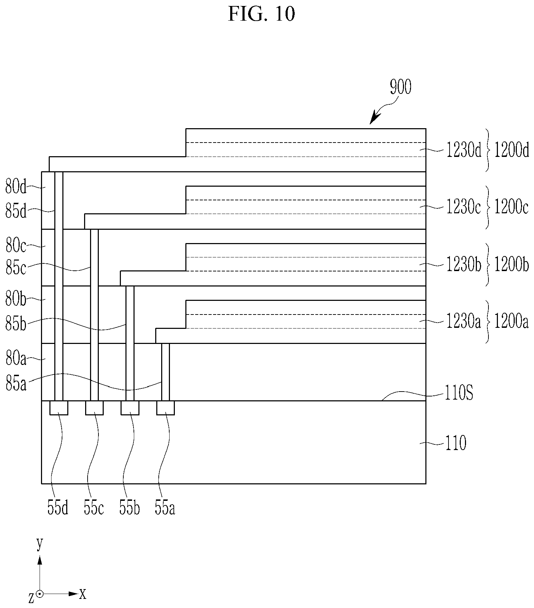

FIG. 10 is a cross-sectional view showing an image sensor according to some example embodiments.

Referring to FIG. 10, the image sensor 900 according to some example embodiments includes a first photoelectric device (e.g., infrared/near infrared photoelectric device 1200d) configured to selectively absorb and/or convert (into electrical signals, e.g., photoelectrically convert) light in an infrared/near infrared wavelength spectrum of incident light (e.g., a first infrared wavelength region), and at least one additional photoelectric device (e.g., 1200a to 1200c) vertically stacked (e.g., in the vertical direction extending perpendicular to the upper surface 110S of the semiconductor substrate 110) between the first photoelectric device and a semiconductor substrate (e.g., 110), each separate photoelectric device of the at least one additional photoelectric device (e.g., 1200a to 1200c) including a separate photoelectric conversion layer and configured to selectively absorb and/or convert (into electrical signals, e.g., photoelectrically convert) a separate (e.g., respective) wavelength region of incident light that is different from the first infrared wavelength region and which may be a separate visible and/or non-visible wavelength region. For example, as shown in FIG. 10, the image sensor 900 may include additional photoelectric devices 1200a to 1200c that include a red photoelectric device 1200a configured to selectively absorb and/or convert (into electrical signals, e.g., photoelectrically convert) light in a red wavelength spectrum of incident light, a green photoelectric device 1200b configured to selectively absorb and/or convert (into electrical signals) light in a green wavelength spectrum of incident light, and a blue photoelectric device 1200c configured to selectively absorb and/or convert (into electrical signals) light in a blue wavelength spectrum of incident light, and they are stacked in the vertical direction that extends perpendicular to the upper surface 110S of the semiconductor substrate 110 (e.g., y direction).

Accordingly, it will be understood that, as shown in FIG. 10, the image sensor 900 may include a plurality of photoelectric devices 1200a to 1200d that are stacked vertically on the semiconductor substrate 110, such that the plurality of photoelectric devices 1200a to 1200d overlap each other in a direction extending perpendicular to an upper surface 110S of the semiconductor substrate 110. While the image sensor 900 includes multiple additional photoelectric devices 1200a to 1200c in addition to the first photoelectric device (e.g., fourth photoelectric device 1200d) configured to selectively absorb and/or convert light in the first near-infrared wavelength region, it will be understood that in some example embodiments the image sensor 900 may be limited to a single additional photoelectric device (e.g., any of 1200a to 1200c) between the photoelectric device 1200d and the semiconductor substrate 110.

The image sensor 900 according to some example embodiments includes a semiconductor substrate 110, a lower insulation layer 80a, an intermediate insulation layer 80b, another intermediate insulation layer 80c, an upper insulation layer 80d, a first photoelectric device 1200a, a second photoelectric device 1200b, a third photoelectric device 1200c, and a fourth photoelectric device 1200d. Each given photoelectric device of the first to fourth photoelectric devices 1200a to 1200d may include first and second electrodes and a photoactive layer (e.g., 1230a to 1230d, respectively) between the respective first and second electrodes of the given photoelectric device. Each given photoelectric device of the first to fourth photoelectric devices 1200a to 1200d may have a same structure and/or material composition as any of the photoelectric devices of FIGS. 1-9 according to any of the example embodiments.

In some example embodiments, the fourth photoelectric device 1200d may be referred to as a first photoelectric device configured to selectively absorb and/or convert (into electrical signals, e.g., photoelectrically convert) light in a first near-infrared wavelength region, and the first to third photoelectric devices 1200a to 1200c may be collectively referred to as at least one additional photoelectric device configured to selectively absorb and/or convert (into electrical signals, e.g., photoelectrically convert) light in one or more separate wavelength regions different from the first near-infrared wavelength region. As shown, the first to fourth photoelectric devices 1200a to 1200d are stacked vertically on the semiconductor substrate 110, such that the first to fourth photoelectric devices 1200a to 1200d overlap each other in a direction extending perpendicular to an upper surface 110S of the semiconductor substrate 110.

The semiconductor substrate 110 may be a silicon substrate, and is integrated with the transmission transistor (not shown) and charge storages.

The first through third photoelectric devices 1200a to 1200c may have a same structure as any of the photoelectric devices according to any of the example embodiments herein, including without limitation the photo-sensing devices 100B, 100G, and 100R shown in any of FIGS. 5 to 7, except each separate photoelectric device 1200a to 1200c may be configured to photoelectrically convert a separate wavelength region of visible and/or non-visible (e.g., near-infrared) light, and the respective photoelectric conversion layers 1230a to 1230c of the first to third photoelectric devices 1200a to 1200c may have a same structure as any of the photoelectric devices according to any of the example embodiments herein, including without limitation the photoelectric device 100 of FIGS. 1 and 3-4, the photoelectric device 100' of FIG. 2, the photo-sensing devices 100B, 100G, 100R, and/or 100IR shown in any of FIGS. 5-7 and/or the first infrared light sensor 100A shown in FIG. 8. The photoelectric conversion layer 1230d may have a same structure and/or composition as the photoactive layer according to any of the example embodiments as described herein, including the photoactive layer 30, 30B, 30G, 30R, and/or 30IR as described herein so as to be configured to selectively absorb and/or convert (into electrical signals, e.g., photoelectrically convert) different visible and/or non-visible wavelength regions of light, and may include the infrared absorption composition.

The fourth photoelectric device 1200d may have a same structure as any of the photoelectric devices according to any of the example embodiments herein, including without limitation the photoelectric device 100 of FIGS. 1 and 3-4, the photoelectric device 100' of FIG. 2, the photo-sensing devices 100B, 100G, 100R, and/or 100IR shown in any of FIGS. 5-7 and/or the first infrared light sensor 100A shown in FIG. 8. The photoelectric conversion layer 1230d may have a same structure and/or composition as the photoactive layer according to any of the example embodiments as described herein, including the photoactive layer 30, 30B, 30G, 30R, and/or 30IR as described herein so as to be configured to selectively absorb and/or convert (into electrical signals, e.g., photoelectrically convert) different visible and/or non-visible wavelength regions of light, and may include the infrared absorption composition.

The first photoelectric device 1200a is formed on the lower insulation layer 80a. The first photoelectric device 1200a includes a photoelectric conversion layer 1230a. The first photoelectric device 1200a may be any one of the photoelectric devices described herein according to any of the example embodiments. The photoelectric conversion layer 1230a may selectively absorb and/or convert (into electrical signals, e.g., photoelectrically convert) light in one of infrared, red, blue, and green wavelength spectra of incident light. For example, the first photoelectric device 1200a may be a blue photoelectric device.

An intermediate insulation layer 80b is formed on the first photoelectric device 1200a.

The second photoelectric device 1200b is formed on the intermediate insulation layer 80b. The second photoelectric 1200b includes a photoelectric conversion layer 1230b. The second photoelectric device 1200b may be any one of the photoelectric devices described herein according to any of the example embodiments. The photoelectric conversion layer 1230b may selectively absorb and/or convert (into electrical signals, e.g., photoelectrically convert) light in one of infrared, red, blue, or green wavelength spectra of incident light. For example, the second photoelectric device 1200b may be a green photoelectric device.

Another intermediate insulation layer 80c is formed on the second photoelectric device 1200b.

The third photoelectric device 1200c is formed on the intermediate insulation layer 80c. The third photoelectric device 1200c includes a photoelectric conversion layer 1230c. The third photoelectric device 1200c any one of the photoelectric devices described herein according to any of the example embodiments. The photoelectric conversion layer 1230c may selectively absorb and/or convert (into electrical signals, e.g., photoelectrically convert) light in one of infrared, red, blue, or green wavelength spectra of incident light. For example, the third photoelectric device 1200c may be a red photoelectric device.

The upper insulation layer 80d is formed on the third photoelectric device 1200c.

The lower insulation layer 80a, the intermediate insulation layers 80b and 80c, and the upper insulation layer 80d have a plurality of through holes, or trenches 85a, 85b, 85c, and 85d exposing the charge storages 55a, 55b, 55c, and 55d, respectively, and said trenches may be partly or completely filled with a filler material (e.g., fillers).

The fourth photoelectric device 1200d is formed on the upper insulation layer 80d. The fourth photoelectric device 1200d includes a photoelectric conversion layer 1230d. The fourth photoelectric device 1200d may be any one of the photoelectric devices described herein according to any of the example embodiments. The photoelectric conversion layer 1230d may selectively absorb and/or convert (into electrical signals, e.g., photoelectrically convert) light in one of infrared, red, blue, or green wavelength spectra of light. For example, the fourth photoelectric device 1200d may be an infrared/near infrared photoelectric device that may include the infrared absorption composition.

In the drawing, the first photoelectric device 1200a, the second photoelectric device 1200b, the third photoelectric device 1200c, and the fourth photoelectric device 1200d are sequentially stacked, but the present disclosure is not limited thereto, and they may be stacked in various orders.

As described above, the first photoelectric device 1200a, the second photoelectric device 1200b, the third photoelectric device 1200c, and the fourth photoelectric device 1200d have a stack structure, and thus the size of an image sensor may be reduced to realize a down-sized image sensor.

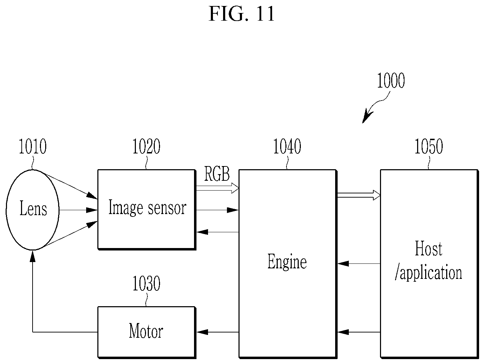

FIG. 11 is a block diagram of a digital camera including an image sensor according to some example embodiments.

Referring to FIG. 11, a digital camera 1000 includes a lens 1010, an image sensor 1020, a motor 1030, and an engine 1040. The image sensor 1020 may be one of image sensors according to any of the example embodiments, including the example embodiments shown in FIGS. 3 to 10.

The lens 1010 concentrates incident light on the image sensor 1020. The image sensor 1020 generates RGB data for received light through the lens 1010.

In some example embodiments, the image sensor 1020 may interface with the engine 1040.

The motor 1030 may adjust the focus of the lens 1010 or perform shuttering in response to a control signal received from the engine 1040. The engine 1040 may control the image sensor 1020 and the motor 1030.

The engine 1040 may be connected to a host/application 1050.

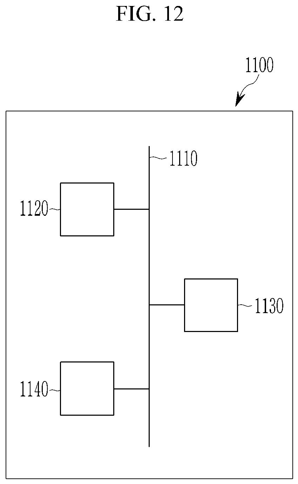

FIG. 12 is a block diagram of an electronic device according to some example embodiments.

Referring to FIG. 12, an electronic device 1100 may include a processor 1120, a memory 1130, and an image sensor 1140 that are electrically coupled together via a bus 1110. The image sensor 1140 may be an image sensor, photoelectric device, camera, or the like according to any of the example embodiments, including the example embodiments shown in FIGS. 3 to 11. The memory 1130 may be a non-transitory computer readable medium and may store a program of instructions. The memory 1130 may be a nonvolatile memory, such as a flash memory, a phase-change random access memory (PRAM), a magnetoresistive RAM (MRAM), a resistive RAM (ReRAM), or a ferro-electric RAM (FRAM), or a volatile memory, such as a static RAM (SRAM), a dynamic RAM (DRAM), or a synchronous DRAM (SDRAM). The processor 1120 may execute the stored program of instructions to perform one or more functions. For example, the processor 1120 may be configured to process electrical signals generated by the image sensor 1140. The processor 1120 may include processing circuitry such as hardware including logic circuits; a hardware/software combination such as a processor executing software; or any combination thereof. For example, the processing circuitry more specifically may include, but is not limited to, a central processing unit (CPU), an arithmetic logic unit (ALU), a digital signal processor, a microcomputer, a field programmable gate array (FPGA), a System-on-Chip (SoC), a programmable logic unit, a microprocessor, application-specific integrated circuit (ASIC), etc. The processor 1120 may be configured to generate an output (e.g., an image to be displayed on a display interface) based on such processing.

One or more of the processor 1120, memory 1130, motor 1030, engine 1040, or host/application 1050 may be included in, include, and/or implement one or more instances of processing circuitry such as hardware including logic circuits, a hardware/software combination such as a processor executing software; or any combination thereof.

In some example embodiments, one or more instances of processing circuitry may include, but are not limited to, a central processing unit (CPU), an application processor (AP), an arithmetic logic unit (ALU), a graphic processing unit (GPU), a digital signal processor, a microcomputer, a field programmable gate array (FPGA), a System-on-Chip (SoC) a programmable logic unit, a microprocessor, or an application-specific integrated circuit (ASIC), etc. In some example embodiments, any of the memories, memory units, or the like as described herein may include a non-transitory computer readable storage device, for example a solid state drive (SSD), storing a program of instructions, and the one or more instances of processing circuitry may be configured to execute the program of instructions to implement the functionality of some or all of any of the processor 1120, memory 1130, motor 1030, engine 1040, or host/application 1050, or the like according to any of the example embodiments as described herein.

Hereinafter, some example embodiments are illustrated in more detail with reference to examples. However, the inventive concepts are not limited to these examples.

### Synthesis Examples

### Synthesis Example 1-1: Synthesis of Polymer (Polymer 1) Including Structural Unit Represented by Chemical Formula 1-1

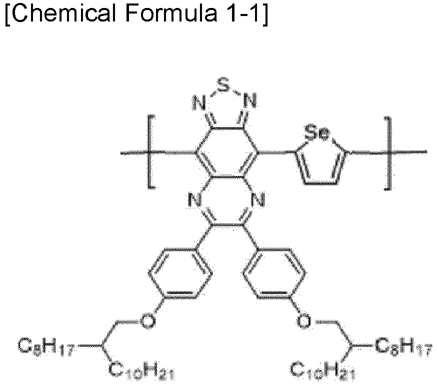

### (1) Synthesis Example 1-1a (Synthesis of Monomer A)

As shown in Reaction Scheme 1-1a, referring to a method described in J. Am. Chem. Soc. 2014, 136, 11901193, Compounds 2 to 6 are synthesized, and Compound 6 is used to synthesize an acceptor structure, Monomer A (4,9-dibromo-6,7-bis(4-((2-octyldodecyl)oxy)phenyl)-[1,2,5]thiadiazolo[3,4-g]quinoxaline).

¹H-NMR (300 MHz, CDCl₃): 7.78 ppm (d, 4H), 6.93 ppm (d, 4H), 3.90 ppm (d, 4H), 1.81 (m, 2H), 1.56-1.24 (m, 64H), 0.90-0.86 (m, 12H).

UPLC-MS: [M+H]⁺ 1089.62

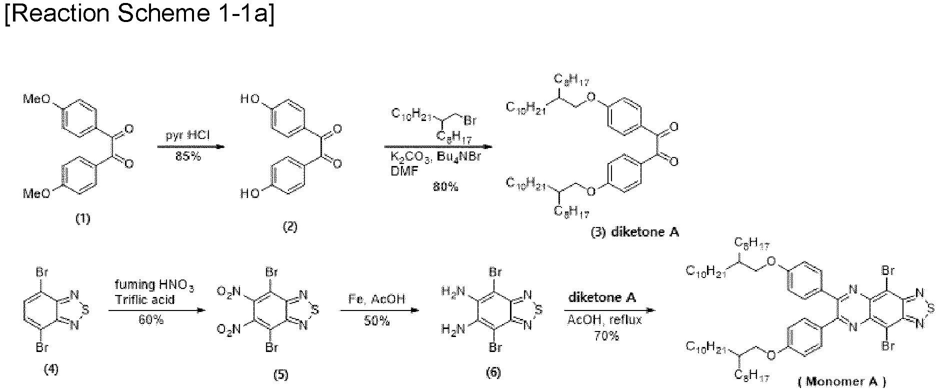

### (2) Synthesis Example 1-1b (Synthesis of Monomer B)

As shown in Reaction Scheme 1-1b, selenophene (5 g, 38 mmol) and anhydrous THF solvent (80 mL) are put in a reaction flask under a nitrogen atmosphere and cooled to -78 °C. Subsequently, an n-butyllithium solution (1.6 M in hexane) (59.6 mL, 95 mmol) is slowly added thereto. The obtained mixture is stirred at -78 °C for 2 hours and then, heated up to room temperature and additionally stirred for 2 hours. The prepared suspension is cooled again to -78 °C, and a trimethyltin chloride solution (1M in THF) (80.4 mL, 80 mmol) is added thereto. The reaction mixture is stirred at -78 °C for 4 hours, heated to room temperature, and additionally stirred at the room temperature for 16 hours. Subsequently, water is added thereto to complete a reaction, and a solution is extracted therefrom with ethylacetate and then, dried on sodium sulfate. After removing a solvent therefrom, the residue is purified through recrystallization with ethanol and dried under a reduced pressure, obtaining 6.55 g (Yield: 38%) of Monomer B (2,5-bis(trimethylstannyl)selenophene).

¹H-NMR (300 MHz, CDCl3): 7.68 ppm (s, 2H), -0.37 ppm (s, 18H). ¹³C-NMR (300 MHz, CDCl3): 150.8 ppm, 139.3 ppm, -7.0 ppm.

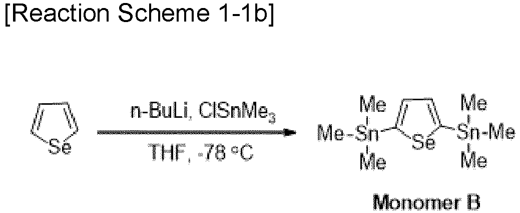

### (3) Synthesis of Polymer (Polymer 1) Including Structural Unit Represented by Chemical Formula 1-1

As shown in Reaction Scheme 1-1, Monomer A (460.2 mg, 0.422 mmol), Monomer B (192.6 mg, 0.422 mmol), Pd₂(dba)₃ (3.9 mg, 4.2 µmol), and tri(o-tolyl)phosphine (10.3 mg, 34.0 µmol) are put in a reaction flask, and the inside of the reaction vessel is sufficiently substituted with nitrogen gas. Subsequently, anhydrous chlorobenzene (12.0 mL) degassed by bubbling with nitrogen gas is added to this reaction vessel, and after dissolving the reactant, the reaction vessel is additionally substituted for 30 minutes with the nitrogen gas. The mixture is stirred for 72 hours, while heated at 130 °C, and then, poured into methanol, forming precipitates. The precipitates are filtered under a reduced pressure and then, purified by Soxhlet with methanol (12 hours), acetone (12 hours), hexane (12 hours), and chloroform (6 hours). Subsequently, a chloroform solution therefrom is concentrated with a rotary evaporator and reprecipitated in methanol, obtaining a polymer (Polymer 1, Number average molecular weight (Mn) = 4,024 g/mol, PDI = 1.66) including 364 mg (Yield: 81%) of a structural unit represented by Chemical Formula 1-1.

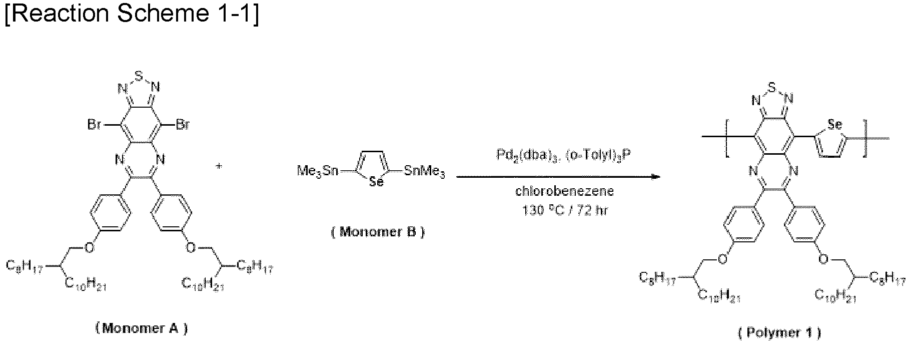

### Synthesis Example 1-2: Synthesis of Polymer (Polymer 2) Including Structural Unit Represented by Chemical Formula 1-2

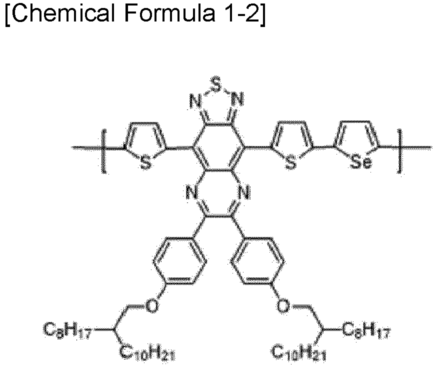

### (1) Synthesis Example 1-2a

As shown in Reaction Scheme 1-2a, Monomer A (2.06 g, 1.9 mmol) of Synthesis Example 1-1a, 2-(tributhylstannyl) thiophene (1.76 g, 4.7 mmol), and Pd(PPh₃)₄ (24 mg, 20.8 µmol) dissolved in anhydrous toluene (30 mL) are put in a reaction flask. Subsequently, the mixture is heated at 110 °C and stirred under reflux for 12 hours. The reactant is cooled to room temperature, and the reaction solvent is distilled under a reduced pressure with a rotatory evaporator. The resulting mixture is purified through column chromatography (Developing solvent: dichloromethane/hexane, Filler: silica (SiO₂) gel), obtaining an intermediate (Yield: 1.80 g, 94%).

UPLC-MS: [M+H]⁺ 1097.83

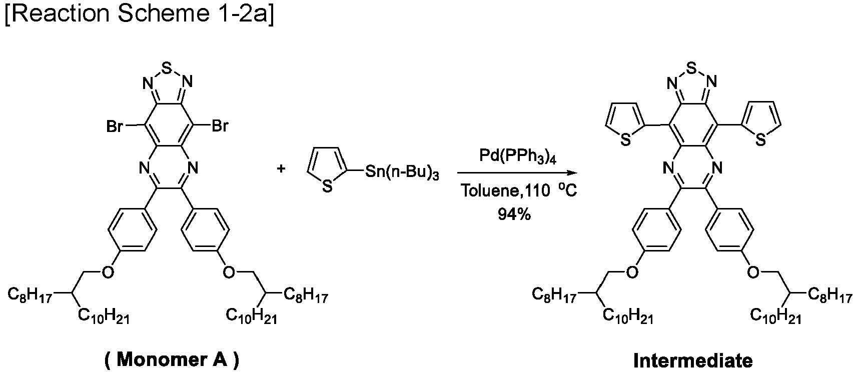

### (2) Synthesis Example 1-2b: Synthesis of Monomer C

The obtained intermediate (1.48 g, 1.35 mmol) is dissolved in dichloromethane (32 mL), and then, N-bromosuccinimide) (0.53 g, 2.98 mmol) is added thereto all at once under an ice bath. After reacting the mixture at room temperature for 12 hours, distilled water (30 mL) is added thereto, completing a reaction. The dichloromethane solution is washed with salt water and dried with anhydrous magnesium sulfate, and the reaction solvent is distilled under a reduced pressure with a rotatory evaporator. The resulting mixture is purified through column chromatography (Developing solvent: dichloromethane/hexane, Filler: silica (SiO₂) gel), obtaining 1.53 g (Yield: 90%) of Monomer C.

UPLC-MS: [M+H]⁺ 1255.64

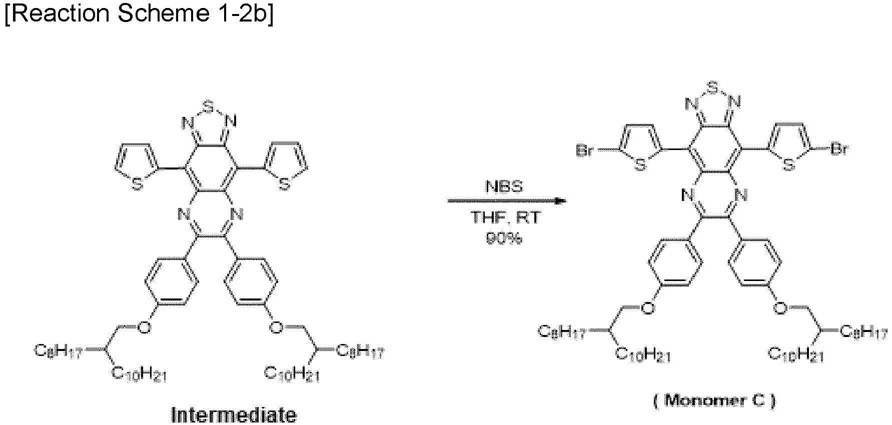

### (3) Synthesis of Polymer (Polymer 2) Including Structural Unit Represented by Chemical Formula 1-2

As shown in Reaction Scheme 1-2, Polymer 2 (Number average molecular weight (Mn) = 6,615 g/mol, PDI = 1.81) including 201 mg (Yield: 93%) of a structural unit represented by Chemical Formula 1-2 is obtained in the same manner as the step (3) of Synthesis Example 1-1 except that Monomer C (221 mg, 0.176 mmol), Monomer B (80.5 mg, 0.176 mmol), Pd₂(dba)₃ (3.2 mg, 3.5 µmol), tri(o-tolyl)phosphine (4.3 mg, 14.2 µmol), and anhydrous chlorobenzene (4.0 mL) are used.

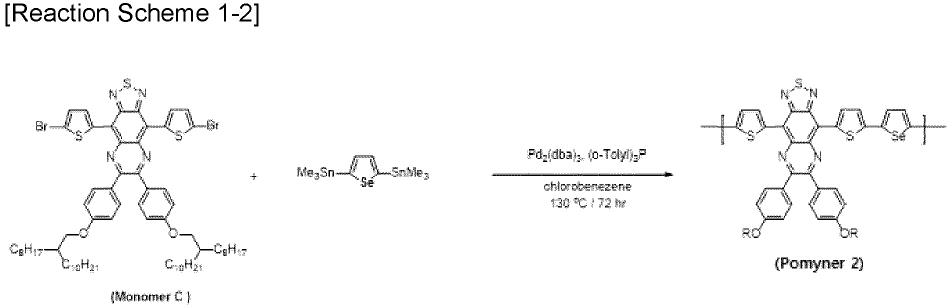

### Synthesis Example 1-3: Synthesis of Polymer (Polymer 3) Including Structural Unit Represented by Chemical Formula 1-3

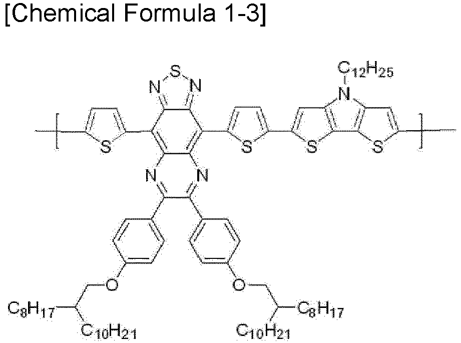

As shown in Reaction Scheme 1-3, a polymer (Polymer 3, Number average molecular weight (Mn) = 1,445 g/mol, PDI = 2.86) including 212 mg (Yield: 90%) of a structural unit represented by Chemical Formula 1-3 is obtained in the same manner as the step (3) of Synthesis Example 1-1 except that Monomer C (204 mg, 0.163 mmol), 4-dodecyl-2,6-bis(trimethylstannyl)-4H-dithieno[3,2-b:2',3'-d]pyrrole (SunaTech Inc.) (109.4 mg, 0.163 mmol), Pd₂(dba)₃ (3.0 mg, 3.3 µmol), tri(o-tolyl)phosphine (4.0 mg, 13.1 µmol), and anhydrous chlorobenzene (4.1 mL) are used.

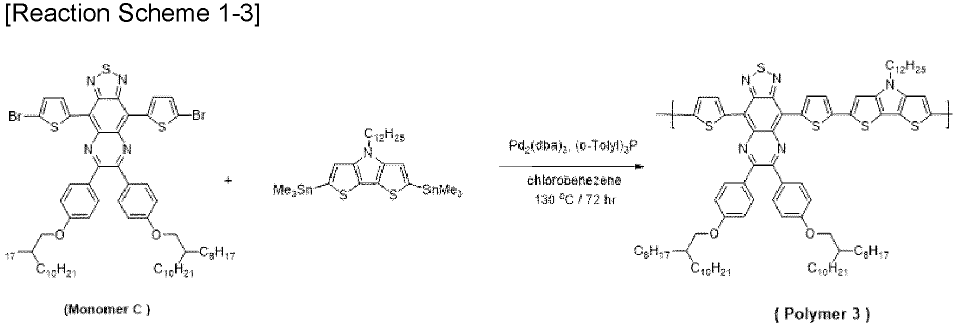

### Synthesis Example 1-4: Synthesis of Polymer (Polymer 4) Including Structural Unit Represented by Chemical Formula 1-4

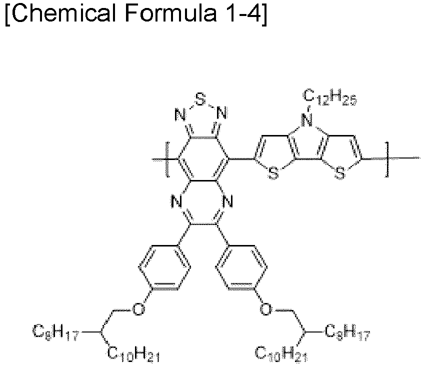

As shown in Reaction Scheme 1-4, a polymer (Polymer 4, Number average molecular weight (Mn) = 5,723 g/mol, PDI = 1.95) including 173 mg (Yield: 76%) of a structural unit represented by Chemical Formula 1-4 is obtained in the same manner as the step (3) of Synthesis Example 1-1 except that Monomer A (178 mg, 0.163 mmol), 4-dodecyl-2,6-bis(trimethylstannyl)-4H-dithieno[3,2-b:2',3'-d]pyrrole (109.8 mg, 0.163 mmol, IN1441, SunaTech Inc.), Pd₂(dba)₃ (3.0 mg, 3.3 µmol), tri(o-tolyl)phosphine (4.0 mg, 13.1 µmol), and anhydrous chlorobenzene (4.0 mL) are used.

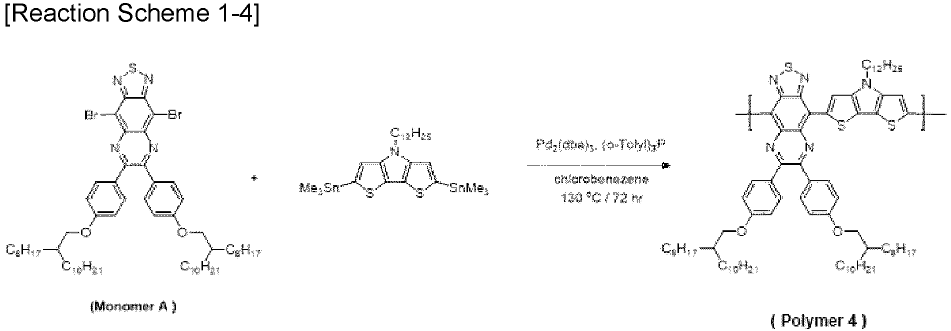

### Synthesis Example 1-5: Synthesis of Polymer (Polymer 5) Including Structural Unit Represented by Chemical Formula 1-5

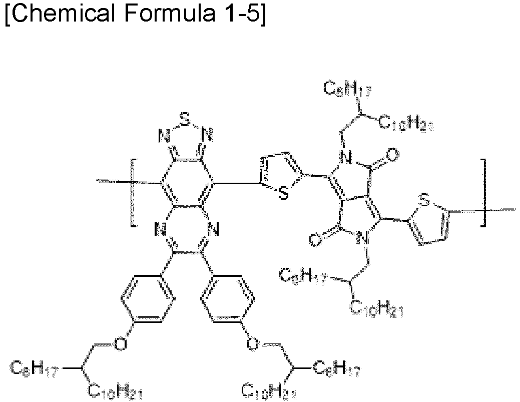

As shown in Reaction Scheme 1-5, Monomer A (215.0 mg, 0.197 mmol), 2,5-bis(2-octyldodecyl)-3,6-bis(5-(4,4,5,5-tetramethyl-1,3,2-dioxaborolan-2-yl)thiophen-2-yl)pyrrolo[3,4-c]pyrrole-1,4(2H,5H)-dione (219.4 mg, 0.197 mmol, SunaTech Inc.), Pd₂(dba)₃ (6.0 mg, 6.5 µmol), tri(o-tolyl)phosphine (12.0 mg, 39.4 µmol), and anhydrous toluene (13.0 mL) are put in a reaction flask to dissolve the reactants. After the inside of the reaction vessel is sufficiently substituted with nitrogen gas, a degassed 1 M K₃PO₄ aqueous solution (1.38 mL) is added thereto. The mixture is stirred for 72 hours, while heated at 100 °C, and then, poured into methanol/water, forming precipitates. The precipitated solid compound is several times washed with water and methanol and filtered under a reduced pressure and then, purified by Soxhlet with methanol (12 hours), acetone (12 hours), hexane (12 hours), and chloroform (6 hours). A chloroform solution therefrom is concentrated with a rotatory evaporator and reprecipitated with methanol, obtaining a polymer (Polymer 5, Number average molecular weight (Mn) = 6,767 g/mol, PDI = 1.98) including 147 mg (Yield: 42%) of a structural unit represented by Chemical Formula 1-5.

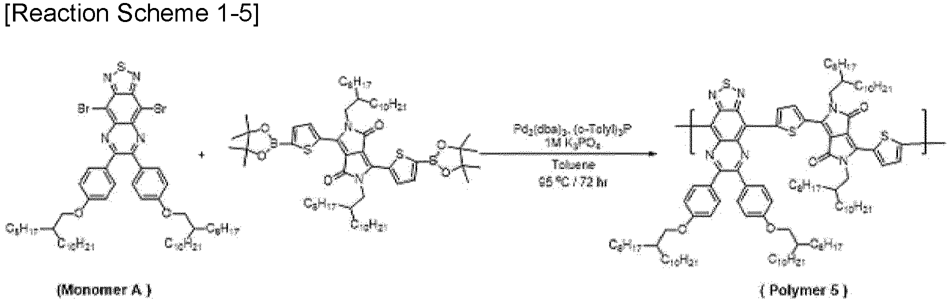

### Synthesis Example 1-6: Synthesis of Polymer (Polymer 6) Including Structural Unit Represented by Chemical Formula 1-6

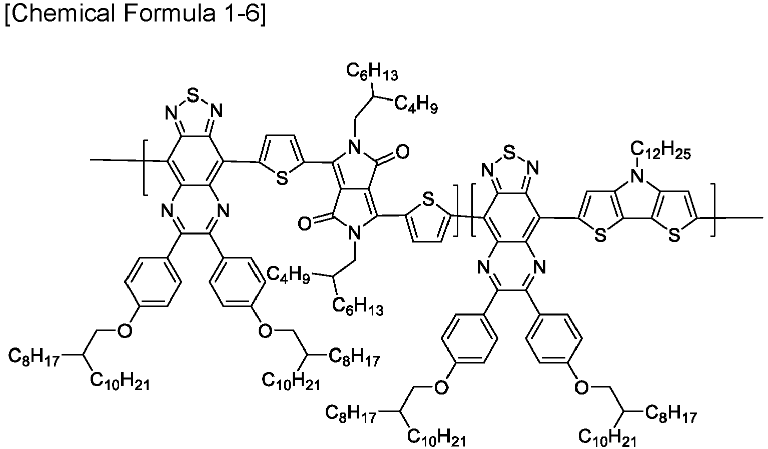

As shown in Reaction Scheme 1-6, a polymer (Polymer 6, Number average molecular weight (Mn) = 3,920 g/mol, PDI = 2.48) including 274 mg (Yield: 64%) of a structural unit represented by Chemical Formula 1-6 is obtained in the same manner as the step (3) of Synthesis Example 1-1 except that Monomer A (326 mg, 0.298 mmol), 2,5-bis(2-butyloctyl)-3,6-bis(5-(trimethylstannyl)thiophen-2-yl)pyrrolo[3,4-c]pyrrole-1,4(2H,5H)-dione (SunaTech Inc.) (143.8 mg, 0.149 mmol), 4-dodecyl-2,6-bis(trimethylstannyl)-4H-dithieno[3,2-b:2',3'-d]pyrrole (SunaTech Inc.) (100.6 mg, 0.149 mmol), Pd₂(dba)₃ (5.5 mg, 6.0 µmol), tri(o-tolyl)phosphine (7.3 mg, 24.0 µmol), and anhydrous chlorobenzene (11.4 mL) are used.

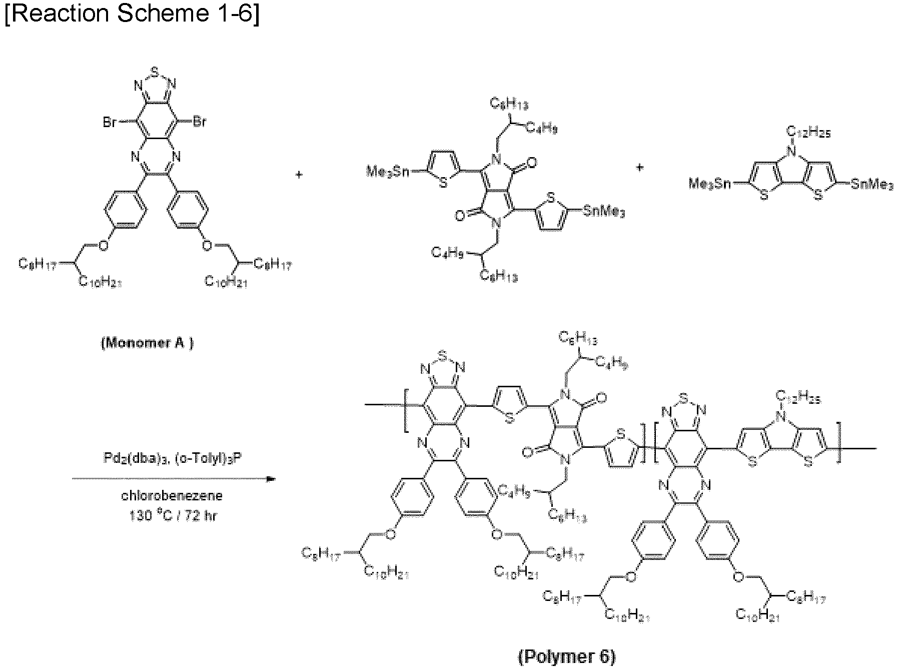

### Synthesis Example 1-7: Synthesis of Polymer (Polymer 7) Including Structural Unit Represented by Chemical Formula 1-7

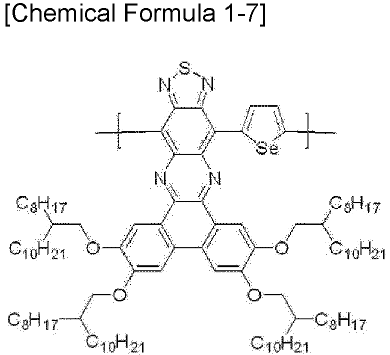

As shown in Reaction Scheme 1-7, a polymer (Polymer 7, Number average molecular weight (Mn) = 5,380 g/mol, PDI = 1.93) including 125 mg (Yield: 60%) of a structural unit represented by Chemical Formula 1-7 is obtained in the same manner as the step (3) of Synthesis Example 1-1 except that Monomer D (10,14-dibromo-2,3,6,7-tetrakis((2-octyldodecyl)oxy)dibenzo[a,c][1,2,5]thiadiazolo[3,4-i]phenazine, 221 mg, 0.126 mmol), Monomer B (57.5 mg, 0.126 mmol), Pd₂(dba)₃ (2.5 mg, 2.8 µmol), tri(o-tolyl)phosphine (6.3 mg, 20.7 µmol), and anhydrous chlorobenzene (4.0 mL) are used.

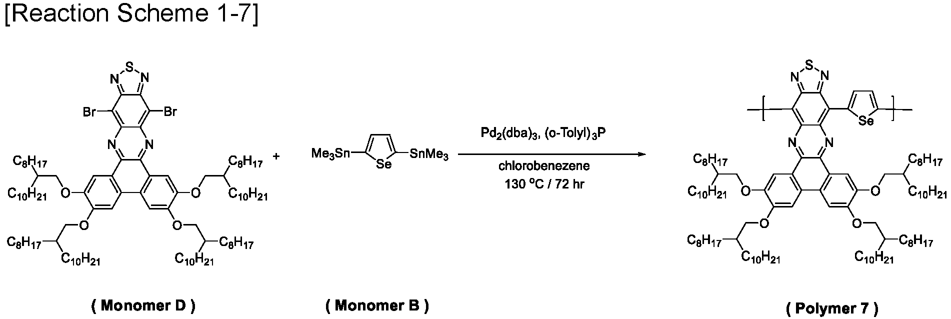

### Synthesis Example 1-8: Synthesis of Polymer (Polymer 8) Including Structural Unit Represented by Chemical Formula 1-8

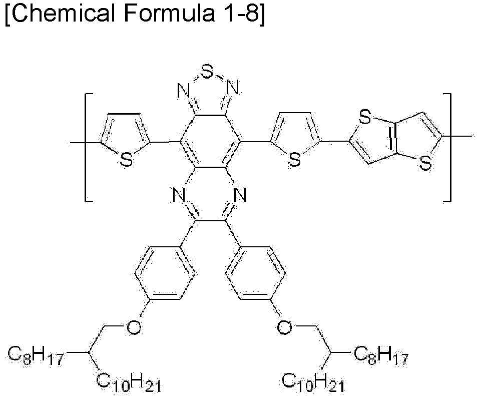

As shown in Reaction Scheme 1-8, a polymer (Polymer 8, Number average molecular weight (Mn) = 2,593 g/mol, PDI = 5.3) including 340 mg (Yield: 98%) of a structural unit represented by Chemical Formula 1-8 is obtained in the same manner as the step (3) of Synthesis Example 1-1 except that a mixture of Monomer C (351 mg, 0.280 mmol), 2,5-bis(trimethylstannyl)thieno[3,2-b]thiophene (130 mg, 0.280 mmol), Pd₂(dba)₃ (10.5 mg, 11.1 µmol), tri(o-tolyl)phosphine (14.0 mg, 44.5 µmol), and dichlorobenzene (5.0 mL) is reacted at 180 °C for 1 hour.

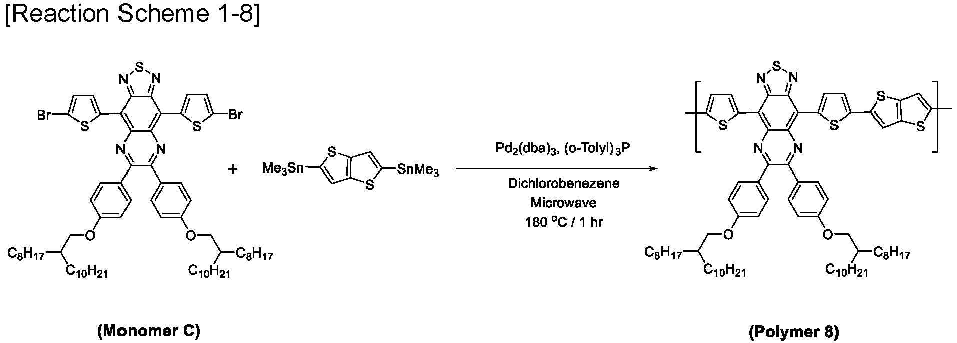

### Synthesis Example 1-9: Synthesis of Polymer (Polymer 9) Including Structural Unit Represented by Chemical Formula 1-9

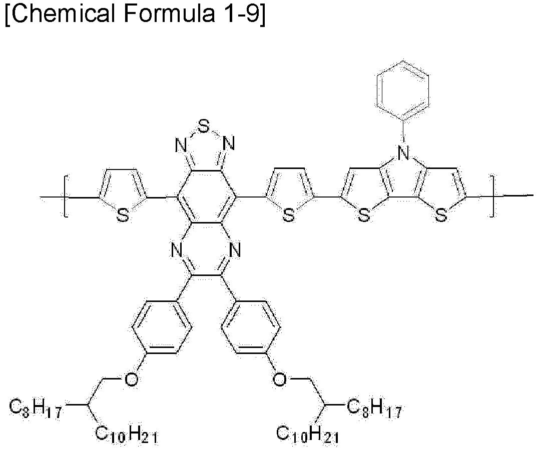

As shown in Reaction Scheme 1-9, a polymer (Polymer 9, Number average molecular weight (Mn) = 2,275 g/mol, PDI = 1.04) including 350 mg (Yield: 93%) of a structural unit represented by Chemical Formula 1-9 is obtained in the same manner as the step (3) of Synthesis Example 1-1 except that a mixture of Monomer C (350 mg, 0.278 mmol), 4-phenyl-2,6-bis(trimethylstannyl)-4H-dithieno[3,2-b:2',3'-d]pyrrole (162 mg, 0.280 mmol), Pd₂(dba)₃ (10.5 mg, 11.1 µmol), tri(o-tolyl)phosphine (14.0 mg, 44.5 µmol), and dichlorobenzene (5.0 mL) is reacted at 180 °C for 1 hour.

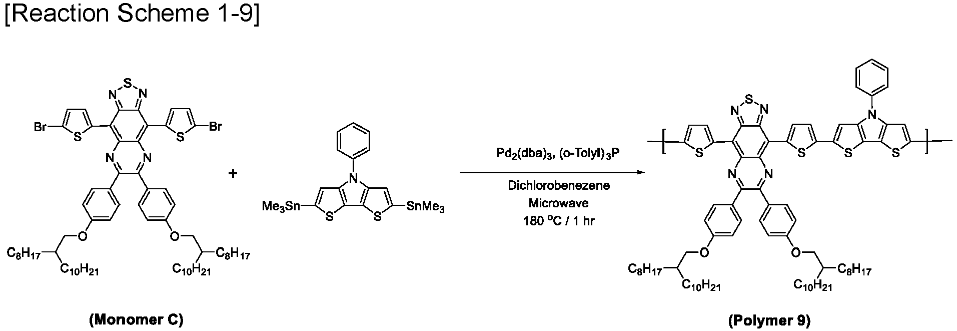

### Synthesis Example 1-10: Synthesis of Polymer (Polymer 10) Including Structural Unit Represented by Chemical Formula 1-10

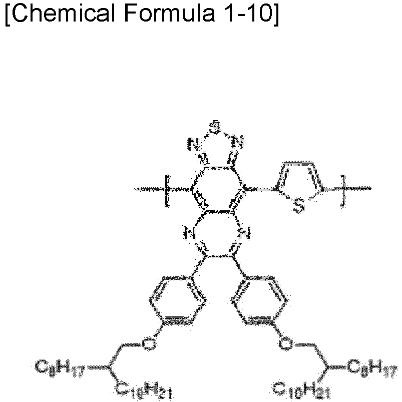

As shown in Reaction Scheme 1-10a, a polymer (Polymer 10, Number average molecular weight (Mn) = 11,833 g/mol, PDI = 1.82) including 510 mg (Yield: 83%) of a structural unit represented by Chemical Formula 1-10 is obtained in the same manner as the step (3) of Synthesis Example 1-1 except that Monomer A (660 mg, 0.605 mmol), 2,5-bis(trimethylstannyl) thiophene (Aldrich Corp.) (248.0 mg, 0.605 mmol), Pd₂(dba)₃ (5.5 mg, 6.1 µmol), tri(o-tolyl)phosphine (14.8 mg, 48.7 µmol), and anhydrous chlorobenzene (12.0 mL) are used.

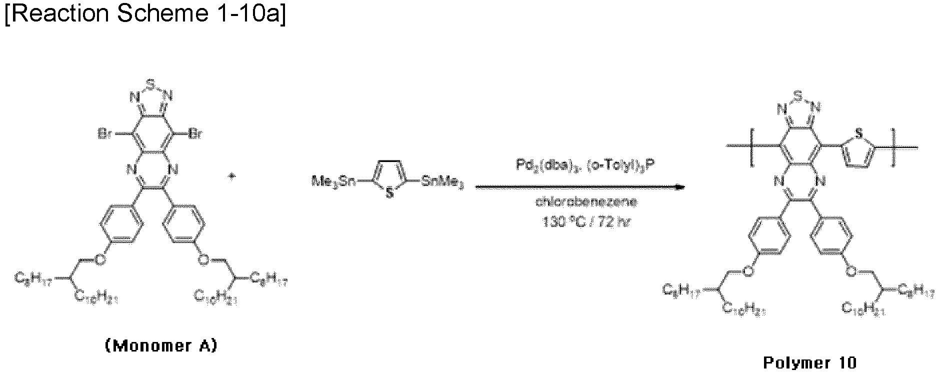

### Synthesis Example 1-11a: Synthesis of Polymer (Polymer 11a) Including Structural Unit Represented by Chemical Formula 1-11a

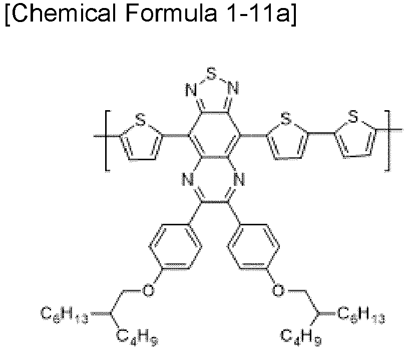

As shown in Reaction Scheme 1-11a, a polymer (Polymer 11a, Number average molecular weight (Mn) = 21k g/mol, PDI = 2.1) including 243 mg (Yield: 95%) of a structural unit represented by Chemical Formula 1-11a is obtained in the same manner as the step (3) of Synthesis Example 1-1 except that a mixture of Monomer C-1 (288.7 mg, 0.280 mmol), 2,5-bis(trimethylstannyl) thiophene (Aldrich Corp.) (114.7 mg, 0.280 mmol), Pd₂(dba)₃ (10.5 mg, 11.1 µmol), tri(o-tolyl)phosphine (14.0 mg, 44.5 µmol), and dichlorobenzene (5.0 mL) is reacted at 160 °C for 1 hour.

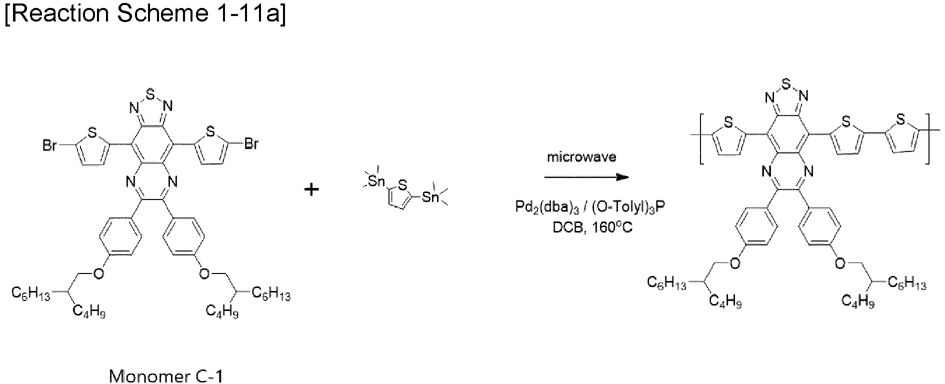

### Synthesis Example 1-11b: Synthesis of Polymer (Polymer 11b) Including Structural Unit Represented by Chemical Formula 1-11b

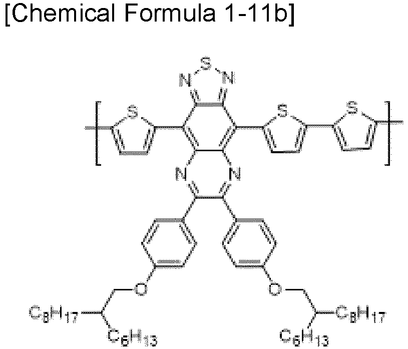

As shown in Reaction Scheme 1-11b, a polymer (Polymer 11b, Number average molecular weight (Mn) = 46k g/mol, PDI = 2.3) including 285 mg (Yield: 93%) of a structural unit represented by Chemical Formula 1-11b is obtained in the same manner as the step (3) of Synthesis Example 1-1 except that a mixture of Monomer C-2 (320 mg, 0.280 mmol), 2,5-bis(trimethylstannyl) thiophene (Aldrich Corp.) (114.7 mg, 0.280 mmol), Pd₂(dba)₃ (10.5 mg, 11.1 µmol), tri(o-tolyl)phosphine (14.0 mg, 44.5 µmol), and dichlorobenzene (5.0 mL) is reacted at 160 °C for 1 hour.

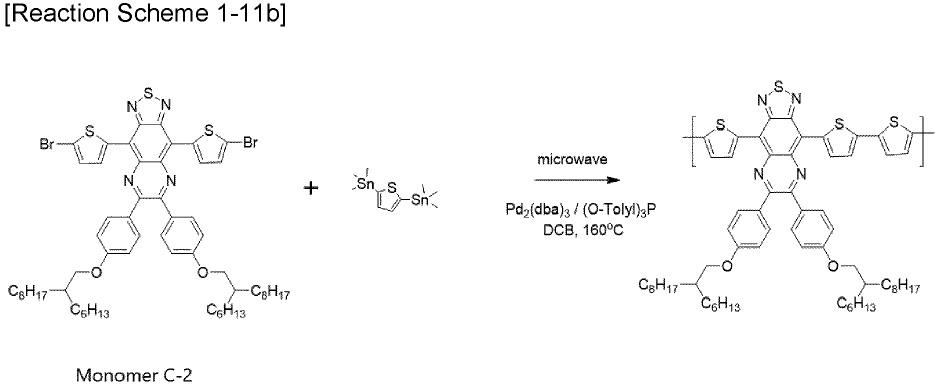

### Synthesis Example 1-11 c: Synthesis of Polymer (Polymer 11c) Including Structural Unit Represented by Chemical Formula 1-11c

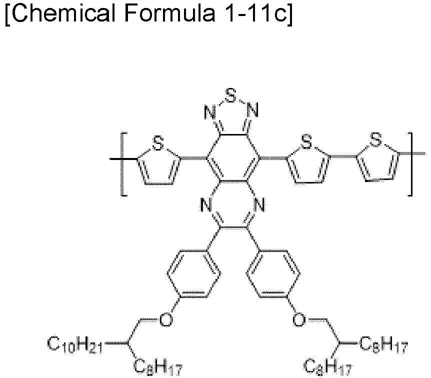

As shown in Reaction Scheme 1-11c, a polymer (Polymer 11c, Number average molecular weight (Mn) = 143k g/mol, PDI = 2.7) including 311 mg (Yield: 94%) of a structural unit represented by Chemical Formula 1-11c is obtained in the same manner as the step (3) of Synthesis Example 1-1 except that a mixture of Monomer C (351.5 mg, 0.280 mmol), 2,5-bis(trimethylstannyl) thiophene (Aldrich Corp.) (114.7 mg, 0.280 mmol), Pd₂(dba)₃ (10.5 mg, 11.1 µmol), tri(o-tolyl)phosphine (14.0 mg, 44.5 µmol), and dichlorobenzene (5.0 mL) is reacted at 160 °C for 1 hour.

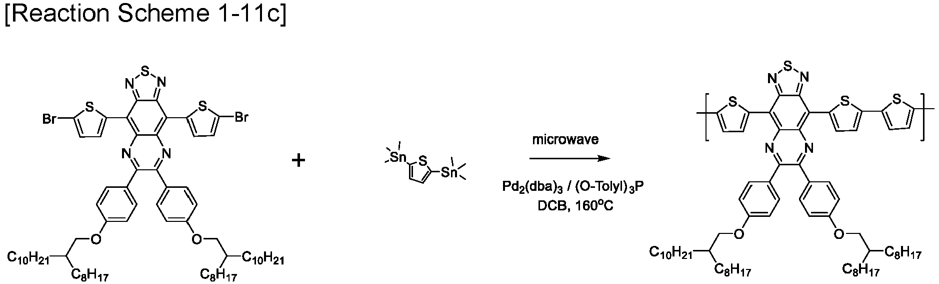

### Preparation Examples 1-1 to 1-11c: Preparation of Infrared Absorption Composition

Each polymer (Polymers 1 to 11c) obtained according to Synthesis Examples 1-1 to 1-11c and an n-type semiconductor compound represented by Chemical Formula 2-1 are mixed in a weight ratio of 1:0.75, preparing infrared absorption compositions.

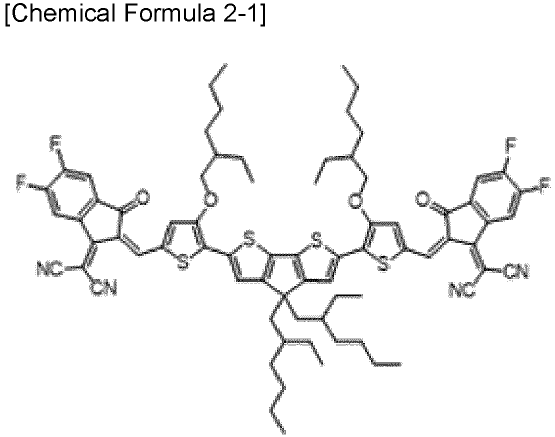

### Preparation Examples 2-1 to 2-11c: Preparation of Infrared Absorption Composition

Each polymer (Polymer 1 to Polymer 11c) according to Synthesis Examples 1-1 to 1-11c and an n-type semiconductor compound represented by Chemical Formula 2-2 are mixed in a weight ratio of 1:0.75, preparing infrared absorption compositions.

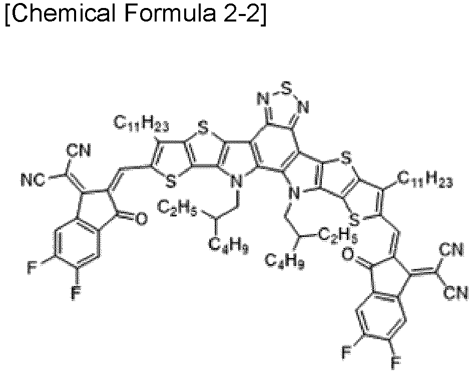

### Preparation Examples 3-1 to 3-11c: Preparation of Infrared Absorption Composition

Each polymer (Polymer 1 to Polymer 11c) according to Synthesis Examples 1-1 to 1-11c and an n-type semiconductor compound represented by Chemical Formula 2-3 are mixed in a weight ratio of 1:0.75, preparing infrared absorption compositions.

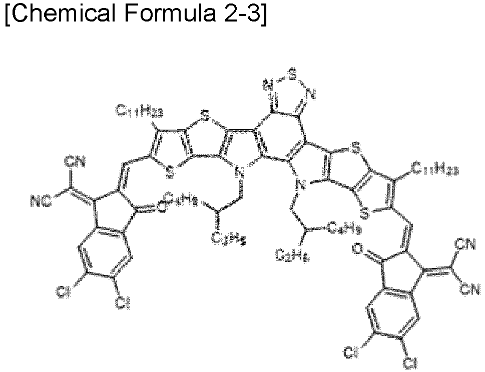

### Preparation Examples 4-1 to 4-11c: Preparation of Infrared Absorption Composition

Each polymer (Polymer 1 to Polymer 11c) according to Synthesis Examples 1-1 to 1-11c and an n-type semiconductor compound represented by Chemical Formula 2-4 are mixed in a weight ratio of 1:0.75, preparing infrared absorption compositions.

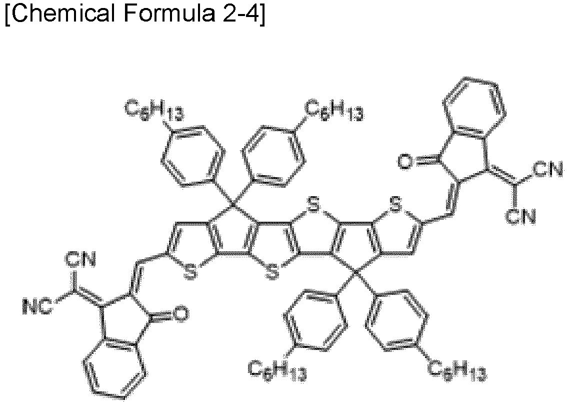

### Preparation Examples 5-1 to 5-11c: Preparation of Infrared Absorption Composition

Each polymer (Polymer 1 to Polymer 11c) according to Synthesis Examples 1-1 to 1-11c and an n-type semiconductor compound represented by Chemical Formula 2-5 are mixed in a weight ratio of 1:0.75, preparing infrared absorption compositions.

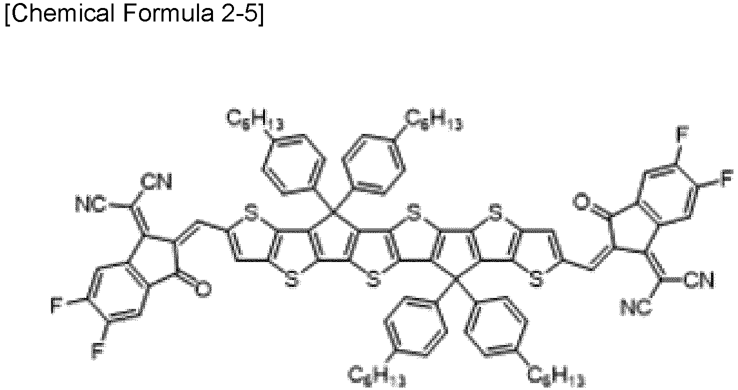

### Preparation Examples 6-1 to 6-11c: Preparation of Infrared Absorption Composition

Each polymer (Polymer 1 to Polymer 11c) according to Synthesis Examples 1-1 to Synthesis Example 1-11c and an n-type semiconductor compound represented by Chemical Formula 2-6 are mixed in a weight ratio of 1:0.75, preparing infrared absorption compositions.

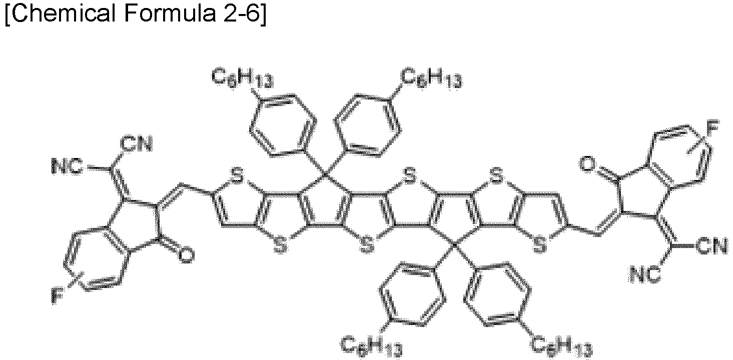

The compound represented by Chemical Formula 2-6 is a mixture of a compound having a symmetric substituted position of F and a compound having an asymmetric substituted position of F (FOIC of 1-Material Inc.).

### Comparative Preparation Examples 1-1 to 1-11c: Preparation of Infrared Absorption Composition

Each polymer (Polymer 1 to Polymer 11c) according to Synthesis Examples 1-1 to 1-11c and PCBM (phenyl-C61-butyric acid methyl ester, n-type semiconductor compound) are mixed in a weight ratio of 1:0.75, preparing infrared absorption compositions.

### Manufacture of Photoelectric Device

### Example 1-1

ITO (10 nm)/Ag (120 nm)/ITO (8 nm) are sputtered on a glass substrate to form an anode and PEDOT (poly(3,4-ethylenedioxythiophene)) is deposited to form a 45 nm-thick hole transport layer (HTL). Then, a solution obtained by dispersing the infrared absorption composition according to Preparation Example 1-1 in a chloroform solvent is spin-coated on the hole transport layer to form a 120 nm-thick photoactive layer. Herein, the p-type semiconductor compound and the n-type semiconductor compound are used in a weight ratio (p:n) of 1:0.75. On the photoactive layer, C60 is deposited to form a 30 nm-thick auxiliary layer. On the auxiliary layer, ITO is deposited to from a 7 nm-thick cathode. Subsequently, on the cathode, a glass plate is used for sealing to manufacture a photoelectric device.

### Examples 1-2 to 6-11c

Each photoelectric device is manufactured in the same manner as Example 1-1 except that the infrared absorption compositions of Preparation Examples 1-2 to 6-11c are respectively used instead of the infrared absorption composition of Preparation Example 1-1 to form each photoactive layer.

### Comparative Example 1-1

ITO (10 nm)/Ag (120 nm)/ITO (8 nm) are sputtered on a glass substrate to form an anode and PEDOT (poly(3,4-ethylenedioxythiophene)) is deposited to form a 45 nm-thick hole transport layer (HTL). Then, a solution obtained by dispersing a polymer (Polymer 1, p-type semiconductor compound) including the structural unit represented by Chemical Formula 1-1 and PCBM in a chlorobenzene solvent is spin-coated on the hole transport layer to form a 150 nm-thick photoactive layer. Herein, the p-type semiconductor compound and the n-type semiconductor compound are used in a weight ratio (p:n) of 1:0.75. On the photoactive layer, C60 is deposited to form a 30 nm-thick auxiliary layer. On the auxiliary layer, ITO is deposited to from a 7 nm-thick cathode. Subsequently, on the cathode, a glass plate is used for sealing to manufacture a photoelectric device.

### Comparative Examples 1-2 to 1-11c

Each photoelectric device is manufactured in the same manner as Comparative Example 1-1 except that Polymer 2 to Polymer 11c of Synthesis Examples 1-2 to 1-11c are respectively used instead of Polymer 1 of Synthesis Example 1-1 as a p-type semiconductor compound to form each photoactive layer.

### Comparative Examples 2-1 to 2-3

Each photoelectric devices of Comparative Examples 2-1 to 2-3 is manufactured in the same manner as Comparative Example 1-1 except that Comparative Polymer 1 (p-type semiconductor compound) having the following structure, and a compound represented by Chemical Formula 2-2, a compound represented by Chemical Formula 2-5, or PCBM as an n-type semiconductor compound are used to form each photoactive layer as shown in Table 4. Comparative Polymer 1 is synthesized as described in Polym. Chem., 2017, 8, 2922-2930.

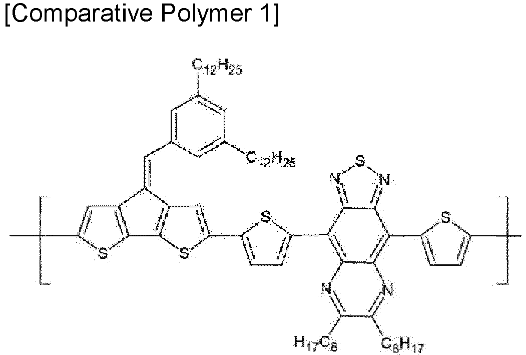

### Comparative Examples 3-1 to 3-3

Each photoelectric device of Comparative Examples 3-1 to 3-3 is manufactured in the same manner as Comparative Example 1-1 except that Comparative Polymer 2 having the following structure (p-type semiconductor compound), and a compound represented by Chemical Formula 2-2, a compound represented by Chemical Formula 2-5, or PCBM as an n-type semiconductor compound are used to form each photoactive layer as shown in Table 5. Comparative Polymer 2 is synthesized according to a method described in J. Mater. Chem. C, 2018, 6, 3634-3641.

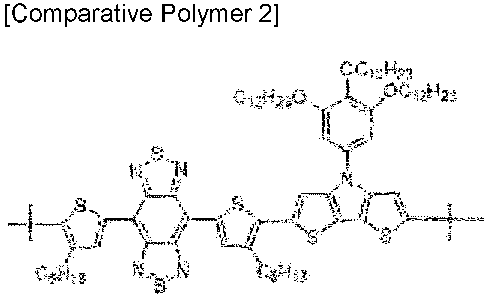

### Evaluation I: Maximum Absorption Wavelength of p-type Semiconductor Compound

The polymers according to Synthesis Examples 1-1 to 1-11c are respectively dissolved in CHCl₃ at a concentration of 1×10⁻⁵ M, preparing solutions and then, evaluated with respect to light absorption characteristics in a solution state. The results are shown in Table 1. The light absorption characteristics are evaluated by measuring a maximum absorption wavelength (λₘₐₓ) with a UV-3600 Plus UV-Vis-NIR spectrometer (Shimadzu Corp.).

In addition, a solution prepared by dissolving 20 mg of each polymer of Synthesis Examples 1-1 to 1-11c in 1.0 mL of anhydrous chlorobenzene is spin-coated on a glass substrate to form thin films, and light absorption characteristics of the polymers in a thin film state are evaluated. The light absorption characteristics are evaluated by measuring a maximum absorption wavelength (λₘₐₓ) with the UV-3600 Plus UV-Vis-NIR spectrometer (Shimadzu Corp.). The results are shown in Table 1.

### Evaluation II: Extinction Coefficient of p-type Semiconductor Compound

Each solution prepared by respectively dissolving 20 mg of the polymers of Synthesis Examples 1-1 to 1-11c in 1.0 mL of anhydrous chlorobenzene is spin-coated on a glass substrate and then, measured with respect to an extinction coefficient in a thin film state. The extinction coefficient is measured by using a UV-3600 Plus UV-Vis-NIR spectrometer (Shimadzu Corp.). Each thin film is measured with respect to a thickness, which is used to calculate an extinction coefficient per unit thickness at the maximum absorption wavelength of an absorption spectrum. The results are shown in Table 1.

**(Table 1)**

| | Maximum absorption wavelength (λₘₐₓ, nm) | | Extinction coefficient (cm⁻¹) (@ maximum absorption wavelength) |
|---|---|---|---|
| | Solution (CHCl₃) | Film | Film |
| Synthesis Example 1-1 (Polymer 1) | 1410 | 1420 | 3.41 × 10⁴ |
| Synthesis Example 1-2 (Polymer 2) | 1125 | 1120 | 3.28 × 10⁴ |
| Synthesis Example 1-3 (Polymer 3) | 1110 | 1164 | 2.97 × 10⁴ |
| Synthesis Example 1-4 (Polymer 4) | 1460 | 1509 | 3.25 × 10⁴ |
| Synthesis Example 1-5 (Polymer 5) | 1014 | 1127 | 3.13 × 10⁴ |
| Synthesis Example 1-6 (Polymer 6) | 1410 | 1440 | 4.51 × 10⁴ |
| Synthesis Example 1-7 (Polymer 7) | 1100 | 1255 | 3.34 × 10⁴ |
| Synthesis Example 1-8 (Polymer 8) | 1070 | 1070 | 3.44 × 10⁴ |
| Synthesis Example 1-9 (Polymer 9) | 1060 | 1170 | 4.51 × 10⁴ |
| Synthesis Example 1-11a (Polymer 11a) | 1095 | 1147 | 4.29 × 10⁴ |
| Synthesis Example 1-11b (Polymer 11b) | 1105 | 1160 | 4.01 × 10⁴ |
| Synthesis Example 1-11c (Polymer 11c) | 1106 | 1147 | 3.66 × 10⁴ |

Referring to Table 1, the polymers according to Synthesis Examples 1-1 to 1-11c all exhibit satisfactory wavelength absorption in an infrared wavelength region.

### Evaluation III: Surface Roughness of Film Including infrared Absorption Composition

The polymer (Polymer 11b) of Synthesis Example 1-11b and an n-type semiconductor compound represented by Chemical Formula 2-3 in a weight ratio of 1:0.75 are dissolved in chloroform (CF) at a concentration of 14 mg/ml to prepare a solution, and this solution is spin-coated on a glass at 5000 rpm under a condition of 60s and dried, forming a film (film formed of the infrared absorption composition according to Preparation Example 1-11b).

The polymer (Polymer 11b) of Synthesis Example 1-11b and PCBM (n-type semiconductor compound) in a weight ratio of 1:0.75 are dissolved in chloroform (CF) at a concentration of 14 mg/ml to prepare a solution, and this solution is spin-coated at 5000 rpm under a condition of 60s on a glass and dried to form a film (film formed of the infrared absorption composition of Comparative Preparation Example 1-11b).

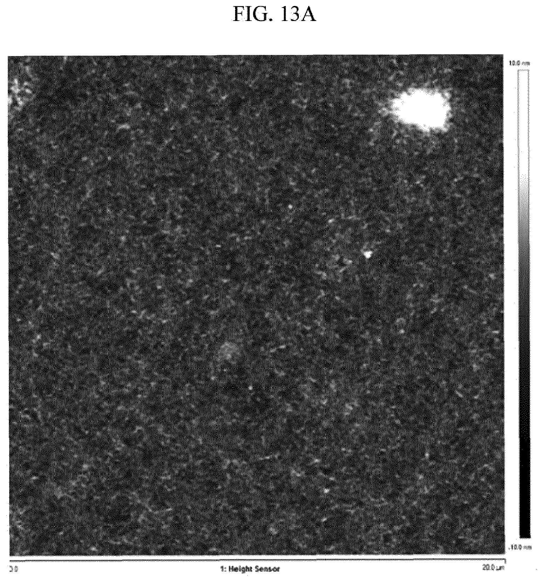

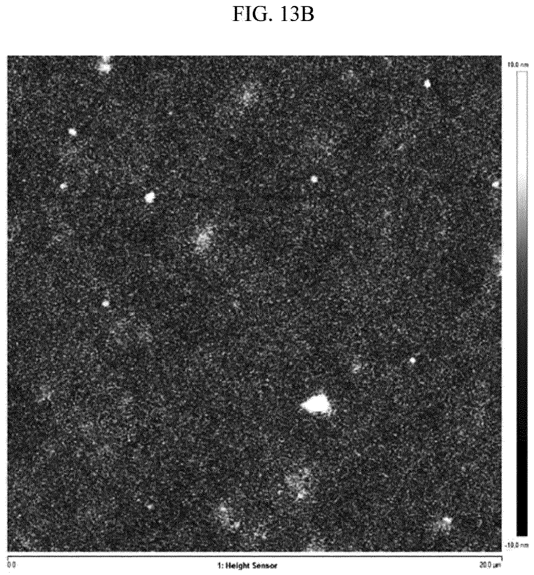

The film (20 µm × 20 µm) formed of the infrared absorption composition of Preparation Example 1-11b and the film (20 µm × 20 µm) formed of the infrared absorption composition of Comparative Preparation Example 1-11b are examined with respect to surface characteristics through atomic force microscopy, and the results are shown in FIGS. 13A and 13B.

FIGS. 13A and 13B are atomic force microscopy analysis photographs of the film made of the infrared absorption composition according to Preparation Examples 1-11b and the film made of the infrared absorption composition according to Comparative Preparation Examples 1-11b, respectively, according to some example embodiments.

Referring to FIG. 13A and FIG. 13B, the film formed of the infrared absorption composition of Preparation Example 1-11b exhibits surface roughness of 1.33 nm, and the film formed of the infrared absorption composition of Comparative Preparation Example 1-11b exhibits surface roughness of 2.38 nm.

In addition, the films (2 µm × 2 µm) formed of the infrared absorption compositions of Preparation Example 1-11b exhibits surface roughness of 1.04 nm, when surface characteristics thereof are examined through atomic force microscopy).

### Evaluation IV: GISAXS Evaluation of Films Including Infrared Absorption Composition

The polymer (Polymer 8) of Synthesis Example 1-8 and an n-type semiconductor compound represented by Chemical Formula 2-3 in a weight ratio of 1:0.75 are dissolved in chloroform (CF) at a concentration of 14 mg/ml, and this solution is spin-coated on a glass at 5000 rpm under a condition of 60s and dried, forming a film (film formed of infrared absorption composition of Preparation Example 1-8). The polymer (Polymer 8) of Synthesis Example 1-8 and PCBM (n-type semiconductor compound) in a weight ratio of 1:0.75 are dissolved in chloroform (CF) at a concentration of 14 mg/ml to prepare a solution, and this solution is spin-coated on a glass at 5000 rpm under 60s and dried, forming a film (film formed of the infrared absorption composition of Comparative Preparation Example 1-8).

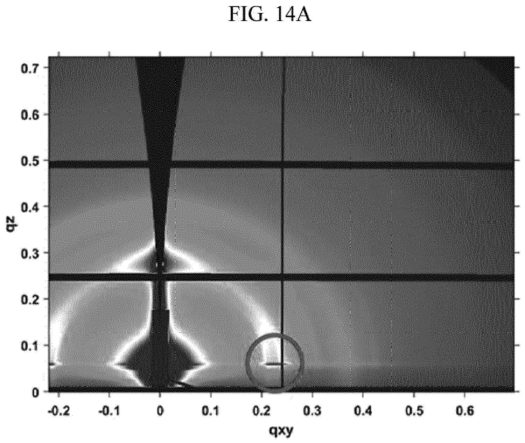

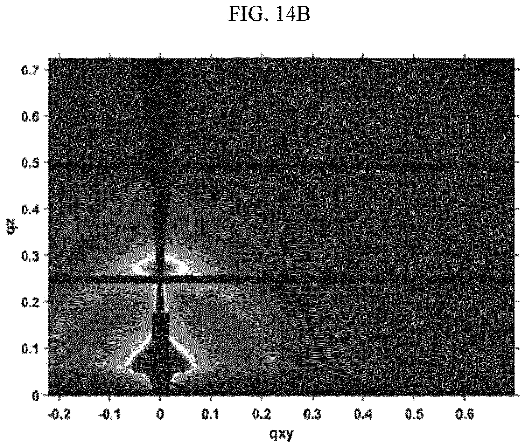

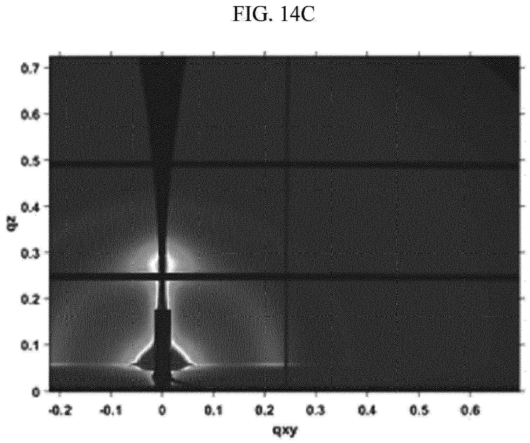

GISAXS (grazing incident small angle x-ray scattering)-analysis of the film formed of the infrared absorption composition of Preparation Example 1-8 and the film formed of the infrared absorption composition of Comparative Preparation Example 1-8 are conducted by using a 3C beamline (Pohang Accelerator Laboratory), and the results are shown in FIG. 14A and FIG. 14B. FIG. 14C shows a GISAXS analysis result of a film formed by using the polymer (Polymer 8) of Synthesis Example 1-8 for comparison.

FIGS. 14A, 14B, and 14C are figures showing the analysis results of GISAXS (grazing incident small angle x-ray scattering) of a film made of the infrared absorption composition according to Preparation Example 1-8, a film made of the infrared absorption composition according to Comparative Preparation Examples 1-8, and a film made of the polymer (Polymer 8) according to Synthesis Examples 1-8, respectively, according to some example embodiments.

Referring to FIG. 14A, the film formed of the infrared absorption composition according to Preparation Example 1-8 exhibits a peak corresponding to a face-on alignment structure (marked by a circle in FIG. 14A), which is not shown in FIG. 14C. On the contrary, FIG. 14B shows no peak corresponding to this face-on alignment structure, which supports that the film formed of the infrared absorption composition of Comparative Preparation Example 1-8 maintains an edge-on alignment structure.

### Evaluation V: Evaluation of External Quantum Efficiency of Photoelectric Devices

The photoelectric devices of Examples 1-1 to 6-11c are evaluated with respect to external quantum efficiency (EQE), and the results are shown in Table 2. The external quantum efficiency (EQE) is evaluated respectively at a wavelength ranging from 400 nm to 1500 nm and 3 V in an Incident Photon to Current Efficiency (IPCE) method. Herein, an equipment is calibrated by using Si and Ge photodiodes reference. The photoelectric devices of Examples 2-8, 3-8, 1-11a, 3-11a, 1-11b, 5-11b, 2-11c, and 3-11c are measured with respect to EQE at 1200 nm, and the results are shown in Table 2.

Table 3 shows EQE of Examples 2-8, 3-8, 1-11a, 3-11a, 1-11b, 5-11b, 2-11c, and 3-11c as a relative value based on 100% of EQE of the photoelectric devices of Comparative Examples 1-8, 1-11a, 1-11b, and 1-11c manufactured by using PCBM instead of the n-type semiconductor compound for comparison.

In addition, the photoelectric devices of Comparative Examples 2-1 to 2-3 using Comparative Polymer 1 as a p-type semiconductor compound and the photoelectric devices of Comparative Examples 3-1 to 3-3 using Comparative Polymer 2 as a p-type semiconductor compound are evaluated with respect to EQE, and the results are respectively shown in Tables 4 and 5.

**(Table 2)**

| | p-type semiconductor compound | n-type semiconductor compound | EQE (%) @ 1200 nm |
|---|---|---|---|
| Example 2-8 | Polymer 8 (Synthesis Example 8) | Chemical Formula 2-2 | 11.91 |
| Example 3-8 | Polymer 8 (Synthesis Example 8) | Chemical Formula 2-3 | 14.08 |
| Example 1-11a | Polymer 11a (Synthesis Example 1-11a) | Chemical Formula 2-1 | 34.14 |
| Example 3-11a | Polymer 11a (Synthesis Example 1-11a) | Chemical Formula 2-3 | 37.96 |
| Example 1-11b | Polymer 11b (Synthesis Example 1-11b) | Chemical Formula 2-3 | 25.29 |
| Example 5-11b | Polymer 11b (Synthesis Example 1-11b) | Chemical Formula 2-5 | 22.1 |
| Example 2-11c | Polymer 11c (Synthesis Example 1-11c) | Chemical Formula 2-2 | 16.99 |
| Example 3-11c | Polymer 11c (Synthesis Example 1-11c) | Chemical Formula 2-3 | 18.56 |

**(Table 3)**

| | p-type semiconductor compound | n-type sem iconductor compound | EQE @ 1200 nm (Relative values based on each comparative example) |
|---|---|---|---|
| Example 2-8 | Polymer 8 (Synthesis Example 8) | Chemical Formula 2-2 | 188.4% |
| Example 3-8 | Polymer 8 (Synthesis Example 8) | Chemical Formula 2-3 | 240.9% |
| Comparative Example 1-8 | Polymer 8 (Synthesis Example 8) | PCBM | 100% |
| Example 1-11a | Polymer 11a (Synthesis Example 1-11a) | Chemical Formula 2-1 | 165.9% |
| Example 3-11a | Polymer 11a (Synthesis Example 1-11a) | Chemical Formula 2-3 | 195.6% |
| Com parative Example 1-11a | Polymer 11a (Synthesis Example 1-11a) | PCBM | 100% |
| Example 1-11b | Polymer 11b (Synthesis Example 1-11b) | Chemical Formula 2-3 | 553.5% |
| Example 5-11b | Polymer 11b (Synthesis Example 1-11b) | Chemical Formula 2-5 | 471.1% |
| Comparative Example 1-11b | Polymer 11b (Synthesis Example 1-11b) | PCBM | 100% |
| Example 2-11c | Polymer 11c (Synthesis Example 1-11c) | Chemical Formula 2-2 | 301.7% |
| Example 3-11c | Polymer 11c (Synthesis Example 1-11c) | Chemical Formula 2-3 | 338.8% |
| Comparative Example 1-11c | Polymer 11c (Synthesis Example 1-11c) | PCBM | 100% |

**(Table 4)**

| | p-type semiconductor compound | n-type semiconductor compound | EQE @ 1030 nm (Relative values based on Comparative Example 2-3) | EQE @ 1200 nm (Relative values based on Comparativ e Example 2-3) |
|---|---|---|---|---|
| Com parative Example 2-1 | Comparative Polymer 1 | Chemical Formula 2-2 | 19.22% | 21.88% |
| Com parative Example 2-2 | Comparative Polymer 1 | Chemical Formula 2-5 | 0.32% | 0.48% |
| Com parative Example 2-3 | Comparative Polymer 1 | PCBM | 100% | 100% |

**(Table 5)**

| | p-type semiconductor compound | n-type semiconductor compound | EQE @ 1200 nm (Relative values based on Comparative Example 3-3) | EQE @ 1290 nm (Relative values based on Comparativ e Example 3-3) |
|---|---|---|---|---|
| Com parative Example 3-1 | Comparative Polymer 2 | Chemical Formula 2-2 | 33.33% | 11.11% |
| Com parative Example 3-2 | Comparative Polymer 2 | Chemical Formula 2-5 | 50% | 55.56% |
| Com parative Example 3-3 | Comparative Polymer 2 | PCBM | 100% | 100% |

Referring to Tables 2 and 3, the photoelectric devices of Examples 2-8, 3-8, 1-11a, 3-11a, 1-11b, 5-11b, 2-11c, and 3-11c exhibit EQE of greater than or equal to 10% at 1200 nm and thus excellent infrared ray absorption characteristics, the photoelectric devices of Examples 2-8 and 3-8 exhibit an EQE increase of about 88% or more compared with the photoelectric device of Comparative Example 1-8, the photoelectric devices of Examples 1-11a and 3-11a exhibit an EQE increase of about 65% or more compared with the photoelectric device of Comparative Example 1-11a, the photoelectric devices of Examples 1-11b and 3-11b exhibit an EQE increase of about 4 times or more compared with the photoelectric device of Comparative Example 1-11b, and the photoelectric devices of Examples 1-11c and 3-11c exhibit an EQE increase of about 3 times or more compared with the photoelectric device of Comparative Example 1-11c.

On the contrary, referring to Tables 4 and 5, the photoelectric devices of Comparative Examples 2-1 and 2-2 exhibit rather decreased EQE compared with the photoelectric device of Comparative Example 2-3, and the photoelectric devices of Comparative Examples 3-1 and 3-2 also exhibit rather decreased EQE compared with the photoelectric device of Comparative Example 3-3.

While these inventive concepts have been described in connection with what is presently considered to be practical example embodiments, it is to be understood that the inventive concepts are not limited to such example embodiments. On the contrary, the inventive concepts are intended to cover various modifications and equivalent arrangements included within the scope of the appended claims.

**<Description of Symbols>**

| | | | |
|---|---|---|---|
| 10: | first electrode | 20: | second electrode |
| 30: | photoactive layer | | |
| 50a, 50b, 50c: | photo-sensing device | | |
| 55: | charge storage | 70a, 70b, 70c: | color filter |
| 80: | insulation layer | 100: | photoelectric device |
| 10B, 10G, 10R, 10IR: | first electrode | | |
| 20B, 20G, 20R, 20IR: | second electrode | | |
| 30B, 30G, 30R, 30IR: | photoactive layer | | |
| 50B: | blue light charge storage. | 50G: | green light charge storage |
| 50R: | red light charge storage | 50IR: | infrared light charge storage |
| 110: | semiconductor substrate | 65: | lower insulation layer |
| 85: | trench | 95: | dual bandpass filter |
| 70: | color filter layer | 90: | upper insulation layer |
| 100B: | blue photo-sensing device | 100G: | green photo-sensing device |
| 100R: | red photo-sensing device | | |
| 100IR: | infrared photo-sensing device | | |
| 300, 400, 500, 600, 700: | image sensor | | |

## Claims

1. An infrared absorption composition, comprising
a p-type semiconductor compound including a first structural unit represented by Chemical Formula 1 and a second structural unit including an electron donating moiety; and
an n-type semiconductor compound represented by Chemical Formula 2: 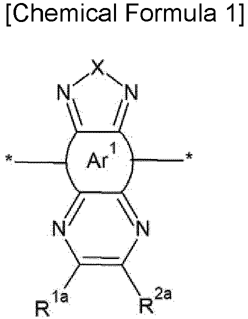
wherein, in Chemical Formula 1,
Ar¹ is a substituted or unsubstituted C6 to C30 aromatic ring, a substituted or unsubstituted C3 to C30 heteroaromatic ring, or any combination thereof,
X is O, S, Se, Te, S(=O), S(=O₂), NR^{a}, CR^{b}R^{c}, SiR^{d}R^{e}, GeR^{f}R^{g}, CR^{h}=CRⁱ, or CR^{hh}=CRⁱⁱ, wherein R^{a}, R^{b}, R^{c}, R^{d}, R^{e}, R^{f}, R^{g}, R^{h}, and Rⁱ are each independently hydrogen, deuterium, a C1 to C6 alkyl group, a C1 to C6 haloalkyl group, a C6 to C14 aryl group, a C3 to C12 heteroaryl group, a halogen, a cyano group, or any combination thereof, and R^{hh} and Rⁱⁱ are each independently a C1 to C6 alkylene group or a C2 to C6 heteroalkylene group and linked to each other to provide an aromatic or heteroaromatic ring,
R^{1a} and R^{2a} are each independently a substituted or unsubstituted C6 to C30 aryl group or a substituted or unsubstituted C3 to C30 heteroaryl group or R^{1a} and R^{2a} are linked to each other to provide a substituted or unsubstituted C6 to C30 arene group or a substituted or unsubstituted C3 to C30 heteroarene group, and
* is a linking point within the p-type semiconductor compound, 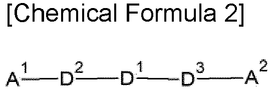
wherein, in Chemical Formula 2,
D¹ is a first electron donating moiety having any one structure of structures represented by Chemical Formulas 3A to 3E,
D² and D³ are each independently a single bond or a second electron donating moiety,
A¹ and A² are each independently an electron accepting moiety of a substituted or unsubstituted C6 to C30 hydrocarbon ring group having at least one functional group of C=O, C=S, C=Se, C=Te, or C=C(CN)₂; a substituted or unsubstituted C2 to C30 heterocyclic group having at least one functional group of C=O, C=S, C=Se, C=Te, or C=C(CN)₂; or a fused ring thereof, 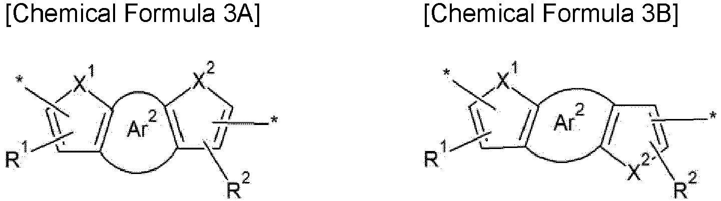 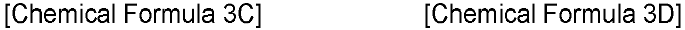 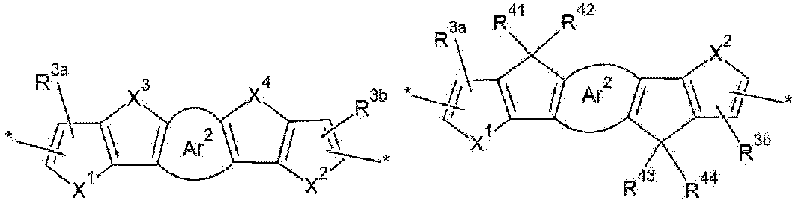 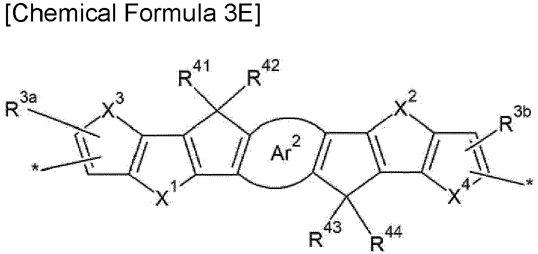
wherein, in Chemical Formulas 3A to 3E,
Ar² is a substituted or unsubstituted C6 to C30 arene group; a substituted or unsubstituted C3 to C30 heterocyclic group including at least one of N, O, S, Se, Te, or Si; a fused ring thereof; or any combination thereof,
X¹, X², X³, and X⁴ are each independently S, Se, or Te,
R⁴¹, R⁴², R⁴³, and R⁴⁴ are each independently a substituted or unsubstituted C1 to C30 alkyl group, a substituted or unsubstituted C1 to C30 alkoxy group, a substituted or unsubstituted C6 to C20 aryl group, or a substituted or unsubstituted C3 to C20 heteroaryl group,
R¹, R², R^{3a} and R^{3b} are each independently hydrogen or a C1 to C10 alkyl group, and
* denotes a linking point within Chemical Formula 2.

2. The infrared absorption composition of claim 1, wherein in Chemical Formula 1, Ar¹ is a benzene ring, a substituted or unsubstituted naphthalene, a substituted or unsubstituted anthracene ring, a substituted or unsubstituted phenanthrene ring, a substituted or unsubstituted tetracene ring, a substituted or unsubstituted pyrene ring, a substituted or unsubstituted quinoline ring, a substituted or unsubstituted isoquinoline ring, a substituted or unsubstituted quinoxaline ring, a substituted or unsubstituted quinazoline ring, or a substituted or unsubstituted phenanthroline ring;
preferably wherein in Chemical Formula 1, Ar¹ is one moiety of moieties represented by Chemical Formula 1A-1 or Chemical Formula 1A-2; 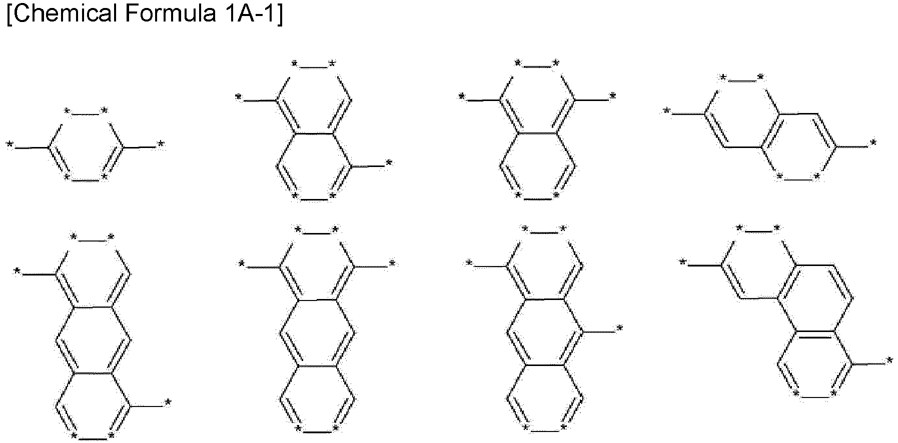
wherein, in Chemical Formula 1A-1,
at least one hydrogen of each aromatic ring is hydrogen or is replaced by deuterium, a halogen, a cyano group, a C1 to C10 alkyl group, a C1 to C10 haloalkyl group, a -SiH₃ group, or a C1 to C10 alkylsilyl group, and
adjacent pairs of *'s inside at least one aromatic ring are linking points with an N-X-N-containing ring and a pyrazine ring of Chemical Formula 1; 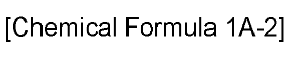 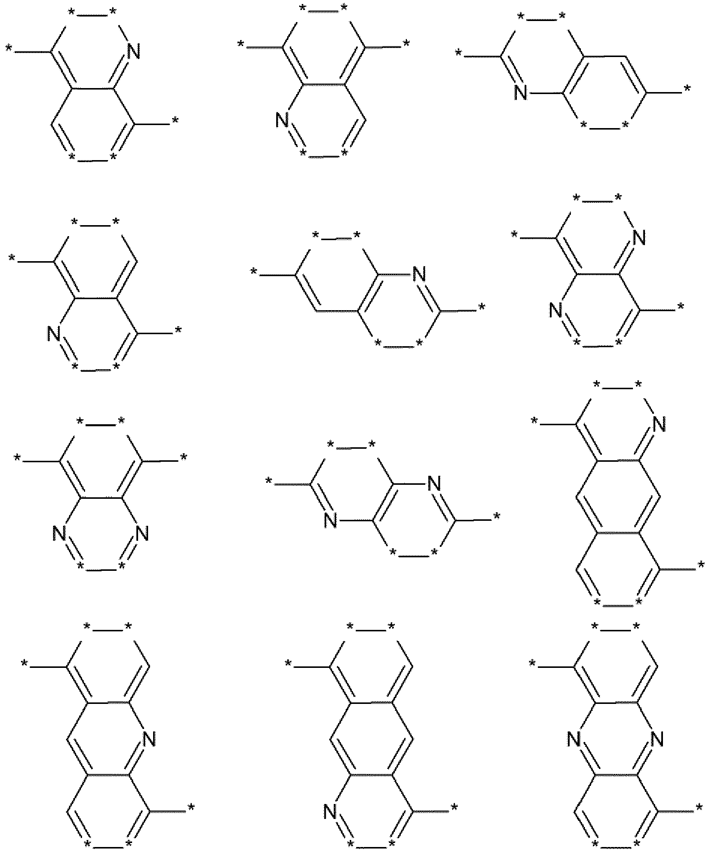
wherein, in Chemical Formula 1A-2,
at least one hydrogen of each aromatic or heteroaromatic ring is hydrogen or is replaced by deuterium, a halogen, a cyano group, a C1 to C10 alkyl group, a C1 to C10 haloalkyl group, a -SiH₃ group, or a C1 to C10 alkylsilyl group, and
adjacent pairs of *'s inside at least one aromatic or heteroaromatic ring are linking points with an N-X-N-containing ring and a pyrazine ring of Chemical Formula 1.

3. The infrared absorption composition of claims 1 or 2, wherein in Chemical Formula 1,
R^{1a} and R^{2a} are linked to each other, and
the substituted or unsubstituted C6 to C30 arene group and the substituted or unsubstituted C3 to C30 heteroarene group formed by linking R^{1a} and R^{2a} to each other are a substituted or unsubstituted benzene ring, a substituted or unsubstituted naphthalene ring, a substituted or unsubstituted acenaphthene ring, a substituted or unsubstituted anthracene ring, a substituted or unsubstituted phenanthrene ring, a substituted or unsubstituted tetracene ring, or a substituted or unsubstituted pyrene ring, a substituted or unsubstituted quinoline ring, a substituted or unsubstituted isoquinoline ring, a substituted or unsubstituted quinoxaline ring, a substituted or unsubstituted quinazoline ring, a substituted or unsubstituted phenanthroline ring, a substituted or unsubstituted pyrimidine ring, or a substituted or unsubstituted benzodithiophene ring; or
wherein in Chemical Formula 1,
R^{1a} and R^{2a} are linked to each other, and
the substituted or unsubstituted C6 to C30 arene group and the substituted or unsubstituted C3 to C30 heteroarene group formed by linking R^{1a} and R^{2a} to each other are one moiety of moieties represented by Chemical Formulas 1B-1 and 1B-2: 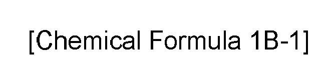 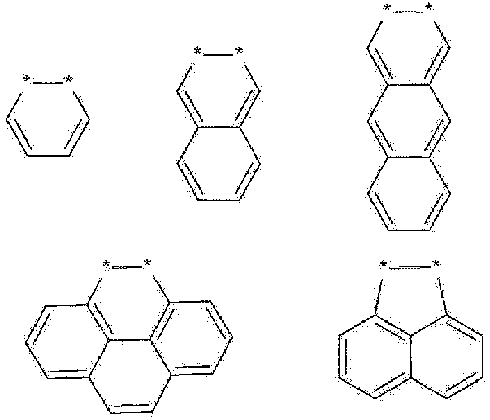
wherein, in Chemical Formula 1B-1,
at least one hydrogen of each aromatic ring is hydrogen or is replaced by a halogen, a cyano group, a C1 to C30 alkyl group, a C1 to C30 alkoxy group, a C1 to C30 haloalkyl group, a -SiH₃ group, a C1 to C30 alkylsilyl group, a C6 to C30 aryl group, a C6 to C30 aryloxy group, or a C3 to C30 heteroaryl group, and
each * is a point bonded to a pyrazine ring of Chemical Formula 1, 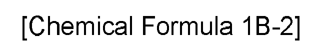 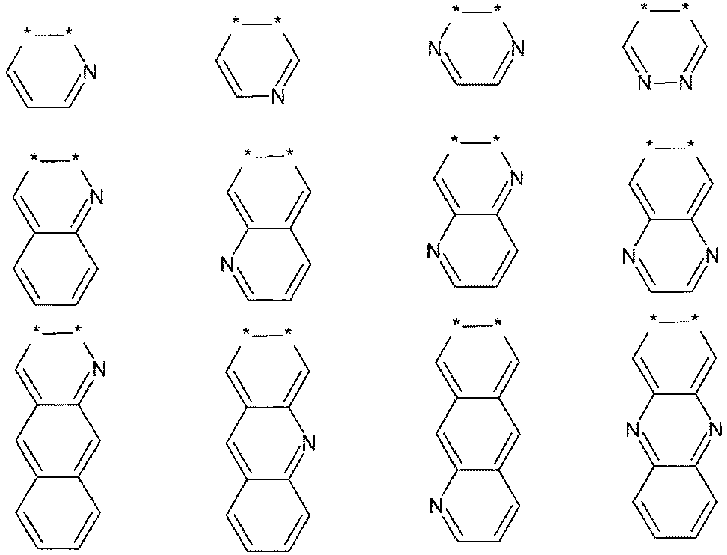
wherein, in Chemical Formula 1B-2,
at least one hydrogen of each aromatic or heteroaromatic ring is hydrogen or is replaced by a halogen, a cyano group, a C1 to C30 alkyl group, a C1 to C30 alkoxy group, a C1 to C30 haloalkyl group, a -SiH₃ group, a C1 to C30 alkylsilyl group, a C6 to C30 aryl group, a C6 to C30 aryloxy group, or a C3 to C30 heteroaryl group, and
each * is a point bonded to a pyrazine ring of Chemical Formula 1; or wherein in Chemical Formula 1,
R^{1a} and R^{2a} are linked to each other, and
the substituted or unsubstituted C6 to C30 arene group and the substituted or unsubstituted C3 to C30 heteroarene group formed by linking R^{1a} and R^{2a} to each other are each independently one moiety of moieties having an aromatic or heteroaromatic ring and represented by Chemical Formula 1B-3 or 1B-4: 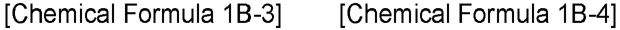 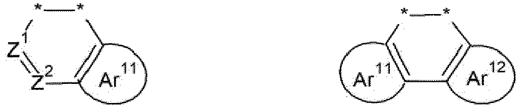
wherein, in Chemical Formulas 1B-3 and 1B-4,
Ar¹¹ and Ar¹² are each independently a substituted or unsubstituted C6 to C30 arene group or a substituted or unsubstituted C3 to C30 heteroarene group,
wherein, in Chemical Formula 1B-3,
Z¹ and Z² are each independently N or CR^{x}, wherein R^{x} is hydrogen, deuterium, a C1 to C10 alkyl group, a C1 to C10 haloalkyl group, a -SiH₃ group, a C1 to C10 alkylsilyl group, a -NH₂ group, a C1 to C10 alkylamine group, a C6 to C10 arylamine group, a C6 to C14 aryl group, a C3 to C12 heteroaryl group, a halogen, a cyano group, or any combination thereof, and each * inside the aromatic or heteroaromatic ring is a point bonded to a pyrazine ring of Chemical Formula 1;
preferably wherein the moiety represented by Chemical Formula 1B-3 is represented by Chemical Formula 1B-3-1, or wherein the moiety represented by Chemical Formula 1B-4 is represented by Chemical Formula 1B-4-1: 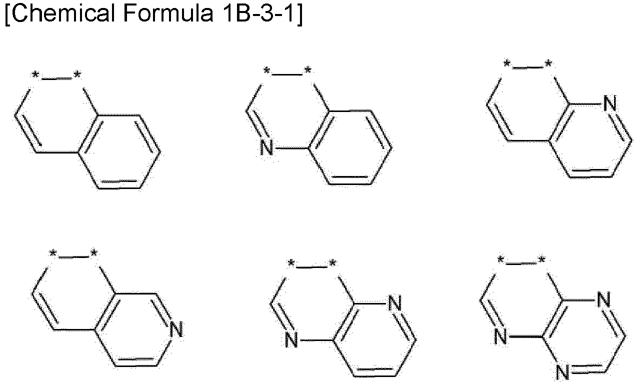
wherein, in Chemical Formula 1B-3-1,
at least one hydrogen of each aromatic or heteroaromatic ring is hydrogen or is replaced by a halogen, a cyano group, a C1 to C30 alkyl group, a C1 to C30 alkoxy group, a C1 to C30 haloalkyl group, a -SiH₃ group, a C1 to C30 alkylsilyl group, a C6 to C30 aryl group, a C6 to C30 aryloxy group, or a C3 to C30 heteroaryl group, and
each * inside the aromatic or heteroaromatic ring is a point bonded to the pyrazine ring of Chemical Formula 1; 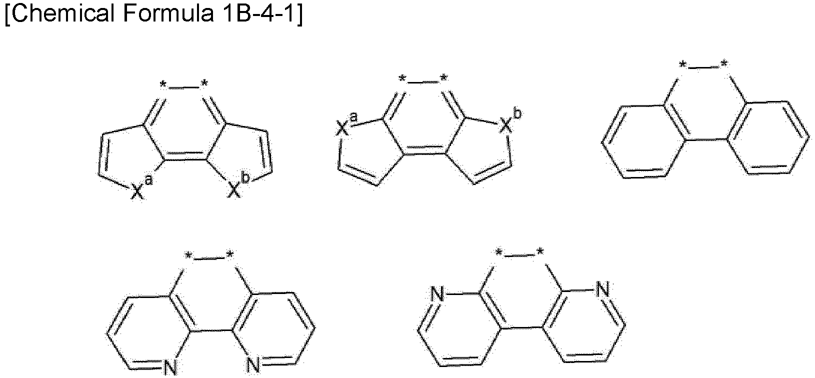
wherein, in Chemical Formula 1B-4-1,
at least one hydrogen of each aromatic or heteroaromatic ring is hydrogen or is replaced by a halogen, a cyano group, a C1 to C30 alkyl group, a C1 to C30 alkoxy group, a C1 to C30 haloalkyl group, a -SiH₃ group, a C1 to C30 alkylsilyl group, a C6 to C30 aryl group, a C6 to C30 aryloxy group, or a C3 to C30 heteroaryl group,
X^{a} and X^{b} are each independently O, S, Se, Te, NR^{a}, SiR^{b}R^{c}, or GeR^{d}R^{e}, wherein R^{a}, R^{b}, R^{c}, R^{d}, and R^{e} are each independently hydrogen, a halogen, a substituted or unsubstituted C1 to C10 alkyl group, or a substituted or unsubstituted C6 to C10 aryl group, and
each * inside the aromatic ring is a point bonded to the pyrazine ring of Chemical Formula 1.

4. The infrared absorption composition of any of claims 1-3, wherein the first structural unit of the p-type semiconductor compound is represented by Chemical Formula 1C: 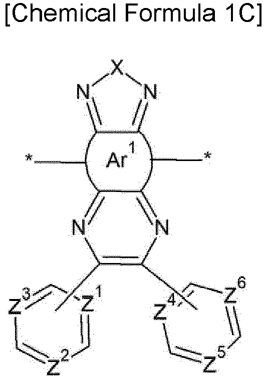
wherein, in Chemical Formula 1C,
Ar¹ is a substituted or unsubstituted C6 to C30 aromatic ring, a substituted or unsubstituted C3 to C30 heteroaromatic ring, or any combination thereof,
X is O, S, Se, Te, S(=O), S(=O₂), NR^{a}, CR^{b}R^{c}, SiR^{d}R^{e}, GeR^{f}R^{g}, CR^{h}=CRⁱ, or CR^{hh}=CRⁱⁱ, wherein R^{a}, R^{b}, R^{c}, R^{d}, R^{e}, R^{f}, R^{g}, R^{h}, and Rⁱ are each independently hydrogen, deuterium, a C1 to C6 alkyl group, a C1 to C6 haloalkyl group, a C6 to C14 aryl group, a C3 to C12 heteroaryl group, a halogen, a cyano group, or any combination thereof, and R^{hh} and Rⁱⁱ are each independently a C1 to C6 alkylene group or a C2 to C6 heteroalkylene group and linked to each other to provide an aromatic or heteroaromatic ring
Z¹ to Z⁶ are each independently N or CR^{x}, wherein R^{x} is hydrogen, deuterium, a C1 to C20 alkyl group, a C1 to C20 haloalkyl group, a -SiH₃ group, a C1 to C20 alkylsilyl group, a -NH₂ group, a C1 to C20 alkylamine group, a C6 to C12 aryl group, a C3 to C12 heteroaryl group, a halogen, a cyano group, or any combination thereof,
at least one hydrogen of each aromatic or heteroaromatic ring is hydrogen or is replaced by deuterium, a halogen, a cyano group, a C1 to C30 alkyl group, a C1 to C30 alkoxy group, a C1 to C30 haloalkyl group, a C6 to C30 aryl group, a C6 to C30 aryloxy group, a -SiH₃ group, or a C1 to C30 alkylsilyl group, and
* is a linking point with the p-type semiconductor compound; and/or
wherein the electron donating moiety included in the second structural unit of the p-type semiconductor compound is a substituted or unsubstituted C6 to C30 arene group, a substituted or unsubstituted divalent C3 to C30 heterocyclic group including at least one of N, O, S, Se, Te, or Si, a fused ring thereof, or any combination thereof, preferably wherein the electron donating moiety included in the second structural unit of the p-type semiconductor compound comprises at least one moiety of moieties represented by Chemical Formulas 4A to 4J of Group 1: 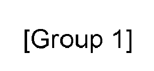 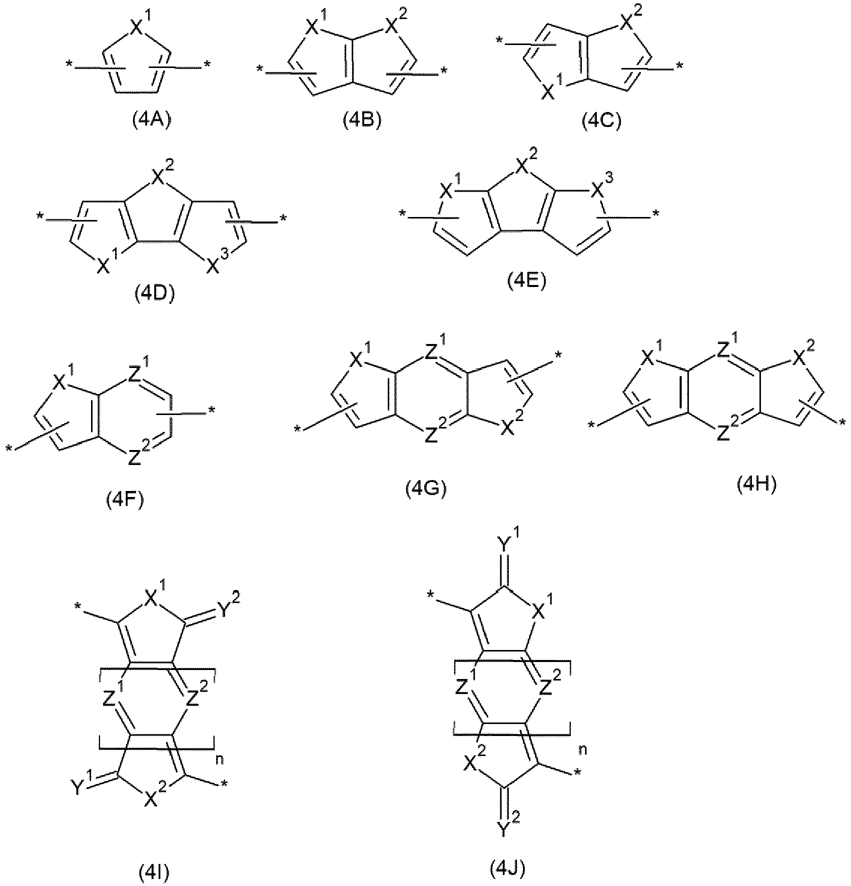
wherein, in Group 1,
X¹ to X³ are each independently S, Se, Te, S(=O), S(=O₂), NR^{a}, SiR^{d}R^{e}, or GeR^{f}R^{g}, wherein R^{a}, R^{b}, R^{c}, R^{d}, R^{e}, R^{f}, and R^{g} are each independently hydrogen, deuterium, a C1 to C20 alkyl group, a C1 to C20 alkoxy group, a C1 to C20 haloalkyl group, a C6 to C20 aryl group, a C3 to C20 heteroaryl group, a halogen, a cyano group, or any combination thereof,
Z¹ and Z² are each independently N or CR^{x}, wherein R^{x} is hydrogen, deuterium, a C1 to C10 alkyl group, a C1 to C10 haloalkyl group, a -SiH₃ group, a C1 to C10 alkylsilyl group, a -NH₂ group, a C1 to C10 alkylamine group, a C6 to C12 aryl group, a C3 to C12 heteroaryl group, a halogen, a cyano group, or any combination thereof,
Y¹ and Y² are each independently O, S, Se, or Te,
n is 0 or 1, and
at least one hydrogen of each aromatic or heteroaromatic ring is hydrogen or is replaced by deuterium, a halogen, a cyano group, a C1 to C30 alkyl group, a C1 to C30 alkoxy group, a C1 to C30 haloalkyl group, a C6 to C30 aryl group, a C6 to C30 aryloxy group, a -SiH₃ group, or a C1 to C30 alkylsilyl group.

5. The infrared absorption composition of any of claims 1-4, wherein the p-type semiconductor compound is a polymer including 20 mol% to 50 mol% of the first structural unit and 50 mol% to 80 mol% of the second structural unit; and/or
wherein the p-type semiconductor compound is configured to exhibit a peak absorption wavelength in a wavelength range of 1000 nm to 3000 nm; and/or
wherein in Chemical Formulas 3A to 3C, Ar² is a moiety having one structure of structures represented by Chemical Formulas 5A to 5K of Group 2: 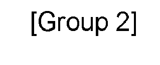 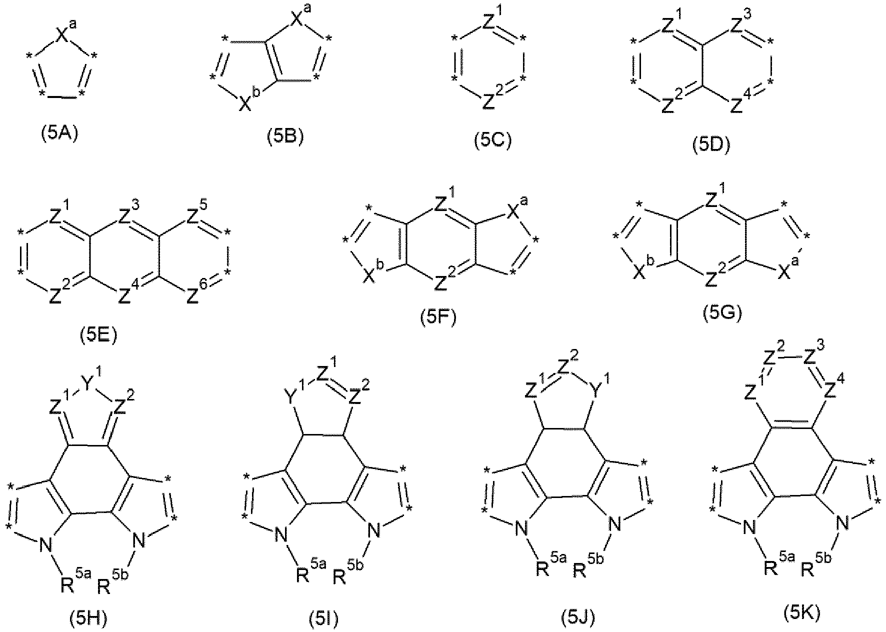
wherein, in Group 2,
X^{a} and X^{b} are each independently CR^{x}R^{y}, S, Se, or Te, wherein R^{x} and R^{y} are each independently a substituted or unsubstituted C1 to C30 alkyl group, a substituted or unsubstituted C1 to C30 alkoxy group, a substituted or unsubstituted C6 to C20 aryl group, or a substituted or unsubstituted C3 to C20 heteroaryl group,
R^{5a} and R^{5b} are each independently hydrogen, a C1 to C20 alkyl group, a C1 to C20 alkoxy group, a C6 to C10 aryl group, or a C2 to C10 heteroaryl group,
Y¹ is CR^{p}R^{q}, NR^{r}, O, S, Se, or Te, wherein R^{p}, R^{q}, and R^{r} are each independently hydrogen or a C1 to C20 alkyl group, and
Z¹ to Z⁶ are each independently CR^{s} or N, wherein R^{s} is hydrogen or a C1 to C20 alkyl group.

6. The infrared absorption composition of any of claims 1-5, wherein in Chemical Formulas 3D and 3E, R⁴¹, R⁴², R⁴³, and R⁴⁴ are each independently a C1 to C20 alkyl group; a C1 to C20 alkoxy group; a C6 to C20 aryl group substituted with a C1 to C20 alkyl group or a C1 to C20 alkoxy group; or a C3 to C20 heteroaryl group substituted with a C1 to C20 alkyl group or a C1 to C20 alkoxy group; and/or
wherein in Chemical Formulas 3D and 3E, R⁴¹, R⁴², R⁴³, and R⁴⁴ are each independently a substituted or unsubstituted C3 to C30 branched alkyl group or a substituted or unsubstituted C3 to C30 branched alkoxy group.

7. The infrared absorption composition of any of claims 1-6, wherein the first electron donating moiety selected from the structures represented by Chemical Formulas 3A to 3E is a moiety represented by Chemical Formula 3A-1, 3B-1, 3C-1, 3D-1, 3D-2, 3E-1, or 3E-2: 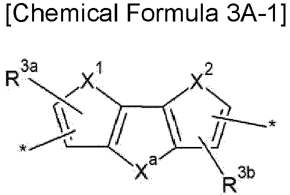
wherein, in Chemical Formula 3A-1,
X^{a} is CR^{x}R^{y}, S, Se, or Te, wherein R^{x} and R^{y} are each independently a substituted or unsubstituted C1 to C30 alkyl group, a substituted or unsubstituted C1 to C30 alkoxy group, a substituted or unsubstituted C6 to C20 aryl group, or a substituted or unsubstituted C3 to C20 heteroaryl group,
X¹ and X² are each independently S, Se, or Te,
R^{3a} and R^{3b} are each independently hydrogen or a C1 to C10 alkyl group, and
* denotes a linking point within Chemical Formula 2, 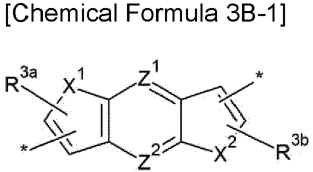
wherein, in Chemical Formula 3B-1,
Z¹ and Z² are each independently CR^{s} or N, wherein R^{s} is hydrogen or a C1 to C20 alkyl group,
X¹ and X² are each independently S, Se, or Te,
R^{3a} and R^{3b} are each independently hydrogen or a C1 to C10 alkyl group, and
* denotes a linking point within Chemical Formula 2, 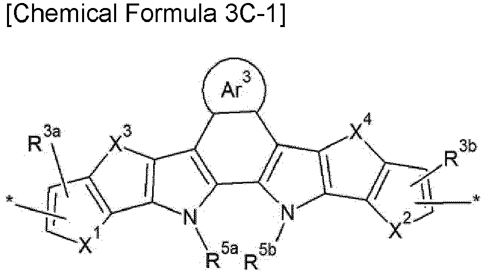
wherein, in Chemical Formula 3C-1,
Ar³ is one moiety of moieties having a ring and represented by Chemical Formula 3C-1a,
X¹, X², X³, and X⁴ are each independently S, Se, or Te,
R^{3a} and R^{3b} are each independently hydrogen or a C1 to C10 alkyl group,
R^{5a} and R^{5b} are each independently hydrogen, a C1 to C20 alkyl group, a C1 to C20 alkoxy group, a C6 to C10 aryl group, or a C2 to C10 heteroaryl group, and
* denotes a linking point within Chemical Formula 2, 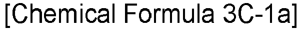 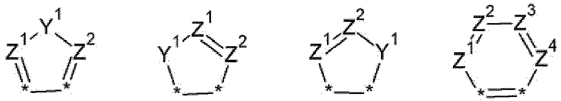
wherein, in Chemical Formula 3C-1a,
Y¹ is CR^{p}R^{q}, NR^{r}, O, S, Se, or Te, wherein R^{p}, R^{q}, and R^{r} are each independently hydrogen or a C1 to C20 alkyl group,
Z¹ to Z⁴ are each independently CR^{s} or N, wherein R^{s} is hydrogen or a C1 to C20 alkyl group, and
* inside the ring denotes a linking point with Chemical Formula 3C-1, 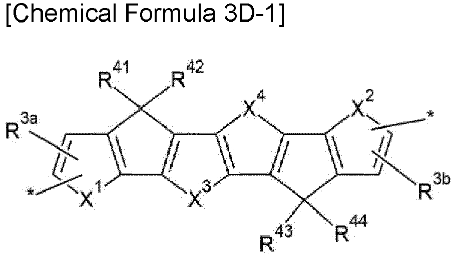
wherein, in Chemical Formula 3D-1,
X¹, X², X³, and X⁴ are each independently S, Se, or Te,
R⁴¹, R⁴², R⁴³, and R⁴⁴ are each independently a substituted or unsubstituted C1 to C30 alkyl group, a substituted or unsubstituted C1 to C30 alkoxy group, a substituted or unsubstituted C6 to C20 aryl group, or a substituted or unsubstituted C3 to C20 heteroaryl group,
R^{3a} and R^{3b} are each independently hydrogen or a C1 to C10 alkyl group, and
* denotes a linking point within Chemical Formula 2, 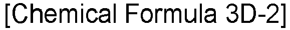 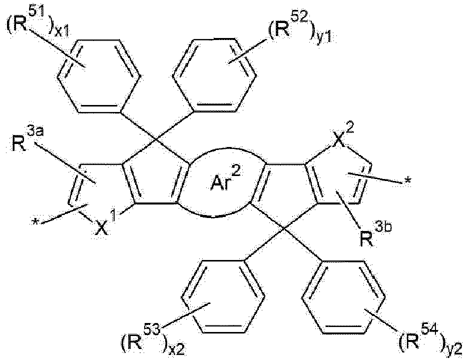
wherein, in Chemical Formula 3D-2,
Ar² is a substituted or unsubstituted C6 to C30 arene group; a substituted or unsubstituted C3 to C30 heterocyclic group including at least one of N, O, S, Se, Te, or Si; a fused ring thereof; or any combination thereof,
X¹ and X² are each independently S, Se, or Te,
R⁵¹, R⁵², R⁵³, and R⁵⁴ are each independently hydrogen, deuterium, a halogen, a substituted or unsubstituted C1 to C20 alkyl group, a substituted or unsubstituted C1 to C20 alkoxy group, a substituted or unsubstituted C6 to C20 aryl group or a substituted or unsubstituted C2 to C20 heteroaryl group,
x1, y1, x2, and y2 are each independently an integer of 0 to 5,
R^{3a} and R^{3b} are each independently hydrogen or a C1 to C10 alkyl group, and
* denotes a linking point within Chemical Formula 2, 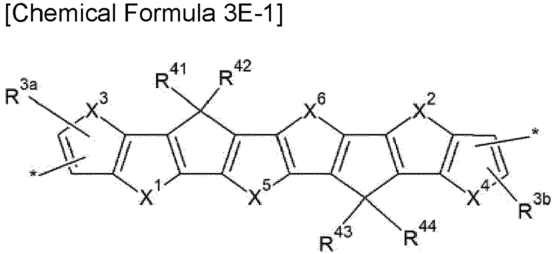
wherein, in Chemical Formula 3E-1,
X¹, X², X³, X⁴, X⁵, and X⁶ are each independently S, Se, or Te,
R⁴¹, R⁴², R⁴³, and R⁴⁴ are each independently a substituted or unsubstituted C1 to C30 alkyl group, a substituted or unsubstituted C1 to C30 alkoxy group, a substituted or unsubstituted C6 to C20 aryl group, or a substituted or unsubstituted C3 to C20 heteroaryl group,
R^{3a} and R^{3b} are each independently hydrogen or a C1 to C10 alkyl group, and
* denotes a linking point within Chemical Formula 2, 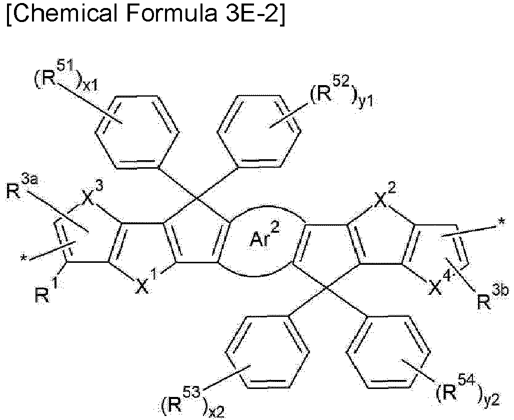
wherein, in Chemical Formula 3E-2,
Ar² is a substituted or unsubstituted C6 to C30 arene group; a substituted or unsubstituted C3 to C30 heterocyclic group including at least one of N, O, S, Se, Te, or Si; a fused ring thereof; or any combination thereof,
X¹, X², X³, and X⁴ are each independently S, Se, or Te,
R⁵¹, R⁵², R⁵³, and R⁵⁴ are each independently hydrogen, deuterium, a halogen, a substituted or unsubstituted C1 to C20 alkyl group, a substituted or unsubstituted C1 to C20 alkoxy group, a substituted or unsubstituted C6 to C20 aryl group or a substituted or unsubstituted C2 to C20 heteroaryl group,
x1, y1, x2, and y2 are each independently an integer of 0 to 5,
R¹, R^{3a} and R^{3b} are each independently hydrogen or a C1 to C10 alkyl group, and
* denotes a linking point within Chemical Formula 2; and/or
wherein in Chemical Formula 2, D² and D³ are each independently one moiety of moieties represented by Chemical Formula 4A to 4J of Group 1: 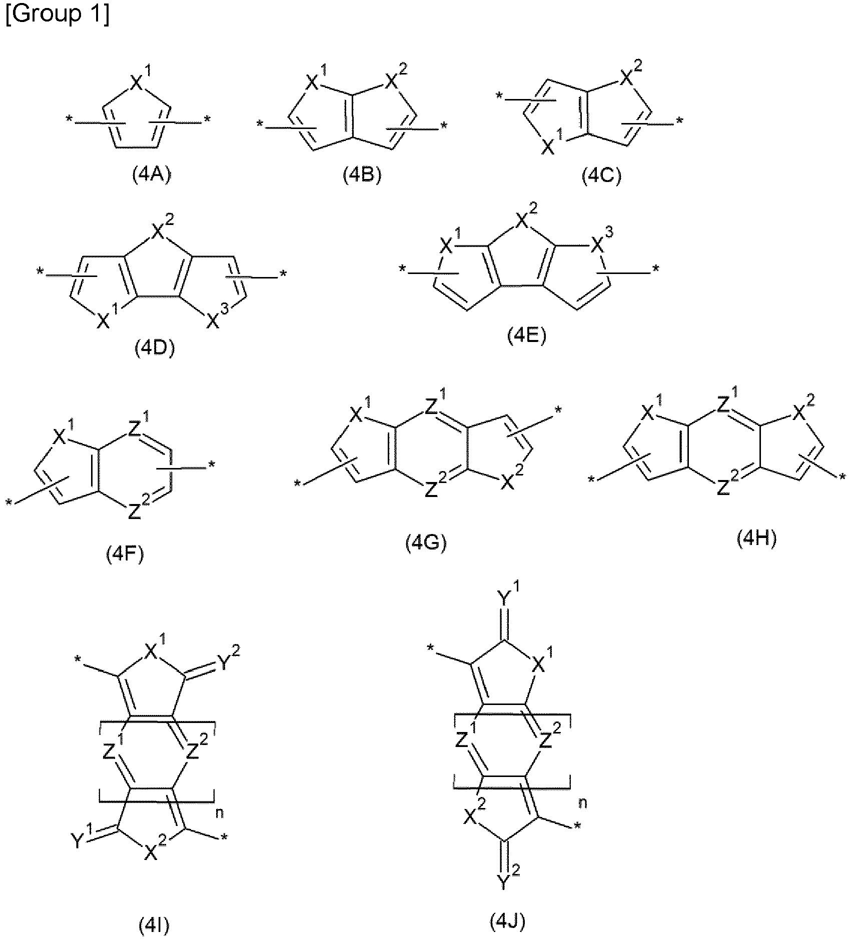
wherein, in Group 1,
X¹ to X³ are each independently S, Se, Te, S(=O), S(=O₂), NR^{a}, SiR^{d}R^{e}, or GeR^{f}R^{g}, wherein R^{a}, R^{b}, R^{c}, R^{d}, R^{e}, R^{f}, R^{g}, R^{h}, and Rⁱ are each independently hydrogen, deuterium, a C1 to C20 alkyl group, a C1 to C20 alkoxy group, a C1 to C20 haloalkyl group, a C6 to C20 aryl group, a C3 to C20 heteroaryl group, a halogen, a cyano group, or any combination thereof,
Z¹ and Z² are each independently N or CR^{x}, wherein R^{x} is hydrogen, deuterium, a C1 to C10 alkyl group, a C1 to C10 haloalkyl group, a -SiH₃ group, a C1 to C10 alkylsilyl group, a -NH₂ group, a C1 to C10 alkylamine group, a C6 to C12 aryl group, a C3 to C12 heteroaryl group, a halogen, a cyano group, or any combination thereof,
Y¹ and Y² are each independently O, S, Se, or Te,
n is 0 or 1,
* denotes a linking point within Chemical Formula 2, and
at least one hydrogen of each aromatic or heteroaromatic ring is hydrogen or is replaced by deuterium, a halogen, a cyano group, a C1 to C30 alkyl group, a C1 to C30 alkoxy group, a C1 to C30 haloalkyl group, a C6 to C30 aryl group, a C6 to C30 aryloxy group, a -SiH₃ group, or a C1 to C30 alkylsilyl group.

8. The infrared absorption composition of any of claims 1-7, wherein A¹ and A² are each independently an electron accepting moiety represented by any one of Chemical Formulas 6A to 6F: 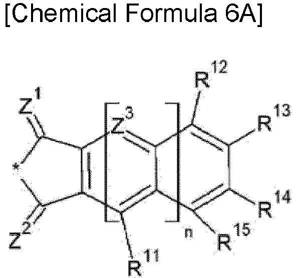
wherein, in Chemical Formula 6A,
Z¹ and Z² are each independently O, S, Se, Te, or CR^{a}R^{b}, wherein R^{a} and R^{b} are each independently hydrogen, a substituted or unsubstituted C1 to C10 alkyl group, or a cyano group,
Z³ is N or CR^{c}, wherein R^{c} is hydrogen, deuterium, or a substituted or unsubstituted C1 to C10 alkyl group,
R¹¹, R¹², R¹³, R¹⁴, and R¹⁵ are the same or different and are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), a cyano-containing group, or any combination thereof, and R¹², R¹³, R¹⁴, and R¹⁵ are each independently present or at least one pair of R¹² and R¹³ and R¹⁴ and R¹⁵ is linked to each other to provide a fused aromatic ring,
n is 0 or 1, and
* denotes a linking point within Chemical Formula 2, 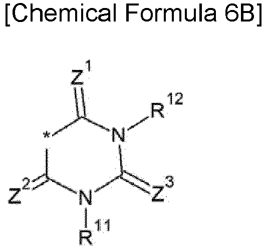
wherein, in Chemical Formula 6B,
Z¹ and Z² are each independently O, S, Se, Te, or CR^{a}R^{b}, wherein R^{a} and R^{b} are each independently hydrogen, a substituted or unsubstituted C1 to C10 alkyl group, or a cyano group,
Z³ is O, S, Se, Te, or C(R^{a})(CN), wherein R^{a} is hydrogen, a cyano group (-CN), or a C1 to C10 alkyl group,
R¹¹ and R¹² are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* denotes a linking point within Chemical Formula 2, 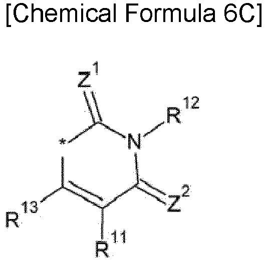
wherein, in Chemical Formula 6C,
Z¹ and Z² are each independently O, S, Se, Te, or CR^{a}R^{b}, wherein R^{a} and R^{b} are each independently hydrogen, a substituted or unsubstituted C1 to C10 alkyl group, or a cyano group,
R¹¹, R¹², and R¹³ are same or different from each other and are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group (-CN), or any combination thereof, and
* denotes a linking point within Chemical Formula 2, 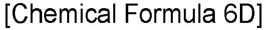 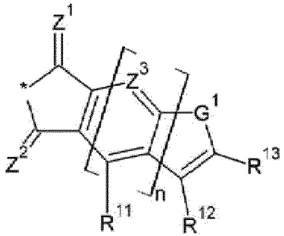
wherein, in Chemical Formula 6D,
Z¹ and Z² are each independently O, S, Se, Te, or CR^{a}R^{b}, wherein R^{a} and R^{b} are each independently hydrogen, a substituted or unsubstituted C1 to C10 alkyl group, or a cyano group,
Z³ is N or CR^{c}, wherein R^{c} is hydrogen or a substituted or unsubstituted C1 to C10 alkyl group,
G¹ is O, S, Se, Te, SiR^{x}R^{y}, or GeR^{z}R^{w}, wherein R^{x}, R^{y}, R^{z}, and R^{w} are same or different from each other and are each independently hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C2 to C20 heteroaryl group,
R¹¹, R¹², and R¹³ are same or different from each other and are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof, and R¹² and R¹³ are each independently present or are linked to each other to provide a fused aromatic ring,
n is 0 or 1, and
* denotes a linking point within Chemical Formula 2, 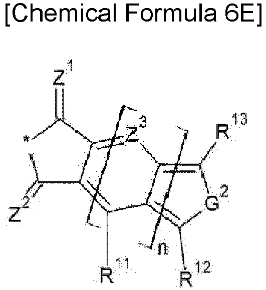
wherein, in Chemical Formula 6E,
Z¹ and Z² are each independently O, S, Se, Te, or CR^{a}R^{b}, wherein R^{a} and R^{b} are each independently hydrogen, a substituted or unsubstituted C1 to C10 alkyl group, or a cyano group,
Z³ is N or CR^{c}, wherein R^{c} is hydrogen or a substituted or unsubstituted C1 to C10 alkyl group,
G² is O, S, Se, Te, SiR^{x}R^{y}, or GeR^{z}R^{w}, wherein R^{x}, R^{y}, R^{z}, and R^{w} are same or different from each other and are each independently hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C2 to C20 heteroaryl group,
R¹¹, R¹², and R¹³ are same or different from each other and are each independently hydrogen, deuterium, a substituted or unsubstituted C1 to C30 alkyl group, a substituted or unsubstituted C1 to C30 alkoxy group, a substituted or unsubstituted C6 to C30 aryl group, a substituted or unsubstituted C4 to C30 heteroaryl group, a halogen, a cyano group, a cyano-containing group, or any combination thereof,
n is 0 or 1, and
* denotes a linking point within Chemical Formula 2, 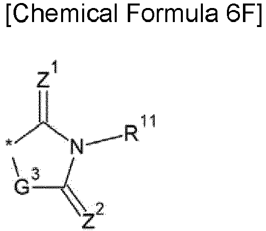
wherein, in Chemical Formula 6F,
Z¹ and Z² are each independently O, S, Se, Te, or CR^{a}R^{b}, wherein R^{a} and R^{b} are each independently hydrogen, a substituted or unsubstituted C1 to C10 alkyl group, or a cyano group,
R¹¹ is hydrogen, deuterium, a substituted or unsubstituted C1 to C30 alkyl group, a substituted or unsubstituted C6 to C30 aryl group, a substituted or unsubstituted C4 to C30 heteroaryl group, a halogen, a cyano group(-CN), a cyano-containing group, or any combination thereof,
G³ is O, S, Se, Te, SiR^{x}R^{y}, or GeR^{z}R^{w}, wherein R^{x}, R^{y}, R^{z}, and R^{w} are same or different from each other and are each independently hydrogen, deuterium, a halogen, a substituted or unsubstituted C1 to C20 alkyl group, a substituted or unsubstituted C1 to C20 alkoxy group, a substituted or unsubstituted C6 to C20 aryl group, or a substituted or unsubstituted C2 to C20 heteroaryl group, and
* is a linking point within Chemical Formula 2; and/or
wherein a weight ratio of the p-type semiconductor compound to the n-type semiconductor compound is in a range of 1:0.1 to 1:10; and/or
wherein the infrared absorption composition is configured to exhibit a peak absorption wavelength in a wavelength region of 1000 nm to 3000 nm.

9. An infrared absorption film comprising the infrared absorption composition of any of claims 1-8;
preferably:
showing a face-on alignment structure in grazing incident small angle x-ray scattering (GISAXS) analysis; and/or
having a surface roughness of less than or equal to 2 nm in atomic force microscopy analysis.

10. A photoelectric device comprising a first electrode and a second electrode facing each other, and a photoactive layer between the first electrode and the second electrode, wherein the photoactive layer includes the infrared absorption composition of any of claims 1-8;
wherein:
an external quantum efficiency at -3V of the photoelectric device is between 10% and 100%; and/or
an external quantum efficiency at -3V of the photoelectric device is increased by at least 80% compared to a photoelectric device including a photoactive layer including the same p-type semiconductor compound and an n-type semiconductor compound of fullerene or a fullerene derivative.

11. A photoelectric device, comprising:
a first electrode and a second electrode facing each other;
a photoactive layer between the first electrode and the second electrode; and
a charge auxiliary layer between
the photoactive layer and the first electrode, or
the photoactive layer and the second electrode,
wherein at least one of the photoactive layer or the charge auxiliary layer includes the infrared absorption composition of any of claims 1-8.

12. A sensor comprising the infrared absorption composition of any of claims 1-8 or the photoelectric device of claims 10 or 11; or an electronic device comprising the photoelectric device of claims 10 or 11.

13. An image sensor, comprising:
a semiconductor substrate;
a first photoelectric device on the semiconductor substrate, the first photoelectric device configured to selectively absorb light in a first infrared wavelength region; and
an additional sensor configured to selectively absorb light in a separate wavelength region that is different from the first infrared wavelength region,
wherein the first photoelectric device includes the infrared absorption composition of any of claims 1-8;
preferably wherein
the additional sensor is an infrared light sensor at least partially embedded within the semiconductor substrate, and the separate wavelength region is a separate infrared wavelength region that is different from the first infrared wavelength region, and
the first photoelectric device and the infrared light sensor overlap in a vertical direction that is perpendicular to an upper surface of the semiconductor substrate;
preferably wherein
the additional sensor includes a plurality of photodiodes at least partially embedded within the semiconductor substrate, the plurality of photodiodes configured to selectively absorb light in separate visible wavelength regions, and
the first photoelectric device and the plurality of photodiodes overlap in a vertical direction that is perpendicular to an upper surface of the semiconductor substrate.

14. The image sensor of claim 13, wherein
the additional sensor includes at least one additional photoelectric device vertically stacked between the first photoelectric device and the semiconductor substrate, each separate photoelectric device of the at least one additional photoelectric device including a separate photoelectric conversion layer and configured to selectively absorb light in a separate, respective wavelength region that is different from the first infrared wavelength region;
and/or
wherein the first photoelectric device includes
a first electrode and a second electrode facing each other; and
a photoactive layer between the first electrode and the second electrode, wherein the photoactive layer includes the infrared absorption composition.

15. The image sensor of claims 13 or 14, wherein the first photoelectric device includes
a first electrode and a second electrode facing each other;
a photoactive layer between the first electrode and the second electrode; and
a charge auxiliary layer between
the photoactive layer and the first electrode, or
the photoactive layer and the second electrode,
wherein the charge auxiliary layer includes the infrared absorption composition.

## Patentansprüche

1. Infrarotabsorptionszusammensetzung, umfassend
eine p-Typ-Halbleiterverbindung, die eine erste Struktureinheit dargestellt durch die chemische Formel 1 und eine zweite Struktureinheit beinhaltend einen Elektronen abgebenden Teil beinhaltet; und
eine n-Typ-Halbleiterverbindung dargestellt durch die chemische Formel 2: 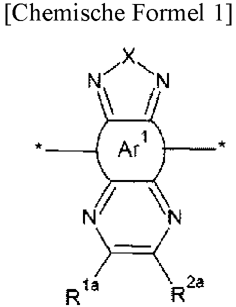
wobei in der chemischen Formel 1
Ar¹ ein substituierter oder unsubstituierter aromatischer C6- bis C30-Ring, ein substituierter oder unsubstituierter heteroaromatischer C6- bis C30-Ring oder eine beliebige Kombination davon ist,
X O, S, Se, Te, S(=O), S(=O₂), NR^{a}, CR^{b}R^{c}, SiR^{d}R^{e}, GeR^{f}R^{g}, CR^{h}=CRⁱ oder CR^{hh}=CRⁱⁱ ist, wobei R^{a}, R^{b}, R^{c}, R^{d}, R^{e}, R^{f}, R^{g}, R^{h} und Rⁱ jeweils unabhängig Wasserstoff, Deuterium, eine C1- bis C6-Alkylgruppe, eine C1- bis C6-Haloalkylgruppe, eine C6- bis C14-Arylgruppe, eine C3- bis C12-Heteroarylgruppe, ein Halogen, eine Cyanogruppe oder eine beliebige Kombination davon sind, und R^{hh} und Rⁱⁱ jeweils unabhängig eine C1- bis C6-Alkylengruppe oder eine C2- bis C6-Heteroalkylengruppe und miteinander verknüpft sind, um einen aromatischen oder heteroaromatischen Ring bereitzustellen,
R^{1a} und R^{2a} jeweils unabhängig eine substituierte oder unsubstituierte C6- bis C30-Arylgruppe oder eine substituierte oder unsubstituierte C3- bis C30-Heteroarylgruppe sind oder R^{1a} und R^{2a} miteinander verknüpft sind, um eine substituierte oder unsubstituierte C6- bis C30-Arengruppe oder eine substituierte oder unsubstituierte C3- bis C30-Heteroarengruppe bereitzustellen, und
* ein Verknüpfungspunkt innerhalb der p-Typ-Halbleiterverbindung ist, 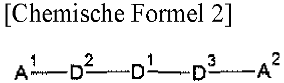
wobei in der chemischen Formel 2
D¹ ein erster Elektronen abgebender Teil mit einer beliebigen Struktur von Strukturen dargestellt durch die chemischen Formeln 3A bis 3E ist,
D² und D³ jeweils unabhängig eine Einfachbindung oder ein zweiter Elektronen abgebender Teil sind,
A¹ und A² jeweils unabhängig ein Elektronen annehmender Teil einer substituierten oder unsubstituierten C6- bis C30-Kohlenwasserstoffringgruppe mit zumindest einer funktionellen Gruppe von C=O, C=S, C=Se, C=Te oder C=C(CN)₂; einer substituierten oder unsubstituierten heterocyclischen C2- bis C30-Gruppe mit zumindest einer funktionellen Gruppe von C=O, C=S, C=Se, C=Te oder C=C(CN)₂; oder ein kondensierter Ring davon sind, 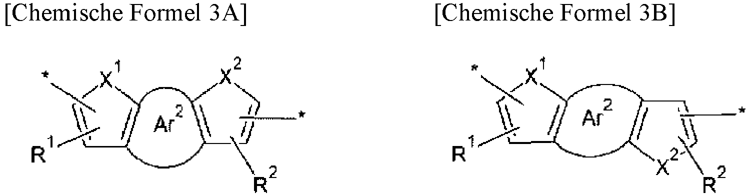 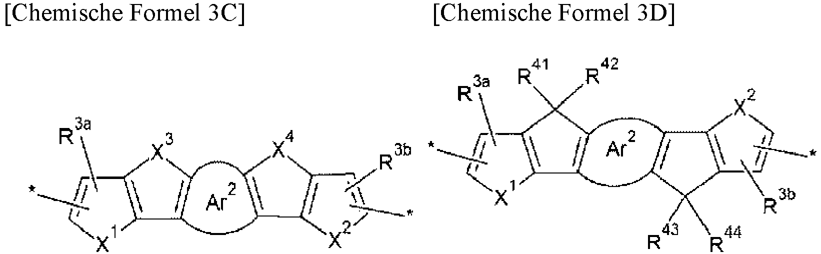 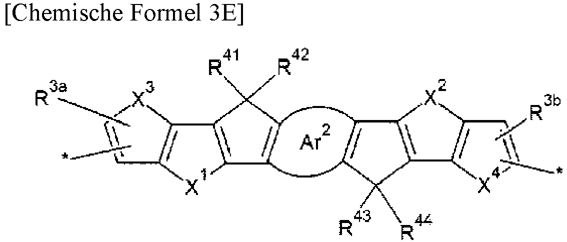
wobei in den chemischen Formeln 3A bis 3E
Ar² eine substituierte oder unsubstituierte C6- bis C30-Arengruppe; eine substituierte oder unsubstituierte heterocyclische C3- bis C30-Gruppe, die zumindest eines von N, O, S, Se, Te oder Si beinhaltet; ein kondensierter Ring davon; oder eine beliebige Kombination davon ist,
X¹, X², X³ und X⁴ jeweils unabhängig S, Se oder Te sind,
R⁴¹, R⁴², R⁴³ und R⁴⁴ jeweils unabhängig eine substituierte oder unsubstituierte C1- bis C30-Alkylgruppe, eine substituierte oder unsubstituierte C1- bis C30-Alkoxygruppe, eine substituierte oder unsubstituierte C6- bis C20-Arylgruppe oder eine substituierte oder unsubstituierte C3- bis C20-Heteroarylgruppe sind,
R¹, R², R^{3a} und R^{3b} jeweils unabhängig Wasserstoff oder eine C1- bis C10-Alkylgruppe sind, und
* einen Verknüpfungspunkt innerhalb der chemischen Formel 2 bezeichnet.

2. Infrarotabsorptionszusammensetzung nach Anspruch 1, wobei in der chemischen Formel 1 Ar¹ ein Benzolring, ein substituiertes oder unsubstituiertes Naphthalin, ein substituierter oder unsubstituierter Anthracenring, ein substituierter oder unsubstituierter Phenanthrenring, ein substituierter oder unsubstituierter Tetracenring, ein substituierter oder unsubstituierter Pyrenring, ein substituierter oder unsubstituierter Chinolinring, ein substituierter oder unsubstituierter Isochinolinring, ein substituierter oder unsubstituierter Chinoxalinring, ein substituierter oder unsubstituierter Chinazolinring oder ein substituierter oder unsubstituierter Phenanthrolinring ist;
wobei bevorzugt in der chemischen Formel 1 Ar¹ ein Teil von Teilen dargestellt durch die chemische Formel 1A-1 oder die chemische Formel 1A-2 ist; 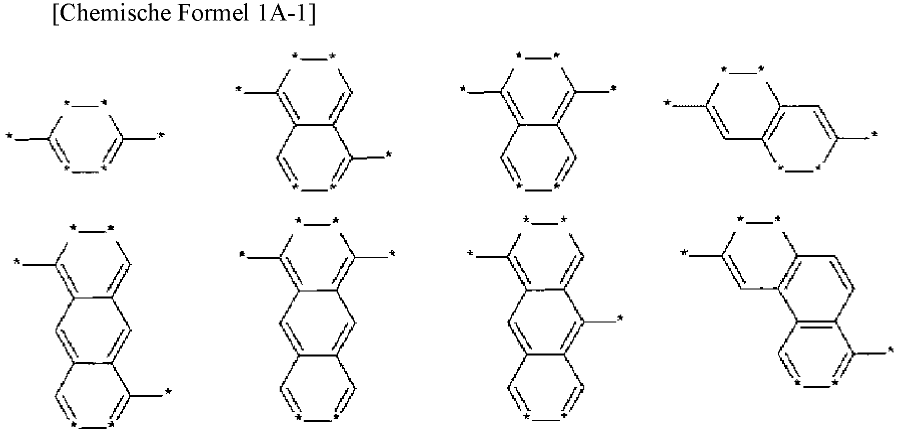
wobei in der chemischen Formel 1A-1
zumindest ein Wasserstoffatom von jedem aromatischen Ring Wasserstoff ist oder durch Deuterium, ein Halogen, eine Cyanogruppe, eine C1- bis C10-Alkylgruppe, eine C1- bis C10-Haloalkylgruppe, eine -SiH₃-Gruppe oder eine C1- bis C10-Alkylsilylgruppe ersetzt ist, und
benachbarte Paare von * innerhalb von zumindest einem aromatischen Ring Verknüpfungspunkte mit einem N-X-N-haltigen Ring und einem Pyrazinring der chemischen Formel 1 sind; 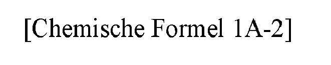 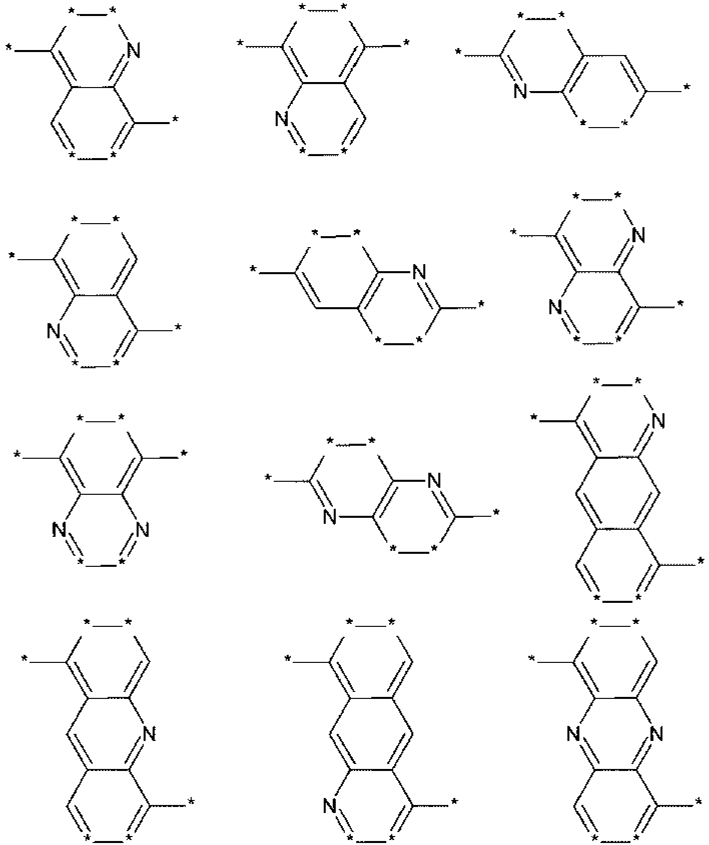
wobei in der chemischen Formel 1A-2
zumindest ein Wasserstoffatom von jedem aromatischen oder heteroaromatischen Ring Wasserstoff ist oder durch Deuterium, ein Halogen, eine Cyanogruppe, eine C1- bis C10-Alkylgruppe, eine C1- bis C10-Haloalkylgruppe, eine -SiH₃-Gruppe oder eine C1- bis C10-Alkylsilylgruppe ersetzt ist, und
benachbarte Paare von * innerhalb von zumindest einem aromatischen oder heteroaromatischen Ring Verknüpfungspunkte mit einem N-X-N-haltigen Ring und einem Pyrazinring der chemischen Formel 1 ist.

3. Infrarotabsorptionszusammensetzung nach Anspruch 1 oder 2, wobei in der chemischen Formel 1
R^{1a} und R^{2a} miteinander verknüpft sind, und
die substituierte oder unsubstituierte C6- bis C30-Arengruppe und die substituierte oder unsubstituierte C3- bis C30-Heteroarengruppe, die durch Verknüpfen von R^{1a} und R^{2a} miteinander gebildet sind, ein substituierter oder unsubstituierter Benzolring, ein substituierter oder unsubstituierter Naphthalinring, ein substituierter oder unsubstituierter Acenaphthenring, ein substituierter oder unsubstituierter Anthracenring, ein substituierter oder unsubstituierter Phenanthrenring, ein substituierter oder unsubstituierter Tetracenring oder ein substituierter oder unsubstituierter Pyrenring, ein substituierter oder unsubstituierter Chinolinring, ein substituierter oder unsubstituierter Isochinolinring, ein substituierter oder unsubstituierter Chinoxalinring, ein substituierter oder unsubstituierter Chinazolinring, ein substituierter oder unsubstituierter Phenanthrolinring, ein substituierter oder unsubstituierter Pyrimidinring oder ein substituierter oder unsubstituierter Benzodithiophenring sind; oder
wobei in der chemischen Formel 1
R^{1a} und R^{2a} miteinander verknüpft sind, und
die substituierte oder unsubstituierte C6- bis C30-Arengruppe und die substituierte oder unsubstituierte C3- bis C30-Heteroarengruppe, die durch Verknüpfen von R^{1a} und R^{2a} miteinander gebildet sind, ein Teil von Teilen sind, die durch die chemischen Formeln 1B-1 und 1B-2 dargestellt sind: 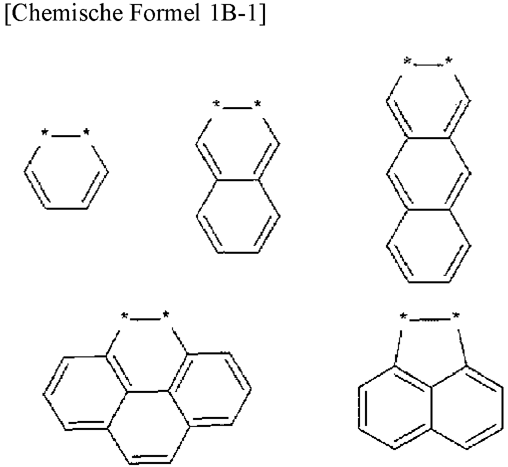
wobei in der chemischen Formel 1B-1
zumindest ein Wasserstoff von jedem aromatischen Ring Wasserstoff ist oder durch ein Halogen, eine Cyanogruppe, eine C1- bis C30-Alkylgruppe, eine C1- bis C30-Alkoxygruppe, eine C1- bis C30-Haloalkylgruppe, eine -SiH₃-Gruppe, eine C1- bis C30-Alkylsilylgruppe, eine C6-bis C30-Arylgruppe, eine C6- bis C30-Aryloxygruppe oder eine C3- bis C30-Heteroarylgruppe ersetzt sind, und
jedes * ein Punkt ist, der an einen Pyrazinring der chemischen Formel 1 gebunden ist, 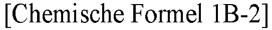 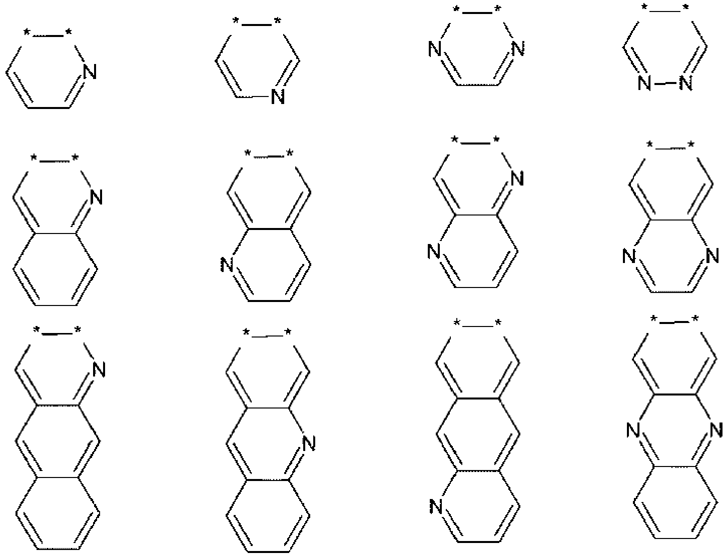
wobei in der chemischen Formel 1B-2
zumindest ein Wasserstoff von jedem aromatischen oder heteroaromatischen Ring Wasserstoff ist oder durch ein Halogen, eine Cyanogruppe, eine C1- bis C30-Alkylgruppe, eine C1- bis C30-Alkoxygruppe, eine C1- bis C30-Haloalkylgruppe, eine -SiH₃-Gruppe, eine C1- bis C30-Alkylsilylgruppe, eine C6- bis C30-Arylgruppe, eine C6- bis C30-Aryloxygruppe oder eine C3- bis C30-Heteroarylgruppe ersetzt ist, und
jedes * ein Punkt ist, der an einen Pyrazinring der chemischen Formel 1 gebunden ist; oder
wobei in der chemischen Formel 1
R^{1a} und R^{2a} miteinander verknüpft sind, und
die substituierte oder unsubstituierte C6- bis C30-Arengruppe und die substituierte oder unsubstituierte C3- bis C30-Heteroarengruppe, die durch Verknüpfen von R^{1a} und R^{2a} miteinander gebildet sind, jeweils unabhängig ein Teil von Teilen mit einem aromatischen oder heteroaromatischen Ring sind und durch die chemische Formel 1B-3 oder 1B-4 dargestellt sind: 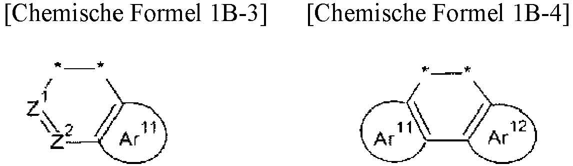
wobei in den chemischen Formeln 1B-3 und 1B-4
Ar¹¹ und Ar¹² jeweils unabhängig eine substituierte oder unsubstituierte C6- bis C30-Arengruppe oder eine substituierte oder unsubstituierte C3- bis C30-Heteroarengruppe sind,
wobei in der chemischen Formel 1B-3
Z¹ und Z² jeweils unabhängig N oder CR^{x} sind, wobei R^{x} Wasserstoff, Deuterium, eine C1- bis C10-Alkylgruppe, eine C1- bis C10-Haloalkylgruppe, eine -SiH₃-Gruppe, eine C1- bis C10-Alkylsilylgruppe, eine -NH₂-Gruppe, eine C1- bis C10-Alkylamingruppe, eine C6- bis C10-Arylamingruppe, eine C6- bis C14-Arylgruppe, eine C3- bis C12-Heteroarylgruppe, ein Halogen, eine Cyanogruppe oder eine beliebige Kombination davon ist, und
jedes * innerhalb des aromatischen oder heteroaromatischen Rings ein Punkt ist, der an einen Pyrazinring der chemischen Formel 1 gebunden ist;
wobei bevorzugt der Teil dargestellt durch die chemische Formel 1B-3 durch die chemische Formel 1B-3-1 dargestellt ist, oder wobei der Teil dargestellt durch die chemische Formel 1B-4 durch die chemische Formel 1B-4-1 dargestellt ist: 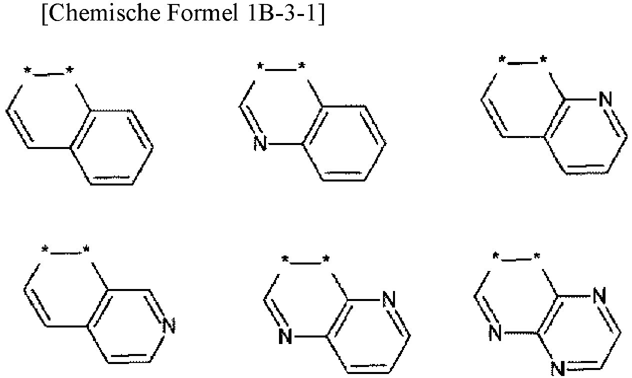
wobei in der chemischen Formel 1B-3-1
zumindest ein Wasserstoff von jedem aromatischen oder heteroaromatischen Ring Wasserstoff ist oder durch ein Halogen, eine Cyanogruppe, eine C1- bis C30-Alkylgruppe, eine C1- bis C30-Alkoxygruppe, eine C1- bis C30-Haloalkylgruppe, eine -SiH₃-Gruppe, eine C1- bis C30-Alkylsilylgruppe, eine C6- bis C30-Arylgruppe, eine C6- bis C30-Aryloxygruppe oder eine C3- bis C30-Heteroarylgruppe ersetzt ist, und
jedes * innerhalb des aromatischen oder heteroaromatischen Rings ein Punkt ist, der an den Pyrazinring der chemischen Formel 1 gebunden ist; 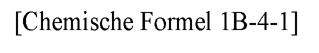 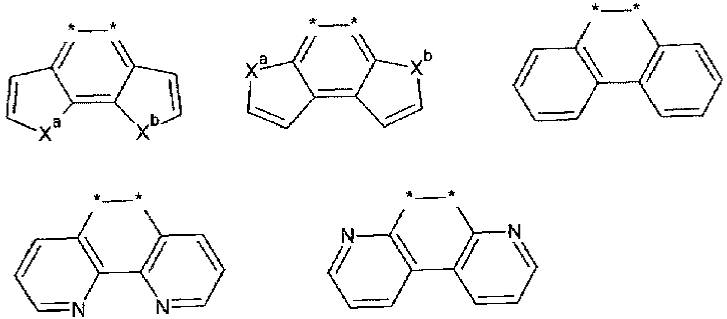
wobei in der chemischen Formel 1B-4-1
zumindest ein Wasserstoff von jedem aromatischen oder heteroaromatischen Ring Wasserstoff ist oder durch ein Halogen, eine Cyanogruppe, eine C1- bis C30-Alkylgruppe, eine C1- bis C30-Alkoxygruppe, eine C1- bis C30-Haloalkylgruppe, eine -SiH₃-Gruppe, eine C1- bis C30-Alkylsilylgruppe, eine C6- bis C30-Arylgruppe, eine C6- bis C30-Aryloxygruppe oder eine C3- bis C30-Heteroarylgruppe ersetzt ist,
X^{a} und X^{b} jeweils unabhängig O, S, Se, Te, NR^{a}, SiR^{b}R^{c} oder GeR^{d}R^{e} sind, wobei R^{a}, R^{b}, R^{c}, R^{d} und R^{e} jeweils unabhängig Wasserstoff, ein Halogen, eine substituierte oder unsubstituierte C1- bis C10-Alkylgruppe oder eine substituierte oder unsubstituierte C6- bis C10-Arylgruppe sind, und
jedes * innerhalb des aromatischen Rings ein Punkt ist, der an den Pyrazinring der chemischen Formel 1 gebunden ist.

4. Infrarotabsorptionszusammensetzung nach einem der Ansprüche 1-3, wobei die erste Struktureinheit der p-Typ-Halbleiterverbindung durch die chemische Formel 1C dargestellt ist: 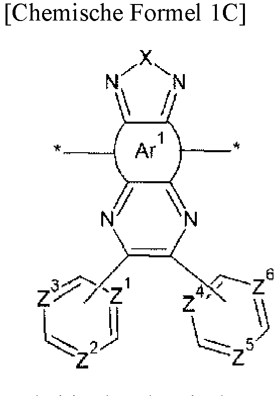
wobei in der chemischen Formel 1C
Ar¹ ein substituierter oder unsubstituierter aromatischer C6- bis C30-Ring, ein substituierter oder unsubstituierter heteroaromatischer C6- bis C30-Ring oder eine beliebige Kombination davon ist,
X O, S, Se, Te, S(=O), S(=O₂), NR^{a}, CR^{b}R^{c}, SiR^{d}R^{e}, GeR^{f}R^{g}, CR^{h}=CRⁱ oder CR^{hh}=CRⁱⁱ ist, wobei R^{a}, R^{b}, R^{c}, R^{d}, R^{e}, R^{f}, R^{g}, R^{h} und Rⁱ jeweils unabhängig Wasserstoff, Deuterium, eine C1- bis C6-Alkylgruppe, eine C1- bis C6-Haloalkylgruppe, eine C6- bis C14-Arylgruppe, eine C3- bis C12-Heteroarylgruppe, ein Halogen, eine Cyanogruppe oder eine beliebige Kombination davon sind, und R^{hh} und Rⁱⁱ jeweils unabhängig eine C1- bis C6-Alkylengruppe oder eine C2- bis C6-Heteroalkylengruppe und miteinander verknüpft sind, um einen aromatischen oder heteroaromatischen Ring bereitzustellen,
Z¹ bis Z⁶ jeweils unabhängig N oder CR^{x} sind, wobei R^{x} Wasserstoff, Deuterium, eine C1-bis C20-Alkylgruppe, eine C1- bis C20-Haloalkylgruppe, eine -SiH₃-Gruppe, eine C1- bis C20-Alkylsilylgruppe, eine -NH₂-Gruppe, eine C1- bis C20-Alkylamingruppe, eine C6- bis C12-Arylgruppe, eine C3- bis C12-Heteroarylgruppe, ein Halogen, eine Cyanogruppe oder eine beliebige Kombination davon sind,
zumindest ein Wasserstoff von jedem aromatischen oder heteroaromatischen Ring Wasserstoff ist oder durch Deuterium, ein Halogen, eine Cyanogruppe, eine C1- bis C30-Alkylgruppe, eine C1- bis C30-Alkoxygruppe, eine C1- bis C30-Haloalkylgruppe, eine C6- bis C30-Arylgruppe, eine C6- bis C30-Aryloxygruppe, eine -SiH₃-Gruppe oder eine C1- bis C30-Alkylsilylgruppe ersetzt sind, und
* ein Verknüpfungspunkt mit der p-Typ-Halbleiterverbindung ist; und/oder
wobei der Elektronen abgebende Teil, der in der zweiten Struktureinheit der p-Typ-Halbleiterverbindung enthalten ist, eine substituierte oder unsubstituierte C6- bis C30-Arengruppe, eine substituierte oder unsubstituierte zweiwertige heterocyclische C3- bis C30-Gruppe, die zumindest eines von N, O, S, Se, Te oder Si beinhaltet, ein kondensierter Ring davon oder eine beliebige Kombination davon ist, wobei bevorzugt der Elektronen abgebende Teil, der in der zweiten Struktureinheit der p-Typ-Halbleiterverbindung enthalten ist, zumindest einen Teil von Teilen dargestellt durch die chemischen Formeln 4A bis 4J der Gruppe 1 umfasst: 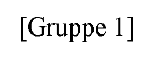 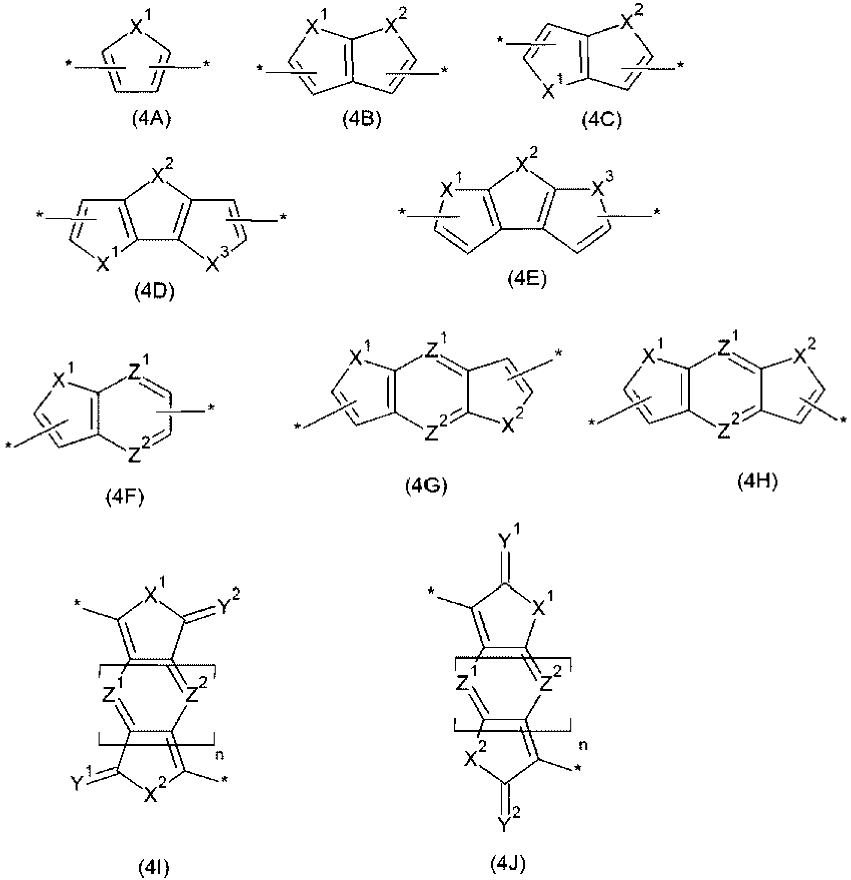
wobei in Gruppe 1
X¹ bis X³ jeweils unabhängig S, Se, Te, S(=O), S(=O₂), NR^{a}, SiR^{d}R^{e} oder GeR^{f}R^{g} sind, wobei R^{a}, R^{b}, R^{c}, R^{d}, R^{e}, R^{f} und R^{g} jeweils unabhängig Wasserstoff, Deuterium, eine C1- bis C20-Alkylgruppe, eine C1- bis C20-Alkoxygruppe, eine C1- bis C20-Haloalkylgruppe, eine C6- bis C20-Arylgruppe, eine C3- bis C20-Heteroarylgruppe, ein Halogen, eine Cyanogruppe oder eine beliebige Kombination davon sind,
Z¹ und Z² jeweils unabhängig N oder CR^{x} sind, wobei R^{x} Wasserstoff, Deuterium, eine C1- bis C10-Alkylgruppe, eine C1- bis C10-Haloalkylgruppe, eine -SiH₃-Gruppe, eine C1- bis C10-Alkylsilylgruppe, eine -NH₂-Gruppe, eine C1- bis C10-Alkylamingruppe, eine C6- bis C12-Arylgruppe, eine C3- bis C12-Heteroarylgruppe, ein Halogen, eine Cyanogruppe oder eine beliebige Kombination davon sind,
Y¹ und Y² jeweils unabhängig O, S, Se oder Te sind,
n 0 oder 1 ist, und
zumindest ein Wasserstoff von jedem aromatischen oder heteroaromatischen Ring Wasserstoff ist oder durch Deuterium, ein Halogen, eine Cyanogruppe, eine C1- bis C30-Alkylgruppe, eine C1- bis C30-Alkoxygruppe, eine C1- bis C30-Haloalkylgruppe, eine C6- bis C30-Arylgruppe, eine C6- bis C30-Aryloxygruppe, eine -SiH₃-Gruppe oder eine C1- bis C30-Alkylsilylgruppe ersetzt ist.

5. Infrarotabsorptionszusammensetzung nach einem der Ansprüche 1-4, wobei die p-Typ-Halbleiterverbindung ein Polymer ist, das 20 Mol-% bis 50 Mol-% der ersten Struktureinheit und 50 Mol-% bis 80 Mol-% der zweiten Struktureinheit beinhaltet; und/oder
wobei die p-Typ-Halbleiterverbindung konfiguriert ist, um eine Spitzenabsorptionswellenlänge in einer Wellenlängenbandbreite von 1000 nm bis 3000 nm aufzuzeigen; und/oder
wobei in den chemischen Formeln 3A bis 3C Ar² ein Teil mit einer Struktur von Strukturen dargestellt durch die chemischen Formeln 5A bis 5K der Gruppe 2 ist: 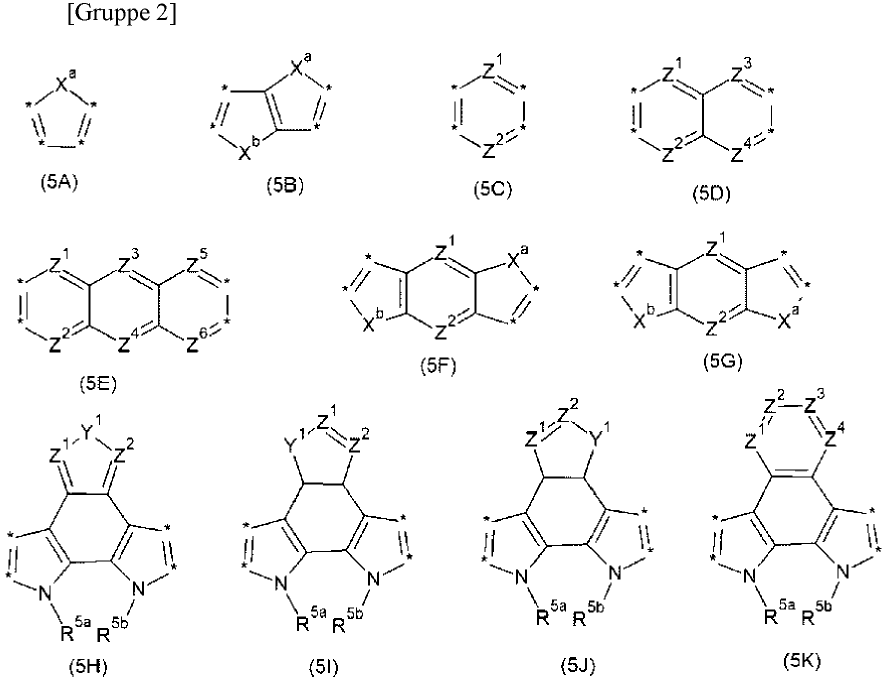
wobei in Gruppe 2
X^{a} und X^{b} jeweils unabhängig CR^{x}R^{y}, S, Se oder Te sind, wobei R^{x} und R^{y} jeweils unabhängig eine substituierte oder unsubstituierte C1- bis C30-Alkylgruppe, eine substituierte oder unsubstituierte C1- bis C30-Alkoxygruppe, eine substituierte oder unsubstituierte C6- bis C20-Arylgruppe oder eine substituierte oder unsubstituierte C3- bis C20-Heteroarylgruppe sind,
R^{5a} und R^{5b} jeweils unabhängig Wasserstoff, eine C1- bis C20-Alkylgruppe, eine C1- bis C20-Alkoxygruppe, eine C6- bis C10-Arylgruppe oder eine C2- bis C10-Heteroarylgruppe sind,
Y¹ CR^{p}R^{q}, NR^{r}, O, S, Se oder Te ist, wobei R^{p}, R^{q} und R^{r} jeweils unabhängig Wasserstoff oder eine C1- bis C20-Alkylgruppe sind, und
Z¹ bis Z⁶ jeweils unabhängig CR^{s} oder N sind, wobei R^{s} Wasserstoff oder eine C1- bis C20-Alkylgruppe ist.

6. Infrarotabsorptionszusammensetzung nach einem der Ansprüche 1-5, wobei in den chemischen Formeln 3D und 3E R⁴¹, R⁴², R⁴³ und R⁴⁴ jeweils unabhängig eine C1- bis C20-Alkylgruppe; eine C1- bis C20-Alkoxygruppe; eine C6- bis C20-Arylgruppe substituiert mit einer C1- bis C20-Alkylgruppe oder einer C1- bis C20-Alkoxygruppe; oder eine C3- bis C20-Heteroarylgruppe substituiert mit einer C1- bis C20-Alkylgruppe oder einer C1- bis C20-Alkoxygruppe sind; und/oder
wobei in den chemischen Formeln 3D und 3E R⁴¹, R⁴², R⁴³ und R⁴⁴ jeweils unabhängig eine substituierte oder unsubstituierte verzweigte C3- bis C30-Alkylgruppe oder eine substituierte oder unsubstituierte verzweigte C3- bis C30-Alkoxygruppe sind.

7. Infrarotabsorptionszusammensetzung nach einem der Ansprüche 1-6, wobei der erste Elektronen abgebende Teil ausgewählt aus den Strukturen dargestellt durch die chemischen Formeln 3A bis 3E ein Teil dargestellt durch die chemische Formel 3A-1, 3B-1, 3C-1, 3D-1, 3D-2, 3E-1 oder 3E-2 ist: 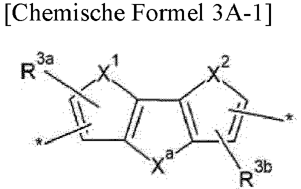
wobei in der chemischen Formel 3A-1
X^{a} CR^{x}R^{y}, S, Se oder Te ist, wobei R^{x} und R^{y} jeweils unabhängig eine substituierte oder unsubstituierte C1- bis C30-Alkylgruppe, eine substituierte oder unsubstituierte C1- bis C30-Alkoxygruppe, eine substituierte oder unsubstituierte C6- bis C20-Arylgruppe oder eine substituierte oder unsubstituierte C3- bis C20-Heteroarylgruppe sind,
X¹ und X² jeweils unabhängig S, Se oder Te sind,
R^{3a} und R^{3b} jeweils unabhängig Wasserstoff oder eine C1- bis C10-Alkylgruppe sind, und
* einen Verknüpfungspunkt innerhalb der chemischen Formel 2 bezeichnet, 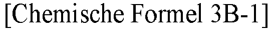 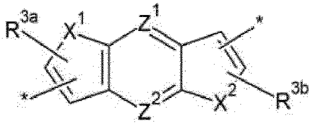
wobei in der chemischen Formel 3B-1
Z¹ und Z² jeweils unabhängig CR^{s} oder N sind, wobei R^{s} Wasserstoff oder eine C1- bis C20-Alkylgruppe ist,
X¹ und X² jeweils unabhängig S, Se oder Te sind,
R^{3a} und R^{3b} jeweils unabhängig Wasserstoff oder eine C1- bis C10-Alkylgruppe sind, und
* einen Verknüpfungspunkt innerhalb der chemischen Formel 2 bezeichnet, 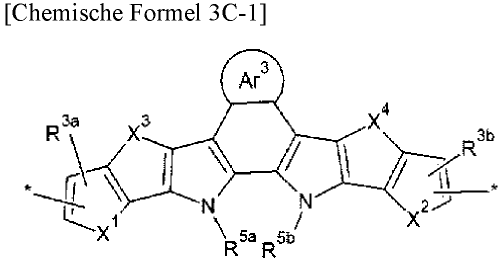
wobei in der chemischen Formel 3C-1
Ar³ ein Teil von Teilen mit einem Ring und dargestellt durch die chemische Formel 3C-1a ist,
X¹, X², X³ und X⁴ jeweils unabhängig S, Se oder Te sind,
R^{3a} und R^{3b} jeweils unabhängig Wasserstoff oder eine C1 bis C10 Alkylgruppe sind,
R^{5a} und R^{5b} sind jeweils unabhängig Wasserstoff, eine C1- bis C20-Alkylgruppe, eine C1-bis C20-Alkoxygruppe, eine C6- bis C10-Arylgruppe oder eine C2- bis C10-Heteroarylgruppe sind, und
* einen Verknüpfungspunkt innerhalb der chemischen Formel 2 bezeichnet, 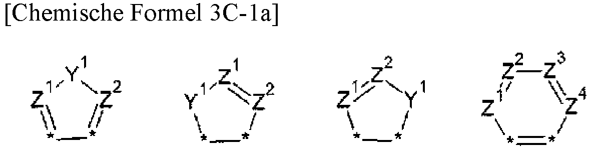
wobei in der chemischen Formel 3C-1a
Y¹ CR^{p}R^{q}, NR^{r}, O, S, Se oder Te ist, wobei R^{p}, R^{q} und R^{f} jeweils unabhängig Wasserstoff oder eine C1- bis C20-Alkylgruppe sind,
Z¹ bis Z⁴ jeweils unabhängig CR^{s} oder N sind, wobei R^{s} Wasserstoff oder eine C1- bis C20-Alkylgruppe ist, und
* innerhalb des Rings einen Verknüpfungspunkt mit der chemischen Formel 3C-1 bezeichnet, 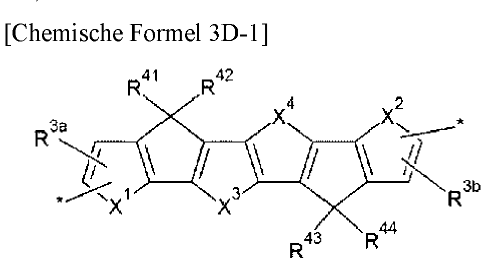
wobei in der chemischen Formel 3D-1
X¹, X², X³ und X⁴ jeweils unabhängig S, Se oder Te sind,
R⁴¹, R⁴², R⁴³ und R⁴⁴ jeweils unabhängig eine substituierte oder unsubstituierte C1- bis C30-Alkylgruppe, eine substituierte oder unsubstituierte C1- bis C30-Alkoxygruppe, eine substituierte oder unsubstituierte C6- bis C20-Arylgruppe oder eine substituierte oder unsubstituierte C3- bis C20-Heteroarylgruppe sind,
R^{3a} und R^{3b} jeweils unabhängig Wasserstoff oder eine C1- bis C10-Alkylgruppe sind, und
* einen Verknüpfungspunkt innerhalb der chemischen Formel 2 bezeichnet, 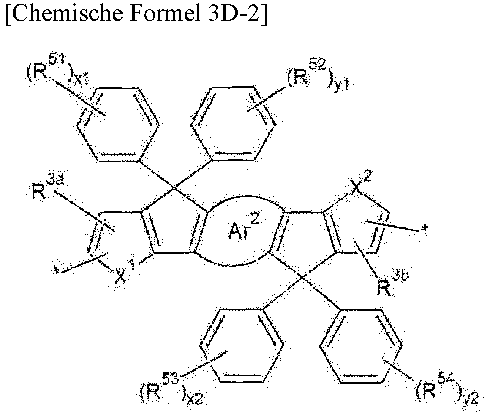
wobei in der chemischen Formel 3D-2
Ar² eine substituierte oder unsubstituierte C6- bis C30-Arengruppe; eine substituierte oder unsubstituierte heterocyclische C3- bis C30-Gruppe, die zumindest eines von N, O, S, Se, Te oder Si beinhaltet; ein kondensierter Ring davon; oder eine beliebige Kombination davon ist,
X¹ und X² jeweils unabhängig S, Se oder Te sind,
R⁵¹, R⁵², R⁵³ und R⁵⁴ jeweils unabhängig Wasserstoff, Deuterium, ein Halogen, eine substituierte oder unsubstituierte C1- bis C20-Alkylgruppe, eine substituierte oder unsubstituierte C1- bis C20-Alkoxygruppe, eine substituierte oder unsubstituierte C6- bis C20-Arylgruppe oder eine substituierte oder unsubstituierte C2- bis C20-Heteroarylgruppe sind,
x1, y1, x2 und y2 jeweils unabhängig eine ganze Zahl von 0 bis 5 sind,
R^{3a} und R^{3b} jeweils unabhängig Wasserstoff oder eine C1- bis C10-Alkylgruppe sind, und
* einen Verknüpfungspunkt innerhalb der chemischen Formel 2 bezeichnet, 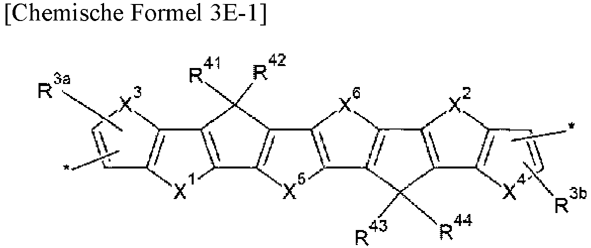
wobei in der chemischen Formel 3E-1
X¹, X², X³, X⁴, X⁵ und X⁶ jeweils unabhängig S, Se oder Te sind,
R⁴¹, R⁴², R⁴³ und R⁴⁴ jeweils unabhängig eine substituierte oder unsubstituierte C1- bis C30-Alkylgruppe, eine substituierte oder unsubstituierte C1- bis C30-Alkoxygruppe, eine substituierte oder unsubstituierte C6- bis C20-Arylgruppe oder eine substituierte oder unsubstituierte C3- bis C20-Heteroarylgruppe sind,
R^{3a} und R^{3b} jeweils unabhängig Wasserstoff oder eine C1- bis C10-Alkylgruppe sind, und
* einen Verknüpfungspunkt innerhalb der chemischen Formel 2 bezeichnet, 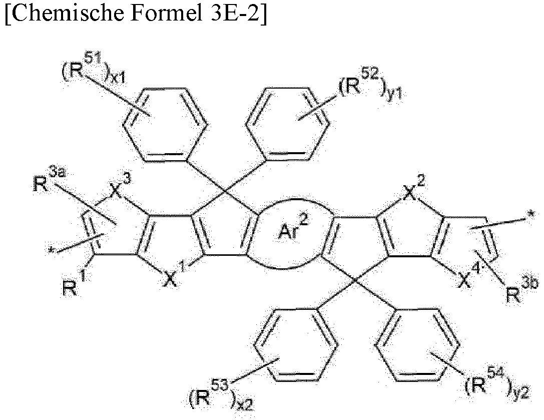
wobei in der chemischen Formel 3E-2
Ar² eine substituierte oder unsubstituierte C6- bis C30-Arengruppe; eine substituierte oder unsubstituierte heterocyclische C3- bis C30-Gruppe, die zumindest eines von N, O, S, Se, Te oder Si beinhaltet; ein kondensierter Ring davon; oder eine beliebige Kombination davon ist,
X¹, X², X³ und X⁴ jeweils unabhängig S, Se oder Te sind,
R⁵¹, R⁵², R⁵³ und R⁵⁴ jeweils unabhängig Wasserstoff, Deuterium, ein Halogen, eine substituierte oder unsubstituierte C1- bis C20-Alkylgruppe, eine substituierte oder unsubstituierte C1- bis C20-Alkoxygruppe, eine substituierte oder unsubstituierte C6- bis C20-Arylgruppe oder eine substituierte oder unsubstituierte C2- bis C20-Heteroarylgruppe sind,
x1, y1, x2 und y2 jeweils unabhängig eine ganze Zahl von 0 bis 5 sind,
R¹, R^{3a} und R^{3b} jeweils unabhängig Wasserstoff oder eine C1- bis C10-Alkylgruppe sind, und
* einen Verknüpfungspunkt innerhalb der chemischen Formel 2 bezeichnet; und/oder
wobei in der chemischen Formel 2 D² und D³ jeweils unabhängig ein Teil von Teilen dargestellt durch die chemischen Formeln 4A bis 4J der Gruppe 1 sind: 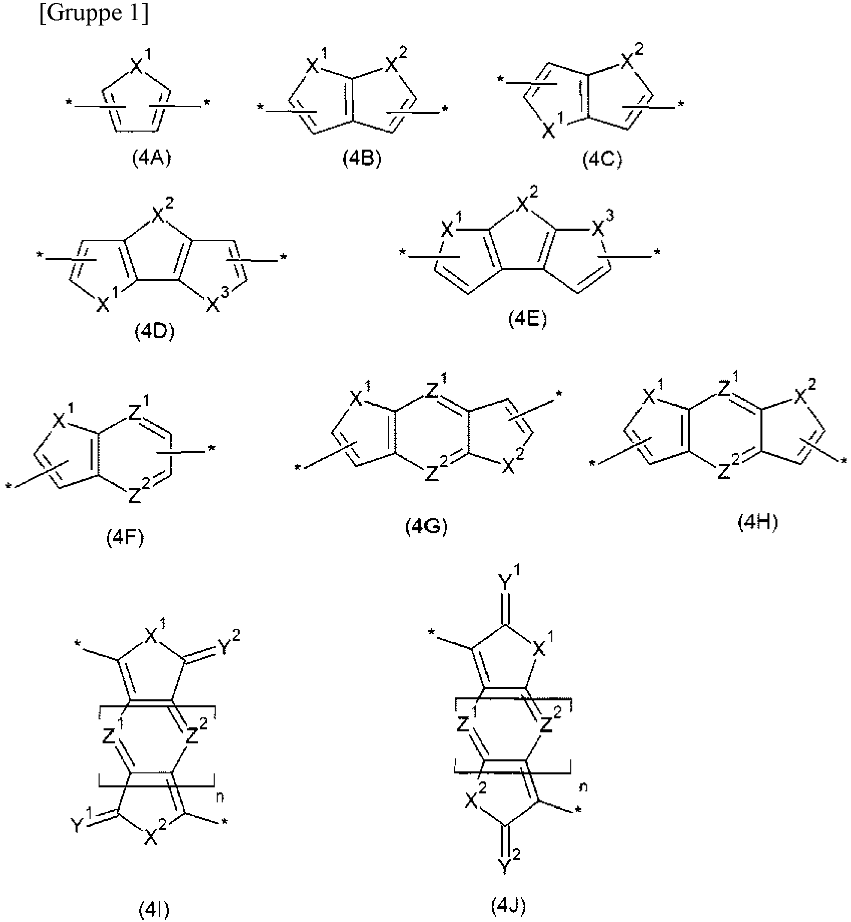
wobei in Gruppe 1
X¹ bis X³ jeweils unabhängig S, Se, Te, S(=O), S(=O₂), NR^{a}, SiR^{d}R^{e} oder GeR^{f}R^{g} sind, wobei R^{a}, R^{b}, R^{c}, R^{d}, R^{e}, R^{f}, R^{g}, R^{h} und Rⁱ jeweils unabhängig Wasserstoff, Deuterium, eine C1-bis C20-Alkylgruppe, eine C1- bis C20-Alkoxygruppe, eine C1- bis C20-Haloalkylgruppe, eine C6- bis C20-Arylgruppe, eine C3- bis C20-Heteroarylgruppe, ein Halogen, eine Cyanogruppe oder eine beliebige Kombination davon sind,
Z¹ und Z jeweils unabhängig N oder CR^{x} sind, wobei R^{x} Wasserstoff, Deuterium, eine C1-bis C10-Alkylgruppe, eine C1- bis C10-Haloalkylgruppe, eine -SiH₃-Gruppe, eine C1- bis C10-Alkylsilylgruppe, eine -NH₂-Gruppe, eine C1- bis C10-Alkylamingruppe, eine C6- bis C12-Arylgruppe, eine C3- bis C12-Heteroarylgruppe, ein Halogen, eine Cyanogruppe oder eine beliebige Kombination davon ist,
Y¹ und Y² jeweils unabhängig O, S, Se oder Te sind,
n 0 oder 1 ist,
* einen Verknüpfungspunkt innerhalb der chemischen Formel 2 bezeichnet, und
zumindest ein Wasserstoff von jedem aromatischen oder heteroaromatischen Ring Wasserstoff ist oder durch Deuterium, ein Halogen, eine Cyanogruppe, eine C1- bis C30-Alkylgruppe, eine C1- bis C30-Alkoxygruppe, eine C1- bis C30-Haloalkylgruppe, eine C6- bis C30-Arylgruppe, eine C6- bis C30-Aryloxygruppe, eine -SiH₃-Gruppe oder eine C1- bis C30-Alkylsilylgruppe ersetzt ist.

8. Infrarotabsorptionszusammensetzung nach einem der Ansprüche 1-7, wobei A¹ und A² jeweils unabhängig ein Elektronen aufnehmender Teil dargestellt durch eine der chemischen Formeln 6A bis 6F sind: 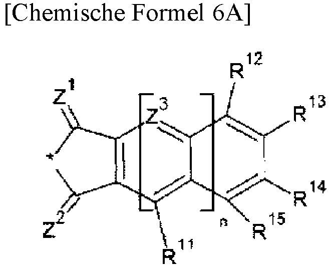
wobei in der chemischen Formel 6A
Z¹ und Z² jeweils unabhängig O, S, Se, Te oder CR^{a}R^{b} sind, wobei R^{a} und R^{b} jeweils unabhängig Wasserstoff, eine substituierte oder unsubstituierte C1-bis C10-Alkylgruppe oder eine Cyanogruppe sind,
Z³ N oder CR^{c} ist, wobei R^{c} Wasserstoff, Deuterium oder eine substituierte oder unsubstituierte C1- bis C10-Alkylgruppe ist,
R¹¹, R¹², R¹³, R¹⁴ und R¹⁵ gleich oder verschieden sind und jeweils unabhängig Wasserstoff, Deuterium, eine substituierte oder unsubstituierte C1- bis C30-Alkylgruppe, eine substituierte oder unsubstituierte C1- bis C30-Alkoxygruppe, eine substituierte oder unsubstituierte C6- bis C30-Arylgruppe, eine substituierte oder unsubstituierte C4- bis C30-Heteroarylgruppe, ein Halogen, eine Cyanogruppe (-CN), eine cyanohaltige Gruppe oder eine beliebige Kombination davon sind, und R¹², R¹³, R¹⁴ und R¹⁵ jeweils unabhängig vorhanden sind oder zumindest ein Paar von R¹² und R¹³ und R¹⁴ und R¹⁵ miteinander verknüpft ist, um einen kondensierten aromatischen Ring bereitzustellen,
n 0 oder 1 ist, und
* einen Verknüpfungspunkt innerhalb der chemischen Formel 2 bezeichnet, 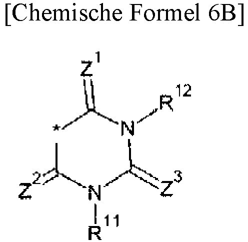
wobei in der chemischen Formel 6B
Z¹ und Z² jeweils unabhängig O, S, Se, Te oder CR^{a}R^{b} sind, wobei R^{a} und R^{b} jeweils unabhängig Wasserstoff, eine substituierte oder unsubstituierte C1-bis C10-Alkylgruppe oder eine Cyanogruppe sind,
Z³ O, S, Se, Te oder C(R^{a})(CN) ist, wobei R^{a} Wasserstoff, eine Cyanogruppe (-CN) oder eine C1- bis C10-Alkylgruppe ist,
R¹¹ und R¹² jeweils unabhängig Wasserstoff, Deuterium, eine substituierte oder unsubstituierte C1- bis C30-Alkylgruppe, eine substituierte oder unsubstituierte C1- bis C30-Alkoxygruppe, eine substituierte oder unsubstituierte C6- bis C30-Arylgruppe, eine substituierte oder unsubstituierte C4- bis C30-Heteroarylgruppe, ein Halogen, eine Cyanogruppe (-CN) oder eine beliebige Kombination davon sind, und
* einen Verknüpfungspunkt innerhalb der chemischen Formel 2 bezeichnet, 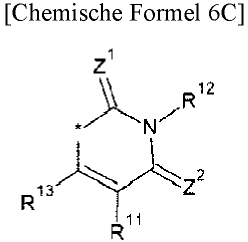
wobei in der chemischen Formel 6C
Z¹ und Z² jeweils unabhängig O, S, Se, Te oder CR^{a}R^{b} sind, wobei R^{a} und R^{b} jeweils unabhängig Wasserstoff, eine substituierte oder unsubstituierte C1-bis C10-Alkylgruppe oder eine Cyanogruppe sind,
R¹¹, R¹² und R¹³ gleich oder verschieden voneinander sind und jeweils unabhängig Wasserstoff, Deuterium, eine substituierte oder unsubstituierte C1- bis C30-Alkylgruppe, eine substituierte oder unsubstituierte C1- bis C30-Alkoxygruppe, eine substituierte oder unsubstituierte C6- bis C30-Arylgruppe, eine substituierte oder unsubstituierte C4- bis C30-Heteroarylgruppe, ein Halogen, eine Cyanogruppe (-CN) oder eine beliebige Kombination davon sind, und
* einen Verknüpfungspunkt innerhalb der chemischen Formel 2 bezeichnet, 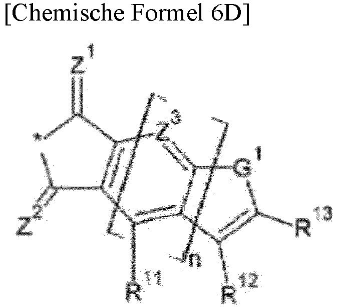
wobei in der chemischen Formel 6D
Z¹ und Z² jeweils unabhängig O, S, Se, Te oder CR^{a}R^{b} sind, wobei R^{a} und R^{b} jeweils unabhängig Wasserstoff, eine substituierte oder unsubstituierte C1-bis C10-Alkylgruppe oder eine Cyanogruppe sind,
Z³ N oder CR^{c} ist, wobei R^{c} Wasserstoff oder eine substituierte oder unsubstituierte C1-bis C10-Alkylgruppe ist,
G¹ O, S, Se, Te, SiR^{x}R^{y} oder GeR^{z}R^{w} ist, wobei R^{x}, R^{y}, R^{z} und R^{w} gleich oder verschieden voneinander sind und jeweils unabhängig Wasserstoff, Deuterium, ein Halogen, eine substituierte oder unsubstituierte C1- bis C20-Alkylgruppe, eine substituierte oder unsubstituierte C1- bis C20-Alkoxygruppe, eine substituierte oder unsubstituierte C6- bis C20-Arylgruppe oder eine substituierte oder unsubstituierte C2- bis C20-Heteroarylgruppe sind,
R¹¹, R¹² und R¹³ gleich oder verschieden voneinander sind und jeweils unabhängig Wasserstoff, Deuterium, eine substituierte oder unsubstituierte C1- bis C30-Alkylgruppe, eine substituierte oder unsubstituierte C1- bis C30-Alkoxygruppe, eine substituierte oder unsubstituierte C6- bis C30-Arylgruppe, eine substituierte oder unsubstituierte C4- bis C30-Heteroarylgruppe, ein Halogen, eine Cyanogruppe, eine cyanohaltige Gruppe oder eine beliebige Kombination davon sind, und R¹² und R¹³ jeweils unabhängig vorhanden sind oder miteinander verknüpft sind, um einen kondensierten aromatischen Ring bereitzustellen,
n 0 oder 1 ist, und
* einen Verknüpfungspunkt innerhalb der chemischen Formel 2 bezeichnet, 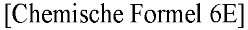 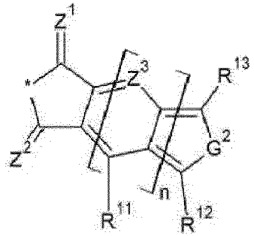
wobei in der chemischen Formel 6E
Z¹ und Z² jeweils unabhängig O, S, Se, Te oder CR^{a}R^{b} sind, wobei R^{a} und R^{b} jeweils unabhängig Wasserstoff, eine substituierte oder unsubstituierte C1-bis C10-Alkylgruppe oder eine Cyanogruppe sind,
Z³ N oder CR^{c} ist, wobei R^{c} Wasserstoff oder eine substituierte oder unsubstituierte C1-bis C10-Alkylgruppe ist,
G² O, S, Se, Te, SiR^{x}R^{y} oder GeR^{z}R^{w} ist, wobei R^{x}, R^{y}, R^{z} und R^{w} gleich oder verschieden voneinander sind und jeweils unabhängig Wasserstoff, Deuterium, ein Halogen, eine substituierte oder unsubstituierte C1- bis C20-Alkylgruppe, eine substituierte oder unsubstituierte C1- bis C20-Alkoxygruppe, eine substituierte oder unsubstituierte C6- bis C20-Arylgruppe oder eine substituierte oder unsubstituierte C2- bis C20-Heteroarylgruppe sind,
R¹¹, R¹² und R¹³ gleich oder verschieden voneinander sind und jeweils unabhängig Wasserstoff, Deuterium, eine substituierte oder unsubstituierte C1- bis C30-Alkylgruppe, eine substituierte oder unsubstituierte C1- bis C30-Alkoxygruppe, eine substituierte oder unsubstituierte C6- bis C30-Arylgruppe, eine substituierte oder unsubstituierte C4- bis C30-Heteroarylgruppe, ein Halogen, eine Cyanogruppe, eine cyanohaltige Gruppe oder eine beliebige Kombination davon sind,
n 0 oder 1 ist, und
* einen Verknüpfungspunkt innerhalb der chemischen Formel 2 bezeichnet, 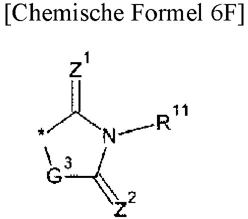
wobei in der chemischen Formel 6F
Z¹ und Z² jeweils unabhängig O, S, Se, Te oder CR^{a}R^{b} sind, wobei R^{a} und R^{b} jeweils unabhängig Wasserstoff, eine substituierte oder unsubstituierte C1-bis C10-Alkylgruppe oder eine Cyanogruppe sind,
R¹¹ Wasserstoff, Deuterium, eine substituierte oder unsubstituierte C1- bis C30-Alkylgruppe, eine substituierte oder unsubstituierte C6- bis C30-Arylgruppe, eine substituierte oder unsubstituierte C4-bis C30-Heteroarylgruppe, ein Halogen, eine Cyanogruppe (-CN), eine cyanohaltige Gruppe oder eine beliebige Kombination davon ist,
G³ O, S, Se, Te, SiR^{x}R^{y} oder GeR^{z}R^{w} ist, wobei R^{x}, R^{y}, R^{z} und R^{w} gleich oder verschieden voneinander sind und jeweils unabhängig Wasserstoff, Deuterium, ein Halogen, eine substituierte oder unsubstituierte C1- bis C20-Alkylgruppe, eine substituierte oder unsubstituierte C1- bis C20-Alkoxygruppe, eine substituierte oder unsubstituierte C6- bis C20-Arylgruppe oder eine substituierte oder unsubstituierte C2- bis C20-Heteroarylgruppe sind, und
* ein Verknüpfungspunkt innerhalb der chemischen Formel 2 ist; und/oder
wobei ein Gewichtsverhältnis der p-Typ-Halbleiterverbindung zu der n-Typ-Halbleiterverbindung in einer Bandbreite von 1:0,1 bis 1: 10 ist; und/oder
wobei die Infrarotabsorptionszusammensetzung konfiguriert ist, um eine Spitzenabsorptionswellenlänge in einem Wellenlängenbereich von 1000 nm bis 3000 nm aufzuzeigen.

9. Infrarotabsorptionsfilm, der die Infrarotabsorptionszusammensetzung nach einem der Ansprüche 1-8 umfasst;
bevorzugt:
zeigend eine frontale Ausrichtungsstruktur bei streifender einfallender Kleinwinkel-Röntgenstreu(GISAXS-)Analyse; und/oder
mit einer Oberflächenrauheit von weniger als oder gleich 2 nm bei Rasterkraftmikroskopieanalyse.

10. Photoelektrische Vorrichtung, umfassend eine erste Elektrode und eine zweite Elektrode, die einander zugewandt sind, und eine photoaktive Schicht zwischen der ersten Elektrode und der zweiten Elektrode, wobei die photoaktive Schicht die Infrarotabsorptionszusammensetzung nach einem der Ansprüche 1-8 beinhaltet;
wobei:
eine externe Quanteneffizienz der photoelektrischen Vorrichtung bei -3 V zwischen 10 % und 100 % ist; und/oder
eine externe Quanteneffizienz der photoelektrischen Vorrichtung bei -3 V um zumindest 80 % erhöht verglichen mit einer photoelektrischen Vorrichtung ist, die eine photoaktive Schicht beinhaltet, die dieselbe p-Typ-Halbleiterverbindung und eine n-Typ-Halbleiterverbindung aus Fulleren oder einem Fulleren-Derivat beinhaltet.

11. Photoelektrische Vorrichtung, umfassend:
eine erste Elektrode und eine zweite Elektrode, die einander zugewandt sind;
eine photoaktive Schicht zwischen der ersten Elektrode und der zweiten Elektrode; und
eine Ladungshilfsschicht zwischen
der photoaktiven Schicht und der ersten Elektrode, oder
der photoaktiven Schicht und der zweiten Elektrode,
wobei zumindest eine von der photoaktiven Schicht oder der Ladungshilfsschicht die Infrarotabsorptionszusammensetzung nach einem der Ansprüche 1-8 beinhaltet.

12. Sensor, der die Infrarotabsorptionszusammensetzung nach einem der Ansprüche 1-8 oder die photoelektrische Vorrichtung nach Anspruch 10 oder 11 umfasst; oder elektronische Vorrichtung, welche die photoelektrische Vorrichtung nach Anspruch 10 oder 11 umfasst.

13. Bildsensor, umfassend:
ein Halbleitersubstrat;
eine erste photoelektrische Vorrichtung auf dem Halbleitersubstrat, wobei die erste photoelektrische Vorrichtung konfiguriert ist, um selektiv Licht in einem ersten Infrarotwellenlängenbereich zu absorbieren; und
einen zusätzlichen Sensor, der konfiguriert ist, um selektiv Licht in einem separaten Wellenlängenbereich zu absorbieren, der sich von dem ersten Infrarotwellenlängenbereich unterscheidet,
wobei die erste photoelektrische Vorrichtung die Infrarotabsorptionszusammensetzung nach einem der Ansprüche 1-8 beinhaltet;
wobei bevorzugt
der zusätzliche Sensor ein Infrarotlichtsensor ist, der zumindest teilweise in das Halbleitersubstrat eingebettet ist, und der separate Wellenlängenbereich ein separater Infrarotwellenlängenbereich ist, der sich von dem ersten Infrarotwellenlängenbereich unterscheidet, und
sich die erste photoelektrische Vorrichtung und der Infrarotlichtsensor in einer vertikalen Richtung überlappen, die senkrecht zu einer oberen Oberfläche des Halbleitersubstrats ist;
wobei bevorzugt
der zusätzliche Sensor eine Vielzahl von Photodioden beinhaltet, die zumindest teilweise in das Halbleitersubstrat eingebettet ist, wobei die Vielzahl von Photodioden konfiguriert ist, um selektiv Licht in separaten sichtbaren Wellenlängenbereichen zu absorbieren, und
sich die erste photoelektrische Vorrichtung und die Vielzahl von Photodioden in einer vertikalen Richtung überlappen, die senkrecht zu einer oberen Oberfläche des Halbleitersubstrats ist.

14. Bildsensor nach Anspruch 13, wobei
der zusätzliche Sensor zumindest eine zusätzliche photoelektrische Vorrichtung beinhaltet, die vertikal zwischen der ersten photoelektrischen Vorrichtung und dem Halbleitersubstrat gestapelt ist, wobei jede separate photoelektrische Vorrichtung von der zumindest einen zusätzlichen photoelektrischen Vorrichtung eine separate photoelektrische Umwandlungsschicht beinhaltet und konfiguriert ist, um selektiv Licht in einem separaten jeweiligen Wellenlängenbereich zu absorbieren, der sich von dem ersten Infrarotwellenlängenbereich unterscheidet;
und/oder
wobei die erste photoelektrische Vorrichtung Folgendes beinhaltet:
eine erste Elektrode und eine zweite Elektrode, die einander zugewandt sind; und
eine photoaktive Schicht zwischen der ersten Elektrode und der zweiten Elektrode, wobei die photoaktive Schicht die Infrarotabsorptionszusammensetzung beinhaltet.

15. Bildsensor nach Anspruch 13 oder 14, wobei die erste photoelektrische Vorrichtung Folgendes beinhaltet:
eine erste Elektrode und eine zweite Elektrode, die einander zugewandt sind;
eine photoaktive Schicht zwischen der ersten Elektrode und der zweiten Elektrode; und
eine Ladungshilfsschicht zwischen
der photoaktiven Schicht und der ersten Elektrode, oder
der photoaktiven Schicht und der zweiten Elektrode,
wobei die Ladungshilfsschicht die Infrarotabsorptionszusammensetzung beinhaltet.

## Revendications

1. Composition d'absorption infrarouge, comprenant
un composé semi-conducteur de type p comprenant un premier motif structural représenté par la formule chimique 1 et un second motif structural comprenant un groupement donneur d'électrons ; et
un composé semi-conducteur de type n représenté par la formule chimique 2 : 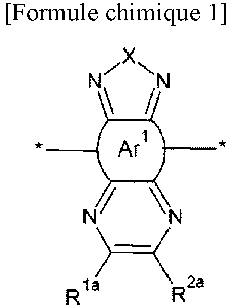
dans lequel, dans la Formule chimique 1,
Ar¹ est un cycle aromatique en C6 à C30 substitué ou non substitué, un cycle hétéroaromatique en C3 à C30 substitué ou non substitué, ou toute combinaison de ceux-ci,
X est O, S, Se, Te, S(=O), S(=O₂), NR^{a}, CR^{b}R^{c}, SiR^{d}R^{e}, GeR^{f}R^{g}, CR^{h}=CRⁱ ou CR^{hh}=CRⁱⁱ, R^{a}, R^{b}, R^{c}, R^{d}, R^{e}, R^{f}, R^{g}, R^{h} et Rⁱ étant chacun indépendamment hydrogène, deutérium, un groupe alkyle en C1 à C6 , un groupe haloalkyle en C1 à C6, un groupe aryle en C6 à C14, un groupe hétéroaryle en C3 à C12, un groupe halogène, un groupe cyano, ou toute combinaison de ceux-ci, et R^{hh} et Rⁱⁱ étant chacun indépendamment un groupe alkylène en C1 à C6 ou un groupe hétéroalkylène en C2 à C6 et liés l'un à l'autre pour former un cycle aromatique ou hétéroaromatique,
R^{1a} et R^{2a} sont chacun indépendamment un groupe aryle en C6 à C30 substitué ou non substitué, un groupe hétéroaryle en C3 à C30 substitué ou non substitué ou R^{1a} et R^{2a} sont liés l'un à l'autre pour former un groupe arène en C6 à C30 substitué ou non substitué ou un groupe hétéroarène en C3 à C30 substitué ou non substitué, et
* est un point de liaison dans le composé semi-conducteur de type p, 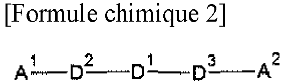
dans lequel, dans la Formule chimique 2,
D¹ est un premier groupement donneur d'électrons comportant l'une quelconque des structures représentées par les formules chimiques 3A à 3E,
D² et D³ sont chacun indépendamment une liaison simple ou un second groupement donneur d'électrons,
A¹ et A² sont chacun indépendamment un groupement accepteur d'électrons d'un groupe cyclique hydrocarboné en C6 à C30 substitué ou non substitué comportant au moins un groupe fonctionnel de C=O, C=S, C=Se, C=Te ou C=C(CN)₂ ; un groupe hétérocyclique en C2 à C30 substitué ou non substitué comportant au moins un groupe fonctionnel de C=O, C=S, C=Se, C=Te ou C=C(CN)₂ ; ou un cycle fusionné de ceux-ci, 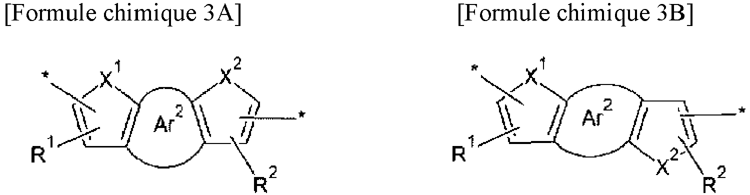 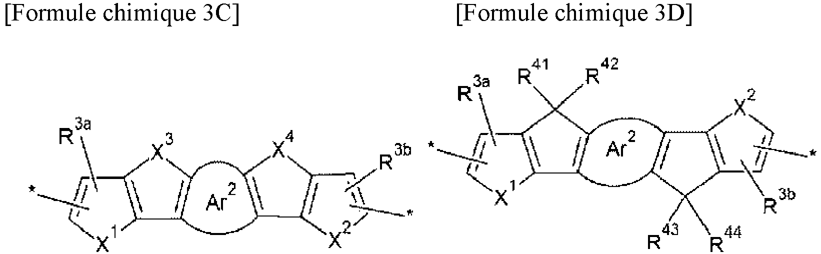 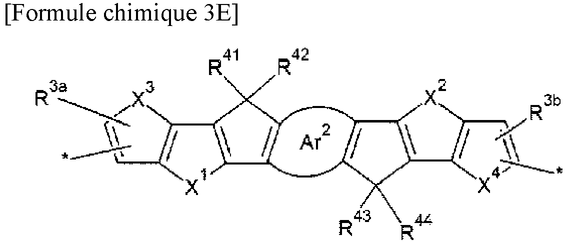
dans lequel, dans les Formules chimiques 3A à 3E,
Ar² est un groupe arène en C6 à C30 substitué ou non substitué ; un groupe hétérocyclique en C3 à C30 substitué ou non substitué comprenant au moins l'un de N, O, S, Se, Te ou Si ; un cycle fusionné de celui-ci ; ou toute combinaison de ceux-ci,
X¹, X², X³ et X⁴ sont chacun indépendamment S, Se ou Te,
R⁴¹, R⁴², R⁴³ et R⁴⁴ sont chacun indépendamment un groupe alkyle en C1 à C30 substitué ou non substitué, un groupe alkoxy en C1 à C30 substitué ou non substitué, un groupe aryle en C6 à C20 substitué ou non substitué, ou un groupe hétéroaryle en C3 à C20 substitué ou non substitué,
R¹, R², R^{3a} et R^{3b} sont chacun indépendamment hydrogène ou un groupe alkyle en C1 à C10, et
* représente un point de liaison dans la formule chimique 2.

2. Composition d'absorption infrarouge selon la revendication 1, dans laquelle dans la formule chimique 1, Ar¹ est un cycle benzène, un naphtalène substitué ou non substitué, un cycle anthracène substitué ou non substitué, un cycle phénanthrène substitué ou non substitué, un cycle tétracène substitué ou non substitué, un cycle pyrène substitué ou non substitué, un cycle quinoline substitué ou non substitué, un cycle isoquinoline substitué ou non substitué, un cycle quinoxaline substitué ou non substitué, un cycle quinazoline substitué ou non substitué, ou un cycle phénanthroline substitué ou non substitué ;
de préférence dans lequel dans la formule chimique 1, Ar¹ est l'un des groupements représentés par la formule chimique 1A-1 ou la formule chimique 1A-2 ; 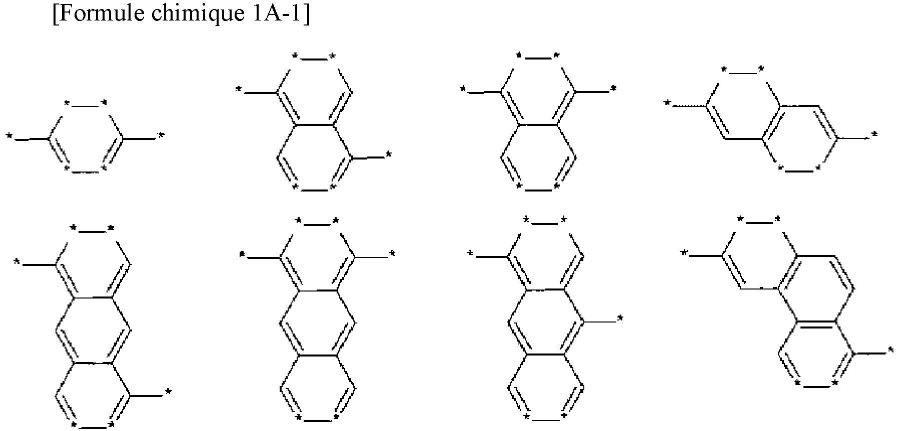
dans lequel, dans la Formule chimique 1A-1,
au moins un hydrogène de chaque cycle aromatique est hydrogène ou est remplacé par deutérium, un halogène, un groupe cyano, un groupe alkyle en C1 à C10, un groupe haloalkyle en C1 à C10, un groupe -SiHs ou un groupe alkylsilyle en C1 à C10, et
les paires adjacentes de * à l'intérieur d'au moins un cycle aromatique sont des points de liaison avec un cycle contenant N-X-N et un cycle pyrazine de formule chimique 1 ; 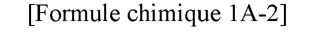 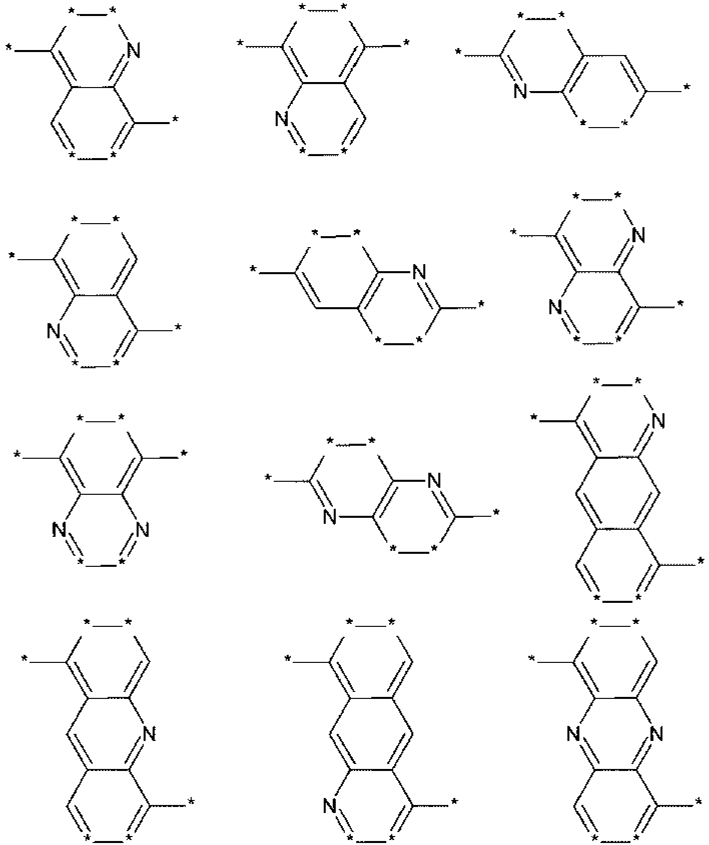
dans lequel, dans la formule chimique 1A-2,
au moins un hydrogène de chaque cycle aromatique ou hétéroaromatique est hydrogène ou est remplacé par deutérium, un halogène, un groupe cyano, un groupe alkyle en C1 à C10, un groupe haloalkyle en C1 à C10, un groupe -SiHs ou un groupe alkylsilyle en C1 à C10, et
les paires adjacentes de * à l'intérieur d'au moins un cycle aromatique ou hétéroaromatique sont des points de liaison avec un cycle contenant N-X-N et un cycle pyrazine de formule chimique 1.

3. Composition d'absorption infrarouge selon les revendications 1 ou 2 dans laquelle dans la formule chimique 1,
R^{1a} et R^{2a} sont liés l'un à l'autre, et
le groupe arène en C6 à C30 substitué ou non substitué et le groupe hétéroarène en C3 à C30 substitué ou non substitué formés par la liaison de R^{1a} et R^{2a} l'un à l'autre sont un cycle benzène substitué ou non substitué, un cycle naphtalène substitué ou non substitué, un cycle acénaphthène substitué ou non substitué, un cycle anthracène substitué ou non substitué, un cycle phénanthrène substitué ou non substitué, un cycle tétracène substitué ou non substitué, ou un cycle pyrène substitué ou non substitué, un cycle quinoline substitué ou non substitué, un cycle isoquinoline substitué ou non substitué, un cycle quinoxaline substitué ou non substitué, un cycle quinazoline substitué ou non substitué, un cycle phénanthroline substitué ou non substitué, un cycle pyrimidine substitué ou non substitué, ou un cycle benzodithiophène substitué ou non substitué ; ou
dans lequel dans la formule chimique 1,
R^{1a} et R^{2a} sont liés l'un à l'autre, et
le groupe arène en C6 à C30 substitué ou non substitué et le groupe hétéroarène en C3 à C30 substitué ou non substitué formés par la liaison de R^{1a} et R^{2a} l'un à l'autre sont l'un des groupements représentés par les formules chimiques 1B-1 et 1B-2 : 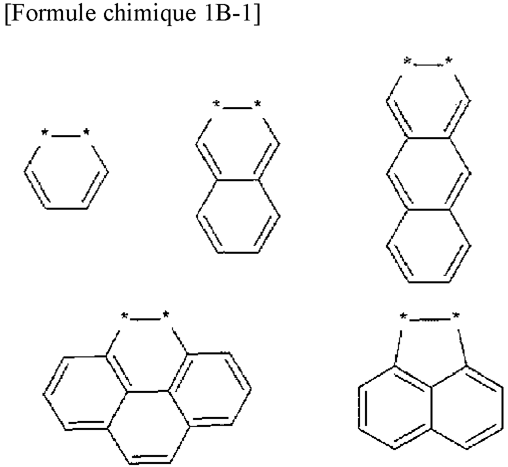
dans lequel, dans la Formule chimique 1B-1,
au moins un hydrogène de chaque cycle aromatique est hydrogène ou est remplacé par un halogène, un groupe cyano, un groupe alkyle en C1 à C30, un groupe alkoxy en C1 à C30, un groupe haloalkyle en C1 à C30, un groupe -SiHs, un groupe alkylsilyle en C1 à C30, un groupe aryle en C6 à C30, un groupe aryloxy en C6 à C30 ou un groupe hétéroaryle en C3 à C30, et
chaque * est un point lié à un cycle pyrazine de formule chimique 1, 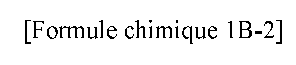 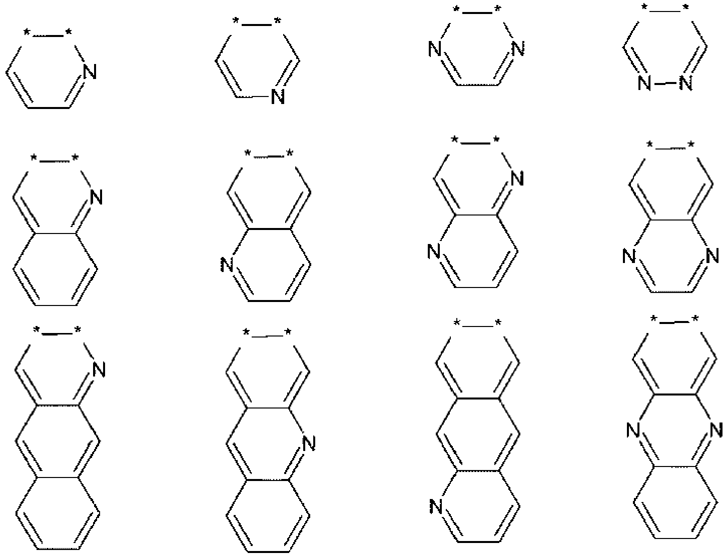
dans lequel, dans la formule chimique 1B-2,
au moins un hydrogène de chaque cycle aromatique ou hétéroaromatique est hydrogène ou est remplacé par un halogène, un groupe cyano, un groupe alkyle en C1 à C30, un groupe alkoxy en C1 à C30, un groupe haloalkyle en C1 à C30, un groupe -SiHs, un groupe alkylsilyle en C1 à C30, un groupe aryle en C6 à C30, un groupe aryloxy en C6 à C30 ou un groupe hétéroaryle en C3 à C30, et
chaque * est un point lié à un cycle pyrazine de formule chimique 1 ; ou
dans lequel dans la formule chimique 1,
R^{1a} et R^{2a} sont liés l'un à l'autre, et
le groupe arène en C6 à C30 substitué ou non substitué et le groupe hétéroarène en C3 à C30 substitué ou non substitué formés par la liaison de R^{1a} et R^{2a} l'un à l'autre sont chacun indépendamment l'un des groupements comportant un cycle aromatique ou hétéroaromatique et représentés par la formule chimique 1B-3 ou 1B-4 : 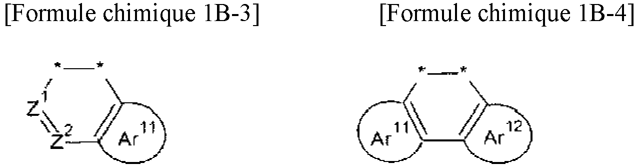
dans lequel, dans les formules chimiques 1B-3 et 1B-4,
Ar¹¹ et Ar¹² sont chacun indépendamment un groupe arène en C6 à C30 substitué ou non substitué ou un groupe hétéroarène en C3 à C30 substitué ou non substitué,
dans lequel, dans la formule chimique 1B-3,
Z¹ et Z² sont chacun indépendamment N ou CR^{x}, R^{x} étant hydrogène, deutérium, un groupe alkyle en C1 à C10, un groupe haloalkyle en C1 à C10, un groupe -SiHs, un groupe alkylsilyle en C1 à C10, un groupe -NH₂, un groupe alkylamine en C1 à C10, un groupe arylamine en C6 à C10, un groupe aryle en C6 à C14, un groupe hétéroaryle en C3 à C12, un halogène, un groupe cyano, ou toute combinaison de ceux-ci, et
chaque * à l'intérieur du cycle aromatique ou hétéroaromatique est un point lié à un cycle pyrazine de formule chimique 1 ;
de préférence dans lequel le groupement représenté par la formule chimique 1B-3 est représenté par la formule chimique 1B-3-1, ou dans lequel le groupement représenté par la formule chimique 1B-4 est représenté par la formule chimique 1B-4-1 : 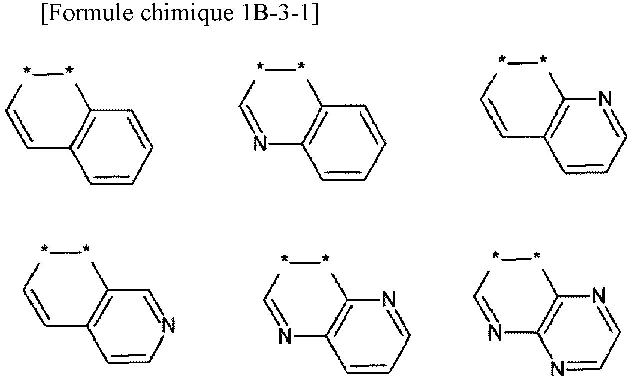
dans lequel, dans la formule chimique 1B-3-1,
au moins un hydrogène de chaque cycle aromatique ou hétéroaromatique est hydrogène ou est remplacé par un halogène, un groupe cyano, un groupe alkyle en C1 à C30, un groupe alkoxy en C1 à C30, un groupe haloalkyle en C1 à C30, un groupe -SiHs, un groupe alkylsilyle en C1 à C30, un groupe aryle en C6 à C30, un groupe aryloxy en C6 à C30 ou un groupe hétéroaryle en C3 à C30, et
chaque * à l'intérieur du cycle aromatique ou hétéroaromatique est un point lié au cycle pyrazine de formule chimique 1 ; 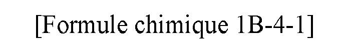 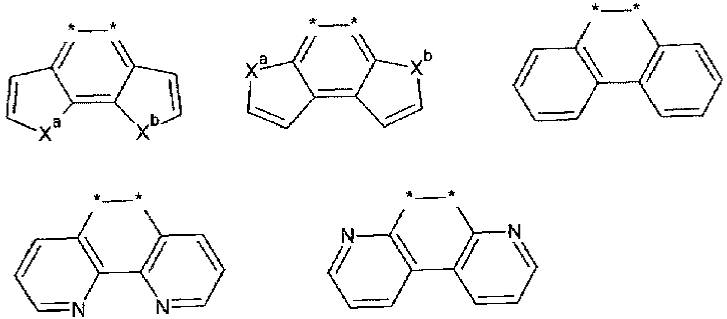
dans lequel, dans la formule chimique 1B-4-1,
au moins un hydrogène de chaque cycle aromatique ou hétéroaromatique est hydrogène ou est remplacé par un halogène, un groupe cyano, un groupe alkyle en C1 à C30, un groupe alkoxy en C1 à C30, un groupe haloalkyle en C1 à C30, un groupe -SiHs, un groupe alkylsilyle en C1 à C30, un groupe aryle en C6 à C30, un groupe aryloxy en C6 à C30 ou un groupe hétéroaryle en C3 à C30,
X^{a} et X^{b} sont chacun indépendamment O, S, Se, Te, NR^{a}, SiR^{b}R^{c} ou GeR^{d}R^{e}, R^{a}, R^{b}, R^{c}, R^{d} et R^{e} étant chacun indépendamment hydrogène, un halogène, un groupe alkyle en C1 à C10 substitué ou non substitué, ou un groupe aryle en C6 à C10 substitué ou non substitué, et
chaque * à l'intérieur du cycle aromatique est un point lié au cycle pyrazine de formule chimique 1.

4. Composition d'absorption infrarouge selon l'une quelconque des revendications 1 à 3, dans laquelle le premier motif structural du composé semi-conducteur de type p est représenté par la formule chimique 1C : 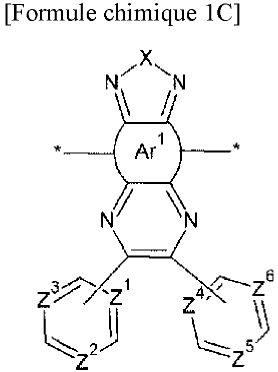
dans lequel, dans la formule chimique 1C,
Ar¹ est un cycle aromatique en C6 à C30 substitué ou non substitué, un cycle hétéroaromatique en C3 à C30 substitué ou non substitué, ou toute combinaison de ceux-ci,
X est O, S, Se, Te, S(=O), S(=O₂), NR^{a}, CR^{b}R^{c}, SiR^{d}R^{e}, GeR^{f}R^{g}, CR^{h}=CRⁱ ou CR^{hh}=CRⁱⁱ, R^{a}, R^{b}, R^{c}, R^{d}, R^{e}, R^{f}, R^{g}, R^{h} et Rⁱ étant chacun indépendamment hydrogène, deutérium, un groupe alkyle en C1 à C6 , un groupe haloalkyle en C1 à C6, un groupe aryle en C6 à C14, un groupe hétéroaryle en C3 à C12, un groupe halogène, un groupe cyano, ou toute combinaison de ceux-ci, et R^{hh} et Rⁱⁱ étant chacun indépendamment un groupe alkylène en C1 à C6 ou un groupe hétéroalkylène en C2 à C6 et liés l'un à l'autre pour former un cycle aromatique ou hétéroaromatique
Z¹ à Z⁶ sont chacun indépendamment N ou CR^{x}, R^{x} étant hydrogène, deutérium, un groupe alkyle en C1 à C20, un groupe haloalkyle en C1 à C20, un groupe -SiHs, un groupe alkylsilyle en C1 à C20, un groupe -NH₂, un groupe alkylamine en C1 à C20, un groupe aryle en C6 à C12, un groupe hétéroaryle en C3 à C12, un halogène, un groupe cyano, ou toute combinaison de ceux-ci,
au moins un hydrogène de chaque cycle aromatique ou hétéroaromatique est hydrogène ou est remplacé par deutérium, un halogène, un groupe cyano, un groupe alkyle en C1 à C30, un groupe alkoxy en C1 à C30, un groupe haloalkyle en C1 à C30, un groupe aryle en C6 à C30, un groupe aryloxy en C6 à C30, un groupe -SiHs, ou un groupe alkylsilyle en C1 à C30, et
* est un point de liaison avec le composé semi-conducteur de type p ; et/ou
dans lequel le groupement donneur d'électrons compris dans le second motif structural du composé semi-conducteur de type p est un groupe arène en C6 à C30 substitué ou non substitué, un groupe hétérocyclique en C3 à C30 divalent substitué ou non substitué comprenant au moins l'un de N, O, S, Se, Te ou Si, un cycle fusionné de celui-ci, ou toute combinaison de ceux-ci, de préférence dans lequel le groupement donneur d'électrons compris dans le second motif structural du composé semi-conducteur de type p comprend au moins l'un des groupements représentés par les formules chimiques 4A à 4J du Groupe 1 : 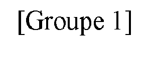 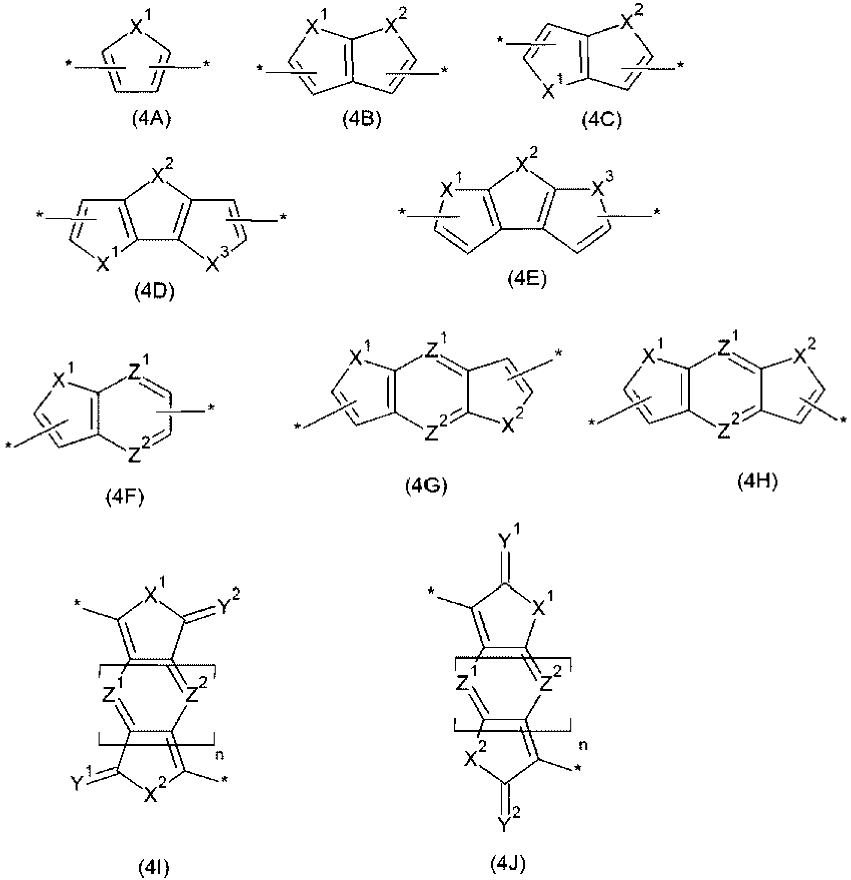
dans lequel, dans le groupe 1,
X¹ à X³ sont chacun indépendamment S, Se, Te, S(=O), S(=O₂), NR^{a}, SiR^{d}R^{e} ou GeR^{f}R^{g}, R^{a}, R^{b}, R^{c}, R^{d}, R^{e}, R^{f} et R^{g} étant chacun indépendamment hydrogène, deutérium, un groupe alkyle en C1 à C20, un groupe alkoxy en C1 à C20, un groupe haloalkyle en C1 à C20, un groupe aryle en C6 à C20, un groupe hétéroaryle en C3 à C20, un halogène, un groupe cyano, ou toute combinaison de ceux-ci,
Z¹ et Z² sont chacun indépendamment N ou CR^{x}, R^{x} étant hydrogène, deutérium, un groupe alkyle en C1 à C10, un groupe haloalkyle en C1 à C10, un groupe -SiHs, un groupe alkylsilyle en C1 à C10, un groupe -NH₂, un groupe alkylamine en C1 à C10, un groupe aryle en C6 à C12, un groupe hétéroaryle en C3 à C12, un halogène, un groupe cyano, ou toute combinaison de ceux-ci,
Y¹ et Y² sont chacun indépendamment O, S, Se ou Te,
n est 0 ou 1, et
au moins un hydrogène de chaque cycle aromatique ou hétéroaromatique est hydrogène ou est remplacé par deutérium, un halogène, un groupe cyano, un groupe alkyle en C1 à C30, un groupe alkoxy en C1 à C30, un groupe haloalkyle en C1 à C30, un groupe aryle en C6 à C30, un groupe aryloxy en C6 à C30, un groupe -SiHs, ou un groupe alkylsilyle en C1 à C30.

5. Composition d'absorption infrarouge selon l'une quelconque des revendications 1 à 4, dans laquelle le composé semi-conducteur de type p est un polymère comprenant 20 mol % à 50 mol % du premier motif structural et 50 mol % à 80 mol % du second motif structural ; et/ou
dans laquelle le composé semi-conducteur de type p est configuré pour présenter une longueur d'onde d'absorption de crête dans une plage de longueurs d'onde de 1000 nm à 3000 nm ; et/ou
dans laquelle, dans les formules chimiques 3A à 3C, Ar² est un groupement comportant l'une des structures représentées par les formules chimiques 5A à 5K du groupe 2 : 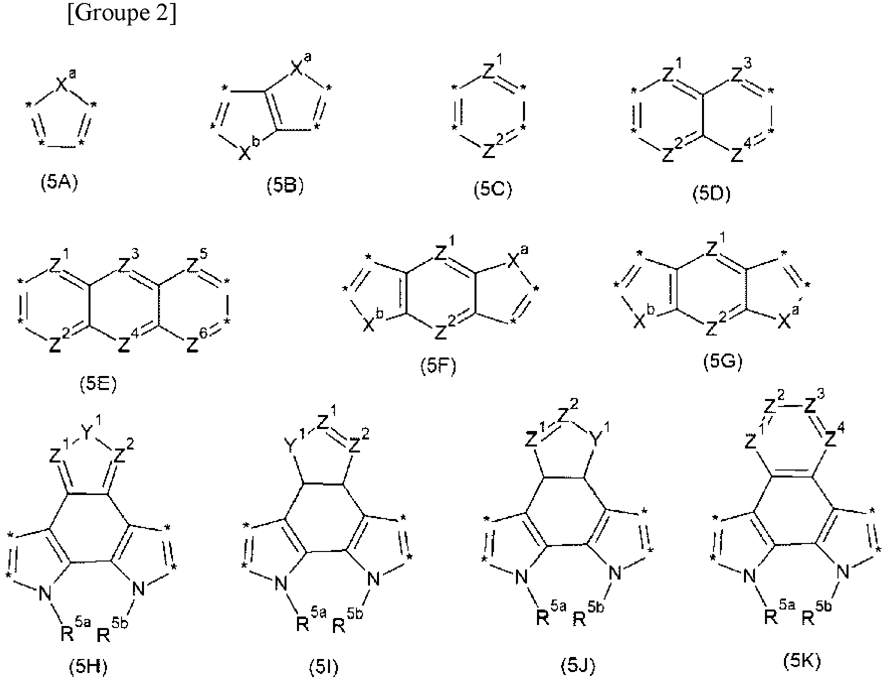
dans lequel, dans le groupe 2,
X^{a} et X^{b} sont chacun indépendamment CR^{x}R^{y}, S, Se ou Te, R^{x} et R^{y} étant chacun indépendamment un groupe alkyle en C1 à C30 substitué ou non substitué, un groupe alkoxy en C1 à C30 substitué ou non substitué, un groupe aryle en C6 à C20 substitué ou non substitué, ou un groupe hétéroaryle en C3 à C20 substitué ou non substitué,
R^{5a} et R^{5b} sont chacun indépendamment hydrogène, un groupe alkyle en C1 à C20, un groupe alkoxy en C1 à C20, un groupe aryle en C6 à C10 ou un groupe hétéroaryle en C2 à C10,
Y¹ est CR^{p}R^{q}, NR^{r}, O, S, Se ou Te, R^{p}, R^{q} et R^{r} étant chacun indépendamment hydrogène ou un groupe alkyle en C1 à C20, et
Z¹ à Z⁶ sont chacun indépendamment CR^{s} ou N, R^{s} étant hydrogène ou un groupe alkyle en C1 à C20.

6. Composition d'absorption infrarouge selon l'une quelconque des revendications 1 à 5, dans laquelle dans les formules chimiques 3D et 3E, R⁴¹, R⁴², R⁴³ et R⁴⁴ sont chacun indépendamment un groupe alkyle en C1 à C20 ; un groupe alkoxy en C1 à C20 ; un groupe aryle en C6 à C20 substitué par un groupe alkyle en C1 à C20 ou un groupe alkoxy en C1 à C20 ; ou un groupe hétéroaryle en C3 à C20 substitué par un groupe alkyle en C1 à C20 ou un groupe alkoxy en C1 à C20 ; et/ou
dans laquelle dans les formules chimiques 3D et 3E, R⁴¹, R⁴², R⁴³ et R⁴⁴ sont chacun indépendamment un groupe alkyle ramifié en C3 à C30 substitué ou non substitué ou un groupe alkoxy ramifié en C3 à C30 substitué ou non substitué.

7. Composition d'absorption infrarouge selon l'une quelconque des revendications 1 à 6, dans laquelle le première groupement donneur d'électrons choisi dans les structures représentées par les formules chimiques 3A à 3E est un groupement représenté par la formule chimique 3A-1, 3B-1, 3C-1, 3D-1, 3D-2, 3E-1 ou 3E-2 : 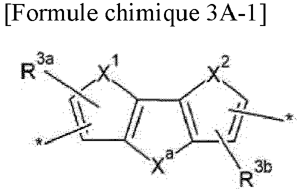
dans lequel, dans la formule chimique 3A-1,
X^{a} est CR^{x}R^{y}, S, Se ou Te, R^{x} et R^{y} étant chacun indépendamment un groupe alkyle en C1 à C30 substitué ou non substitué, un groupe alkoxy en C1 à C30 substitué ou non substitué, un groupe aryle en C6 à C20 substitué ou non substitué, ou un groupe hétéroaryle en C3 à C20 substitué ou non substitué,
X¹ et X² sont chacun indépendamment S, Se ou Te,
R^{3a} et R^{3b} sont chacun indépendamment hydrogène ou un groupe alkyle en C1 à C10, et
* représente un point de liaison dans la formule chimique 2, 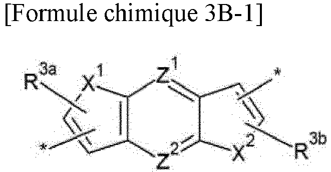
dans lequel, dans la Formule chimique 3B-1,
Z¹ et Z² sont chacun indépendamment CR^{s} ou N, R^{s} étant hydrogène ou un groupe alkyle en C1 à C20,
X¹ et X² sont chacun indépendamment S, Se ou Te,
R^{3a} et R^{3b} sont chacun indépendamment hydrogène ou un groupe alkyle en C1 à C10, et
* représente un point de liaison dans la formule chimique 2, 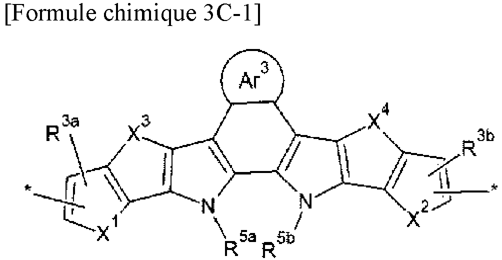
dans lequel, dans la formule chimique 3C-1,
Ar³ est l'un des groupements comportant un cycle et représenté par la formule chimique 3C-1a,
X¹, X², X³ et X⁴ sont chacun indépendamment S, Se ou Te,
R^{3a} et R^{3b} sont chacun indépendamment hydrogène ou un groupe alkyle en C1 à C10,
R^{5a} et R^{5b} sont chacun indépendamment hydrogène, un groupe alkyle en C1 à C20, un groupe alkoxy en C1 à C20, un groupe aryle en C6 à C10 ou un groupe hétéroaryle en C2 à C10, et
* représente un point de liaison dans la formule chimique 2, 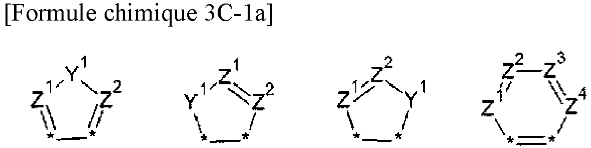
dans lequel, dans la formule chimique 3C-1a,
Y¹ est CR^{p}R^{q}, NR^{r}, O, S, Se ou Te, R^{p}, R^{q} et R^{r} étant chacun indépendamment hydrogène ou un groupe alkyle en C1 à C20,
Z¹ à Z⁴ sont chacun indépendamment CR^{s} ou N, R^{s} étant hydrogène ou un groupe alkyle en C1 à C20, et
* à l'intérieur du cycle représente un point de liaison avec la formule chimique 3C-1, 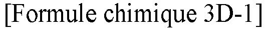 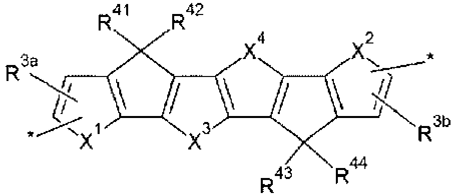
dans lequel, dans la formule chimique 3D-1,
X¹, X², X³ et X⁴ sont chacun indépendamment S, Se ou Te,
R⁴¹, R⁴², R⁴³ et R⁴⁴ sont chacun indépendamment un groupe alkyle en C1 à C30 substitué ou non substitué, un groupe alkoxy en C1 à C30 substitué ou non substitué, un groupe aryle en C6 à C20 substitué ou non substitué, ou un groupe hétéroaryle en C3 à C20 substitué ou non substitué,
R^{3a} et R^{3b} sont chacun indépendamment hydrogène ou un groupe alkyle en C1 à C10, et
* représente un point de liaison dans la formule chimique 2, 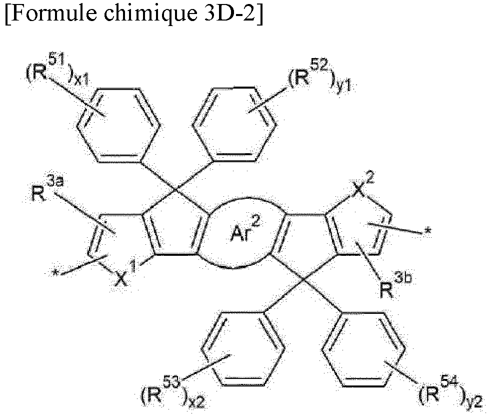
dans lequel, dans la formule chimique 3D-2,
Ar² est un groupe arène en C6 à C30 substitué ou non substitué ; un groupe hétérocyclique en C3 à C30 substitué ou non substitué comprenant au moins l'un de N, O, S, Se, Te ou Si ; un cycle fusionné de celui-ci ; ou toute combinaison de ceux-ci,
X¹ et X² sont chacun indépendamment S, Se ou Te,
R⁵¹, R⁵², R⁵³ et R⁵⁴ sont chacun indépendamment hydrogène, deutérium, un halogène, un groupe alkyle en C1 à C20 substitué ou non substitué, un groupe alkoxy en C1 à C20 substitué ou non substitué, un groupe aryle en C6 à C20 substitué ou non substitué, ou un groupe hétéroaryle en C2 à C20 substitué ou non substitué,
x1, y1, x2 et y2 sont chacun indépendamment un nombre entier de 0 à 5,
R^{3a} et R^{3b} sont chacun indépendamment hydrogène ou un groupe alkyle en C1 à C10, et
* représente un point de liaison dans la formule chimique 2, 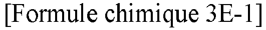 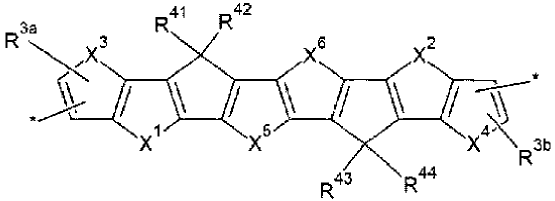
dans lequel, dans la formule chimique 3E-1,
X¹, X², X³, X⁴, X⁵ et X⁶ sont chacun indépendamment S, Se ou Te,
R⁴¹, R⁴², R⁴³ et R⁴⁴ sont chacun indépendamment un groupe alkyle en C1 à C30 substitué ou non substitué, un groupe alkoxy en C1 à C30 substitué ou non substitué, un groupe aryle en C6 à C20 substitué ou non substitué, ou un groupe hétéroaryle en C3 à C20 substitué ou non substitué,
R^{3a} et R^{3b} sont chacun indépendamment hydrogène ou un groupe alkyle en C1 à C10, et
* représente un point de liaison dans la formule chimique 2, 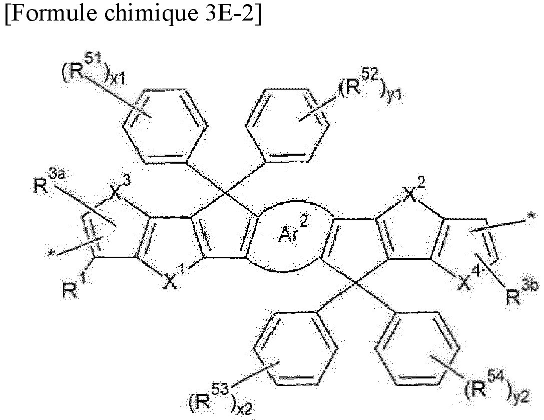
dans lequel, dans la formule chimique 3E-2,
Ar² est un groupe arène en C6 à C30 substitué ou non substitué ; un groupe hétérocyclique en C3 à C30 substitué ou non substitué comprenant au moins l'un de N, O, S, Se, Te ou Si ; un cycle fusionné de celui-ci ; ou toute combinaison de ceux-ci,
X¹, X², X³ et X⁴ sont chacun indépendamment S, Se ou Te,
R⁵¹, R⁵², R⁵³ et R⁵⁴ sont chacun indépendamment hydrogène, deutérium, un halogène, un groupe alkyle en C1 à C20 substitué ou non substitué, un groupe alkoxy en C1 à C20 substitué ou non substitué, un groupe aryle en C6 à C20 substitué ou non substitué, ou un groupe hétéroaryle en C2 à C20 substitué ou non substitué,
x1, y1, x2 et y2 sont chacun indépendamment un nombre entier de 0 à 5,
R¹, R^{3a} et R^{3b} sont chacun indépendamment hydrogène ou un groupe alkyle en C1 à C10, et
* représente un point de liaison dans la formule chimique 2 ; et/ ou
dans lequel dans la formule chimique 2, D² et D³ sont chacun indépendamment l'un des groupements représentés par la formule chimique 4A à 4J du groupe 1 : 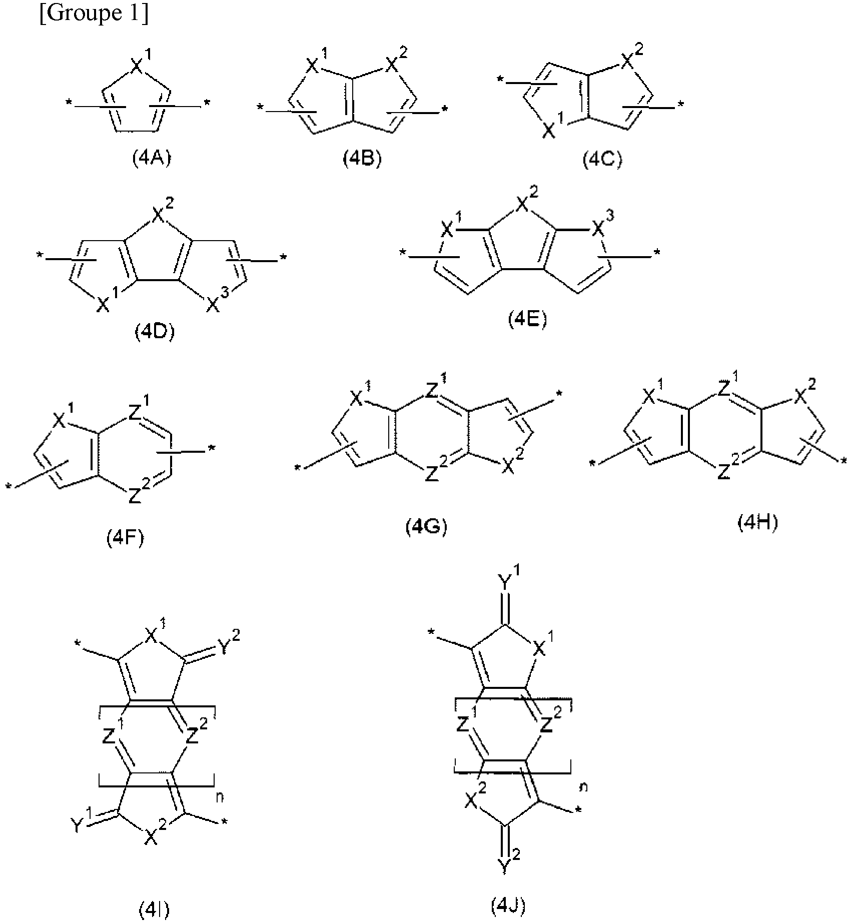
dans lequel, dans le groupe 1,
X¹ à X³ sont chacun indépendamment S, Se, Te, S(=O), S(=O₂), NR^{a}, SiR^{d}R^{e} ou GeR^{f}R^{g}, R^{a}, R^{b}, R^{c}, R^{d}, R^{e}, R^{f}, R^{g}, R^{h} et Rⁱ étant chacun indépendamment hydrogène, deutérium, un groupe alkyle en C1 à C20, un groupe alkoxy en C1 à C20, un groupe haloalkyle en C1 à C20, un groupe aryle en C6 à C20, un groupe hétéroaryle en C3 à C20, un halogène, un groupe cyano, ou toute combinaison de ceux-ci,
Z¹ et Z² sont chacun indépendamment N ou CR^{x}, R^{x} étant hydrogène, deutérium, un groupe alkyle en C1 à C10, un groupe haloalkyle en C1 à C10, un groupe -SiHs, un groupe alkylsilyle en C1 à C10, un groupe -NH₂, un groupe alkylamine en C1 à C10, un groupe aryle en C6 à C12, un groupe hétéroaryle en C3 à C12, un halogène, un groupe cyano, ou toute combinaison de ceux-ci,
Y¹ et Y² sont chacun indépendamment O, S, Se ou Te,
n est 0 ou 1,
* représente un point de liaison dans la formule chimique 2, et
au moins un hydrogène de chaque cycle aromatique ou hétéroaromatique est hydrogène ou est remplacé par deutérium, un halogène, un groupe cyano, un groupe alkyle en C1 à C30, un groupe alkoxy en C1 à C30, un groupe haloalkyle en C1 à C30, un groupe aryle en C6 à C30, un groupe aryloxy en C6 à C30, un groupe -SiHs, ou un groupe alkylsilyle en C1 à C30.

8. Composition d'absorption infrarouge selon l'une quelconque des revendications 1 à 7, dans laquelle A¹ et A² sont chacun indépendamment un groupement accepteur d'électrons représenté par l'une quelconque des formules chimiques 6A à 6F : 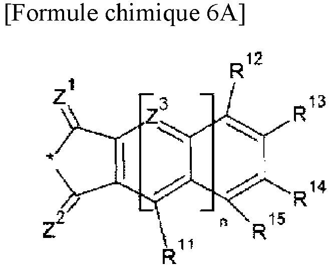
dans lequel, dans la formule chimique 6A,
Z¹ et Z² sont chacun indépendamment O, S, Se, Te ou CR^{a}R^{b}, R^{a} et R^{b} étant chacun indépendamment hydrogène, un groupe alkyle en C1 à C10 substitué ou non substitué, ou un groupe cyano,
Z³ est N ou CR^{c}, R^{c} étant hydrogène, deutérium ou un groupe alkyle en C1 à C10 substitué ou non substitué,
R¹¹, R¹², R¹³, R¹⁴ et R¹⁵ sont identiques ou différents et sont chacun indépendamment hydrogène, deutérium, un groupe alkyle en C1 à C30 substitué ou non substitué, un groupe alkoxy en C1 à C30 substitué ou non substitué, un groupe aryle en C6 à C30 substitué ou non substitué, un groupe hétéroaryle en C4 à C30 substitué ou non substitué, un halogène, un groupe cyano (-CN), un groupe contenant un cyano, ou toute combinaison de ceux-ci, et R¹², R¹³, R¹⁴ et R¹⁵ sont chacun indépendamment présents ou au moins une paire de R¹² et R¹³ et R¹⁴ et R¹⁵ est liée l'une à l'autre pour produire un cycle aromatique fusionné,
n est 0 ou 1, et
* représente un point de liaison dans la formule chimique 2, 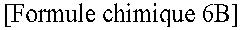 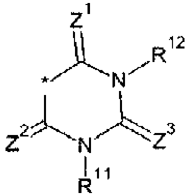
dans lequel, dans la formule chimique 6B,
Z¹ et Z² sont chacun indépendamment O, S, Se, Te ou CR^{a}R^{b}, R^{a} et R^{b} étant chacun indépendamment hydrogène, un groupe alkyle en C1 à C10 substitué ou non substitué, ou un groupe cyano,
Z³ est O, S, Se, Te ou C(R^{a})(CN), R^{a} étant hydrogène, un groupe cyano (-CN) ou un groupe alkyle en C1 à C10,
R¹¹ et R¹² sont chacun indépendamment hydrogène, deutérium, un groupe alkyle en C1 à C30 substitué ou non substitué, un groupe alkoxy en C1 à C30 substitué ou non substitué, un groupe aryle en C6 à C30 substitué ou non substitué, un groupe hétéroaryle en C4 à C30 substitué ou non substitué, un halogène, un groupe cyano (-CN), ou toute combinaison de ceux-ci, et
* représente un point de liaison dans la formule chimique 2, 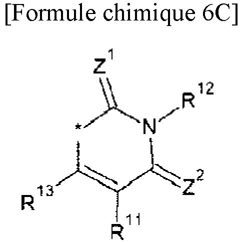
dans lequel, dans la formule chimique 6C,
Z¹ et Z² sont chacun indépendamment O, S, Se, Te ou CR^{a}R^{b}, R^{a} et R^{b} étant chacun indépendamment hydrogène, un groupe alkyle en C1 à C10 substitué ou non substitué, ou un groupe cyano,
R¹¹, R¹² et R¹³ sont identiques ou différents les uns des autres et sont chacun indépendamment hydrogène, deutérium, un groupe alkyle en C1 à C30 substitué ou non substitué, un groupe alkoxy en C1 à C30 substitué ou non substitué, un groupe aryle en C6 à C30 substitué ou non substitué, un groupe hétéroaryle en C4 à C30 substitué ou non substitué, un halogène, un groupe cyano (-CN), ou toute combinaison de ceux-ci, et
* représente un point de liaison dans la formule chimique 2, 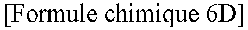 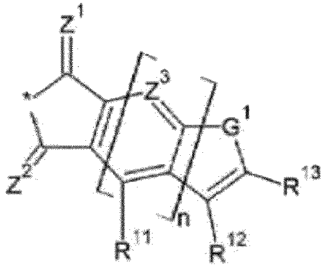
dans lequel, dans la formule chimique 6D,
Z¹ et Z² sont chacun indépendamment O, S, Se, Te ou CR^{a}R^{b}, R^{a} et R^{b} étant chacun indépendamment hydrogène, un groupe alkyle en C1 à C10 substitué ou non substitué, ou un groupe cyano,
Z³ est N ou CR^{c}, R^{c} étant hydrogène ou un groupe alkyle en C1 à C10 substitué ou non substitué,
G¹ est O, S, Se, Te, SiR^{x}R^{y} ou GeR^{z}R^{w}, R^{x}, R^{y}, R^{z} et R^{w} étant identiques ou différents les uns des autres et étant chacun indépendamment hydrogène, deutérium, un halogène, un groupe alkyle en C1 à C20 substitué ou non substitué, un groupe alkoxy en C1 à C20 substitué ou non substitué, un groupe aryle en C6 à C20 substitué ou non substitué ou un groupe hétéroaryle en C2 à C20 substitué ou non substitué,
R¹¹, R¹² et R¹³ sont identiques ou différents les uns des autres et sont chacun indépendamment hydrogène, deutérium, un groupe alkyle en C1 à C30 substitué ou non substitué, un groupe alkoxy en C1 à C30 substitué ou non substitué, un groupe aryle en C6 à C30 substitué ou non substitué, un groupe hétéroaryle en C4 à C30 substitué ou non substitué, un halogène, un groupe cyano, un groupe contenant un cyano, ou toute combinaison de ceux-ci, et R¹² et R¹³ sont chacun indépendamment présent ou sont liés l'un à l'autre pour former un cycle aromatique fusionné,
n est 0 ou 1, et
* représente un point de liaison dans la formule chimique 2, 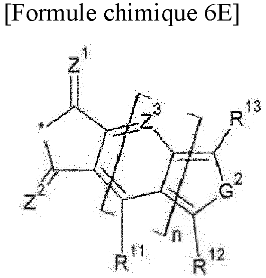
dans lequel, dans la formule chimique 6E,
Z¹ et Z² sont chacun indépendamment O, S, Se, Te ou CR^{a}R^{b}, R^{a} et R^{b} étant chacun indépendamment hydrogène, un groupe alkyle en C1 à C10 substitué ou non substitué, ou un groupe cyano,
Z³ est N ou CR^{c}, R^{c} étant hydrogène ou un groupe alkyle en C1 à C10 substitué ou non substitué,
G² est O, S, Se, Te, SiR^{x}R^{y} ou GeR^{z}R^{w}, R^{x}, R^{y}, R^{z} et R^{w} étant identiques ou différents les uns des autres et étant chacun indépendamment hydrogène, deutérium, un halogène, un groupe alkyle en C1 à C20 substitué ou non substitué, un groupe alkoxy en C1 à C20 substitué ou non substitué, un groupe aryle en C6 à C20 substitué ou non substitué ou un groupe hétéroaryle en C2 à C20 substitué ou non substitué,
R¹¹, R¹² et R¹³ sont identiques ou différents les uns des autres et sont chacun indépendamment hydrogène, deutérium, un groupe alkyle en C1 à C30 substitué ou non substitué, un groupe alkoxy en C1 à C30 substitué ou non substitué, un groupe aryle en C6 à C30 substitué ou non substitué, un groupe hétéroaryle en C4 à C30 substitué ou non substitué, un halogène, un groupe cyano, un groupe contenant un cyano, ou toute combinaison de ceux-ci,
n est 0 ou 1, et
* représente un point de liaison dans la formule chimique 2, 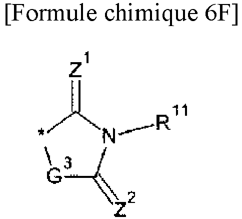
dans lequel, dans la formule chimique 6F,
Z¹ et Z² sont chacun indépendamment O, S, Se, Te ou CR^{a}R^{b}, R^{a} et R^{b} étant chacun indépendamment hydrogène, un groupe alkyle en C1 à C10 substitué ou non substitué, ou un groupe cyano,
R¹¹ est hydrogène, deutérium, un groupe alkyle en C1 à C30 substitué ou non substitué, un groupe aryle en C6 à C30 substitué ou non substitué, un groupe hétéroaryle en C4 à C30 substitué ou non substitué, un halogène, un groupe cyano (-CN), un groupe contenant un cyano, ou toute combinaison de ceux-ci,
G³ est O, S, Se, Te, SiR^{x}R^{y} ou GeR^{z}R^{w}, R^{x}, R^{y}, R^{z} et R^{w} étant identiques ou différents les uns des autres et étant chacun indépendamment hydrogène, deutérium, un halogène, un groupe alkyle en C1 à C20 substitué ou non substitué, un groupe alkoxy en C1 à C20 substitué ou non substitué, un groupe aryle en C6 à C20 substitué ou non substitué ou un groupe hétéroaryle en C2 à C20 substitué ou non substitué, et
* est un point de liaison dans la formule chimique 2 ; et/ ou
dans lequel un rapport de poids du composé semi-conducteur de type p au composé semi-conducteur de type n se situe dans une plage de 1:0,1 à 1:10 ; et/ou
dans lequel la composition d'absorption infrarouge est configurée pour présenter une longueur d'onde d'absorption de crête dans une plage de longueurs d'onde de 1000 nm à 3000 nm.

9. Film d'absorption infrarouge comprenant la composition d'absorption infrarouge selon l'une quelconque des revendications 1 à 8 ;
de préférence :
présentant une structure d'alignement face à face dans l'analyse par diffusion aux petits angles de rayons X en incidence rasante (GISAXS) ; et/ou
comportant une rugosité de surface inférieure ou égale à 2 nm dans une analyse par microscopie à force atomique.

10. Dispositif photoélectrique comprenant une première électrode et une seconde électrode se faisant face, et une couche photoactive entre la première électrode et la seconde électrode, ladite couche photoactive comprenant la composition d'absorption infrarouge selon l'une quelconque des revendications 1 à 8 ;
dans lequel :
un rendement quantique externe à -3 V du dispositif photoélectrique est compris entre 10 % et 100 % ; et/ou
un rendement quantique externe à -3 V du dispositif photoélectrique est augmenté d'au moins 80 % par rapport à un dispositif photoélectrique comprenant une couche photoactive comprenant le même composé semi-conducteur de type p et un composé semi-conducteur de type n de fullerène ou d'un dérivé de fullerène.

11. Dispositif photoélectrique, comprenant :
une première électrode et une seconde électrode se faisant face ;
une couche photoactive entre la première électrode et la seconde électrode ; et
une couche auxiliaire de charge entre
la couche photoactive et la première électrode, ou
la couche photoactive et la seconde électrode,
au moins l'une parmi la couche photoactive ou la couche auxiliaire de charge comprenant la composition d'absorption infrarouge selon l'une quelconque des revendications 1 à 8.

12. Capteur comprenant la composition d'absorption infrarouge selon l'une quelconque des revendications 1 à 8 ou le dispositif photoélectrique selon les revendications 10 ou 11 ; ou dispositif électronique comprenant le dispositif photoélectrique selon les revendications 10 ou 11.

13. Capteur d'image, comprenant :
un substrat semi-conducteur ;
un premier dispositif photoélectrique sur le substrat semi-conducteur, le premier dispositif photoélectrique étant configuré pour absorber sélectivement la lumière dans une première région de longueur d'onde infrarouge ; et
un capteur supplémentaire configuré pour absorber sélectivement la lumière dans une région de longueur d'onde séparée qui est différente de la première région de longueur d'onde infrarouge,
dans lequel le premier dispositif photoélectrique comprend la composition d'absorption infrarouge selon l'une quelconque des revendications 1 à 8 ;
de préférence dans lequel,
le capteur supplémentaire est un capteur de lumière infrarouge au moins partiellement intégré dans le substrat semi-conducteur, et la région de longueur d'onde séparée est une région de longueur d'onde infrarouge séparée qui est différente de la première région de longueur d'onde infrarouge, et
le premier dispositif photoélectrique et le capteur de lumière infrarouge se chevauchent dans une direction verticale qui est perpendiculaire à une surface supérieure du substrat semi-conducteur ;
de préférence dans lequel
le capteur supplémentaire comprend une pluralité de photodiodes au moins partiellement intégrées dans le substrat semi-conducteur, la pluralité de photodiodes étant configurée pour absorber sélectivement la lumière dans des régions de longueur d'onde visibles séparées, et
le premier dispositif photoélectrique et la pluralité de photodiodes se chevauchent dans une direction verticale qui est perpendiculaire à une surface supérieure du substrat semi-conducteur.

14. Capteur d'image selon la revendication 13, dans lequel
le capteur supplémentaire comprend au moins un dispositif photoélectrique supplémentaire empilé verticalement entre le premier dispositif photoélectrique et le substrat semi-conducteur, chaque dispositif photoélectrique séparé du ou des dispositifs photoélectriques supplémentaires comprenant une couche de conversion photoélectrique séparée conversion et étant configuré pour absorber sélectivement la lumière dans une région de longueur d'onde respective séparée qui est différente de la première région de longueur d'onde infrarouge ;
et/ou
dans lequel le premier dispositif photoélectrique comprend
une première électrode et une seconde électrode se faisant face ; et
une couche photoactive entre la première électrode et la seconde électrode, ladite couche photoactive comprenant la composition d'absorption infrarouge.

15. Capteur d'image selon les revendications 13 ou 14, dans lequel le premier dispositif photoélectrique comprend
une première électrode et une seconde électrode se faisant face ;
une couche photoactive entre la première électrode et la seconde électrode ; et
une couche auxiliaire de charge entre
la couche photoactive et la première électrode, ou
la couche photoactive et la seconde électrode,
dans lequel la couche auxiliaire de charge comprend la composition d'absorption infrarouge.
